# EUROPEAN PATENT SPECIFICATION

(11) **EP 1 227 084 B1**
(45) Date of publication and mention of the grant of the patent: **14.12.2005**
(21) Application number: 00970155.8
(22) Date of filing: 27.10.2000
(51) Int. Cl.: C07D 213/30, C07D 277/26, C07D 277/28, C07D 277/32, C07D 277/44, C07D 215/14, C07D 215/56, C07D 307/52, C07D 513/04, C07D 263/56, A61K 31/425, A61K 31/44

(54) **DRUG DISCHARGE PUMP INHIBITORS**
PUMPENINHIBITOREN ZUR FREISETZUNG VON MEDIKAMENTEN
INHIBITEURS DE LA POMPE PAR LIBERATION DE MEDICAMENTS

(30) Priority: 28.10.1999 US 428466; 26.10.2000 JP 2000326713
(43) Date of publication of application: 31.07.2002
(60) Divisional application: 05019514.8
(73) Proprietor: Trine Pharmaceuticals, Inc., Waltham, MA 02451 (US); DAIICHI PHARMACEUTICAL CO., LTD., Chuo-ku, Tokyo 103-8234 (JP)
(72) Inventor: LEGER, Roger, Saint-Lambert, Quebec J4R 2V8 (CA); WATKINS, William, John, Sunnyvale, CA 94087 (US); ZHANG, Jason, Zhijia, Foster City, CA 94404 (US); RENAU, Thomas, Eric, Santa Clara, CA 95051 (US); LEE, Ving, Jack, Los Altos, CA 94024 (US); OHTA, Toshiharu, Daiichi Pharmaceutical Co., Ltd.,, Edogawa-ku, Tokyo 134-8630 (JP); NAKAYAMA, Kiyoshi, Daiichi Pharmaceutical Co., Ltd, Edogawa-ku, Tokyo 134-8630 (JP); ISHIDA, Yohhei, Daiichi Pharmaceutical Co., Ltd., Edogawa-ku, Tokyo 134-8630 (JP); OHTSUKA, Masami, Daiichi Pharmaceutical Co., Ltd., Edogawa-ku, Tokyo 134-8630 (JP); KAWATO, Haruko, Daiichi Pharmaceutical Co., Ltd., Edogawa-ku, Tokyo 134-8630 (JP)
(74) Representative: Sternagel, Fleischer, Godemeyer & Partner
(86) International application number: PCT/JP2000/007565
(87) International publication number: WO 2001/030757

(56) References cited:
- EP-A- 0 279 239
- EP-A- 0 513 387
- EP-A- 0 513 974
- EP-A- 0 594 861
- EP-A1- 0 786 457
- WO-A-96/33285
- WO-A-98/02430
- WO-A-99/37667
- WO-AS-99/36442
- WO-A1-93/22312
- WO-A1-93/22315
- WO-A1-96/02507
- WO-A1-96/33285
- WO-A1-97/03967
- WO-A1-98/17625
- WO-A1-98/57957
- WO-A1-99/50304
- DE-A- 3 417 840
- FR-A- 1 458 066
- GB-A- 1 305 820
- JP-B1- 44 002 219
- JP-B1- 44 013 952
- US-A- 3 870 712
- US-A- 3 907 808
- US-A- 4 013 647
- US-A- 4 404 207
- US-A- 4 665 079
- US-A- 4 891 428
- US-A- 5 936 977
- PATENT ABSTRACTS OF JAPAN vol. 015, no. 478 (C-0891), 4 December 1991 (1991-12-04) -& JP 03 207789 A (DAINIPPON INK & CHEM INC;OTHERS: 01), 11 September 1991 (1991-09-11)
- PATENT ABSTRACTS OF JAPAN vol. 014, no. 526 (C-0779), 19 November 1990 (1990-11-19) -& JP 02 219892 A (DAINIPPON INK & CHEM INC;OTHERS: 01), 3 September 1990 (1990-09-03)
- PATENT ABSTRACTS OF JAPAN vol. 017, no. 345 (C-1077), 30 June 1993 (1993-06-30) -& JP 05 043878 A (DAINIPPON INK & CHEM INC), 23 February 1993 (1993-02-23)
- PATENT ABSTRACTS OF JAPAN vol. 1998, no. 04, 31 March 1998 (1998-03-31) -& JP 09 323971 A (SANKYO CO LTD), 16 December 1997 (1997-12-16)
- PATENT ABSTRACTS OF JAPAN vol. 1999, no. 02, 26 February 1999 (1999-02-26) -& JP 10 310578 A (YOSHITOMI PHARMACEUT IND LTD), 24 November 1998 (1998-11-24)
- NISHIGAKI S. ET AL.: 'Synthesis of 1-substituted 1,4-dihydro-7-(2-(5-nitro-2-furyl)vinyl)-4- oxo-1,8-naphthyridine derivatives. I.' CHEM. PHARM. BULL. vol. 17, no. 9, 1969, pages 1827 - 1831, XP002944105
- LI X.-A., LIVERMORE D.M., NIKAIDO H.: 'Role of efflux pumps(s) in intrinsic resistance of pseudomonas aeruginosa: Resistance to tetracycline, chloramphenicol and norfloxacin' ANTIMICROB. CHEMTH. vol. 38, no. 8, 1994, pages 1732 - 1734, XP002944106
- GREENE J. ET AL.: 'Chemical function queries for 3D database search' J. CHEM. INF. COMPUT. SCI. no. 34, 1994, pages 1297 - 1308, XP002944107
- CHUPP J.P., MOLYNEAUX J.M.: 'Derivation of fluorine-containing pyridinedicarboxylates. III. Regioselective anion chemistry at the 2- and 4-position' J. HETEROCYCL. CHEM. vol. 26, no. 6, 1989, pages 1771 - 1780, XP002944108
- DEJOHN D. ET AL.: 'Functionalization of substituted 2(1H)- and 4(1H)-pyridones. III. The preparation of substituted 6-vinyl-1,2-dihydro-2-oxo- and 1,4-dihydro-4-oxo-3-pyridinecarboxylic acids through the chemistry of pyridone dianions' J. HETEROCYCL. CHEM. vol. 20, no. 5, 1983, pages 1295 - 1302, XP002944109
- CHEMICAL ABSTRACTS, vol. 115, 1991, Columbus, Ohio, US; abstract no. 105447, ROZENBLIT A. ET AL.: 'Computer-aided prediction and study of biological properties of nitrofurylvinyl(polyenyl)quinolines' & KHIM.-FARM. ZH. vol. 25, no. 5, 1991, pages 55 - 58
- CHEMICAL ABSTRACTS, vol. 92, 1980, Columbus, Ohio, US; abstract no. 157718, SUKHOVA N.M. ET AL.: 'Search for new drugs in a series of furan derivatives' & KONF. KHIM. TEKHNOL. FURANOVYKH SOEDIN., (TEZISY DOKL.), 3RD 1978, pages 97 - 98
- CHEMICAL ABSTRACTS, vol. 72, 1970, Columbus, Ohio, US; abstract no. 111193, LIPKIN A.E., BESPALOVA ZH. P.: 'Synthesis based on 2-methyl-4-quinolinecarboxylic acid' & KHIM.-FARM. ZH. vol. 4, no. 1, 1970, pages 24 - 26
- CHEMICAL ABSTRACTS, vol. 66, 1967, Columbus, Ohio, US; abstract no. 75885, FUJITA AKIO ET AL.: 'Nitrofuran derivatives. VI. Synthesis of 2-(2-(5-nitro-2-furyl)vinyl)pyridinemethano l and 2-(2-(5-nitro-2-furyl)vinyl)pyridinecarboxy lic acid derivatives' & YAKUGAKU ZASSHI vol. 86, no. 11, 1966, pages 1014 - 1021
- CHEMICAL ABSTRACTS, vol. 108, 1988, Columbus, Ohio, US; abstract no. 142146, & INORG. CHEM. vol. 27, no. 7, 1988, pages 1167 - 1173
- CHEMICAL ABSTRACTS, vol. 106, 1987, Columbus, Ohio, US; abstract no. 196227, & TAP CHI HOA HOC vol. 23, no. 5, 1985, pages 9 - 11
- CHEMICAL ABSTRACTS, vol. 103, 1985, Columbus, Ohio, US; abstract no. 87754, & YIYAO GONGYE vol. 16, no. 2, 1985, pages 66 - 68, 91
- CHEMICAL ABSTRACTS, vol. 100, 1984, Columbus, Ohio, US; abstract no. 138915, & KHIM. GETEROTSIKL. SOEDIN. no. 11, 1983, pages 1521 - 1523
- CHEMICAL ABSTRACTS, vol. 92, 1980, Columbus, Ohio, US; abstract no. 128023, & KHIM. GETEROTSIKL. SOEDIN. no. 9, 1979, pages 1194 - 1200
- CHEMICAL ABSTRACTS, vol. 89, 1978, Columbus, Ohio, US; abstract no. 109013, & TEZISY DOKL. - RESP. KONF. MOLODYKH UCH.-KHIM., 2ND vol. 1, 1977, AKAD. NAUK EST. SSR, INST. KHIM.: TALLINN, USSR, pages 14 - 15
- CHEMICAL ABSTRACTS, vol. 78, 1973, Columbus, Ohio, US; abstract no. 97460, & J. HETEROCYCL. CHEM. vol. 9, no. 6, 1972, pages 1203 - 1207

## Description

### Technical Field

The present invention relates to the use of a compound for the production of a medicament useful for preventive and therapeutic treatment of microbial infectious diseases.

### Background Art

For preventive or therapeutic treatment of infectious diseases caused by microorganisms, various antibacterial agents have so far been developed, and drugs such as β-lactam antibiotics (penicillins, cephems, monobactams, carbapenems, and penems), aminoglycosides, quinolones, macrolides, tetracyclines, rifamycins, chloramphenicols, and phosphomycins have been practically used. However, with the increase of clinically used amount of antibacterial agents, remarkable numbers of resistant bacterial strains to these antibacterial agents have emerged, which becomes a serious problem in the treatment of infectious diseases.

Examples of problematic bacteria, which cause particularly intractable or serious infectious diseases among those caused by resistant bacteria, include *Pseudomonas aeruginosa* and methicillin-resistant *Staphylococcus aureus* (MRSA). Antibacterial agents effective against these bacteria have been limited so far, and it is not certain whether or not therapeutic efficacy of the currently available drugs will be expected in the future. In particular, no drug is available at present by which specifically high efficacy against resistant *Pseudomonas aeruginosa* can be achieved. With the increase of aged population and the popularization of sophisticated medical technologies including human organ transplantation and anti-cancer treatments, infections frequently occurring particularly in patients with reduced immunity, i.e., so-called opportunistic infections, have become an extremely serious problem in the clinical field, and under the circumstances, early developments of measures against the resistant bacteria are desired.

Recently, the presence of drug efflux pumps has recognized as a bacterial excretion mechanism of drugs through researches on resistance acquiring mechanisms of resistant bacteria. In earlier researches, a pump that specifically excretes a tetracycline antibacterial agent from bacterial cells was identified in 1980 by the group of Levy, and the discovery was noted as a major factor of the resistance to tetracycline (L. McMurry, Proc. Natl. Acad. Sci. U.S.A., 77, 3974, 1980). Furthermore, based on recent researches, the presence of multidrug-excreting drug efflux pumps was reported in *Escherichia coli, Pseudomonas aeruginosa, Bacillus subtilis, Staphylococcus bacteria, Diplococcus pneumoniae,* and *Neisseria gonorrhoeae.* Four multidrug efflux pumps have so far been reported as homological drug efflux pumps deriving from *Pseudomonas aeruginosa,* and they have been considered as a cause of low drug sensitivity inherent to *Pseudomonas aeruginosa* (K. Poole et al., J. Bacteriol., 175, 7363, 1993; K. Poole et al., M. Microbiol., 21, 713, 1996; T. Kohler et al., M. Microbiol., 23, 345, 1997; T. Mine et al., Antimicrob. Agents Chemother., 43, 415, 1999).

The drug efflux pumps of *Pseudomonas aeruginosa* excrete various drugs including β-lactams, tetracyclines, chloramphenicols, and quinolones, to which the drug resistance of *Pseudomonas aeruginosa* is attributable.

In order to overcome the problem, it will be effective to develop an antibacterial agent that has a novel structure, by which resistance acquisition due to a drug efflux pump, one of factors of resistance acquisition, can be avoided, or develop an agent for a combinational use with currently available antibacterial agents that can restore their efficacy by inhibiting functions of drug efflux pumps.

In recent years, the rational drug design has been recognized as an important approach in the drug design. The method can be divided mainly into two categories. One is a method applied when three dimensional information of a target protein or the like has been revealed, by which novel compounds are designed on the basis of the data. If a crystal structure is revealed in a ligand-bound state, designs will be made easier. The other method is applied when the coordinates of a target have not been revealed. In this case, a method is adopted wherein a modeling is carried out on the basis of structure-activity correlation between a compound having an activity and derivatives thereof as well as their three dimensional structures, and results are utilized for a novel design.

Examples of typical techniques for the latter method include, for example, methods using the following programs and the like: CATALYST™ (Greene et al., J. Chem. Inf. Comp. Sci., 1994, 34, 1297-1308), DISCO (Martin Y. C., et al., J. Comp. Aided Mol. Design, 1993, 7, 83-102), COMFA (Cramer R. D., J. Am. Chem. Soc., 1988, 110, 5959-5967). By using these programs and by imputing and analyzing the information of the correlation between structures and activities as well as information of the structures, three dimensional coordinates (pharmacophore) that indicate conditions required for expression of the activity can be obtained. Use of accurate pharmacophore improves accuracy of the design, and highly contributes to efficient creation of effective medicaments. In addition, activity of a compound can be expected by using the resulting pharmacophore. Recently, such approaches have been intensively made. For example, International Publication WO98/04913 discloses the backgrounds and the like, and examples for reduction to practice as well as examples of practical applications.

### Disclosure of the Invention

Therefore, an object of the present invention is to provide a novel medicament for the treatment of infectious diseases that improves therapeutic efficacy of an agent against pathogenic microorganisms, in particular, a medicament that acts on a microorganism with acquired resistance to an antimicrobial agent, and eliminates the resistance of the bacteria by inhibiting a drug efflux pump so as to improve preventive and/or therapeutic effect of the antimicrobial agent.

The inventors of the present invention conducted various researches to achieve the foregoing object. Namely, they selected compounds having resistant-eliminating effect from novel and known classes of compounds by applying criteria relating to improvement of the eliminating action on the resistant to antimicrobial agents using *Pseudomonas aeruginosa* having acquired resistance, and prepared derivatives thereof and investigated their activities. Moreover, they succeeded in constructing a pharmacophore indicated by specific coordinates by using techniques which involve conformational analysis of the derivatives and analysis of correlation between the conformations and activities. As a result, they found that the compounds that fit the pharmacophore had an inhibitory activity on drug efflux pumps of *Pseudomonas aeruginosa*. The present invention was achieved on the basis of these findings.

It is disclosed a medicament for preventive and/or therapeutic treatment of microbial infections, which comprises as an active ingredient a compound represented by the following general formula (I), a physiologically acceptable salt thereof, or hydrates thereof, wherein: 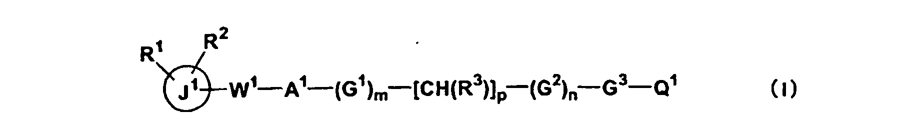 R¹ and R² independently represent hydrogen atom, a halogen atom, carboxyl group, an alkoxycarbonyl group, an amino group which may be substituted, an alkyl group which may be substituted, an aryl group which may be substituted, or a heterocyclic group which may be substituted, or R¹ and R² may bind to each other to represent a saturated or unsaturated 5- to 7-membered ring formed together with two adjacent ring-membered atoms of J¹ to which R¹ and R² bind;
R³ represents hydrogen atom, hydroxyl group, or an alkoxy group;
J¹ represents a 5- or 6-membered heteroaromatic ring;
W¹ represents a group selected from the group consisting of -CH=CH-, -C≡C-, -CH₂CH₂-, -OCH₂-, -SCH₂-, -OCH₂O-, -CH₂O-, -CH₂-, -CO-, -CH₂CH₂CH₂-, -CH₂NH-, -NHCH₂-, -CH₂S-, -CONH-, -CH₂SCH₂-, -CH=CH-CONH- and -CH₂OCH₂- (each group binds at its left end to a ring-membered atom of J¹) or single bond;
A¹ represents a phenylene group which may be substituted, a pyridinediyl group which may be substituted, a furandiyl group which may be substituted, a thiophenediyl group which may be substituted, a benzofurandiyl group which may be substituted, a benzo[b]thiophenediyl group which may be substituted, a benzoxazolediyl group which may be substituted, a benzothiazolediyl group which may be substituted, a pyrido[1,2-a]pyrimidinediyl group which may be substituted, a quinazolinediyl group which may be substituted, a benzotriazinediyl group which may be substituted, a 2H-chromenediyl group which may be substituted, a quinolin-4-one-diyl group which may be substituted, an azaquinolin-4-one-diyl group which may be substituted, a quinolinediyl group which may be substituted, a thiadiazolo[3,2-a]pyrimidinediyl group which may be substituted, or a thiazolo[3,2-a]pyrimidinediyl group which may be substituted;
G¹ represents oxygen atom, carbonyl group, ethynyl group, -CH=N-, -N(R⁴)-CO-, -(CH₂)-N(R⁵)-CO-, -N(R⁶)-, -N(R⁷)-SO₂-, -SO₂-N(R⁸)-, -CON(R⁹)-, -C(=CHR¹⁰)-, -C(R¹¹)=C(R¹²)-, -NHCO-C(R¹³)(R¹⁴)-, -CONH-C(R¹⁵)(R¹⁶)- or -CH₂O(CH₂)_{q}- wherein R⁴, R⁵, R⁶, R⁷, R⁸ and R⁹ independently represent hydrogen atom, hydroxyl group or an alkyl group which may be substituted; R¹⁰ represents cyano group, carboxyl group or an alkoxycarbonyl group which may be substituted; R¹¹ and R¹² independently represents hydrogen atom, a halogen atom, an alkyl group which may be substituted, or an aryl group which may be substituted, or R¹¹ and R¹² represent a ring formed by binding to each other; R¹³ and R¹⁴ independently represent hydrogen atom or an alkyl group, or R¹³ and R¹⁴ represent an alkylene group formed by binding to each other;
R¹⁵ and R¹⁶ independently represent hydrogen atom or an alkyl group, or R¹⁵ and R¹⁶ represent an alkylene group formed by binding to each other; and q represents an integer of from 0 to 5;
p represents an integer of from 0 to 3;
G² represents a phenylene group which may be substituted, a furandiyl group which may be substituted, a tetrahydrofurandiyl group which may be substituted, a pyridinediyl group which may be substituted, a thiazolinediyl group which may be substituted, an isoxazolidinediyl group which may be substituted, a 1,3-dioxolanediyl group which may be substituted, a thiophenediyl group which may be substituted, a pyrimidinediyl group which may be substituted, -C(R¹⁷)=C(R¹⁸)-[C(R¹⁹)=C(R²⁰)]_{y}- wherein R¹⁷, R¹⁸, R¹⁹ and R²⁰ independently represent hydrogen atom, a halogen atom, an alkyl group which may be substituted, or an aryl group which may be substituted, or represent a ring formed by R¹⁷ and R¹⁸ bound to each other, and/or a ring formed by R¹⁹ and R²⁰ bound to each other, and y represents an integer of from 0 to 3, or -C(R²¹)(R²²)-C(R²³)(R²⁴)- wherein R²¹, R²², R²³ and R²⁴ independently represent hydrogen atom, phenyl group, or an alkyl group having 1 to 6 carbon atoms, or represent a ring formed by R²¹ and R²² bound to each other and/or a ring formed by R²³ and R²⁴ bound to each other, or alternatively, represent a ring formed by R²¹ and R²³ bound to each other;
G³ represents -CH₂- or single bond;
m and n independently represent an integer of 0 or 1; and
Q¹ represents an acidic group.

As other aspects it is disclosed a medicament for eliminating resistance of a microorganism with acquired drug resistance, which comprises a compound represented by the aforementioned general formula (I), a physiologically acceptable salt thereof, or a hydrate thereof as an active ingredient; and a medicament for enhancing effect of an antimicrobial agent, which comprises a compound represented by the aforementioned general formula (I), a physiologically acceptable salt thereof, or hydrates thereof as an active ingredient. The aforementioned medicaments wherein the microorganism is *Pseudomonas aeruginosa* are preferred embodiments of the present invention. The present invention also provides a pharmaceutical composition for preventive and/or therapeutic treatment of microbial infections, which comprises a compound represented by the aforementioned general formula (I), a physiologically acceptable salt thereof, or a hydrate thereof together with an antimicrobial agent.

As further aspects it is disclosed a method for preventive and/or therapeutic treatment of microbial infections, which comprises the step of administering to a mammal including human a preventively and/or therapeutically effective amount of a compound represented by the aforementioned general formula (I), a physiologically acceptable salt thereof, or a hydrate thereof; a method for eliminating resistance of a microorganism with acquired resistance to an antimicrobial agent, which comprises the step of contacting with the microorganism an effective amount of a compound represented by the aforementioned general formula (I), a physiologically acceptable salt thereof, or a hydrate thereof; a method for inhibiting acquisition of resistance to an antimicrobial agent by a microorganism, which comprises the step of contacting with a microorganism an effective amount of a compound represented by the aforementioned general formula (I), a physiologically acceptable salt thereof, or a hydrate thereof; a method for enhancing sensitivity of a microorganism to an antimicrobial agent, which comprises the step of contacting with a microorganism an effective amount of a compound represented by the aforementioned general formula (I), a physiologically acceptable salt thereof, or a hydrate thereof; and a method for improving effect of an antimicrobial agent, which comprises the step of administering to a mammal including human an effective amount of a compound represented by the aforementioned general formula (I), a physiologically acceptable salt thereof, or a hydrate thereof. The compounds represented by the aforementioned general formula (I), physiologically acceptable salts thereof, or hydrates thereof are usually administered with one or more of antimicrobial agents simultaneously or separately, or successively. The present invention also provides a use of the compounds represented by the aforementioned general formula (I), physiologically acceptable salts thereof, or hydrates thereof for the manufacture of the aforementioned medicaments.

The present invention provides a compound represented by the following general formula (II) or a salt thereof, and a medicament which comprises a compound represented by the following general formula (II), a physiologically acceptable salt thereof, or hydrates thereof as an active ingredient: 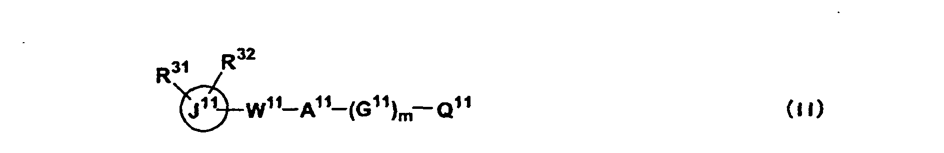 wherein, R³¹ and R³² independently represent hydrogen atom, a halogen atom, carboxyl group, an alkoxycarbonyl group, an amino group which may be substituted, an alkyl group which may be substituted, an aryl group which may be substituted, or a heterocyclic group which may be substituted, or R³¹ and R³² represent a 6-membered ring formed by R³¹ and R³² bound to each other together with two adjacent ring-membered atoms of J¹¹;
J¹¹ represents a 5- or 6-membered heteroaromatic ring;
W¹¹ represents a group selected from the group consisting of -CH=CH-, -CH₂CH₂-, -OCH₂-, -SCH₂-, -OCH₂O-, -CH₂O-, -CH₂-, -CO-, -CH₂CH₂CH₂-, -CH₂NH-, -NHCH₂-, -CH₂S-, -CONH-, -CH₂SCH₂-, -CH=CH-CONH- and -CH₂OCH₂- (each group binds at its left end to a ring-membered atom of J¹¹) or single bond;
A¹¹ represents a pyridinediyl group which may be substituted, a pyrido[1,2-a]pyrimidinediyl group which may be substituted, a quinolin-4-one-diyl group which may be substituted, an azaquinolin-4-one-diyl group which may be substituted, a quinolinediyl group which may be substituted, a thiadiazolo[3,2-a]pyrimidinediyl group which may be substituted, or a thiazolo[3,2-a]pyrimidinediyl group which may be substituted;
G¹¹ represents oxygen atom, carbonyl group, ethynyl group, -CH=N-, -N(R³³)CO-, -N(R³⁴)-SO₂-, -SO₂-N(R³⁵)-, -CO-N(R³⁶)-, -C(=CHR³⁷)- or -C(R³⁸)=C(R³⁹) wherein R³³, R³⁴, R³⁵, R³⁶, R³⁷, R³⁸ and R³⁹ independently represent hydrogen atom or an alkyl group which may be substituted;
m represents an integer of 0 or 1; and
Q¹¹ represents an acidic group.

Moreover, it is disclosed a compound which has a partial structure occupying the following four sites in the following tolerance, and has an action for inhibiting drug efflux pumps of *Pseudomonas aeruginosa*; a salt thereof; or a hydrate thereof.

| Site | Feature | x(Å) | y(Å) | z(Å) | Tolerance (Å) |
|---|---|---|---|---|---|
| 1 | Hydrophobic | 8.839 | -0.324 | 1.49 | 2 |
| 2 | Hydrophobic | 4.819 | 1.836 | -0.29 | 1.7 |
| 3 | Hydrophobic | -1.722 | -0.964 | 0.89 | 1.7 |
| 4 | Negative Ionizable | -8.636 | -1.067 | -2.554 | 2 |

According to a preferred embodiment the aforementioned compound, the salt thereof, or the hydrates thereof have an inhibitory activity on drug efflux pump of *Pseudomonas aeruginosa* (MPC4: the inhibitory activity against drug efflux pump of *Pseudomonas aeruginosa* means a minimum concentration of a medicament required to reduce a minimum inhibitory concentration of an antibacterial agent against the bacterium to a quarter.) of about 40 µg/mL or less, preferably 10 *µ*g/mL or less, further preferably 1 *µ*g/mL or less.

As another aspect it is disclosed a drug efflux pump inhibitor which comprises the aforementioned compound, a physiologically acceptable salt thereof, or a hydrate thereof. It is also disclosed a method for screening a compound having an inhibitory activity against drug efflux pump of *Pseudomonas aeruginosa* which comprises the step of determining whether or not a partial structure of a test compound occupies the aforementioned four sites in the aforementioned tolerance by means of a computer program and/or an experimental three dimensional structure analysis.

It is further disclosed a drug efflux pump inhibitor of *Pseudomonas aeruginosa* which comprises a compound obtained by the aforementioned screening. As for the compound screened as mentioned above, it is preferred to verify that an inhibitory activity thereof against drug efflux pumps of *Pseudomonas aeruginosa* (MPC4) is 40 µg/mL or less, preferably 10 µg/mL or less, further preferably 1 µg/mL or less.

Examples of the compound which has a partial structure occupying the aforementioned four sites in the aforementioned tolerance and has an inhibitory action against drug efflux pumps of *Pseudomonas aeruginosa* or a salt thereof, include the compound of the aforementioned general formula (I) or (II) or salts thereof.

### Brief Explanation of the Drawings

Fig. 1 shows the pharmacophore having the highest adequacy among the results obtained by calculating the pharmacophore of the compounds shown in Table 2 by means of CATALYST/HipHop, one of the functions of generating a pharmacophore (Hypothesis) of CATALYST™.
Fig. 2 shows the result obtained by overlaying all the compounds shown in Table 2 on the pharmacophore shown in Fig. 1.
Fig. 3 shows the result obtained by overlaying the compound of Example 1 on the pharmacophore shown in Fig. 1.
Fig. 4 shows the result obtained by overlaying the compound of Example 10 on the pharmacophore shown in Fig. 1.
Fig. 5 shows the result obtained by overlaying the compound of Example 15 on the pharmacophore shown in Fig. 1.
Fig. 6 shows the result obtained by overlaying the compound of Example 27 on the pharmacophore shown in Fig. 1.
Fig. 7 shows the result obtained by overlaying the compound of Example 37 on the pharmacophore shown in Fig. 1.
Fig. 8 shows the result obtained by overlaying the compound of Example 49 on the pharmacophore shown in Fig. 1.
Fig. 9 shows the result obtained by overlaying the compound of Example 70 on the pharmacophore shown in Fig. 1.

### Best Mode for Carrying out the Invention

The terms used in the present specification have the following meanings. An alkyl group or an alkyl moiety of a substituent containing the alkyl moiety (e.g., an alkoxy group) may be linear, branched, cyclic, or a combination thereof unless otherwise specifically mentioned. The alkyl groups have 1 to 8 carbon atoms, preferably about 1 to 6 carbon atoms unless otherwise specifically mentioned, which may be represented by the term "lower". More specifically, examples of the alkyl groups include methyl group, ethyl group, n-propyl group, isopropyl group, cyclopropyl group, n-butyl group, isobutyl group, sec-butyl group, tert-butyl group, cyclobutyl group, cyclopropylmethyl group, n-pentyl group, isopentyl group, neopentyl group, cyclopentyl group, 1,1-dimethylpropyl group, n-hexyl group, isohexyl group, cyclohexyl group, n-heptyl group, n-octyl group and the like. When the term "halogen atom" is referred to, any of fluorine atom, chlorine atom, bromine atom, and iodine atom may be used.

Examples of the aryl group include those having 5 to 14 carbon atoms, preferably those having 5 to 10 carbon atoms (e.g., benzene ring, naphthalene ring). The aryl ring may be either a monocyclic ring or a condensed ring.

As the heterocyclic group, a residue of a hetero ring may be used which has 1 to 4 hetero atoms selected from oxygen atom, sulfur atom and nitrogen atom, and 3 to 10 ring-membered atoms in total. Examples of the hetero ring that constitutes the heterocyclic group include, for example, furan ring, dihydrofuran ring, tetrahydrofuran ring, pyrane ring, dihydropyrane ring, tetrahydropyran ring, benzofuran ring, isobenzofuran ring, chromene ring, chroman ring, isochroman ring, thiophene ring, benzothiophene ring, pyrrole ring, pyrroline ring, pyrrolidine ring, imidazole ring, imidazoline ring, imidazolidine ring, pyrazole ring, pyrazoline ring, pyrazolidine ring, triazole ring, tetrazole ring, pyridine ring, pyridine oxide ring, piperidine ring, pyrazine ring, piperazine ring, pyrimidine ring, pyridazine ring, indolizine ring, indole ring, indoline ring, isoindole ring, isoindoline ring, indazole ring, benzimidazole ring, purine ring, quinolidine ring, quinoline ring, phthalazine ring, naphthylidine ring, quinoxaline ring, quinazoline ring, cinnoline ring, pteridine ring, oxazole ring, oxazolidine ring, isoxazole ring, isoxazolidine ring, thiazole ring, benzothiazole ring, thiazylidine ring, isothiazole ring, isothiazolidine ring, dioxane ring, dithian ring, morpholine ring, thiomorpholine ring, phthalimide ring and the like.

In the specification, when a functional group is defined as "which may be substituted", the definition means that the functional group may have one or more substituents. The number of substituents, as well as types and substitution positions thereof are not particularly limited, and when two or more substituents exist, they may be the same or different. Examples of the substituent include, for example, an alkyl group; an alkenyl group (preferably those having 2 to 8 carbon atoms, more preferably 2 to 6 carbon atoms, and most preferably 2 to 5 carbon atoms, for example, vinyl group, allyl group, 2-butenyl group, 3-pentenyl group and the like), an alkynyl group (preferably those having 2 to 8 carbon atoms, more preferably 2 to 6 carbon atoms, and most preferably 2 to 5 carbon atoms, for example, propargyl group, 3-pentynyl group and the like), an aryl group (preferably those having 6 to 14 carbon atoms, more preferably 6 to 12 carbon atoms, and most preferably 6 to 10 carbon atoms, for example, phenyl group, naphthyl group and the like), an aralkyl group (preferably those having 7 to 15 carbon atoms, more preferably 7 to 13 carbon atoms, further preferably 7 to 11, and most preferably 7 to 9 carbon atoms, for example, benzyl group, α-methylbenzyl group, 2-phenylethyl group, naphthylmethyl group and the like), amino group, a substituted amino group (preferably those having 0 to 16 carbon atoms, more preferably 0 to 12 carbon atoms, and most preferably 0 to 8 carbon atoms, for example, methylamino group, dimethylamino group, diethylamino group, dibenzylamino group and the like), an alkoxyl group (preferably those having 1 to 8 carbon atoms, more preferably 1 to 6 carbon atoms, and most preferably 1 to 4 carbon atoms, for example, methoxy group, ethoxy group, butoxy group and the like), an aryloxy group (preferably those having 6 to 14 carbon atoms, more preferably 6 to 12 carbon atoms, and most preferably 6 to 10 carbon atoms, for example, phenyloxy group, 2-naphthyloxy group and the like), an acyl group (preferably those having 1 to 14 carbon atoms, more preferably 1 to 12 carbon atoms, and most preferably 1 to 10 carbon atoms, for example, acetyl group, benzoyl group, formyl group, pivaloyl group and the like), an alkoxycarbonyl group (preferably those having 2 to 9 carbon atoms, more preferably 2 to 7 carbon atoms, and most preferably 2 to 5 carbon atoms, for example, methoxycarbonyl group, ethoxycarbonyl group and the like), an aryloxycarbonyl group (preferably those having 7 to 15 carbon atoms, more preferably 7 to 13 carbon atoms, and most preferably 7 to 11 carbon atoms, for example, phenyloxycarbonyl group and the like), an acyloxy group (preferably those having 2 to 15 carbon atoms, more preferably 2 to 13 carbon atoms, and most preferably 2 to 11 carbon atoms, for example, acetoxy group, benzoyloxy group and the like), an acylamino group (preferably those having 2 to 15 carbon atoms, more preferably 2 to 13 carbon atoms, and most preferably 2 to 11 carbon atoms, for example, acetylamino group, benzoylamino group and the like), an alkoxycarbonylamino group (preferably those having 2 to 9 carbon atoms, more preferably 2 to 7 carbon atoms, and most preferably 2 to 5 carbon atoms, for example, methoxycarbonylamino group and the like), an aryloxycarbonylamino group (preferably those having 7 to 15 carbon atoms, more preferably 7 to 13 carbon atoms, and most preferably 7 to 11 carbon atoms, for example, phenyloxycarbonylamino group and the like), a sulfonylamino group (preferably those having 1 to 14 carbon atoms, more preferably 1 to 12 carbon atoms, and most preferably 1 to 10 carbon atoms, for example, methanesulfonylamino group, benzenesulfonylamino group and the like), a sulfamoyl group (preferably those having 0 to 14 carbon atoms, more preferably 0 to 12 carbon atoms, and most preferably 0 to 10 carbon atoms, for example, sulfamoyl group, methylsulfamoyl group, dimethylsulfamoyl group, phenylsulfamoyl group and the like), a carbamoyl group (preferably those having 1 to 14 carbon atoms, more preferably 1 to 12 carbon atoms, and most preferably 1 to 10 carbon atoms, for example, carbamoyl group, methylcarbamoyl group, diethylcarbamoyl group, phenylcarbamoyl group and the like), an alkylthio group (preferably those having 1 to 8 carbon atoms, more preferably 1-6 carbon atoms, and most preferably 1 to 4 carbon atoms, for example, methylthio group, ethylthio group and the like), an arylthio group (preferably those having 6 to 14 carbon atoms, more preferably 6 to 12 carbon atoms, and most preferably 6 to 10 carbon atoms, for example, phenylthio group and the like), a sulfonyl group (preferably those having 1 to 14 carbon atoms, more preferably 1 to 12 carbon atoms, and most preferably 1 to 10 carbon atoms, for example, mesyl group, tosyl group and the like), a sulfinyl group (preferably those having 1 to 14 carbon atoms, more preferably 1 to 12 carbon atoms, and most preferably 1-10 carbon atoms, for example, methanesulfinyl group, benzenesulfinyl group and the like), a ureido group (one having preferably 1-14 carbon atoms, more preferably 1-12 carbon atoms, and most preferably 1 to 10 carbon atoms, for example, ureido group, methylureido group, phenylureido group and the like), a phosphoric acid amide group (preferably those having 1 to 14 carbon atoms, more preferably 1 to 12 carbon atoms, and most preferably 1 to 10 carbon atoms, for example, diethylphosphoric acid amide, phenylphosphoric acid amide and the like), hydroxyl group, mercapto group, a halogen atom, cyano group, sulfo group, carboxyl group, nitro group, oxo group, hydroxamic acid group, sulfino group, hydrazino group, heterocyclic group and the like. The substituents may further have one or more substituents exemplified above.

Examples of the alkyl group represented by R¹ and R² include, for example, a linear or branched alkyl group having 1 to 8 carbon atoms, preferably a linear or branched alkyl group having 1 to 5 carbon atoms (for example, methyl group, ethyl group, n-propyl group, isopropyl group, n-butyl group, isobutyl group, sec-butyl group, tert-butyl group, n-pentyl group and the like), and a cyclic alkyl group having 3 to 8 carbon atoms, preferably a cyclic alkyl group having 3 to 5 carbon atoms (for example, cyclopropyl group, cyclobutyl group, cyclopentyl group and the like). The cyclic alkyl group may have a substituent such as an alkyl group and a halogen atom on its ring.

Examples of the aryl group represented by R¹ and R² include, for example, an aryl group having 5 to 14 carbon atoms, preferably an aryl group having 5 to 10 carbon atoms (for example, those having benzene ring, naphthalene ring and the like). The aryl group may have a substituent such as an alkyl group, hydroxyl group and a halogen atom on its ring.

Examples of the heterocyclic group represented by R¹ and R² include, for example, a heterocyclic group having 3 to 8 ring-membered atoms, preferably 3 to 6 ring-membered atoms, which may be saturated or partially saturated, or may be an aromatic ring. Examples of substituents on the heterocyclic group include, for example, tert-butoxycarbonyl group, benzyloxycarbonyl group and the like.

The ring formed by R¹ and R² bound to each other may be a saturated ring, partially saturated ring, or a fused aromatic ring. The ring may preferably be a 6-membered ring, and one or more substituents may be present on the ring. For example, R¹ and R² may preferably bind to each other to form a condensed benzene ring, condensed pyridine ring, or condensed tetrahydropyridine ring, or may preferably bind to each other to form tetramethylene group, butamonoenylene group, butadienylene group, azabutadienylene group or the like. One or more substituents such as a halogen atom, a lower alkoxy group, a lower alkoxycarbonyl group, and an alkyl group having 1 to 3 carbon atoms may substitute on the ring.

R³ may preferably be hydrogen or hydroxyl group.

The 5- or 6-membered heteroaromatic rings represented by J¹ are not particularly limited so far that the rings are heteroaromatic rings containing at least one hetero atom selected from the group consisting of sulfur atom, nitrogen atom and oxygen atom. For example, the ring may preferably be a heteroaromatic ring represented by the following formula (III): 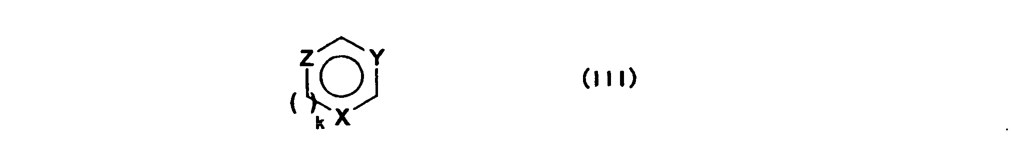 wherein X, Y and Z independently represent sulfur atom, nitrogen atom, oxygen atom or carbon atom, provided that at least one of X, Y and Z represents a hetero atom selected from the group consisting of sulfur atom, nitrogen atom and oxygen atom; and k represents 0 or 1. More preferably, the heteroaromatic rings shown below may be used.

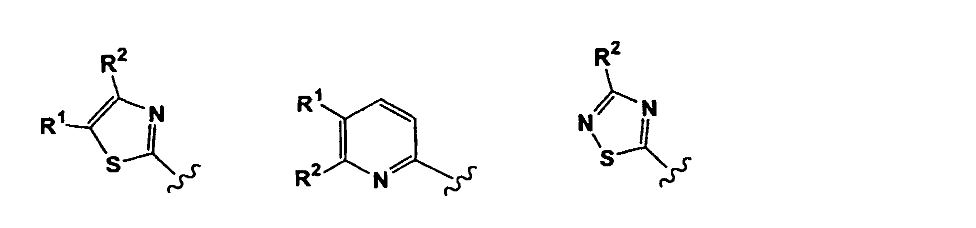

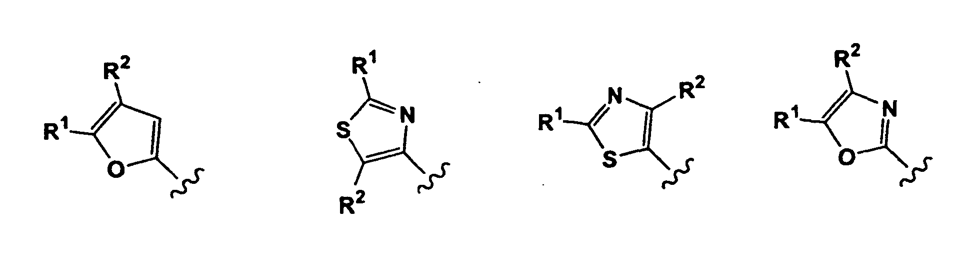

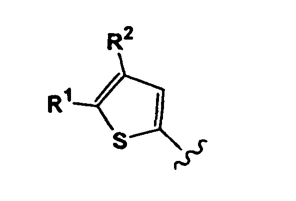

W¹ may preferably be -CH₂O-, -CH=CH- or ethynyl group.

A¹ may preferably be a phenylene group which may be substituted, a pyridinediyl group which may be substituted, a benzofurandiyl group which may be substituted, a benzo[b]thiophenediyl group which may be substituted, a benzoxazolediyl group which may be substituted, a benzothiazolediyl group which may be substituted, a 4-oxo-pyrido[1,2-a]pyrimidinediyl group which may be substituted, a quinolin-4-one-diyl group which may be substituted, or an azaquinolin-4-one-diyl group which may be substituted. Positions of bonding on these cyclic divalent groups are not particularly limited. Any two of ring-membered carbon atoms can form the bondings. For example, the phenylene group may be any of p-phenylene group, m-phenylene group, and o-phenylene group.

The types and number as well as positions of substituents which may be present on the divalent cyclic group represented by A¹ are not particularly limited. Examples of the substituents include, for example, an alkyl group which may be substituted (for example, alkyl groups having 1 to 8 carbon atoms, preferably those having 1 to 5 carbon atoms explained above, and examples of substituents on the alkyl group include, for example, a halogen atom, hydroxyl group, an alkyloxy group and the like), a phenyl group which may be substituted, an alkylenephenyl group which may be substituted, an alkylenepyridyl group which may be substituted, hydroxyl group, amino group and the like. More preferably, a halogen atom, an alkyl group which may be substituted, hydroxyl group, amino group and the like may be used.

G¹ may preferably be -N(R⁴)-CO-, -CO-N(R⁹)- or -C(R¹¹)=C(R¹²)-. These groups are particularly preferred when A¹ is a substituted phenylene group or a substituted pyridinediyl group. Furthermore, G¹ may preferably be -C(R¹¹)=C(R¹²)- or m is 0, when A¹ is a benzofurandiyl group which may be substituted, a benzo[b]thiophenediyl group which may be substituted, a benzoxazolediyl group which may be substituted, a benzothiazolediyl group which may be substituted, a 4-oxo-pyrido[1,2-a]pyrimidinediyl group which may be substituted, a quinolin-4-one-diyl group which may be substituted, or an azaquinolin-4-one-diyl group which may be substituted.

The symbol "p" represents an integer of from 0 to 3. When A¹ is a substituted phenylene group or a substituted pyridinediyl group, p may preferably be 0 or 1. When A¹ is a benzofurandiyl group which may be substituted, a benzo[b]thiophenediyl group which may be substituted, a benzoxazolediyl group which may be substituted, a benzothiazolediyl group which may be substituted, a 4-oxo-pyrido[1,2-a]pyrimidinediyl group which may be substituted, a quinolin-4-one-diyl group which may be substituted, or an azaquinolin-4-one-diyl group which may be substituted, p may preferably be 0.

When A¹ is a benzofurandiyl group which may be substituted, a benzo[b]thiophenediyl group which may be substituted, a benzoxazolediyl group which may be substituted, a benzothiazolinediyl group which may be substituted, a 4-oxo-pyrido[1,2-a]pyrimidinediyl group which may be substituted, a quinolin-4-one-diyl group which may be substituted, or an azaquinolin-4-one-diyl group which may be substituted, both of p and n may preferably be 0.

When G² represents a phenylene group which may be substituted, examples of preferred substituents on the benzene ring include, for example, -CH₂OH, -COR^{c} [wherein R^{c} is -NH(R^{d}) or -N(R^{e})(R^{f}) (R^{d} represents hydrogen atom or an alkyl group, and R^{e} and R^{f} independently represent hydrogen atom or an alkyl group, or R^{e} and R^{f} binds to each other to represent an alkylene group), -CH₂COR^{g} [R^{g} represents hydroxyl group, an alkoxyl group, -NH(R^{d}) or -N(R^{e})(R^{f}) (wherein R^{d}, Re and R^{f} have the same meanings as those defined above)], aminoethoxy group, hydroxyethoxy group, a halogen atom, an alkoxy group, an acyloxy group, hydroxyl group, an alkyl group, nitro group, trifluoromethyl group, cyano group and the like.

When G² represents -C(R²¹)(R²²)-C(R²³)(R²⁴)-, the ring formed by R²¹ and R²² bound to each other, the ring formed by R²³ and R²⁴ bound to each other, or the ring formed by R²¹ and R²³ bound to each other may be either saturated or unsaturated, and the ring may contain one or more hetero atoms selected from the group consisting of sulfur atom, nitrogen atom, and oxygen atom. One or more substituents (for example, an alkyl group) may be present on the ring.

Types of the acidic groups represented by Q¹ are not particularly limited, and the acidic group may be a cyclic or non-cyclic substituent, or a combination thereof. Examples include a lower alkoxyl group, hydroxyl group, carboxyl group, N-cyanocarboxamide group, a methanesulfonylamide group having 1 to 3 fluorine atoms, an alkoxycarbonyl group which may be substituted, -CONH-(5-tetrazolyl) group, a 5-tetrazolyl group which may be substituted, a 1,2,3-triazolyl group which may be substituted, a 2,4-dioxothiazolidin-5-ylidenyl group which may be substituted, a 4-oxo-2-thioxothiazolidin-5-ylidenyl group which may be substituted, a 5-oxo-4-tetrazolyl group which may be substituted, a 3-(5-oxo)-[1.2.4]oxadiazolidinyl group which may be substituted, a 2-(3,5-dioxo)-[1.2.4]oxadiazolidinyl group which may be substituted, 5-(3-oxo)-[1.2.4]oxadiazolidinyl group which may be substituted, a 3-(5-oxo)-[1.2.4]isoxazolydinyl group and the like. More preferred examples include carboxyl group, N-cyanocarboxamide group, methanesulfonylamide group that has 1-3 fluorine atoms, an alkoxycarbonyl group which may be substituted, -CONH-(5-tetrazolyl) group, a 5-tetrazolyl group which may be substituted and the like.

As R³¹, R³², J¹¹, m and Q¹¹ in the compounds represented by the general formula (II), those explained above as for R¹, R², J¹, m and Q¹ may preferably be used, respectively.
-CH₂O-, -CH=CH- or -CH₂CH₂- may preferably be used as W¹¹. A pyridinediyl group which may be substituted, a 4-oxo-pyrido[1,2-a]pyrimidinediyl group which may be substituted, a quinolin-4-one-diyl group which may be substituted, or an azaquinolin-4-one-diyl group may preferably be used as A¹¹, and preferred substituents on the ring include a halogen atom, an alkyl group which may be substituted, hydroxyl group, an alkoxy group, amino group or the like. G¹¹ may preferably be -N(R³³)-CO-, -CO-N(R³⁶)- or -C(R³⁸)=C(R³⁹)- (wherein R³³, R³⁶, R³⁸ and R39 independently represent hydrogen atom or an alkyl group which may be substituted). Those wherein m is 0 are also preferred.

The compounds represented by the aforementioned general formulas (I) and (II) may form a salt. Where an acidic group is present, examples of the salt include salts with alkali metals and alkaline earth metals such as lithium, sodium, potassium, magnesium and calcium; salts with ammonia, monomethylamine, dimethylamine, trimethylamine, dicyclohexylamine, tris(hydroxymethyl)aminomethane, N,N-bis(hydroxyethyl)piperazine, 2-amino-2-methyl-1-propanol, ethanolamine, N-methylglucamine, L-glucamine and the like; and salts with basic amino acids such as lysine, δ-hydroxylysine, arginine and the like. Where a basic group is present, examples of the salt include, for example, salts with mineral acids such as hydrochloric acid, hydrobromic acid, sulfuric acid, nitric acid and phosphoric acid; salts with organic acids such as methanesulfonic acid, benzenesulfonic acid, p-toluenesulfonic acid, acetic acid, propionic acid, tartaric acid, fumaric acid, maleic acid, malic acid, oxalic acid, succinic acid, citric acid, benzoic acid, mandelic acid, cinnamic acid, lactic acid, glycolic acid, glucuronic acid, ascorbic acid, nicotinic acid and salicylic acid; and salts with acidic amino acids such as aspartic acid and glutamic acid.

As an active ingredient of a medicament, the compounds represented by the general formula (I) or salts thereof, as well as solvates thereof or hydrates thereof can be used. The compounds represented by the aforementioned general formula (I) may have one or more asymmetric carbon atoms. As for the stereochemistry of the asymmetric carbon atoms, each asymmetric carbon atom may independently be in either (R)-configuration or (S)-configuration, and the compounds may exist as stereoisomers such as optical isomers and diastereoisomers. As the active ingredient of a medicament, any one of possible stereoisomers in a pure form, any mixtures thereof, racemates thereof and the like may be used. Where the compounds represented by the aforementioned general formula (I) have a double bond, the compounds may be in either (E)-configuration or (Z)-configuration, and any geometrical isomer in a pure form or any mixture thereof can be used as an active ingredient of the medicament of the present invention. The compounds represented by the aforementioned general formula (I) may exist as tautomers depending on a type of a substituent, and any tautomers may be used as an active ingredient of the medicament of the present invention.

The scope of the present invention relating to the novel compounds represented by the aforementioned general formula (II) encompasses compounds in free form and salts thereof as well as solvates thereof and hydrates thereof. The compounds represented by the aforementioned general formula (II) may have one or more asymmetric carbon atoms. As for the stereochemistry of the asymmetric carbon atoms, each asymmetric carbon atom may independently in either (R)-configuration or (S)-configuration, and the compounds may exist as stereoisomers such as optical isomers and diastereoisomers. Any stereoisomers thereof in a pure form, any mixtures thereof, racemates thereof and the like fall within the scope of the present invention. Where the compounds represented by the aforementioned general formula (II) have a double bond, the compounds may be in either (E)-configuration or (Z)-configuration, and geometrical isomers in pure form and any mixtures thereof fall within the scope of the present invention. The compounds represented by the aforementioned general formula (II) may exist as tautomers depending on a type of a substituent, and any tautomers fall within the scope of the present invention.

The compounds represented by the aforementioned general formula (I) can be prepared by known methods. For example, those wherein J¹ is a thiazole derivative can be prepared according to the methods described in Japanese Patent Unexamined Publication Nos. 62-142168/1987, 6-80654/1994, 10-195063/1998, International Publication Nos. WO96/33181, WO 98/57935 and the like. The compounds wherein J¹ is a quinoline derivative can be prepared by, for example, the method described in Japanese Patent Unexamined Publication No. 7-179426/1995. Other derivatives can also be prepared in similar manners.

For example, a compound wherein G¹ moiety is -NHCO- can be prepared according to the method shown in Scheme 1 below.

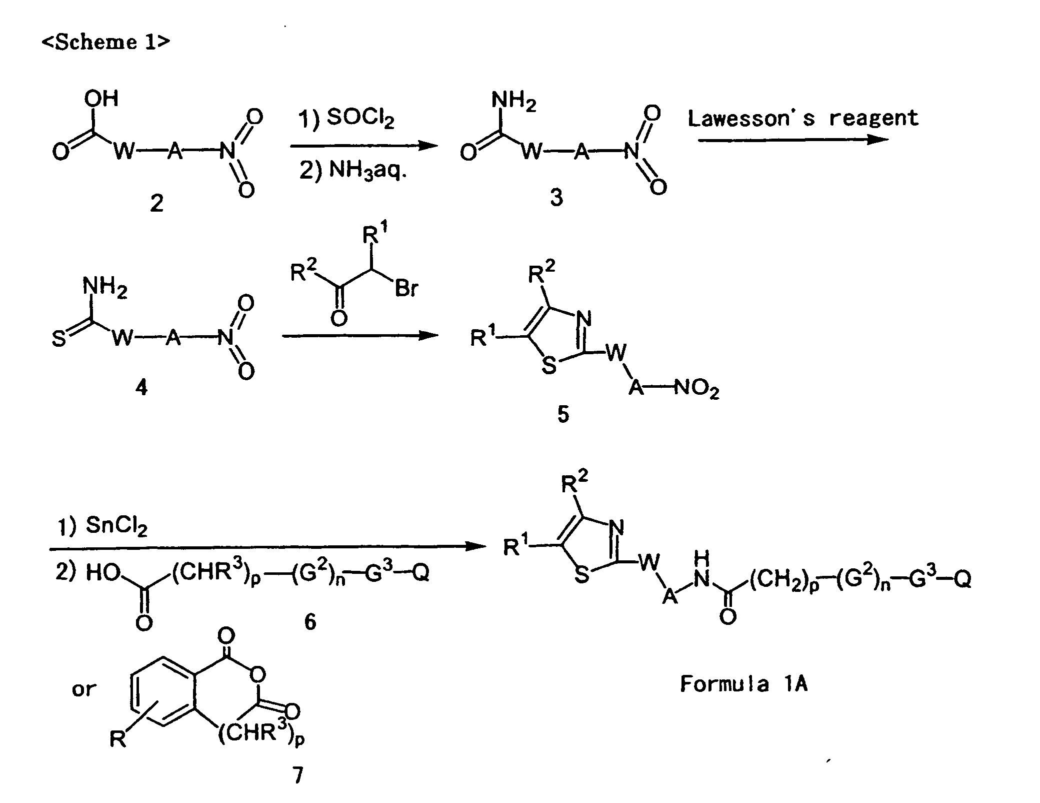

A nitroaryl derivative having carboxyl group is allowed to react with a chlorination agent such as thionyl chloride, and then the product is treated with aqueous ammonia to obtain a carboxamide compound. The carboxamide compound can be converted into a thioamide compound by using the Lawesson's reagent in an inert solvent such as diethyl ether, tetrahydrofuran (THF), toluene and benzene, and the thioamide compound is then condensed with a haloketone, which is commercially available or known, per se, or can be produced by a known method, to obtain Compound 5. An amine compound is prepared by treating the nitro group with a known reducing agent such as tin chloride in an inert alcoholic solvent such as ethanol, or under a reducing condition such as catalytic hydrogenation. Then, the resulting amine derivative is allowed to react with a corresponding carboxylic acid derivative, corresponding carboxylic acid anhydride derivative or the like to obtain a condensate. The reaction may be performed in the presence or absence of a condensing reagent for carboxylic acid and amine, for example, dicyclohexylcarbodiimide, N,N-bis(2-oxo-3-oxazolidinyl)phosphinic acid chloride, dimethylaminopyridine and the like. A compound whose acidic group moiety of Q¹ is protected is subjected to deprotection by using a suitable reagent to obtain a compound represented by the formula 1A. The deprotection can be performed, for example, by applying alkali or acid hydrolysis or catalytic hydrogenation to a carboxylic acid ester, or by treating p-methoxybenzyl-protected tetrazole group with a strong acid such as trifluoroacetic acid in the presence or absence of anisole and the like.

A compound wherein W¹ moiety is ethynyl group can also be prepared by the method shown in Scheme 2 set out below.

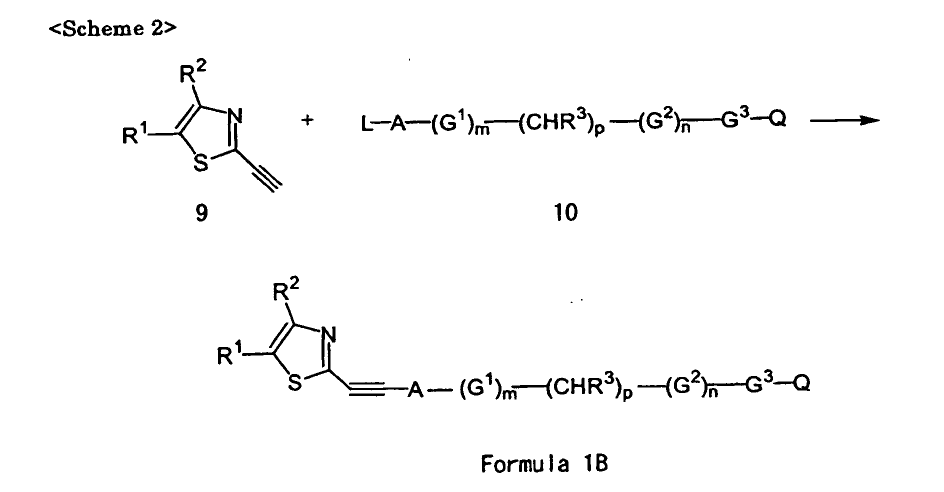

2-Ethynylthiazole that can be prepared by a known method and Compound 10 wherein L represents a leaving group such as a halogen atom and trifluoromethanesulfonate can be coupled to obtain a condensate in an organic amine solvent such as triethylamine or diisopropylamine in the presence of an inert solvent, if required, (for example, tetrahydrofuran, 1,2-dimethoxyethane, diethylformamide and the like) at a temperature within a range of 0°C to the boiling point of the solvent according to a method known per se. The reaction may be performed in the presence or absence of triphenylphosphine and cuprous iodide, or by adding a catalytic amount of palladium [2] acetate, tetrakis(triphenylphosphine)palladium [0], or bis(triphenylphosphine)palladium [2] dichloride. After the coupling, a protective group for the acidic group moiety of Q can be eliminated, if required, to obtain a compound represented by the formula 1B.

A compound wherein W¹ moiety is bound by means of an ether bond can also be prepared by the method shown in the following Scheme 3.

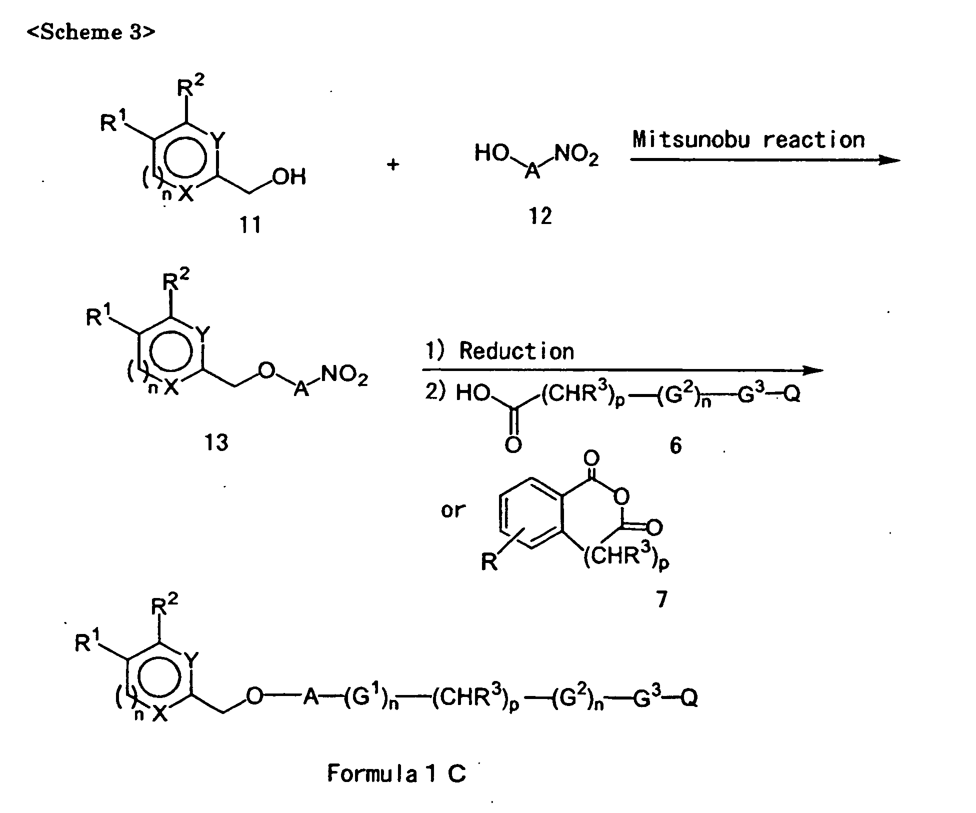

An alcoholic derivative and a phenol derivative can be subjected to the Mitsunobu reaction to obtain a condensate, i.e., treating the derivatives in the presence of diethyl azodicarboxylate and triphenylphosphine in an inert solvent such as tetrahydrofuran. The resulting condensate can be converted into a compound represented by the formula 1C in the same manners as those shown in Scheme 1.

A compound wherein W¹ moiety is bound with ethenyl group can also be prepared by the method shown in the following Scheme 4.

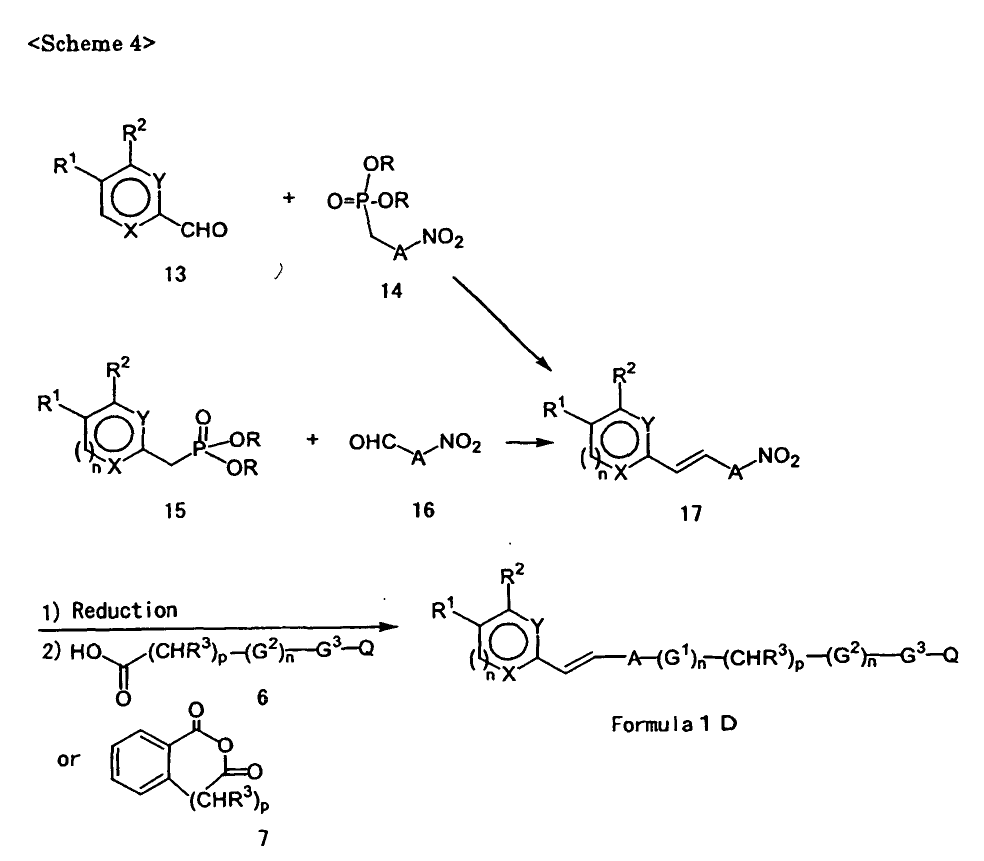

A compound represented by the formula 1D can be obtained by subjecting Compound 13 and Compound 14, or alternatively Compound 15 and Compound 16, to the Horner-Emonds reaction, and then reducing the nitro group of the resulting Compound 17 with stannous chloride, followed by condensing the product with a corresponding carboxylic acid or a corresponding carboxylic acid anhydride.

The compounds of the present invention represented by the general formula (II) can be prepared according to the following methods. However, the methods for preparing the compounds of the present invention are not limited to the methods explained below. Preparations of typical compounds of the present invention are specifically explained in the examples of the specification, and accordingly, any compound falling within the general formula (II) can be prepared by appropriately choosing starting materials, reagents, reaction conditions and the like by referring to the following general explanations of synthetic methods and specific explanations in examples, and by adding modifications or alterations to the disclosed methods, if necessary.

A compound whose J¹¹ is an heteroaromatic ring represented by the aforementioned formula (III) can be prepared according to the method shown in the following scheme 5.

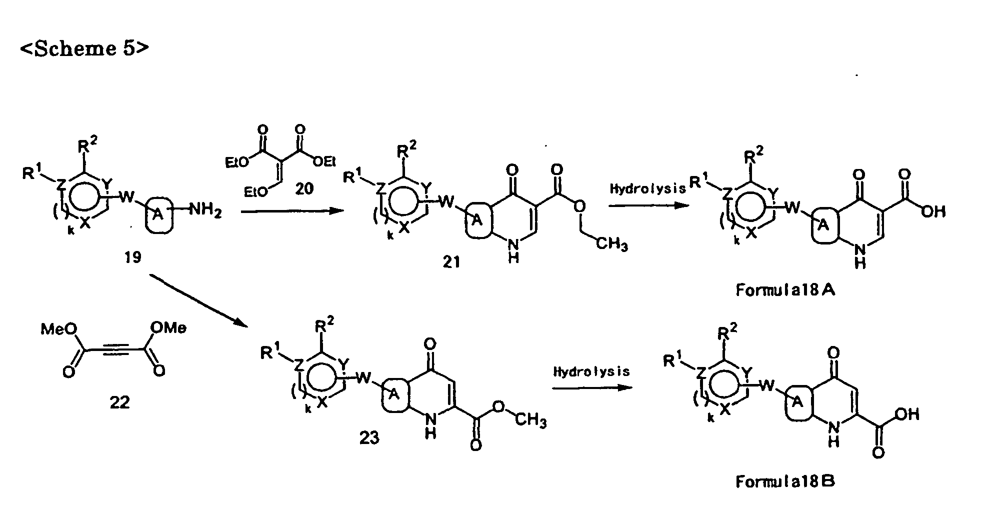

Compound 21 can be obtained by heating Compound 19, which is known per se, or can easily be obtained by a known compound, with diethyl ethoxymethylenemalonate in a solvent such as Dowtherm A, propionic acid, PPA and PPE. The resulting Compound 21 can be treated under conditions of ordinary hydrolysis to obtain a carboxylic acid (compound of the formula 18A). Similarly, Compound 23 can be obtained by condensing Compound 19 with dimethyl acetylenedicarboxylate in a similar manner. The resulting Compound 23 can be treated under conditions of ordinary hydrolysis to obtain a carboxylic acid (compound of the formula 18B).

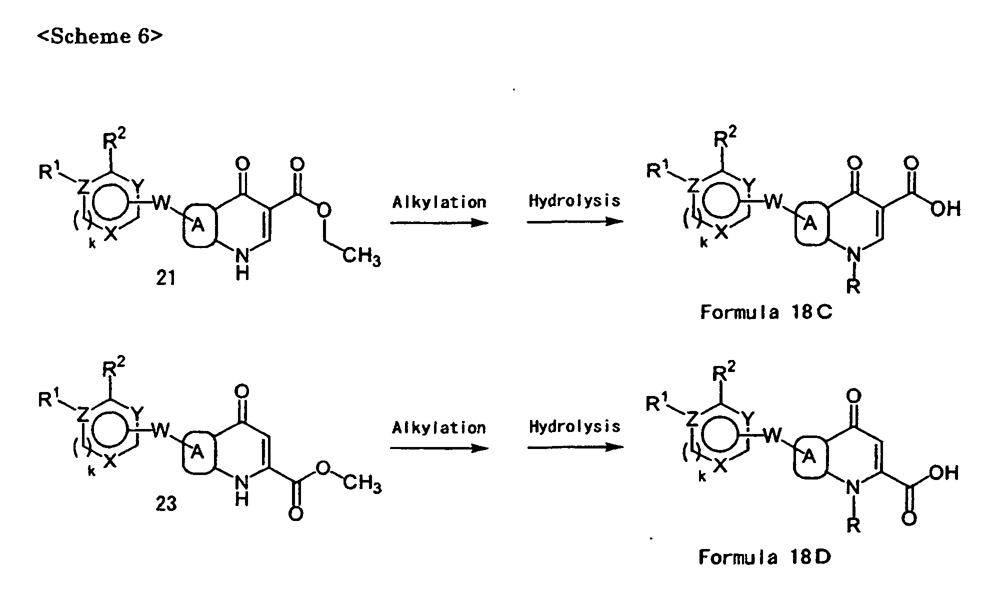

Where the nitrogen atoms of Compound 21 and Compound 23 have hydrogen atom, compounds having a substituent introduced on the nitrogen atoms (compounds of the formulas 18C and 18D) can be obtained by treating the starting compounds with a base such as sodium hydride and potassium carbonate in a solvent such as dimethylformamide (DMF) and THF, and then reacting with an electrophilic agent such as an alkyl halide, followed by hydrolyzing the products.

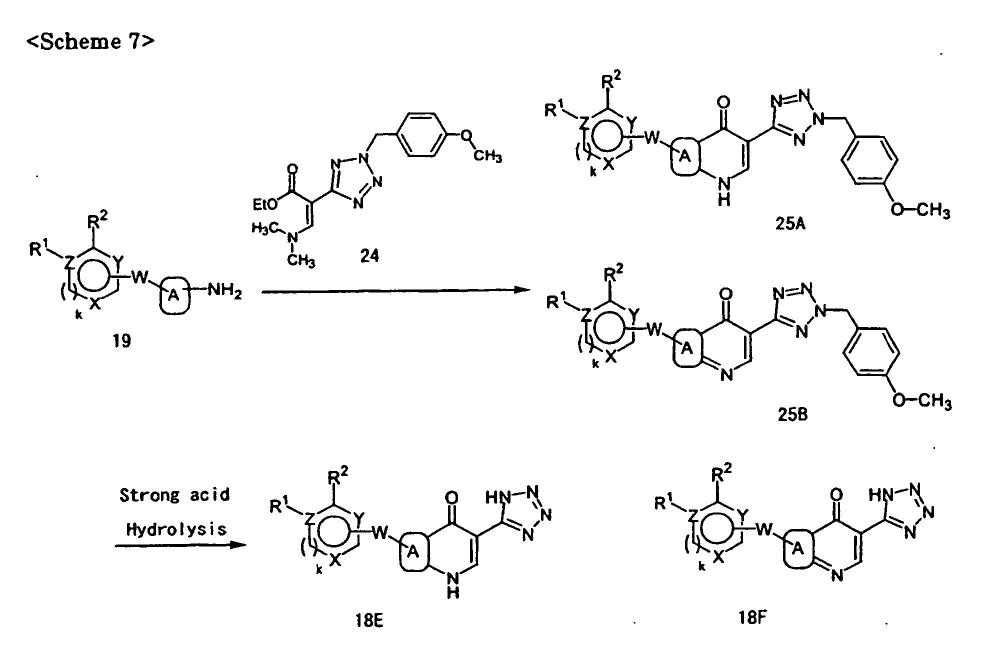

A compound having tetrazole instead of the carboxylic acid (Compounds 18E, F) can be prepared by carrying out the condensation by using known Compound 24 (Chem. Pharm. Bull., 1991, 39, 1099) instead of the diethyl ethoxymethylenemalonate, and treating the product with a strong acid such as trifluoroacetic acid.

A compound of the formula 19A can be prepared by the method shown in the following Scheme 8.

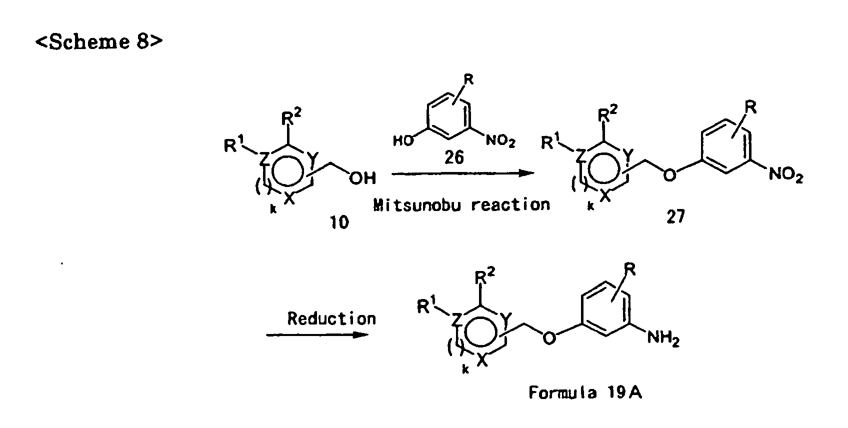

A condensate (Compound 27) can be obtained by carrying out the Mitsunobu reaction by using a 3-hydroxynitrobenzene derivative (Compound 26) and Compound 10. A compound of the formula 19A can be obtained by treating the nitro group of Compound 27 with stannous chloride in an alcohol, or subjecting the nitro group to catalytic reduction.

A compound of the formula 19B can be prepared according to the method shown in the following Scheme 9.

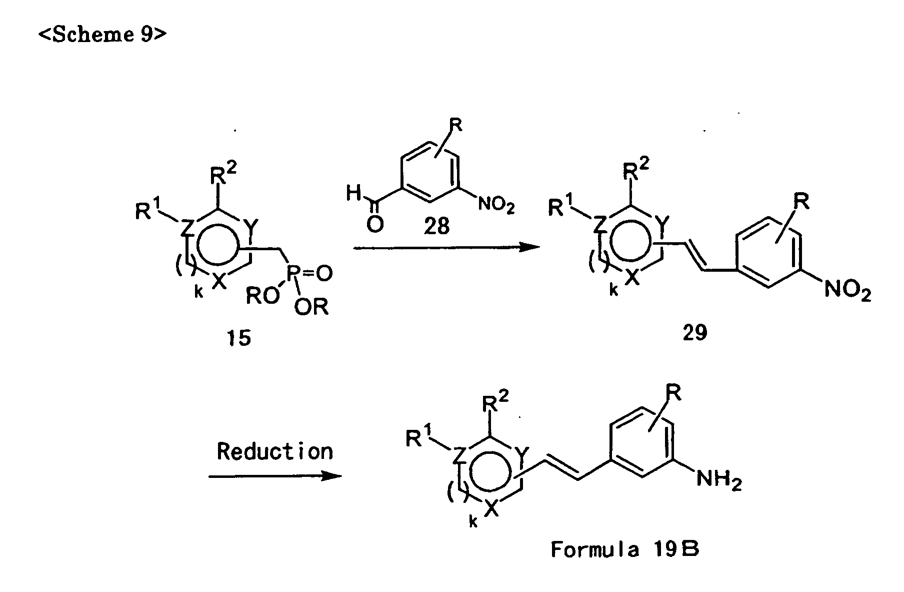

A compound of the formula 19B can be obtained by treating a 3-nitrobenzaldehyde derivative (Compound 28) and Compound 15 with a base such as sodium methoxide in methanol, and then reducing the nitro group by a treatment with stannous chloride in an alcohol.

A compound of the formula 19C can be prepared by the method shown in the following Scheme 10.

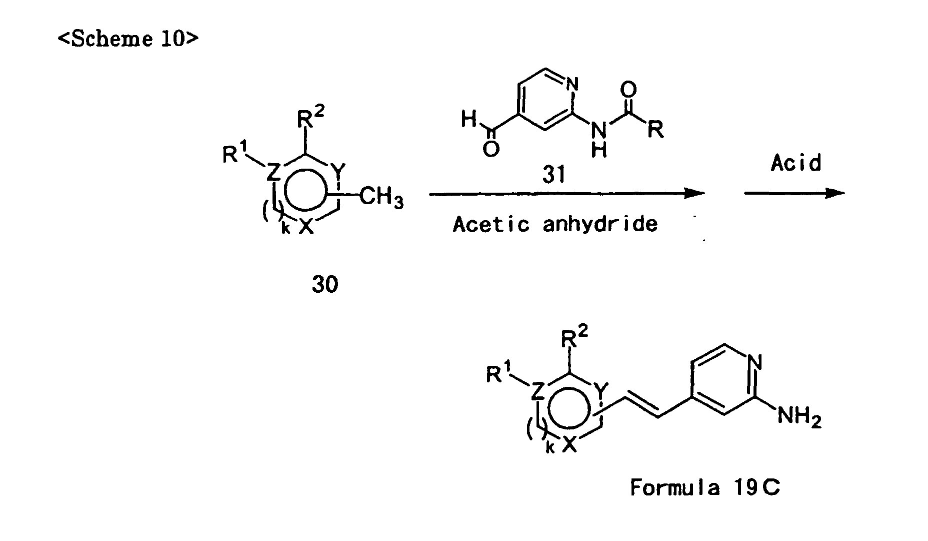

A compound of the formula 19C can be obtained by heating N-acetyl- or N-pivaloyl-2-pyridine-4-aldehyde (Compound 31, International Publication No. WO93/07141) and Compound 30 in acetic anhydride, and then hydrolyzing the product in hydrochloric acid.

Compounds of the formulas 19D and 19E can be prepared by the method shown in the following Scheme 11.

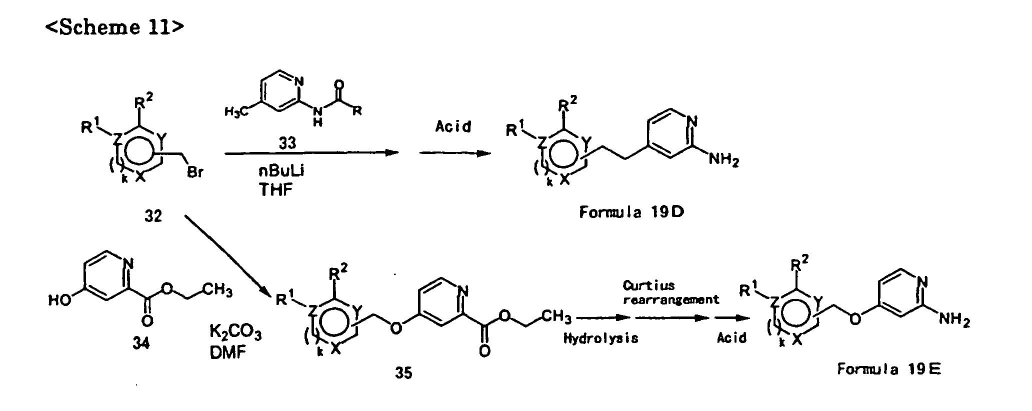

A compound of the formula 19D can be obtained by treating tert-butyl N-(4-methyl-2-pyridyl)carbamate (Compound 33: J. Org. Chem., 1996, 61, 4810) with n-butyllithium in THF and then reacting with a corresponding alkyl halide (Compound 32), followed by treating the product with a strong acid such as trifluoroacetic acid. Compound 35 can be obtained by treating ethyl 4-hydroxypyridine-2-carboxylate (Compound 34, EP-A-0330353) with a base such as potassium carbonate in DMF, and allowing the product to react with a corresponding alkyl halide (Compound 32). A compound of the formula 19E can be obtained by hydrolyzing the ester moiety of Compound 35, subjecting the hydrolysate to the Curtius rearrangement using diphenylphosphoryl azide and t-butanol, and then treating the resulting t-butyl carbonate with a strong acid such as trifluoroacetic acid.

A compound of the formula 18G can be prepared by the method shown in the following Scheme 12.

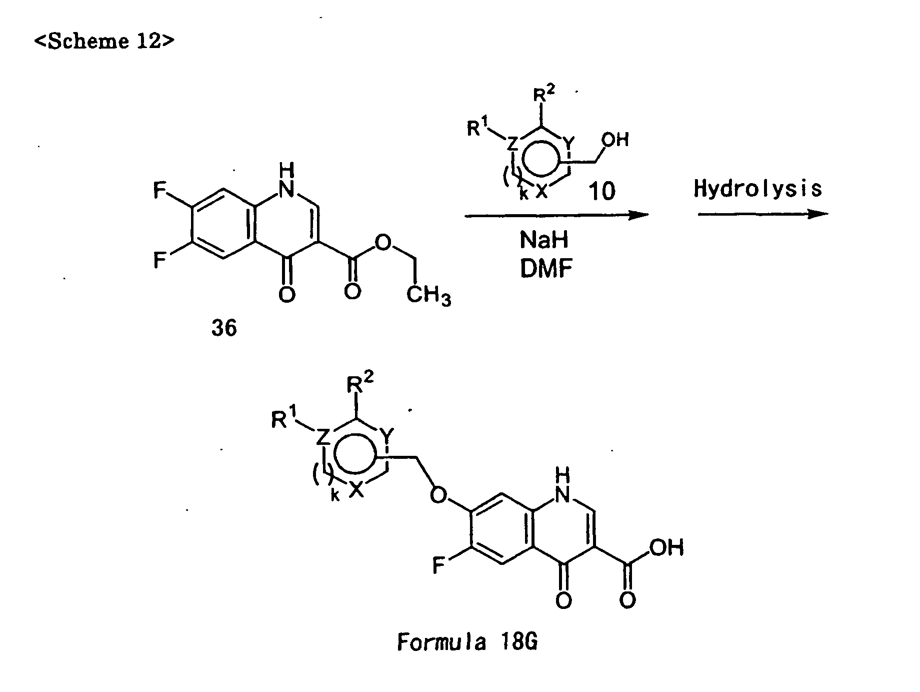

Compound 10 can be converted into an alkoxide by a treatment with a strong base such as sodium hydride in DMF or THF, and then subjecting to reaction with known Compound 36 (for example, Japanese Patent Unexamined Publication Nos. 61-246188/1986, 62-12760/1987 and 60-197686/1985). Then, the resulting product can be converted into a carboxylic acid (compound of the formula 18G) by hydrolysis.

The synthetic intermediates and the target compounds in the aforementioned preparations can be isolated and purified by using methods for isolation and purification ordinarily used in the field of organic synthetic chemistry, for example, neutralization, filtration, extraction, drying, concentration, recrystallization, various chromatographic techniques and the like. The synthetic intermediates may be used in subsequent reactions without purification. When a salt of compound of the general formula (I) or (II) is desired, a product obtained in the form of a salt may be purified without any treatment. When a product is obtained in a free form, a salt can be formed by dissolving or suspending the product in a suitable organic solvent, and then adding an acid or base. It is also possible to convert a compound represented by the general formula (I) or (II) obtained in the form of a salt into a compound in a free form, and then convert the result into an appropriate salt.

Although it is not intended to be bound by any specific theory, the compounds represented by the general formula (I) have an activity for inhibiting drug efflux pumps of microorganisms. More specifically, the compounds represented by the general formula (I) can act on a microorganism with acquired resistance to an antimicrobial agent to inhibit its drug efflux pump, and eliminate the resistance of the microorganism. In addition, the compounds represented by the general formula (I) can act on a microorganism to inhibit a drug efflux pump, thereby suppress the acquisition of resistance to an antimicrobial agent by a microorganism. Therefore, the medicament that comprises a compound represented by the general formula (I) as an active ingredient is useful for preventive and/or therapeutic treatment of microbial infections, generally by a combinational administration with an antimicrobial agent. The medicament is extremely useful as a medicament for preventive and/or therapeutic treatment of, in particular, infectious diseases caused by a microorganism with acquired resistance to one or more antimicrobial agents.

Methods for using the medicament of the present invention are not particularly limited. Examples include a method of administering one or more antimicrobial agents, and also administering the medicament of the present invention simultaneously, separately, or successively to enhance the activity of the antimicrobial agent(s); and a method of preparing a pharmaceutical composition comprising one or more antimicrobial agents and the medicament of the present invention (so-called a compound drug) and the administering the composition.

Kinds of microbial infections that are applicable by the medicament of the present invention are not particularly limited. Bacteria are suitable as target microorganisms. The medicament of the present invention can be used for various infections by microorganisms including Gram-positive or Gram-negative bacteria, aerobic or anaerobic bacteria and the like. The medicament of the present invention can most suitably be used for infections by *Pseudomonas aeruginosa* with acquired resistance to one or more antimicrobial agents, or infections by *Pseudomonas aeruginosa* with low sensitivity to antimicrobial agents. The medicament of the present invention can be used for microbial infections of mammals including human.

Drugs having variety of structures have been known as antimicrobial agents, and various drugs are clinically used. Kinds of antimicrobial agents that can be administered in combination with the medicament of the present invention are not particularly limited, and examples include, for example, penicillin (penam) antibiotics, cephalosporin (cephem) antibiotics, oxacephem antibiotics, penem antibiotics, carbapenem antibiotics, monobactam antibiotics, aminoglycoside antibiotics, macrolide antibiotics, chloramphenicol antibiotics, tetracycline antibiotics, glycopeptide antibiotics, phosphomycin antibiotics, lincomycin antibiotics, sulfonamide preparations, p-aminosalicylic acid preparations, isonicotinic acid hydrazide preparations, quinolone synthetic antimicrobial agents and the like. However, antimicrobial agents are not limited to these examples. When a pharmaceutical composition comprising one or more antimicrobial agents together with the medicament of the present invention is manufactured, the antimicrobial agents exemplified above can also be used.

As the active ingredient of the medicament of the present invention, a substance selected from the group consisting of the compounds represented by the formula (I) and pharmaceutically acceptable salts thereof, and hydrates thereof and solvates thereof can be used. Two or more of the substances may be used in combination. The aforementioned substance, per se, may be administered as the medicament of the present invention. Generally, however, it is desirable that the substance is administered in the form of a pharmaceutical composition comprising one or more of the aforementioned substances as the active ingredient together with one or more pharmaceutical additives. The pharmaceutical composition may optionally be added with one or more of other pharmaceutically active ingredients such as the aforementioned antimicrobial agents and β-lactamase inhibitors.

A pharmaceutical composition for the use of administration *in vivo* can be readily prepared by mixing one or more of the aforementioned substances as the active ingredient and one or more pharmaceutically acceptable additives for pharmaceutical preparations according to methods for formulation ordinarily used in the field of manufacturing pharmacy. The route of administration of the medicament of the present invention is not particularly limited; however, it is desirable to appropriately chose the most effective administration route for preventive and/or therapeutic treatment of a target infectious disease. Examples of pharmaceutical compositions suitable for oral administration include, for example, capsules, powders, tablets, granules, subtilized granules, emulsions, syrups, solutions, suspensions and the like. Examples of pharmaceutical compositions suitable for parenteral administration include, for example, inhalants, sprays, intrarectal preparations, injections, drip infusions, ointments, creams, transdermal preparations, transmucosal preparations, eye drops, nasal drops, ear drops, tape preparations, patches and the like. However, the forms of the medicament of the present invention are not limited to these examples.

Among the pharmaceutical compositions suitable for oral administration, liquid preparations such as emulsions and syrups can be prepared by using pharmaceutical additives including water; saccharides such as sucrose, sorbitol, fructose; glycols such as polyethylene glycol and propylene glycol; oils such as sesame oil, olive oil and soybean oil; antiseptics such as p-hydroxybenzoic acid esters; flavors such as strawberry flavor and peppermint and the like. Solid preparations such as capsules, tablets, powders and granules can be prepared by using excipients such as lactose, glucose, sucrose and mannitol; disintegrating agents such as starch and sodium alginate; lubricants such as magnesium stearate and talc; binders such as polyvinyl alcohol, hydroxypropylcellulose and gelatin; surfactants such as fatty acid esters; plasticizers such as glycerin and the like.

Among the pharmaceutical compositions suitable for parenteral administration, liquid preparations such as injections, drip infusions and eye drops can preferably be prepared as sterilized isotonic liquid preparations. For example, injections can be prepared by using an aqueous medium such as a solution of sodium chloride, a solution of glucose, or a mixture of saline and glucose solution. The intrarectal preparations can be prepared generally in the form of suppositories by using carriers such as cacao butter, hydrogenated fat and hydrogenated carboxylic acid. For the preparation of sprays, a non-irritable carrier can be used that enables fine dispersion and enhances absorption of the aforementioned substances as the active ingredient. Examples of such a carrier include lactose, glycerin and the like. Aerosols, dry powders or the like can also be chosen as the form of preparation. However, pharmaceutical additives used for the manufacture of the medicament of the present invention are not limited to those mentioned above, and any additives available for those skilled in the art can be used.

A dose and frequency of administration of the medicament of the present invention are not particularly limited, and a suitable dose can be chosen depending on the type and severity of a microbial infection, the presence or absence of an underlying disease, the age and body weight of a patient and the like.

The compound provided is characterized in that its partial structure occupies the following four sites in the following tolerance. The compounds provided have a pharmacophore comprising the following four sites as a common pharmacophore to exert an inhibitory activity against drug efflux pumps of *Pseudomonas aeruginosa* Each of Sites 1 to 3 shows a hydrophobic feature, and Site 4 shows a negatively ionizable (acidic group) feature. Definitions of these features are described in the following literature or the cited references in the literature (J. Chem. Inf., and Comp. Sci., 34, 1297-1308).

| Site | Feature | x(Å) | y(Å) | z(Å) | Tolerance (Å) |
|---|---|---|---|---|---|
| 1 | Hydrophobic | 8.839 | -0.324 | 1.49 | 2 |
| 2 | Hydrophobic | 4.819 | 1.836 | -0.29 | 1.7 |
| 3 | Hydrophobic | -1.722 | -0.964 | 0.89 | 1.7 |
| 4 | Negative Ionizable | -8.636 | -1.067 | -2.554 | 2 |

In the aforementioned pharmacophore, more specifically, the hydrophobic feature represents a position occupied by a hydrophobic substituent in the partial structure of the compound. Examples of the hydrophobic substituent include functional groups such as, for example, an alkyl group, a cycloalkyl group, an aryl group, a heterocyclic group, or a halogen atom. However, the substituents are not limited to these examples. The cite of an acidic group represents a position occupied by an acidic group in the partial structure of the compound.

Examples of the substituent occupying Site 1 include, for example, an alkyl group, an aryl group, a heterocyclic group, a halogen atom and the like, and an alkyl group, an aryl group, or a heterocyclic group is preferred. These groups may further have one or more substituents.

Examples of the substituent occupying Site 2 include an alkyl group, an aryl group, a heterocyclic group, a halogen atom and the like, and an aryl group or a heterocyclic group is preferred. These groups may further have one or more substituents.

Examples of the substituent occupying Site 3 include an alkyl group, an aryl group, a heterocyclic group, a halogen atom and the like, and an aryl group or a heterocyclic group is preferred. These groups may further have one or more substituents.

The type of the acidic group occupying Site 4 is not particularly limited, and the acidic group may be cyclic, acyclic, or a combination thereof. The acidic group may further have one or more substituents.

A structural moiety connecting each of the sites is referred to as a linker. The linker comprises, for example, carbon atom, oxygen atom, nitrogen atom, sulfur atom or the like as a constitutional element, and has a length required to have the partial structures of the compound fit in each of the sites. However, the kind of the linker is not particularly limited. Specifically, examples of the linker include those selected from the group consisting of -CH=CH-, -C≡C-, -CH₂CH₂-, -OCH₂-, -SCH₂-, -OCH₂O-, -CH₂O-, -CH₂-, -CO-, -CH₂CH₂CH₂-, -CH₂NH-, -NHCH₂-, -CH₂S-, -CONH-, -CH₂SCH₂-, -CH=CH-CONH- and -CH₂OCH₂-, and those containing a combination of these linkers. Some of the aforementioned linkers have geometrical isomerism, and any of such isomers may be used.

Examples of the compound having the aforementioned pharmacophore include those represented by the aforementioned general formula (I) or (II). However, the compounds are not limited to these compounds.

R¹, R², R³¹ and R³² are partial structures occupying Site 1, J¹ and J¹¹ are those occupying Site 2, A¹ and A¹¹ are those occupying Site 3, and Q¹ and Q¹¹ correspond to those occupying Site 4. The term "occupy" means that each structure itself or atoms constituting each structure (substituent) take an arbitrary locus in an inner space of a sphere defined as the site.

By using the aforementioned pharmacophore, it can be determined whether or not a compound has an inhibitory activity against the drug efflux pump of *Pseudomonas aeruginosa*. In addition, a compound having an inhibitory activity against the drug efflux pump of *Pseudomonas aeruginosa* can be selected by carrying out screening in which the aforementioned pharmacophore model is applied and a library of compounds having generated conformations is used. Moreover, a virtual compound can be created by calculating conformations in the same manner on the basis of a class of virtual compounds, and then carrying out docking study with the aforementioned pharmacophore. A useful medicament can be created by practically synthesizing the virtually created compound and assaying the inhibitory activity against the drug efflux pump of *Pseudomonas aeruginosa*. Appropriate combination of two or more of these techniques may be applicable to find a compound having more potent inhibitory activity against the drug efflux pump of *Pseudomonas aeruginosa*.

The compounds can be efficiently overlaid by using a software having a docking function such as CATALYST (Greene et al., J. Chem. Inf. Comp. Sci., 1994, 34, 1297-1308; The software is commercially available from Molecular Simulation Inc.), and alternatively, the overlay can manually be performed. As for the creation of a pharmacophore using CATALYST, specific techniques are disclosed in detail in, for example, International Publication WO/04913 and the like. In general, compounds that fit for a pharmacophore are those wherein, for each of sites, constitutional atoms in a partial structure that conform with the feature of a site are located within 0.5 to 3 Å from the site when they are optimally overlaid with the pharmacophore. A compound whose partial structure occupied within the aforementioned tolerance for all of the four sites of the aforementioned pharmacophore (within 2 Å for Site 1 and Site 4, and within 1.7 Å for Site 2 and Site 3) has an inhibitory action against the drug efflux pump of *Pseudomonas aeruginosa,* and can be expected to have an effect of combination with an antibacterial agent in preventive and/or therapeutic treatment of infectious diseases. The compounds represented by the general formula (I) and (II), as compounds having an inhibitory action against the drug efflux pump of *Pseudomonas aeruginosa,* can be used as active ingredients of medicaments for preventive and/or therapeutic treatment of infectious diseases as well as inhibitors of the drug efflux pump of *Pseudomonas aeruginosa*.

### Examples

The present invention will be explained more specifically with reference to the following examples. However, the scope of the present invention is not limited to these examples. In the examples a (*) means, that the compound falls outside the claimed use.

### Example 1: 2-(2-Oxo-2-3-[(E)-2-(4-phenyl-1,3-thiazol-2-yl)-1-ethenyl]anilinoethyl)-benzoic acid (*)

3-[(E)-2-(4-Phenyl-1,3-thiazol-2-yl)-1-ethenyl]aniline (Japanese Patent Unexamined Publication No. 62-142168/1987, 500 mg, 1.8 mmol) and homophthalic anhydride (440 mg, 2.7 mmol) were dissolved in toluene (10 mL), and the solution was heated under reflux for 30 minutes. After the reaction mixture was cooled, the deposited crystals were collected by filtration to obtain the title compound as a white solid (720 mg, 91%).
¹H-NMR (DMSO-d₆) δ : 4.11 (2H, s), 7.30-7.54 (12H, m), 7.89 (1H, d, J=8.3Hz), 7.96 (1H, s), 7.99 (1H, d, J=7.2Hz), 8.09 (1H, s), 10.18 (1H, s)
FAB-MS; m/z: 441 (MH⁺)

### Example 2: 2-{2-Oxo-2-[3-(2-quinolylmethoxy)anilino]ethyl}benzoic acid

### (A) 2-[(3-Nitrophenyloxy)methyl]quinoline (*)

m-Nitrophenol (500 mg, 3.59 mmol) and 2-chloromethylquinoline (846 mg, 3.95 mmol) were dissolved in DMF (10 ml), and the solution was added with potassium carbonate (745 mg) and stirred overnight at room temperature. The reaction mixture was concentrated, diluted with ethyl acetate, washed with saturated aqueous sodium hydrogencarbonate and saturated brine, and then dried over magnesium sulfate. The solvent was evaporated under reduced pressure, and the residue was purified by silica gel chromatography (n-hexane:ethyl acetate, 8:1, v/v) to obtain the title compound (245 mg, 24%).
¹H-NMR (CDCl₃) δ : 5.45 (2H, s), 7.35 (1H, dd, J=8.3, 2.4Hz), 7.44 (1H, t, J=8.3Hz), 7.58 (1H, t, J=7.8Hz), 7.65 (1H, d, J=8.8Hz), 7.76 (1H, t, J=7.8Hz), 7.84-7.86 (2H, m), 7.92 (1H, d, J=2.0Hz), 8.10 (1H, d, J=8.8Hz), 8.23 (1H, d, J=8.3Hz)

### (B) 3-(2-Quinolylmethoxy)aniline

The 2-[(3-nitrophenyloxy)methyl]quinoline obtained in (A) (245 mg, 0.874 mmol) was dissolved in ethanol (10 ml), and the solution was added with stannous chloride (580 mg) and heated under reflux for 3 hours. After the reaction mixture was left stand for cooling, the mixture was added with 5 M aqueous sodium hydroxide (20 ml) and then stirred for 15 minutes. The reaction mixture was extracted with chloroform. The organic layer was dried over magnesium sulfate, and the solvent was evaporated under reduced pressure to obtain the title compound (247 mg, quantitative).

### (C) 2-{2-Oxo-2-[3-(2-quinolylmethoxy)anilino]ethyl}benzoic acid

The 3-(2-quinolylmethoxy)aniline obtained in (B) (219 mg, 0.875 mmol) and homophthalic anhydride (213 mg, 0.131 mmol) were dissolved in toluene (10 ml), and the solution was heated under reflux for 30 minutes. The deposited solid was collected by filtration to obtain the title compound as a white solid (223 mg, 62%).
¹H-NMR (DMSO-d₆) δ : 4.06 (2H, s), 5.32 (2H, s), 6.71 (1H, m), 7.14 (1H, m), 7.19 (1H, t, J=7.8Hz), 7.33-7.42 (3H, m), 7.50 (1H, t, J=7.3Hz), 7.63 (2H, m), 7.78 (1H, m), 7.87 (1H, d, J=6.8Hz), 8.00 (2H, t, J=8.8Hz), 8.40 (1H, d, J=8.3Hz), 10.1 (1H, br)
FAB-MS; m/z: 413 (MH⁺)

### Example 3: 2-(2-3-[(E)-2-(4-Isopropyl-5-chloro-1,3-thiazol-2-yl)-1-ethenyl]anilino-2-oxoethyl)benzoic acid (*)

### (A) 4-Isopropyl-5-chloro-2-[(E)-2-(3-nitrophenyl)-1-ethenyl]-1,3-thiazole hydrochloride

4-Isopropyl-2-[(E)-2-(3-nitrophenyl)-1-ethenyl]-1,3-thiazole (Japanese Patent Unexamined Publication No.62-142168/1987, 270 mg, 0.98 mmol) was dissolved in carbon tetrachloride (5 mL), and the solution was added with N-chlorosuccinimide (160 mg, 1.2 mmol) and heated under reflux for 17 hours. The reaction mixture was distributed in methylene chloride and saturated aqueous sodium hydrogencarbonate. The organic layer was washed with saturated brine, and dried over magnesium sulfate, and then the solvent was evaporated under reduced pressure. The residue was added with a small amount of hydrochloric acid in dioxane, and the solvent was evaporated again. The residue was purified by silica gel chromatography (hexane:ethyl acetate, 4:1, v/v) to obtain the title compound as a white solid (236 mg, 69%).
¹H-NMR (CDCl₃) δ : 1.31 (6H, d, J=6.8Hz), 3.24 (1H, m), 7.30 (2H, s), 7.56 (1H, t, J=7.8Hz), 7.81 (1H, d, J=7.8Hz), 8.16 (1H, dd, J=7.8, 2.0Hz), 8.37 (1H, t, J=2.0Hz)

### (B) 2-(2-3-[(E)-2-(4-Isopropyl-5-chloro-1,3-thiazol-2-yl)-1-ethenyl]anilino-2-oxoethyl)-benzoic acid

The 4-isopropyl-5-chloro-2-[(E)-2-(3-nitrophenyl)-1-ethenyl]-1,3-thiazole hydrochloride obtained in (A) (225 mg, 0.652 mmol) was dissolved in ethanol (10 mL), and the solution was added with tin (II) chloride (590 mg, 3.11 mmol) and heated under reflux for 2 hours. The reaction mixture was added with 4 N aqueous sodium hydroxide, and extracted with ethyl acetate. After the organic layer was washed with saturated brine and dried over sodium sulfate, the solvent was evaporated under reduced pressure. The residue was dissolved in toluene (10 mL), and the solution was added with homophthalic anhydride (126 mg, 0.78 mmol) and heated under reflux for 30 minutes. The reaction mixture was cooled, and the deposited crystals were collected by filtration to obtain the title compound as a white solid (88 mg, 30%).
¹H-NMR (DMSO-d₆) δ : 1.23 (6H, d, J=6.8Hz), 3.15 (1H, m), 3.98 (2H, s), 7.25-7.50 (8H, m), 7.80-7.90 (2H, m)
FAB-MS; m/z: 441 (MH⁺)

### Example 4: 2-[2-Oxo-2-{3-[(E)-(5-phenyl-2-furyl)-1-ethenyl]anilino}ethyl]benzoic acid

### (A) N-Methyl-N-methoxy-5-bromo-2-furanamide (*)

5-Bromo-2-furancarboxylic acid (3.00g, 15.7 mmol) was dissolved in methylene chloride (60 ml), and the solution was added with N-methyl-N-methoxy-amine hydrochloride (94 mg), triethylamine (2.19 ml, 15.7 mmol) and HOBt (1.06 g, 7.85 mmol). Then, the mixture was added with WSCD·HCl (3.31 g, 17.3 mmol) under ice cooling and then stirred at room temperature for 18 hours. The reaction mixture was diluted with methylene chloride, washed with 10% aqueous citric acid, saturated aqueous sodium hydrogencarbonate and saturated brine, and then dried over magnesium sulfate. The solvent was evaporated under reduced pressure. The residue was purified by silica gel chromatography (n-hexane:ethyl acetate, 4:1, v/v) to obtain the title compound as colorless amorphous (3.18 g, 86%).
¹H-NMR (CDCl₃) δ ; 3.33 (3H, s), 3.77 (3H, d, J=2.0Hz), 6.45 (1H, dd, J=3.9, 2.0Hz), 7.10 (1H, d, J=3.9Hz)

### (B) N-Methyl-N-methoxy-5-phenyl-2-furanamide

The N-methyl-N-methoxy-5-bromo-2-furanamide obtained in (A) (3.18 g, 13.6 mmol) was dissolved in toluene/water (60 ml, 2:1, v/v), and the mixture was added with phenylboric acid (1.74 g, 14.3 mmol), tetrakis(triphenylphosphine)palladium (785 mg) and sodium carbonate (4.32 g, 40.8 mmol), and then heated under reflux overnight. The reaction mixture was left stand for cooling, and then extracted with ethyl acetate. After the organic layer was dried over magnesium sulfate, the solvent was evaporated under reduced pressure. The residue was purified by silica gel chromatography (n-hexane:ethyl acetate, 4:1, v/v) to obtain the title compound as white crystals (2.27 g, 72%).
¹H-NMR (CDCl₃) δ ; 3.39 (3H, s), 3.84 (3H, s), 6.77 (1H, d, J=3.9Hz), 7.25 (1H, d, J=3.9Hz), 7.34 (1H, m), 7.43 (2H,t, J=7.3Hz), 7.80 (2H, d, J=7.3Hz)

### (C) 5-Phenyl-2-furaldehyde

Aluminum lithium hydride (410 mg, 10.8 mmol) was suspended in THF (15 ml), and the mixture was added with the N-methyl-N-methoxy-5-phenyl-2-furanamide obtained in (B) (500 mg, 2.16 mmol) under ice cooling and stirred for 30 minutes at 0°C. The reaction mixture was poured into ice water, and the mixture was added with potassium hydrogensulfate and stirred for a while. The reaction mixture was extracted with ethyl acetate, and the organic layer was dried over magnesium sulfate. After the drying agent was removed by filtration, the solvent was evaporated under reduced pressure. The residue was purified by silica gel chromatography (n-hexane:ethyl acetate, 20:1, v/v) to obtain the title compound (289 mg, 78%).
¹H-NMR (CDCl₃) δ ; 6.83 (1H, d, J=3.9Hz), 7.31 (1H, d, J=3.9Hz), 7.38-7.44 (3H, m), 7.82 (2H, m), 9.64 (1H, s)

### (D) Diethyl (3-nitrobenzyl)phosphonate

3-Nitrobenzyl bromide (1.00 g, 4.63 mmol) was dissolved in triethyl phosphite (15 ml), and the mixture was stirred at 100°C for 3 hours. The reaction mixture was left stand for cooing, and then added with ether. The deposited insoluble substances were removed by filtration, and the mother liquor was evaporated. The residue was purified by silica gel chromatography (n-hexane:ethyl acetate, 4:1 → 1:1, v/v) to obtain the title compound (1.14 g, 90%).
¹H-NMR (CDCl₃) δ : 1.27 (6H, t, J=7.3Hz), 3.21 (1H, s), 3.27 (1H, s), 4.07 (4H, q, J=7.3Hz), 7.50 (1H, dd, J=8.3, 7.8Hz), 7.66 (1H, d, J=7.8Hz), 8.14 (1H, d, J=8.3Hz), 8.16 (1H, d, J=2.0Hz)

### (E) 2-[(E)-2-(3-Nitrophenyl)-1-ethenyl]-5-phenylfuran

The 5-phenyl-2-furaldehyde obtained in (C) (188 mg, 1.09 mmol) and the diethyl (3-nitrobenzyl)phosphonate obtained in (D) (597 mg, 2.18 mmol) were dissolved in DMF (3 ml), and the solution was added with sodium methoxide (118 mg) and stirred at room temperature for 6 hours. The reaction mixture was further added with 5-phenyl-2-furaldehyde (191 mg) and sodium methoxide (118 mg), then added with 60% sodium hydride (88.0 mg) and stirred at room temperature overnight. The reaction mixture was added with methanol, and concentrated under reduced pressure. The resulting residue was diluted with ethyl acetate, and washed with saturated brine. The organic layer was dried over magnesium sulfate, and the solvent was evaporated under reduced pressure. The residue was purified by silica gel chromatography (n-hexane:ethyl acetate, 20:1, v/v) to obtain the title compound as yellow crystals (288 mg, 45%).
¹H-NMR (CDCl₃) δ ; 6.54 (1H, d, J=3.4Hz), 6.73 (1H, d, J=3.4Hz), 7.04 (1H, d, J=16.1Hz), 7.15 (1H, d, J=16.1Hz), 7.30 (1H, t, J=7.8Hz), 7.43 (1H, t, J=7.8Hz), 7.51 (1H, t, J=7.8Hz), 7.76 (3H, m), 8.07 (1H, m), 8.35 (1H, s)

### (F) 3-[(E)-(5-Phenyl-2-furyl)-1-ethenyl]aniline

The 2-[(E)-2-(3-nitrophenyl)-1-ethenyl]-5-phenylfuran obtained in (E) (288 mg, 0.989 mmol) was dissolved in ethanol (15 ml), and the solution was added with stannous chloride (656 mg) and heated under reflux for 3 hours. The reaction mixture was left stand for cooling, and then added with 5 M aqueous sodium hydroxide (20 ml) and stirred for 15 minutes. The mixture was then extracted with chloroform, and the organic layer was dried over magnesium sulfate. The solvent was evaporated under reduced pressure to obtain the title compound (259 mg, quantitative).

### (G) 2-[2-Oxo-2-{3-[(E)-(5-phenyl-2-furyl)-1-ethenyl]anilino}ethyl]benzoic acid

The 3-[(E)-(5-phenylfuryl)-1-ethenyl]aniline obtained in (F) (259 mg, 0.987 mmol) and homophthalic anhydride (240 mg, 0.148 mmol) were dissolved in toluene (7 ml), and the solution was heated under reflux for 30 minutes. The deposited solids were collected by filtration to obtain the title compound as a white solid (302 mg, 72%).
¹H-NMR (DMSO-d₆) δ : 4.11 (2H, s), 6.69 (1H, d, J=3.42), 7.02 (1H, d, J=16.2Hz), 7.03 (1H, d, J=3.4Hz), 7.09 (1H, d, J=16.2Hz), 7.27-7.46 (8H, m), 7.53 (1H, t, J=7.3Hz), 7.79 (2H, d, J=7.8Hz), 7.89 (2H, d, J=7.3Hz), 7.92 (1H, s), 10.1 (1H, s)
FAB-MS; m/z: 423 (M⁺)

### Example 5: 2-{2-[3-((E)-2-{5-Amino-4-isopropyl-1,3-thiazol-2-yl}-1-ethenyl)amino]-2-oxoethyl}benzoic acid (*)

### (A) Ethyl 4-isopropyl-2-[(E)-2-(3-nitrophenyl)-1-ethenyl]-1,3-thiazole-5-carboxylate

(E)-3-(3-Nitrophenyl)-2-propenethioamide (2.07 g, 9.99 mmol) and ethyl 2-chloro-4-methyl-3-oxopentanoate (1.92 g, 9.99 mmol) were dissolved in ethanol (100 ml), and the solution was heated under reflux for 26 hours. The reaction mixture was left stand for cooling and then concentrated. The residue was purified by silica gel chromatography (n-hexane:ethyl acetate, 20:1, v/v) to obtain the title compound (812 mg, 24%).
¹H-NMR (CDCl₃) δ : 1.34 (6H, d, J=9.8Hz), 1.39 (3H, t, J=7.3Hz), 3.99 (1H, m), 4.36 (2H, q, J=7.3Hz), 7.36 (1H, d, J=16.1Hz), 7.56 (1H, d, J=16.1Hz), 7.58 (1H, t, J=8.3Hz), 7.85 (1H, d, J=8.3Hz), 8.19 (1H, dd, J=8.3, 2.0Hz), 8.41 (1H, brs)

### (B) 4-Isopropyl-2-[(E)-2-(3-nitrophenyl)-1-ethenyl]-1,3-thiazole-5-carboxylic acid

The ethyl 4-isopropyl-2-[(E)-2-(3-nitrophenyl)-1-ethenyl]-1,3-thiazole-5-carboxylate obtained in (A) (250 mg, 0.722 mmol) was dissolved in a mixed solvent of THF, methanol and water (6 ml, 3:2:1, v/v), and the solution was added with lithium hydroxide monohydrate (60.6 mg) and stirred at room temperature for 3 hours. The reaction mixture was neutralized with 1 M hydrochloric acid, and extracted with ethyl acetate. The organic layer was dried over magnesium sulfate. The solvent was evaporated under reduced pressure to obtain the title compound (247 mg, quantitative).

### (C) tert-Butyl N-{4-isopropyl-2-[(E)-2-(3-nitrophenyl)-1-ethenyl]-1,3-thiazol-5-yl}carbamate

The 4-isopropyl-2-[(E)-2-(3-nitrophenyl)-1-ethenyl]-1,3-thiazole-5-carboxylic acid obtained in (B) (230 mg, 0.722 mmol) was dissolved in tert-butanol (8 ml), and the solution was added with triethylamine (0.121 ml) and diphenyl phosphoric acid azide (0.187 ml), and then heated under reflux for 6 hours. The reaction mixture was left stand for cooling, and then diluted with ethyl acetate and washed with 10% aqueous citric acid, saturated aqueous sodium hydrogencarbonate, and then with saturated brine. The reaction mixture was dried over magnesium sulfate, and the solvent was evaporated under reduced pressure. The residue was purified by silica gel chromatography (n-hexane:ethyl acetate, 8:1, v/v) to obtain the title compound (272 mg, 97%).
¹H-NMR (CDCl₃) δ ; 1.32 (6H, d, J=6.8Hz), 1.54 (9H, s), 2.93 (1H, m), 6.66 (1H, br), 7.23 (1H, d, J=16.1Hz), 7.33 (1H, d, J=16.1Hz), 7.52 (1H, dd, J=8.3, 7.8Hz), 7.76 (1H, d, J=7.8Hz), 8.10 (1H, dd, J=8.3, 1.5Hz), 8.33 (s, 1H)

### (D) tert-Butyl N-{4-isopropyl-2-[(E)-2-(3-aminophenyl)-1-ethenyl]-1,3-thiazol-5-yl}carbamate

The tert-butyl N-{4-isopropyl-2-[(E)-2-(3-nitrophenyl)-1-ethenyl]-1,3-thiazol-5-yl}carbamate obtained in (C) (272 mg, 0.698 mmol) was dissolved in ethanol (10 ml), and the solution was added with stannous chloride (463 mg) and heated under reflux for 3 hours. The reaction mixture was left stand for cooling, and then added with 5 M aqueous sodium hydroxide (20 ml) and stirred for 15 minutes. The reaction mixture was extracted with ethyl acetate, and the organic layer was dried over magnesium sulfate. The solvent was evaporated under reduced pressure to obtain the title compound (241 mg, 96%).

### (E) 2-{2-[3-((E)-2-{5-[(tert-Butoxycarbonyl)amino]-4-isopropyl-1,3-thiazol-2-yl}-1-ethenyl)amino]-2-oxoethyl}benzoic acid

The tert-butyl N-{4-isopropyl-2-[(E)-2-(3-amino phenyl)-1-ethenyl]-1,3-thiazol-5-yl}carbamate obtained in (D) (241 mg, 0.670 mmol) and homophthalic anhydride (163 mg, 1.01 mmol) were dissolved in toluene (8 ml), and the solution was heated under reflux for one hour. The deposited solid was collected by filtration to obtain the title compound as a white solid (244 mg, 70%).
¹H-NMR (DMSO-d₆) δ : 1.15 (6H, d, J=6.8Hz), 1.46 (9H, s), 2.49 (1H, m), 4.09 (2H, s), 7.28-7.44 (6H, m), 7.82-7.88 (2H, m), 9.65 (1H, br), 10.3 (1H, br)
FAB-MS; m/z: 522 (MH⁺)

### (F) 2-(2-[3-((E)-2-{5-Amino-4-isopropyl-1,3-thiazol-2-yl}-1-ethenyl)amino]-2-oxoethyl)-benzoic acid

The 2-{2-[3-((E)-2-{5-[(tert-butoxycarbonyl)amino]-4-isopropyl-1,3-thiazol-2-yl}-1-ethenyl)amino]-2-oxoethyl}benzoic acid obtained in (E) (150 mg, 0.288 mmol) was dissolved in 4 N hydrochloric acid in dioxane (3 ml), and the solution was stirred at room temperature for 2 hours. After the solvent was evaporated under reduced pressure, diethyl ether was added to the residue. The deposited crystals were collected by filtration, and dried to obtain dihydrochloride of the title compound as white powder (148 mg, quantitative).
¹H-NMR (DMSO-d₆) δ : 1.20 (6H, d, J=6.8Hz), 3.39 (1H, m), 4.11 (2H, s), 7.11 (1H, d, J=16.1Hz), 7.24-7.42 (7H, m), 7.52 (1H, t, J=7.8Hz), 7.89 (1H, d, J=7.8Hz), 7.96 (1H, s), 10.1 (1H, s)
FAB-MS; m/z: 422 (MH⁺)

### Example 6: 2-(2-Oxo-2-{3-[(E)-2-(pyrido[3,2-d][1,3]thiazol-2-yl)-1-ethenyl]anilino}ethyl)benzoic acid (*)

### (A) N1-(2-Chloro-3-pyridyl)-(E)-3-(3-nitrophenyl)-2-propeneamide

(E)-3-(3-Nitrophenyl)-2-propenoic acid (6.02 g, 31.2 mmol) was suspended in toluene (250 ml), and the suspension was added dropwise with thionyl chloride (2.7 ml, 37.4 mmol), and then heated under reflux for one and half hours. After the reaction mixture was cooled, the solvent and excessive reagents were evaporated. The residue was subjected to azeotropy by using toluene to obtain (E)-3-(3-nitrophenyl)-2-propenoyl chloride as a white solid.

This solid was suspended in THF (100 ml), and the suspension was added with 3-amino-2-chloropyridine (4.01 g, 31.2 mmol) under ice cooling, and then stirred at the same temperature for 5 minutes and further at room temperature for 19 hours. The reaction mixture was added with saturated aqueous sodium hydrogencarbonate, and the insoluble solids were collected by filtration. The solids were again suspended in chloroform:methanol (10:1, v/v), washed with saturated aqueous sodium hydrogencarbonate and saturated brine, and then dried over anhydrous sodium sulfate. The solvent was evaporated to obtain the title compound as a white solid (1.00 g, 10.6%).
¹H-NMR (CDCl₃) δ : 6.97 (1H, d, J=15.6Hz), 7.36 (1H, t, J=4.9Hz), 7.64 (1H, t, J=7.8Hz), 7.83 (1H, d, J=15.6Hz), 7.92 (1H, d, J=8.3Hz), 8.16 (1H, d, J=4.9Hz), 8.26 (1H, dd, J=8.3, 1.0Hz), 8.49 (1H, m), 8.75 (1H, d, J=6.8Hz)

### (B) 2-[(E)-2-(3-Nitrophenyl)-1-ethenyl]pyrido[3,2-d][1,3]thiazole

The N¹-(2-chloro-3-pyridyl)-(E)-3-(3-nitrophenyl)-2-propeneamide obtained in (A) (0.69 g, 2.27 mmol) was suspended in pyridine (20 ml), and the suspension was added with phosphorous pentasulfide (0.51 g, 2.27 mmol), and then heated with stirring at about 100°C for 5 hours, and further heated under reflux for 30 minutes. After the reaction mixture was cooled, the mixture was added with saturated aqueous sodium hydrogencarbonate, and extracted with chloroform:methanol (10:1, v/v). The extract was washed with saturated brine, dried over anhydrous sodium sulfate, and then the solvent was evaporated. The residue was purified by silica gel column chromatography (chloroform, and hexane:ethyl acetate = 1:1 → 1:4, v/v → chloroform) to obtain the title compound as a pale orange solid (0.17 g, 26.4%).
¹H-NMR (CDCl₃) δ : 7.46 (1H, dd, J=7.8, 4.4Hz), 7.50 (1H, d, J=15.6Hz), 7.62 (1H, t, J=7.8Hz), 7.65 (1H, d, J=15.6Hz), 7.91 (1H, d, J=7.8Hz), 8.21-8.40 (1H, m), 8.26 (1H, dd, J=8.3, 1.5Hz), 8.45-8.48 (1H, m), 8.59 (1H, dd, J=4.4, 1.5Hz)

### (C) 3-[(E)-2-(Pyrido[3,2-d][1,3]thiazol-2-yl)-1-ethenyl]aniline

The 2-[(E)-2-(3-nitrophenyl)-1-ethenyl]pyrido[3,2-d][1,3]thiazole obtained in (B) (0.10 g, 0.35 mmol) was suspended in ethanol (15 ml), and the suspension was added with tin (II) chloride monohydrate (278 mg, 1.24 mmol), and heated under reflux for 3.5 hours. The reaction mixture was added with 1.5 N aqueous sodium hydroxide under ice cooling, and the mixture was extracted with chloroform. The organic layer was washed with saturated brine, and dried over anhydrous sodium sulfate. Then, the solvent was evaporated to obtain the title compound as a yellow solid (92 mg, quantitative).
¹H-NMR (CDCl₃) δ : 3.80 (2H, br), 6.72 (1H, dd, J=7.8, 2.4Hz), 6.91 (1H, m), 7.01 (1H, d, J=7.3Hz), 7.21 (1H, t, J=7.8Hz), 7.32 (1H, d, J=16.1Hz), 7.40 (1H, dd, J=8.3, 4.4Hz), 7.49 (1H, d, J=16.1Hz), 8.19 (1H, dd, J=8.3, 1.5Hz), 8.54 (1H, dd, J=4.4, 1.5Hz)

### (D) 2-(2-Oxo-2-(3-[(E)-2-(pyrido[3,2-d][1,3]thiazol-2-yl)-1-ethenyl]anilino)ethyl)benzoic acid

The 3-[(E)-2-(pyrido[3,2-d][1,3]thiazol-2-yl)-1-ethenyl]aniline obtained in (C) (90 mg, 0.36 mmol) was dissolved in toluene (2 ml), and the solution was added with homophthalic anhydride (about 75%, 86 mg, 0.53 mmol) and heated under reflux for 30 minutes. After the reaction mixture was cooled, the deposited solids were collected by filtration and washed with ether to obtain the title compound as pale orange powder (121 mg, 82.0%).
¹H-NMR (DMSO-d₆) δ : 4.12 (2H, s), 7.36-7.42 (3H, m), 7.48 (1H, d, J=16.1Hz), 7.46-7.61 (4H, m), 7.69 (1H, d, J=16.1Hz), 7.90 (1H, d, J=7.3Hz), 7.99 (1H, br), 8.35 (1H, d, J=8.3Hz), 8.60 (1H, dd, J=4.4, 1.0Hz), 10.25 (1H, br), 12.80 (1H, br) EI/MS; m/z: 415 (M⁺)
FAB/MS; m/z: 416 (MH⁺)

### Example 7: 2-(2-3-[(E)-2-(1,3-Benzoxazol-2-yl)-1-ethenyl]anilino-2-oxoethyl)benzoic acid (*)

2-[(Diethoxyphosphinyl)methyl]benzoxazole (J. Org. Chem. 1993, 58, 7009) and 3-nitrobenzaldehyde were treated in the same manner as in Example 15 (F), and then in the same manner as in Example 3 (B) successively to obtain the title compound.
¹H-NMR (DMSO-d₆) δ : 4.12 (2H, s), 7.18 (1H, d), 7.38 (7H, m), 7.51 (1H, m), 7.61 (1H, d), 7.72 (3H, m), 7.91 (2H, m)
MS (ES-); m/z: 397 (M⁺-1)

### Example 8: 2-[2-Oxo-2-(3-(E)-2-[4-(4-pyridyl)-1,3-thiazol-2-yl]-1-ethylanilino)ethyl]-benzoic acid (*)

### (A) 4-Bromoacetylpyridine

4-Acetylpyridine (1 g) was dissolved in acetic acid (5 mL), added with pyridinium hydrobromide perbromide (2.64 g), and stirred at 50°C for 3 hours. The reaction mixture was cooled with ice, and the deposited crystals were collected by filtration, washed with toluene, and dried to obtain the title compound as white powder (0.934 g).
¹H-NMR (CD₃OD) δ : 3.70 (1H, d, J=11.1Hz), 3.80 (1H, d, J=10.8Hz), 8.25 (2H, d, J=6.0Hz), 8.90 (2H, d, J=6.0Hz)

### (B) 2-[(E)-2-(3-Nitrophenyl)-1-ethenyl]-4-(4-pyridyl)-1,3-thiazole

The 4-bromoacetylpyridine obtained in (A) was treated according to the method described in Japanese Patent Unexamined Publication No.6-80654/1994 to obtain the title compound.
¹H-NMR (CD₃OD) δ : 7.68 (1H, d, J=15.9Hz), 7.70 (1H, d, J=7.5Hz), 7.81 (1H, d, J=16.2Hz), 8.09 (1H, d, J=7.8Hz), 8.23 (1H, d, J=8.4Hz), 8.53 (1H, t, J=1.8Hz), 6.66 (2H, d, J=6.9Hz), 8.76 (1H, s), 8.87 (2H, d, J=6.9Hz)

### (C) 2-[2-Oxo-2-(3-(E)-2-[4-(4-pyridyl)-1,3-thiazol-2-yl]-1-ethylanilino)ethyl]benzoic acid

The 2-[(E)-2-(3-Nitrophenyl)-1-ethenyl]-4-(4-pyridyl)-1,3-thiazole obtained in (B) was treated in the same manner as in Example 3 (B) to obtain the title compound.
¹H-NMR (DMSO-d₆) δ : 4.12 (2H, s), 7.35-7.56 (5H, m), 7.90 (1H, d, J=8.1Hz), 7.96 (2H, m), 8.44 (1H, s), 8.65 (2H, m), 10.18 (1H, s)
MS (ES-); m/z: 440 (M⁺-1)

### Example 9: 2-(2-3-[(6-Isopropyl-2-pyridyl)methoxy]anilino-2-oxoethyl)benzoic acid

### (A) 2-Isopropylpyridine-N-oxide (*)

2-Isopropylpyridine (5 g) was dissolved in acetic acid (27 mL), and the solution was added dropwise with peracetic acid (9.2 mL, 37% solution) and stirred at 70°C for 16 hours. The solvent was carefully evaporated under reduced pressure, and the residue was added with toluene and the remaining acetic acid was removed by azeotropy under reduced pressure. The residue was purified by distillation to obtain the title compound (4.9 g) from the fraction at 127°C/1 mmHg.
¹H-NMR (CDCl₃) δ : 1.40 (6H, d, J=6.9Hz), 3.91 (1H, heptuplet, J=6.9Hz), 7.28 (1H, m), 7.38-7.47 (2H, m), 8.50 (1H, d, J=6.3Hz)

### (B) 6-Isopropyl-2-cyanopyridine

The 2-isopropylpyridine-N-oxide obtained in (A) (4.9 g) was dissolved in dimethylformamide (25 mL), and the solution was added with sodium cyanide (5.26 g) and triethylamine (20 mL). The solution was added dropwise with chlorotrimethylsilane (13.6 mL) and then stirred at 120°C for 16 hours. After the reaction mixture was cooled, the solvent was evaporated under reduced pressure. The residue was distributed in ethyl acetate and 7% aqueous lithium chloride. The organic layer was washed with saturated brine, and dried over sodium sulfate. Then, the solvent was evaporated under reduced pressure, and the residue was purified by silica gel chromatography (hexane:ethyl acetate, 85:15, v/v) to obtain the title compound as oil (978 mg).
¹H-NMR (CDCl₃) δ : 1.42 (6H, d, J=7.2Hz), 3.22 (1H, heptuplet, J=7.2Hz), 7.50 (1H, dd, J=7.9, 1.2Hz), 7.62 (1H, dd, J=7.6, 1.0Hz), 7.83 (1H,t, J=8.1Hz)

### (C) 6-Isopropylpicolinic acid

The 6-isopropyl-2-cyanopyridine obtained in (B) (978 mg) was added with 5 N hydrochloric acid (13.5 mL), and the mixture was stirred at 110°C for 16 hours. The reaction mixture was cooled, and then adjusted to pH 5.5 with 1 N sodium hydroxide. The deposited solids were collected by filtration, and dried to obtain the title compound (534 mg). The mother liquid was adjusted to pH 5.5 with 1 N hydrochloric acid, and then extracted five times with methylene chloride. The organic layer was washed with saturated brine and dried over sodium sulfate, and then the solvent was evaporated under reduced pressure to further obtain 324 mg of the title compound.
¹H-NMR (CDCl₃) δ : 1.41 (6H, d, J=6.9Hz), 3.23 (1H, heptuplet, J=6.9Hz), 7.55 (1H, d, J=8.1Hz), 7.96 (1H, t, J=7.8Hz), 8.14 (1H, d, J=7.5Hz)

### (D) 6-Isopropyl-2-hydroxymethylpyridine

The 6-isopropylpicolinic acid obtained in (C) (200 mg) was dissolved in tetrahydrofuran (6 mL), and the solution was added dropwise with borane-tetrahydrofuran complex (1 M, 6 mL) at 0°C under nitrogen atmosphere. The reaction mixture was stirred at the same temperature for 3.5 hours, and added with methanol, and then the solvent was evaporated under reduced pressure. The residue was added with 1 N hydrochloric acid, and stirred for 20 minutes. The reaction mixture was adjusted to pH 11 with 5 N sodium hydroxide, and extracted four times with methylene chloride. The organic layer was dried over sodium sulfate, and the solvent was evaporated under reduced pressure. The residue was purified by silica gel chromatography (methylene chloride:methanol, 99:1, v/v) to obtain the title compound as colorless oil (145 mg).
¹H-NMR (CDCl₃) δ : 1.41 (d, J=6.9, 6H), 3.18 (heptuplet, J=6.9, 1H), 4.84 (s, 2H), 7.12 (d, J=7.8, 1H), 7.18 (d, J=7.5, 1H), 7.71 (d, J=7.8, 1H)

### (E) 3-Nitro-1-[(6-isopropyl-2-pyridyl)methoxy]benzene

The 6-isopropyl-2-hydroxymethylpyridine obtained in (D) (118 mg), triphenylphosphine (248 mg) and 3-nitrophenol (132 mg) were dissolved in anhydrous tetrahydrofuran (7 mL), and the solution was added with diethyl azodicarboxylate (150 µL) at 0°C under nitrogen atmosphere, and stirred at room temperature for 16 hours. The solvent was evaporated under reduced pressure, and the residue was distributed between ethyl acetate and 1 N sodium hydroxide. The organic layer was washed with saturated brine, and dried over sodium sulfate. The solvent was evaporated under reduced pressure, and the residue was purified by silica gel chromatography (hexane:ethyl acetate, 1:1, v/v) to obtain the title compound as yellow oil (202 mg).
¹H-NMR (CDCl₃) δ : 1.43 (6H, d, J=6.9Hz), 3.19 (1H, heptuplet, J=6.9Hz), 5.37 (1H, s), 7.24 (1H, d, J=7.5Hz), 7.42 (1H, d, J=7.8Hz), 7.44 (1H, ddd, J=8.4, 2.7, 1.2Hz), 7.54 (1H, t, J=8.4), 7.76 (1H, t, J=7.5Hz), 7.94 (1H, ddd, J=8.1, 2.4, 1.2Hz), 8.00 (1H, t, J=2.4Hz)

### (F) 2-[2-Oxo-2-(3-(E)-2-[4-(4-pyridyl)-1,3-thiazol-2-yl]-1-ethylanilino)ethyl]benzoic acid

The 3-nitro-1-[(6-isopropyl-2-pyridyl)methoxy]benzene obtained in (E) was treated in the same manner as in Example 3 (B) to obtain the title compound.
¹H-NMR (CDCl₃) δ : 1.43 (6H, d, J=6.9Hz), 3.32 (1H, heptuplet, J=6.9Hz), 4.08 (1H, s), 5.32 (1H, s), 6.34 (1H, broad s), 7.15 (2H, m), 7.29 (2H, m), 7.40-7.63 (3H, m), 7.57 (2H, m), 7.82 (1H, td, J=7.5, 2.4Hz), 8.11 (1H, d, J=8.1Hz)
MS (ES-); m/z: 403 (M⁺-1)

### Example 10: 2-(2-Oxo-2-{3-[2-(4-phenyl-1,3-thiazol-2-yl)ethyl]anilino}ethyl)benzoic acid (*)

### (A) Methyl (E)-3-(3-nitrophenyl)-2-propenate

3-Nitrocinnamic acid (10.00 g, 51.8 mmol) was suspended in methanol (300 ml), and the suspension was added dropwise with concentrated sulfuric acid (10 ml), and heated under reflux for 6 hours while dehydration was carried out by means of a dropping funnel filled with molecular sieves. The solvent was evaporated, and the residue was added with water, and then extracted with ethyl acetate. The organic layer was washed with saturated aqueous sodium hydrogencarbonate and saturated brine, and dried over anhydrous sodium sulfate. The solvent was evaporated to obtain the title compound as a white solid (10.72 g, quantitative).
¹H-NMR (CDCl₃) δ : 3.84 (3H, s), 6.59 (1H, d, J=16.1Hz), 7.59 (1H, t, J=7.8Hz), 7.73 (1H, d, J=16.1Hz), 7.83 (1H, d, J=7.8Hz), 8.24 (1H, td, J=7.3, 1.5 Hz), 8.38 (1H, t, J=1.5Hz)

### (B) Methyl 3-(3-aminophenyl)propanate

The methyl (E)-3-(3-nitrophenyl)-2-propenate obtained in (A) (2.01 g, 9.70 mmol) was dissolved in methanol:ethyl acetate (1:1, v/v, 500 ml), and the solution was added with 10% palladium/carbon (0.2 g) and stirred at room temperature for 24 hours under hydrogen flow (1 atm.). The catalyst was removed by filtration, and the solvent was evaporated to obtain the title compound as pale orange oil (1.73 g, quantitative yield).
¹H-NMR (CDCl₃) δ : 2.59 (2H, t, J=7.8Hz), 2.84 (2H, t, J=7.8Hz), 3.66 (3H, s), 3.66 (2H, br), 6.47-6.53 (2H, m), 6.58 (1H, d, J=7.3Hz), 7.06 (1H, td, J=7.8, 2.9Hz)

### (C) Methyl 3-{3-[(N-tert-butoxycarbonyl)amino]phenyl}propanate

The methyl 3-(3-aminophenyl)propanate (1.73 g, 9.65 mmol) obtained in (B) was dissolved in chloroform (80 ml). The solution was added with di-tert-butyl dicarbonate (3.16 g, 14.5 mmol), added dropwise with triethylamine (336 µl, 2.41 mmol), and then stirred at room temperature for 21 hours. Since remaining starting material was observed by TLC, the reaction mixture was further added with di-tert-butyl dicarbonate (3.16 g, 14.5 mmol) and triethylamine (336 µl, 2.41 mmol), and stirred at room temperature for 5 hours. Still remaining starting material was observed by TLC, the reaction mixture was further added with di-tert-butyl dicarbonate (3.16 g, 14.5 mmol), and stirred at room temperature for 17 hours. After the solvent was evaporated, the residue was purified by silica gel column chromatography (hexane:ethyl acetate = 2:1, v/v → chloroform) to obtain the title compound as colorless oil (2.34 g, 86.8%).
¹H-NMR (CDCl₃) δ : 1.52 (9H, s), 2.62 (2H, t, J=7.8Hz), 2.92 (2H, t, J=7.8Hz), 3.67 (3H, s), 6.46 (1H, br), 6.87 (1H, d, J=7.3Hz), 7.13-7.25 (2H, m), 7.26 (1H, br)

### (D) tert-Butyl N-[3-(3-amino-3-oxopropyl)phenyl]carbamate

The methyl 3-{3-[(N-tert-butoxycarbonyl)amino]phenyl}propanate obtained in (C) (1.84 g, 6.59 mmol) was dissolved in methanol (10 ml), and the solution was added dropwise with an ammonia solution in methanol (30 ml), and stirred at room temperature for 18 hours. Since a large amount of remaining starting material was observed by TLC, the reaction mixture was further added with an ammonia solution in methanol (100 ml), and stirred at room temperature for 6 hours. Since still remaining starting material was observed by TLC, the reaction mixture was further added with concentrated aqueous ammonia (28%, 50 ml), and further stirred at room temperature for 17 hours. After the methanol was evaporated, the residue was extracted with ethyl acetate, washed with saturated brine, and then dried over anhydrous sodium sulfate. The solvent was evaporated to obtain the title compound as colorless oil (1.60 g, 91.9%).
¹H-NMR (CDCl₃) δ : 1.51 (9H, s), 2.52 (2H, t, J=7.8Hz), 2.94 (2H, t, J=7.8Hz), 5.44 (2H, br), 6.54 (1H, br), 6.89 (1H, d, J=7.3Hz), 7.10-7.22 (2H, m), 7.31 (1H, br)

### (E) tert-Butyl N-[3-(3-amino-3-thioxopropyl)phenyl]carbamate

The tert-butyl N-[3-(3-amino-3-oxopropyl)phenyl]carbamate obtained in (D) (1.58 g, 5.98 mmol) was dissolved in THF (30 ml), and inside of the reaction system was replaced with nitrogen. The reaction mixture was added with the Lawesson's reagent (1.21 g, 2.99 mmol), stirred at room temperature for 10 minutes, and then heated at about 60°C for 2.5 hours. After the solvent was evaporated, the residue was purified by silica gel column chromatography (chloroform → chloroform:methanol = 30:1, v/v) to obtain the title compound as light yellow oil (1.08 g, 64.7%).
¹H-NMR (CDCl₃) δ : 1.52 (9H, s), 2.94 (2H, t, J=7.3Hz), 3.08 (2H, t, J=7.8Hz), 6.51 (1H, br), 6.81 (1H, br), 6.92 (1H, d, J=7.8Hz), 7.11 (1H, d, J=8.3Hz), 7.21 (1H, t, J=7.8Hz), 7.35 (2H, br)

### (F) 3-[2-(4-Phenyl-1,3-thiazol-2-yl)ethyl]aniline

The tert-Butyl N-[3-(3-amino-3-thioxopropyl)phenyl]carbamate obtained in (E) (200 mg, 0.72 mmol) was dissolved in ethanol (5 ml), and the solution was added with phenacyl bromide (142 mg, 0.72 mmol) and heated under reflux for one hour and 20 minutes. After the solvent was evaporated, the residue was added with saturated aqueous sodium hydrogencarbonate, and the mixture was extracted with chloroform and washed with saturated brine. The organic layer was dried over anhydrous sodium sulfate, and the solvent was evaporated. The residue was dissolved in methylene chloride (5 ml), and the solution was added dropwise with trifluoroacetic acid (5 ml) under ice cooling, and stirred for 30 minutes at the same temperature. After the solvent was evaporated, the residue was added with saturated aqueous sodium hydrogencarbonate, and then extracted with chloroform. The organic layer was washed with saturated brine, and dried over anhydrous sodium sulfate, and then the solvent was evaporated. The residue was purified by silica gel column chromatography (chloroform) to obtain the title compound as yellow oil (194 mg, 96.9%).
¹H-NMR (CDCl₃) δ : 3.06 (2H, t, J=7.8Hz), 3.33 (2H, t, J=7.8Hz), 3.61 (2H, br), 6.52-6.58 (2H, m), 6.65 (1H, d, J=7.3Hz), 7.08 (1H, t, J=7.3Hz), 7.28-7.35 (2H, m), 7.41 (2H, t, J=7.8Hz), 7.89 (2H, d, J=7.8Hz)

### (G) 2-(2-Oxo-2-{3-[2-(4-phenyl-1,3-thiazol-2-yl)ethyl]anilino}ethyl)benzoic acid

The 3-[2-(4-phenyl-1,3-thiazol-2-yl)ethyl]aniline obtained in (F) (194 mg, 0.69 mmol) was dissolved in toluene (5 ml), and the solution was added with homophthalic anhydride (about 75%, 168 mg, 1.04 mmol) and heated under reflux for 30 minutes. After the reaction mixture was cooled, the insoluble solids were collected by filtration, and washed with ether to obtain the title compound as white powder (187 mg, 61.0%).
¹H-NMR (DMSO-d₆) δ : 3.05 (2H, t, J=7.8Hz), 3.33 (2H, t, J=7.8Hz), 4.08 (2H, s), 6.95 (1H, d, J=7.8Hz), 7.20 (1H, t, J=7.8Hz), 7.30-7.45 (7H, m), 7.51 (1H, td, J=7.3, 1.5Hz), 7.55 (1H, br), 7.88 (1H, dd, J=7.8, 1.5Hz), 7.92-7.96 (2H, m), 10.04 (1H, br), 12.82 (1H, br)
FAB/MS; m/z: 443 (MH⁺)

### Example 11: 2-(2-Oxo-2-{3-[2-(4-phenyl-1,3-thiazol-2-yl)cyclopropyl]anilino}ethyl)-benzoic acid (*)

### (A) Methyl 2-(3-nitrophenyl)-1-cyclopropanecarboxylate

Ether (30 ml) and 40% aqueous potassium hydroxide (10 ml) were vigorously stirred under ice cooling, and the mixture was added portionwise with N-methyl-N'-nitro-N-nitrosoguanidine (3.55 g, 24.1 mmol), and stirred for 30 minutes at the same temperature. The reaction mixture was allowed to stand to prepare a diazomethane/ether solution as an upper layer.

Separately, the methyl (E)-3-(3-nitrophenyl)-2-propenate (500 mg, 2.41 mmol) synthesized in Example 10 (A) was dissolved in ether (300 ml), and the solution was added with palladium acetate (5 mg, 0.024 mmol) and atmosphere in the reaction system was replaced with nitrogen gas. The reaction mixture was slowly added with the diazomethane/ether solution prepared above under ice cooling, and the mixture was stirred at the same temperature for one hour and further at room temperature for one hour. The reaction mixture was added with acetic acid, and the solvent was evaporated. The residue was subjected to azeotropy by using toluene, and the residue was purified by silica gel column chromatography (hexane:ethyl acetate =4:1, v/v) to obtain the title compound as light yellow oil (504 mg, 94.4%).
¹H-NMR (CDCl₃) δ : 1.34-1.42 (1H, m), 1.64-1.73 (1H, m), 1.93-2.01 (1H, m), 1.58-1.66 (1H, m), 3.74 (3H, s), 7.46 (2H, d, J=5.4Hz), 7.94 (1H, s), 8.03-8.10 (1H, m)

### (B) 2-(3-Nitrophenyl)-1-cyclopropanecarboxylic acid

The methyl 2-(3-nitrophenyl)-1-cyclopropanecarboxylate obtained in (A) (497 mg, 2.25 mmol) was dissolved in methanol (5 ml), and the solution was added dropwise with 1 N aqueous sodium hydroxide (5 ml, 5.0 mmol) and stirred at room temperature for one hour. The solvent was evaporated, and the residue was adjusted to approximately pH 1 by addition of 1 N aqueous hydrochloric acid, and then the mixture was extracted with ethyl acetate. The organic layer was washed with saturated brine, and dried over anhydrous sodium sulfate. The solvent was evaporated to obtain the title compound as white solid (471 mg, quantitative).
¹H-NMR (CD₃OD) δ : 1.30-1.39 (0.5H, m), 1.40-1.49 (0.5H, m), 1.54-1.64 (1H, m), 1.92-2.05 (1H, m), 2.50-2.56 (0.5H, m), 2.58-2.64 (0.5H, m), 7.48-7.58 (2H, m), 8.00 (1H, d, J=8.8 Hz), 8.02-8.10 (1H, m)

### (C) 2-(3-Nitrophenyl)-1-cyclopropanecarboxamide

The 2-(3-nitrophenyl)-1-cyclopropanecarboxylic acid obtained in (B) (460 mg, 2.22 mmol) was added with thionyl chloride (10 ml), and the mixture was heated under reflux for 30 minutes. After the reaction mixture was cooled, excessive reagents were evaporated and the residue was subjected to azeotropy by using toluene. The residue was added with concentrated aqueous ammonia (28%, 20 ml) under ice cooling, and stirred at the same temperature for 2 hours and further at room temperature for 14 hours. The deposited solids were collected by filtration, washed with water, and dried to obtain the title compound as light yellow powder (398 mg, 87.1%).
¹H-NMR (CD₃OD) δ : 1.32-1.39 (0.5H, m), 1.52-1.62 (1H, m), 1.66-1.73 (0.5H, m), 1.95-2.01 (1H, m), 1.48-1.55 (0.5H, m), 2.78-2.87 (0.5H, m), 2.49-2.64 (2H, m), 7.97-8.14 (2H, m)

### (D) 2-(3-Nitrophenyl)-1-cyclopropanecarbothioamide

The 2-(3-nitrophenyl)-1-cyclopropanecarboxamide obtained in (C) (183 mg, 0.89 mmol) was suspended in THF (5 ml), and atmosphere in the reaction system was replaced with nitrogen gas. The reaction mixture was added with the Lawesson's reagent (179 mg, 0.44 mmol), and the mixture was stirred at about 70°C for 1.5 hours. After the reaction mixture was cooled, the solvent was evaporated, and the residue was purified by silica gel column chromatography (chloroform → chloroform:methanol = 30:1, v/v) to obtain the title compound as light yellow oil (122 mg, 62.0%).
¹H-NMR (CDCl₃) δ : 1.50-1.62 (1H, m), 1.97-2.08 (1H, m), 2.15-2.24 (1H, m), 2.81-2.91 (1H, m), 7.21 (1H, br), 7.46 (1H, t, J=7.8Hz), 7.53 (1H, d, J=7.8Hz), 7.60 (1H, br), 7.92 (1H, s), 8.05 (1H, d, J=7.8Hz)

### (E) 2-[2-(3-Nitrophenyl)cyclopropyl]-4-phenyl-1,3-thiazole

The 2-(3-nitrophenyl)-1-cyclopropanecarbothioamide obtained in (D) (122 mg, 0.55 mmol) was dissolved in ethanol (5 ml), and the solution was added with 2-bromoacetophenone (109 mg, 0.55 mmol) and heated under reflux for one hour. After the reaction mixture was cooled, the solvent was evaporated. The residue was triturated in ethanol and ether as powder and then collected by filtration, and the powders were washed with ether to obtain the title compound as pale orange powder (158 mg, 89.0%).
¹H-NMR (CDCl₃) δ : 1.70-1.80 (1H, m), 1.91-2.00 (1H, m), 2.09-2.19 (1H, m), 2.85-2.96 (1H, m), 7.47 (1H, s), 7.48-7.60 (4H, m), 7.73 (1H, d, J=7.3Hz), 8.05-8.20 (4H, m)

### (F) 3-[2-(4-Phenyl-1,3-thiazol-2-yl)cyclopropyl]aniline

The 2-[2-(3-nitrophenyl)cyclopropyl]-4-phenyl-1,3-thiazole obtained in (E) (151 mg, 0.47 mmol) was dissolved in ethanol (10 ml), and the solution was added with tin (II) chloride monohydrate (372 mg, 1.65 mmol) and heated under reflux for 5 hours. The reaction mixture was added with 1.5 N aqueous sodium hydroxide under ice cooling, and then extracted with chloroform. The organic layer was washed with saturated brine, and dried over anhydrous sodium sulfate. The solvent was evaporated to obtain the title compound as pale orange oil (114 mg, 83.1%).

### (G) 2-(2-Oxo-2-{3-[2-(4-phenyl-1,3-thiazol-2-yl)cyclopropyl]anilino}ethyl)benzoic acid

The 3-[2-(4-phenyl-1,3-thiazol-2-yl)cyclopropyl]aniline obtained in (F) (114 mg, 0.39 mmol) was dissolved in toluene (3 ml), and the solution was added with homophthalic anhydride (about 75%, 95 mg, 0.59 mmol) and heated under reflux for 30 minutes. After the reaction mixture was cooled, the insoluble solids were collected by filtration, and washed with ether to obtain the title compound as white powder (139 mg, 78.4%).
¹H-NMR (DMSO-d₆) δ : 1.53-1.63 (1H, m), 1.70-1.80 (1H, m), 2.47-2.57 (1H, m), 2.61-2.71 (1H, m), 4.07 (2H, s), 6.90 (1H, d, J=7.8Hz), 7.21 (1H, t, J=7.8Hz), 7.29-7.52 (8H, m), 7.84-7.89 (2H, m), 7.89-8.50 (2H, m), 10.05 (1H, s), 12.75 (1H, br) EI/MS; m/z: 454 (M⁺)
FAB/MS; m/z: 455 (MH⁺)

### Example 12: 2-(2-Oxo-2-(3-((4-phenyl-1,3-thiazol-2-yl)amino)carbonylanilino)ethyl)-benzoic acid (*)

### (A) 4-Phenyl-1,3-thiazole-2-amine

2-Bromoacetophenone (13.1 g, 65.7 mmol) and thiourea (5.00 g, 65.7 mmol) were dissolved in ethanol (100 mL), and the solution was heated under reflux for 2 hours. The reaction mixture was concentrated under reduced pressure, and the residue was recrystallized from 2-propanol to obtain the title compound as a white solid (16.5 g, 91%).
¹H-NMR (CDCl₃) δ : 7.10 (1H, s), 7.48-7.54 (3H, m), 7.67 (2H, dd, J=1.5, 7.8Hz)

### (B) N1-(4-Phenyl-1,3-thiazol-2-yl)-3-nitrobenzamide

The 4-phenyl-1,3-thiazole-2-amine (250 mg, 0.972 mmol) obtained in (A) and 3-nitrobenzoic acid (163 mg, 0.972 mmol) were dissolved in methylene chloride (10 mL), and the solution was added with diisopropylamine (0.508 mL, 2.92 mmol) and N,N'-bis(2-oxo-3-oxazolidinyl)phosphorodiamizic chloride (297 mg, 1.17 mmol) at 0°C, and further added with DMF (5 mL). The reaction mixture was stirred at room temperature for 15 hours, and concentrated under reduced pressure. The residue was distributed in ethyl acetate and 1 N aqueous hydrochloric acid. The organic layer was washed with saturated aqueous sodium hydrogencarbonate and the with saturated brine, and dried over magnesium sulfate. The solvent was evaporated under reduced pressure. The residue was purified by silica gel chromatography (hexane:ethyl acetate, 5:1, v/v) to obtain the title compound as a white solid (59.9 mg, 19%). The fraction of the mixture was recrystallized from hexane to obtain the title compound as a white solid (44.7 mg, 14%).
¹H-NMR (CDCl₃) δ : 7.14-7.23 (4H, m), 7.45-7.49 (1H, m), 7.46 (1H, t, J=8.3Hz), 7.48 (1H, t, J=7.8Hz), 8.10 (1H, dd, J=1.0, 7.8Hz), 8.22 (1H, dt, J=8.3, 1.0Hz), 8.51 (1H, t, J=2.0Hz), 12.09 (1H, br)

### (C) N1-(4-Phenyl-1,3-thiazol-2-yl)-3-aminobenzamide

The N1-(4-phenyl-1,3-thiazol-2-yl)-3-nitrobenzamide obtained in (B) (105 mg, 0.322 mmol) was dissolved in ethanol (5 mL), and the solution was added with tin (II) chloride dihydrate (363 mg, 1.61 mmol) and heated under reflux for 2 hours. The reaction mixture was added with 1 N aqueous sodium hydroxide, and extracted with ethyl acetate. The organic layer was washed with saturated brine, dried over sodium sulfate, and then added with hexane. The deposited solids were collected by filtration to obtain the title compound as a white solid (75.8 mg, 80%).
¹H-NMR (CD₃OD) δ : 7.04-7.07 (1H, m), 7.28-7.44 (7H, m), 7.93-7.95 (2H, m)

### (D) 2-(2-Oxo-2-(3-((4-phenyl-1,3-thiazol-2-yl)amino)carbonylanilino)ethyl)benzoic acid

The N1-(4-phenyl-1,3-thiazol-2-yl)-3-aminobenzamide obtained in (C) (75.8 mmol, 0.257 mmol) was dissolved in toluene (5 mL), and the solution was added with homophthalic anhydride (62.4 mg, 0.385 mmol) and heated under reflux for 2 hours. The reaction mixture was concentrated under reduced pressure, and then added with toluene. Then, the deposited solids were collected by filtration to obtain the title compound as white amorphous (105 mg, 89%).
¹H-NMR (DMSO-d₆) δ : 4.14 (2H, s), 7.32-7.48 (7H, m), 7.52-7.56 (1H, m), 7.69 (1H, s), 7.82 (2H, d, J=7.8Hz), 7.90 (1H, dd, J=1.5, 6.8Hz), 7.85 (2H, d, J=7.3Hz), 8.28 (1H, br), 10.31 (1H, br), 12.75 (1H, br)
FAB-MS; m/z: 458 (MH⁺)

### Example 13: 2-(2-2,4-Difluoro-5-((3-isopropyl-1,2,4-thiadiazol-5-yl)methoxy)anilino-2-oxoethyl)benzoic acid (*)

### (A) Ethyl 3-isopropyl-1,2,4-thiadiazole-5-carboxylate

Isobutyric acid amide (9.1 g, 0.104 mol) was suspended in toluene (300 mL), and the suspension was added with chlorocarbonylsulphenyl chloride (15 g, 0.115 mol), and heated under reflux for 2 hours. The solvent was evaporated under reduced pressure, and the residue was added with ethyl cyanoformate (50 mL) and xylene (40 mL), and then heated under reflux for 3 hours. The solvent was evaporated under reduced pressure, and the residue was purified by silica gel chromatography (hexane:ethyl acetate, 4:1, v/v) to obtain the title compound as light yellow oil (14 g, 67%).
¹H-NMR (CDCl₃) δ : 1.52 (6H, d, J=7.2Hz), 1.54 (3H, t, J=7.2Hz), 3.56 (1H, m), 4.60 (2H, q, J=7.2Hz)

### (B) (3-Isopropyl-1,2,4-thiadiazol-5-yl)methanol

The ethyl 3-isopropyl-1,2,4-thiadiazole-5-carboxylate obtained in (A) (13.8 g, 68.9 mmol) was dissolved in methanol (500 mL), and the solution was added with sodium borohydride (4.4 g) under ice cooling and stirred for 30 minutes. The solvent was evaporated under reduced pressure, and the residue was distributed in ethyl acetate and water. After the organic layer was separated, the aqueous layer was made weakly acidic, and then extracted with ethyl acetate again. The organic layers were combined and dried over magnesium sulfate. The solvent was evaporated under reduced pressure to obtain the title compound as white powder (10.7 g, 98%).
¹H-NMR (CDCl₃) δ : 1.52 (6H, d, J=7.2Hz), 3.41 (1H, m), 5.09 (2H, s)

### (C) 2,4-Difluoro-5-nitrophenyl [(3-isopropyl-1,2,4-thiadiazol-5-yl)methyl] ether

The (3-isopropyl-1,2,4-thiadiazol-5-yl)methanol) obtained in (B) (4.5 g, 28.4 mmol), 2,4-difluoro-5-nitrophenol (5.0 g, 28.6 mmol) and triphenylphosphine (10 g) were dissolved in THF (200 ml), and the solution was added dropwise with diethyl azodicarboxylate (6 ml) under ice cooling. After the addition was completed, the reaction mixture was stirred at room temperature for 19 hours, and the solvent was evaporated under reduced pressure. The residue was purified by silica gel chromatography (hexane:methylene chloride, 1:2, v/v) to obtain the title compound as light yellow oil(6.0 g, 67%).
¹H-NMR (CDCl₃) δ : 1.41 (6H, d, J=7.2Hz), 3.35 (1H, m), 5.57 (2H, s), 7.15 (1H, t, J=10.0Hz), 7.89 (1H, t, J=6.8Hz)

### (D) 2-(2-2,4-Difluoro-5-[(3-isopropyl-1,2,4-thiadiazol-5-yl)methoxy]anilino-2-oxoethyl)-benzoic acid

The 2,4-Difluoro-5-nitrophenyl [(3-isopropyl-1,2,4-thiadiazol-5-yl)methyl] ether obtained in (C) (2.1 g, 6.66 mmol) was dissolved in ethyl acetate (50 ml), and the solution was added with 10% palladium/carbon (water content 50%, 200 mg), and then the mixture was subjected to catalytic reduction at 50°C for 4 hours. After the catalyst was removed by filtration, the solvent was evaporated under reduced pressure, and the residue was purified by silica gel chromatography (hexane:ethyl acetate, 1:2, v/v) to obtain light brown oil (510 mg, 27%). A 100 mg portion of the oil was dissolved in toluene (3 ml), and the solution was added with homophthalic anhydride (60 mg) and stirred at 80°C for 1 hour. The solvent was evaporated under reduced pressure, and the residue was purified by silica gel chromatography (chloroform:methanol, 20:1, v/v) to obtain the title compound as white powder (136 mg, 86%).
¹H-NMR (CD₃OD) δ : 1.36 (6H, d, J=6.8Hz), 3.28 (1H, m), 4.03 (2H, s), 5.52 (2H, s), 7.08 (1H, m), 7.30-7.45 (3H, m), 7.88 (1H, d, J=7.6Hz), 7.96 (1H, t, J=8.8Hz)
FAB-MS; m/z: 448 (MH⁺)

### Example 14: 2-(3-Amino-5-((4-isopropyl-1,3-thiazol-2-yl)methoxy)anilino-2-oxoethyl)-benzoic acid (*)

### (A) 2-(Chloromethyl)-4-isopropyl-1,3-thiazole

(4-Isopropyl-1,3-thiazol-2-yl)methanol (1.04 g, 6.59 mmol) was dissolved in methylene chloride (10 mL), and the solution was added with thionyl chloride (721 µL, 9.88 mmol) at 0°C and stirred at room temperature for 2 hours. The solvent was concentrated under reduced pressure, and the residue was subjected to azeotropy by using toluene. The residue was diluted with diethyl ether, and washed with saturated aqueous sodium hydrogencarbonate and saturated brine. The organic layer was dried over magnesium sulfate, and concentrated under reduced pressure to obtain the title compound as brown oil (982 mg, 85%).
¹H-NMR (CDCl₃) δ : 1.31 (6H, d, J=6.8Hz), 3.09 (1H, septet, J=6.8Hz), 4.83 (2H, s), 6.91 (1H, d, J=1.0Hz)

### (B) 2-((3,5-Dinitrophenoxy)methyl)-4-isopropyl-1,3-thiazole

A solution of 3,5-dinitrophenol (686 mg, 3.37 mmol) in DMF (5 mL) was added with sodium hydride (60% in oil, 224 mg, 5.59 mmol) at 0°C, and the mixture was stirred at 0°C for 10 minutes. The reaction mixture was added dropwise with a solution of the 2-(chloromethyl)-4-isopropyl-1,3-thiazole obtained in (A) (982 mg, 5.59 mmol) in DMF (5 mL) at 0°C, and then stirred at room temperature for 16 hours. The reaction mixture was diluted with 1 N aqueous hydrochloric acid at 0°C, and then extracted with ethyl acetate. The organic layers were combined, and washed with water and saturated brine. The organic layer was dried over magnesium sulfate, and concentrated under reduced pressure. The residue was purified by silica gel column chromatography (hexane:ethyl acetate, 10:1, v/v) to obtain the title compound as brown crystals (435 mg, 36%).
¹H-NMR (CDCl₃) δ : 1.33 (6H, d, J=6.8Hz), 3.12 (1H, septet, J=6.8Hz), 5.52 (2H, s), 6.98 (1H, s), 8.26 (2H, d, J=2.0Hz), 8.68 (1H, t, J=2.0Hz)

### (C) 5-((4-Isopropyl-1,3-thiazol-2-yl)methoxy)-1,3-benzenediamine

A solution of 2-((3,5-dinitrophenoxy)methyl)-4-isopropyl-1,3-thiazole obtained in (B) (435 mg, 1.34 mmol) in ethanol (10 mL) was added with tin (II) chloride dihydrate (3.04 g, 13.5 mmol), and the mixture was heated under reflux for 2 hours. The reaction mixture was added with 4 N aqueous sodium hydroxide at 0°C, and then extracted with methylene chloride. The organic layer was dried over magnesium sulfate, and concentrated under reduced pressure to obtain the title compound as brown oil (351 mg, 71%).
¹H-NMR (CDCl₃) δ : 1.31 (6H, d, J=7.3H), 1.61 (2H, br), 3.10 (1H, septet, J=7.3Hz), 3.59 (2H, br), 5.25 (2H, s), 5.70 (1H, t, J=2.0Hz), 5.80 (2H, d, J=2.0Hz), 6.87 (1H, s)

### (D) tert-Butyl N-3-amino-5-((4-isopropyl-1,3-thiazol-2-yl)methoxy)phenylcarbamate

A solution of the 5-((4-isopropyl-1,3-thiazol-2-yl)methoxy)-1,3-benzenediamine obtained in (C) (351 mg, 1.33 mmol) in THF (10 mL) was added with di-tert-butyl dicarbonate (291 mg, 1.33 mmol) at 0°C, and the mixture was stirred for 15 hours. The reaction mixture was concentrated under reduced pressure, and the residue was purified by silica gel column chromatography (chloroform → chloroform:methanol, 98:2, v/v) to obtain the title compound as brown oil (238 mg, 49%).
¹H-NMR (CDCl₃) δ : 1.32 (6H, d, J=6.8Hz), 1.50 (9H, s), 1.58 (2H, br), 3.10 (1H, septet, J=6.8Hz), 5.26 (2H, s), 6.03 (1H, t, J=2.0Hz), 6.36 (1H, t, J=2.0Hz), 6.55 (1H, s), 6.88 (1H, s)

### (E) 2-(3-(tert-Butoxycarbonyl)amino)-5-((4-isopropyl-1,3-thiazol-2-yl)methoxy)anilino-2-oxoethyl)benzoic acid

The tert-butyl N-3-amino-5-((4-isopropyl-1,3-thiazol-2-yl)methoxy)phenylcarbamate obtained in (D) was dissolved in toluene (5 mL), and the solution was added with homophthalic anhydride (159 mg, 0.983 mmol) and heated under reflux for 1.5 hours. The reaction mixture was concentrated under reduced pressure, and the residue was purified by silica gel column chromatography (chloroform → chloroform:methanol, 98:2 → 96:4, v/v) to obtain the title compound as white crystals (292 mg, 85%).
¹H-NMR (CDCl₃) δ : 1.30 (6H, d, J=6.8Hz), 1.47 (9H, s), 3.12 (1H, septet, J=6.8Hz), 3.99 (2H, s), 5.26 (2H, s), 6.79 (1H, s), 6.87 (1H, s), 6.93 (1H, s), 7.12-7.58 (4H, m), 8.05 (1H, d, J=7.8Hz), 8.95 (1H, s)

### (F) 2-(3-Amino-5-((4-isopropyl-1,3-thiazol-2-yl)methoxy)anilino-2-oxoethyl)benzoic acid

The 2-(3-(tert-butoxycarbonyl)amino)-5-((4-isopropyl-1,3-thiazol-2-yl) methoxy)anilino-2-oxoethyl)benzoic acid obtained in (E) (100 mg, 0.190 mmol) was added dropwise with 4 N hydrochloric acid in dioxane (2 mL) at 0°C. The reaction mixture was stirred at a room temperature for 3 hours, and then concentrated under reduced pressure by azeotropy using toluene and ether. The residue was added with ether and collected by filtration to obtain dihydrochloride of the title compound as white crystals (78.8 mg, 83%).
¹H-NMR (DMSO-d₆-CDCl₃) δ : 1.35 (6H, d, J=6.8Hz), 3.18 (1H, septet, J=6.8Hz), 3.92 (2H, br), 4.06 (2H, s), 5.41 (2H, s), 6.86 (1H, s), 7.11 (1H, s), 7.30-7.52 (6H, m), 7.99 (1H, d, J=7.8Hz), 10.18 (1H, s)
FAB-MS; m/z: 426 (MH⁺)

### Example 15: (4S,5S)-5-[(5-[(E)-2-(4-Isopropyl-1,3-thiazol-2-yl)-1-ethenyl]-3-pyridylamino)carbonyl]-2,2-dimethyl-1,3-dioxolane-4-carboxylic acid

### (A) 5-[(tert-Butoxycarbonyl)amino]nicotinic acid

5-Pyridinedicarboxylic acid (5.00 g, 29.9 mmol) was suspended in t-butanol (35 ml), and the solution was added dropwise with triethylamine (8.34 ml, 59.8 mmol) and diphenylphosphoric acid azide (7.10 ml, 32.9 mmol), and heated under reflux for 3 hours. The reaction mixture was adjusted to a pH lower than 10 by addition of 1 N aqueous sodium hydroxide under ice cooling, and then washed with ether. The reaction mixture was again cooled with ice, and adjusted to about pH 4 with 1 N aqueous sodium hydroxide. The deposited solids were collected by filtration, and washed with water and ether to obtain the title compound as white powder (3.27 g, 45.9%).
¹H-NMR (DMSO-d₆) δ : 1.49 (9H, s), 8.45 (1H, s), 8.66 (1H, s), 8.75 (1H, s), 9.83 (1H, br)

### (B) tert-Butyl N-(5-[methoxy(methyl)amino]carbonyl-3-pyridyl)carbamate

The 5-[(tert-butoxycarbonyl)amino]nicotinic acid obtained in (A) (3.25 g, 13.6 mmol) was suspended in methylene chloride (100 ml), and the suspension was added with N,O-dimethylhydroxylamine hydrochloride (2.00 g, 20.5 mmol), and then added with N,N-bis-(2-oxo-3-oxazolidinyl)phosphinate chloride (6.95 g, 27.3 mmol) under ice cooling. The mixture was further added dropwise with diisopropylethylamine (9.51 ml, 54.6 mmol). The reaction mixture was stirred at the same temperature for 10 minutes and further at room temperature for 24 hours, and then the solvent was evaporated. The residue was added with water, and extracted with chloroform. The organic layer was washed successively with 5% aqueous citric acid, water, saturated aqueous sodium hydrogencarbonate and saturated brine, and dried over anhydrous sodium sulfate. The solvent was evaporated to obtain the title compound as a mixture of light yellow foam and oil (1.80 g, 46.9%).
¹H-NMR (CDCl₃) δ : 1.53 (9H, s), 3.38 (3H, s), 3.59 (3H, s), 7.27 (1H, br), 8.24 (1H, br), 8.59 (1H, d, J=1.9Hz), 8.64 (1H, d, J=1.9Hz)

### (C) tert-Butyl N-(5-formyl-3-pyridyl)carbamate

The tert-butyl N-(5-[methoxy(methyl)amino]carbonyl-3-pyridyl)carbamate obtained in (B) (1.80 g, 6.40 mmol) was dissolved in THF (20 ml), and atmosphere in the reaction system was replaced with nitrogen gas. The reaction mixture was cooled to -78°C, and then added portionwise with a suspension of aluminum lithium hydride (291 mg, 7.68 mmol) in THF (10 ml) over 30 minutes. The mixture was stirred at the same temperature for 2.5 hours and under ice cooling for 20 minutes. The reaction mixture was again cooled to -78°C, and added with 5% aqueous potassium hydrogensulfate (20 ml), and then the temperature was raised to room temperature. After the solvent was evaporated, the residue was extracted with ethyl acetate, washed with 1 N aqueous sodium hydroxide and saturated brine, and dried over anhydrous sodium sulfate. The solvent was evaporated to obtain the title compound as a mixture of yellow foam and oil (1.42 g, quantitative).
¹H-NMR (CDCl₃) δ : 1.55 (9H, s), 6.78 (1H, br), 8.44 (1H, br), 8.69 (1H, d, J=1.9Hz), 8.75 (1H, d, J=1.9Hz), 10.10 (1H, s)

### (D) 2-(Bromomethyl)-4-isopropyl-1,3-thiazole

(4-Isopropyl-1,3-thiazol-2-yl)methanol (1.55 g, 9.86 mmol) were dissolved in methylene chloride (20 ml). The solution was added with triphenylphosphine (3.10 g, 11.8 mmol), added further dropwise with a solution of carbon tetrabromide (3.92 g, 11.8 mmol) in methylene chloride (10 ml), and then stirred at room temperature for 30 minutes. After the solvent was evaporated, the residue was purified by silica gel column chromatography (hexane → hexane:ethyl acetate = 10:1, v/v) to obtain the title compound as yellow oil (1.39 g, 64.1%).
¹H-NMR (CDCl₃) δ : 1.30 (6H, d, J=7.3Hz), 3.01-3.15 (1H, m), 4.72 (2H, s), 6.92 (1H, s)

### (E) Diethyl [(4-isopropyl-1,3-thiazol-2-yl)methyl]phosphonate

The 2-(bromomethyl)-4-isopropyl-1,3-thiazole obtained in (D) (1.39 g, 6.31 mmol) was dissolved in toluene (30 ml), and the solution was added dropwise with triethyl phosphite (1.08 ml, 6.31 mmol) and heated under reflux for 21 hours. After the solvent was evaporated, the residue was again dissolved in toluene (30 ml), and then, the solution was added dropwise with triethyl phosphite (1.08 ml, 6.31 mmol) and heated under reflux for 20 hours. The reaction mixture was further added with triethyl phosphite (1.08 ml, 6.31 mmol), and heated under reflux for additional 10 hours. The solvent was evaporated, and the residue was purified by silica gel column chromatography (chloroform → chloroform:ethyl acetate = 1:1 → 1:2, v/v) to obtain the title compound as light yellow oil (1.92 g, quantitative).
¹H-NMR (CDCl₃) δ : 1.29 (6H, d, J=6.8Hz), 1.30 (6H, t, J=6.8Hz), 3.01-3.15 (1H, m), 3.61 (2H, d, J=21.5Hz), 4.11 (2H, q, J=6.8Hz), 4.13 (2H, q, J=6.8Hz), 6.81 (1H, s)

### (F) tert-Butyl N-5-[(E)-2-(4-isopropyl-1,3-thiazol-2-yl)-1-ethenyl]-3-pyridylcarbamate

The diethyl [(4-isopropyl-1,3-thiazol-2-yl)methyl]phosphonate obtained in (E) (0.87 g, 3.14 mmol) was dissolved in methanol (30 ml), and the solution was added dropwise with a solution of the tert-butyl N-(5-formyl-3-pyridyl)carbamate obtained in (C) (0.70 g, 3.14 mmol) in methanol (10 ml), added further with sodium methoxide (203 mg, 3.76 mmol) under ice cooling, and then stirred at room temperature for 3 hours. The reaction mixture was further added with sodium methoxide (203 mg, 3.76 mmol), and stirred at room temperature for 5 hours. After the solvent was evaporated, the residue was added with ethyl acetate, and washed with water and saturated brine. The layer was then dried over anhydrous sodium sulfate, and the solvent was evaporated. The residue was purified by silica gel column chromatography (chloroform → chloroform:ethyl acetate = 3:1 → 2:1, v/v) to obtain the title compound as a white solid (0.82 g, 75.7%).
¹H-NMR (DMSO-d₆) δ : 1.33 (6H, d, J=6.8Hz), 1.54 (9H, s), 3.07-3.20 (1H, m), 6.70 (1H, s), 6.85 (1H, s), 7.33 (1H, d, J=16.1Hz), 7.39 (1H, d, J=16.1Hz), 8.29 (2H, br), 8.41 (1H, s)

### (G) 5-[(E)-2-(4-Isopropyl-1,3-thiazol-2-yl)-1-ethenyl]-3-pyridineamine

The tert-butyl N-5-[(E)-2-(4-isopropyl-1,3-thiazol-2-yl)-1-ethenyl]-3-pyridylcarbamate obtained in (F) (153 mg, 0.45 mmol) was dissolved in methylene chloride (5 ml), and the solution was added dropwise with trifluoroacetic acid (5 ml) under ice cooling, and stirred at the same temperature for one hour and further at room temperature for 2.5 hours. After the solvent was evaporated, the residue was added with water, and washed with ether. The mixture was adjusted to about pH 8 with saturated aqueous sodium hydrogencarbonate, and then extracted with chloroform. The organic layer was washed with saturated brine, and dried over anhydrous sodium sulfate. The solvent was evaporated to obtain the title compound as a light yellow solid (72 mg, 66.7%).
¹H-NMR (CDCl₃) δ : 1.33 (6H, d, J=7.3Hz), 3.06-3.18 (1H, m), 3.74 (2H, br), 6.84 (1H, s), 7.09-7.12 (1H, m), 7.24-7.29 (2H, m), 8.03 (1H, d, J=2.9Hz), 8.16 (1H, d, J=1.5Hz).

### (H) Methyl (4S,5S)-5-[(5-[(E)-2-(4-isopropyl-1,3-thiazol-2-yl)-1-ethenyl]-3-pyridylamino)carbonyl]-2,2-dimethyl-1,3-dioxolane-4-carboxylate

The 5-[(E)-2-(4-isopropyl-1,3-thiazol-2-yl)-1-ethenyl]-3-pyridineamine obtained in (G) (72 mg, 0.30 mmol) was dissolved in methylene chloride (5 ml), and the solution was added with the (4S,5S)-5-(methoxycarbonyl)-2,2-dimethyl-1,3-dioxolane-4-carboxylic acid synthesized in Example 28 (A) (90 mg, 0.44 mmol), and then added dropwise with diisopropylethylamine (123 µl, 0.71 mmol) under ice cooling. The mixture was further added with N,N-bis-(2-oxo-3-oxazolidinyl)-phosphinic acid chloride (90 mg, 0.35 mmol), and stirred at the same temperature for 10 minutes and further at room temperature for 16 hours. After the solvent was evaporated, the residue was added with water, and then extracted with chloroform. The organic layer was washed with saturated aqueous sodium hydrogencarbonate and saturated brine, and dried over anhydrous sodium sulfate, and then, the solvent was evaporated. The residue was purified by silica gel column chromatography (chloroform → chloroform:methanol = 20:1, v/v) to obtain the title compound as light yellow oil (78 mg, 61.2%).
¹H-NMR (CDCl₃) δ : 1.34 (6H, d, J=6.8Hz), 1.55 (3H, s), 1.59 (3H, s), 3.05-3.20 (1H, m), 3.89 (3H, s), 4.87 (1H, d, J=5.4Hz), 4.94 (1H, d, J=5.4Hz), 6.88 (1H, s), 7.28 (1H, s), 7.35-7.45 (2H, m), 8.42-8.65 (3H, m)

### (I) (4S,5S)-5-[(5-[(E)-2-(4-Isopropyl-1,3-thiazol-2-yl)-1-ethenyl]-3-pyridylamino)-carbonyl]-2,2-dimethyl-1,3-dioxolane-4-carboxylic acid

The methyl (4S,5S)-5-[(5-[(E)-2-(4-isopropyl-1,3-thiazol-2-yl)-1-ethenyl]-3-pyridylamino)carbonyl]-2,2-dimethyl-1,3-dioxolane-4-carboxylate obtained in (H) (78 mg, 0.18 mmol) was dissolved in THF:methanol (3:1, v/v, 4 ml), and the solution was added dropwise with a solution of lithium hydroxide monohydrate (8 mg, 0.20 mmol) in water (1 ml) under ice cooling, and the mixture was stirred at room temperature for 3.5 hours. After the solvent was evaporated, the residue was added with a small amount of water, and the mixture was washed with ether, adjusted to about pH 7 with 1 N aqueous hydrochloric acid, and then extracted with chloroform. The organic layer was washed with saturated brine, and dried over anhydrous sodium sulfate, and then the solvent was evaporated. The residue was added with hexane and collected by filtration as powder. The powder was washed with hexane to obtain the title compound as light yellow powder (75 mg, quantitative).
¹H-NMR (DMSO-d₆) δ : 1.27 (6H, d, J=6.8Hz), 1.45 (6H, s), 2.99-3.10 (1H, m), 4.73-4.83 (2H, m), 7.30 (1H, s), 7.46 (1H, d, J=16.1Hz), 7.52 (1H, d, J=16.1Hz), 8.39 (1H, s), 8.63 (1H, s), 8.71 (1H, s), 10.64 (1H, br)
EI/MS; m/z: 417 (M⁺)
FAB/MS; m/z: 418 (MH⁺)

### Example 16: 2-(3-((4-Isopropyl-1,3-thiazol-2-yl)methoxy)-5-(methoxycarbonyl)anilino)-2-oxoethylbenzoic acid (*)

### (A) Dimethyl 5-((4-isopropyl-1 ,3-thiazol-2-yl)methoxy)isophthalate

A solution of dimethyl 5-hydroxyphthalate (1.34 g, 6.36 mmol), (4-isopropyl-1,3-thiazol-2-yl)methanol (1.00 g, 6.36 mmol) and triphenylphosphine (1.67 g, 6.36 mmol) in THF (20 mL) was added with diethyl azodicarboxylate (1.00 mL, 6.36 mmol) at -15°C. The reaction mixture was stirred at room temperature for 3 hours, and the solvent was concentrated under reduced pressure by azeotropy using methylene chloride. The residue was added with methylene chloride, and the insoluble solids were removed by filtration and washed with methylene chloride. The filtrate and the washing liquids were combined, and concentrated under reduced pressure. The residue was purified by silica gel column chromatography (hexane:ethyl acetate, 5:1, v/v) to obtain the title compound as a mixture of colorless oil and crystals (2.20 g, 99%).
¹H-NMR (CDCl₃) δ : 1.33 (6H, d, J=6.8Hz), 3.12 (1H, septet, J=6.8Hz), 3.94 (6H, s), 5.42 (2H, s), 6.92 (1H, s), 7.87 (2H, s), 8.33 (1H, d, J=1.2Hz)

### (B) 3-((4-Isopropyl-1,3-thiazol-2-yl)methoxy)-5-(methoxycarbonyl)benzoic acid

A solution of the dimethyl 5-((4-isopropyl-1,3-thiazol-2-yl)methoxy)isophthalate obtained in (A) (1.99 g, 5.69 mmol) in methanol (40 mL) was added with a solution of barium hydroxide octahydrate (897 mg, 2.84 mmol) in methanol (20 mL) at 0°C, and the mixture was stirred at room temperature for 16 hours. The solvent was concentrated under reduced pressure, and the residue was diluted with 1 N aqueous hydrochloric acid and extracted with ethyl acetate, and then washed with saturated aqueous sodium hydrogencarbonate. The organic layer was dried over magnesium sulfate, and then concentrated under reduced pressure. The residue was purified by silica gel column chromatography (chloroform → chloroform:methanol, 10:1 → 5:1, v/v) to obtain the title compound (496 mg). The starting material (1.31 g) was also recovered. Each of the compounds was a mixture containing ethyl ester whose separation was impossible.
¹H-NMR (DMSO-d₆) δ : 1.25 (6H, d, J=6.8Hz), 3.04 (1H, septet, J=6.8Hz), 3.87 (3H, s), 5.52 (2H, s), 7.31 (1H, s), 7.73 (1H, s), 7.81 (1H, s), 8.13 (1H, s)

### (C) Methyl 3-((tert-butoxycarbonyl)amino)-5-((4-isopropyl-1,3-thiazol-2-yl)methoxy)-benzoate

A solution of the 3-((4-isopropyl-1,3-thiazol-2-yl)methoxy)-5-(methoxycarbonyl)benzoic acid obtained in (B) (102 mg) in tert-butanol (5 mL) was added with triethylamine (51.0 µL, 0.365 mmol) and diphenylphosphoric acid azide (72.2 µL, 0.335 mmol), and the mixture was heated under reflux for 13.5 hours. The reaction mixture was concentrated under reduced pressure, and the residue was purified by silica gel column chromatography (chloroform → chloroform:methanol, 99:1, v/v → methanol) to obtain the title compound as a mixture containing its ethyl ester difficult to be separated (72.2 mg).
¹H-NMR (CDCl₃) δ : 1.32 (6H, d, J=6.8Hz), 1.52 (9H, s), 3.11 (1H, septet, J=6.8Hz), 3.89 (3H, s), 5.36 (2H, s), 6.61 (1H, br), 6.89 (1H, s), 7.35 (1H, s), 7.53-7.54 (2H, m)

### (D) 3-((tert-Butoxycarbonyl)amino)-5-((4-isopropyl-1,3-thiazol-2-yl)methoxy)benzoic acid

A solution of the 3-((tert-butoxycarbonyl)amino)-5-((4-isopropyl-1,3-thiazol-2-yl)methoxy)benzoic acid obtained in (C) (72.2 mg) in dioxane (2 mL) was added with 1 N aqueous sodium hydroxide (1 mL). Since a uniform mixture was not formed, the reaction mixture was added with methanol (1 mL), and then stirred at room temperature for 18 hours. The solvent was concentrated under reduced pressure, and the residue was diluted with 1 N aqueous hydrochloric acid, and extracted with ethyl acetate. The organic layer was washed with saturated aqueous sodium hydrogencarbonate, dried over magnesium sulfate, and concentrated under reduced pressure to obtain the title compound as a white solid (60.3 mg).
¹H-NMR (CD₃OD) δ : 1.31 (6H, d, J=6.8Hz), 1.52 (9H, s), 3.09 (1H, septet, J=6.8Hz), 5.36 (2H, s), 7.14 (1H, d, J=0.7Hz), 7.31 (1H, d, J=1.2, 2.4Hz), 7.45 (1H, br), 7.64-7.65 (1H, m)

### (E) Methyl 3-((tert-butoxycarbonyl)amino)-5-((4-isopropyl-1,3-thiazol-2-yl)methoxy)-benzoate

A solution of the 3-((tert-butoxycarbonyl)amino)-5-((4-isopropyl-1,3-thiazol-2-yl)methoxy)benzoic acid obtained in (D) (60.3 mg) in THF(3 mL) and methanol (1 mL) was added with trimethylsilyldiazomethane (2.0 M in hexane, 85.5 µL, 0.169 mmol) at 0°C, and the mixture was stirred at room temperature for 15.5 hours. The solvent was concentrated under reduced pressure, and the residue was purified by silica gel column chromatography (hexane:ethyl acetate, 5:1, v/v) to obtain the title compound as colorless oil (57.2 mg, 92%).
¹H-NMR (CDCl₃) δ : 1.32 (6H, d, J=6.8Hz), 1.52 (9H, s), 3.11 (1H, septet, J=6.8Hz), 3.89 (3H, s), 5.36 (2H, s), 6.61 (1H, br), 6.89 (1H, s), 7.35 (1H, s), 7.53-7.54 (2H, m)

### (F) Methyl 3-amino-5-((4-isopropyl-1,3-thiazol-2-yl)methoxy)benzoate

The methyl 3-((tert-butoxycarbonyl)amino)-5-((4-isopropyl-1,3-thiazol-2-yl)-methoxy)benzoate obtained in (E) (57.2 mg, 0.140 mmol) was added with 4 N hydrochloric acid in dioxane (2 mL) at 0°C, and the mixture was stirred at room temperature for 2 hours. The solvent was concentrated under reduced pressure by azeotropy using chloroform, and the residue was diluted with saturated aqueous sodium hydrogencarbonate, and then extracted with chloroform. The organic layer was dried over magnesium sulfate, and concentrated under reduced pressure to obtain the title compound as light yellow oil (44.4 mg, quantitative).
¹H-NMR (CDCl₃) δ : 1.32 (6H, d, J=6.8Hz), 3.11 (1H, septet, J=6.8Hz), 3.88 (3H, s), 5.33 (2H, s), 6.53 (1H, t, J=1.8Hz), 6.90 (1H, d, J=1.0Hz), 7.03 (1H, t, J=1.0Hz), 7.08 (1H, d, J=1.7Hz)

### (G) 2-(3-((4-Isopropyl-1,3-thiazol-2-yl)methoxy)-5-(methoxycarbonyl)anilino)-2-oxo-ethylbenzoic acid

A solution of the methyl 3-amino-5-((4-isopropyl-1,3-thiazol-2-yl)methoxy)-benzoic acid obtained in (F) (44.4 mg, 0.145 mmol) in toluene (2 mL) was added with homophthalic anhydride (23.5 mg, 0.145 mmol), and the mixture was heated under reflux for one hour. The reaction mixture was cooled at 0°C, and the resulting solids were collected by filtration, and washed with toluene to obtain the title compound as white crystals (48.3 mg, 71%).
¹H-NMR (DMSO-d₆) δ : 1.24 (6H, d, J=6.8Hz), 3.03 (1H, septet, J=6.8Hz), 3.83 (3H, s), 4.09 (2H, s), 5.42 (2H, s), 7.27 (1H, s), 7.29 (1H, s), 7.35-7.40 (2H, m), 7.51 (1H, t, J=7.2Hz), 7.60 (1H, s), 7.86 (1H, s), 7.89 (1H, d, J=7.6Hz), 10.4 (1H, s), 12.82 (1H, s)
EI-MS; m/z: 468 (M⁺)

### Example 17: 2-[2-(5-[(E)-2-(4-Isopropyl-1,3-thiazol-2-yl)-1-ethenyl]-3-pyridyiamino)-2-oxoethyl]benzoic acid (*)

The 5-[(E)-2-(4-isopropyl-1,3-thiazol-2-yl)-1-ethenyl]-3-pyridineamine obtained in Example 15 (G) was treated under the same conditions as shown in Example 1 to obtain the title compound.
¹H-NMR (DMSO-d₆) δ : 1.36 (6H, d, J=6.7Hz), 3.14 (1H, heptuplet), 4.24 (2H, s), 7.38 (1H, s), 7.50 (1H, t, J=6.0Hz), 7.53 (2H, s), 7.63 (1H, t, J=6.0Hz), 7.99 (1H, d, J<1Hz), 8.01 (1H, d, J=1.8Hz), 8.42 (1H, d, J=2.1Hz), 8.65 (1H, d, J=1.5Hz), 8.70 (1H, d, J=2.1Hz), 10.48 (1H, s), 12.94 (1H, s)
MS (ES-); m/z: 406 (M⁺-1)

### Example 18: 2-[2-(6-[(E)-2-(4-Isopropyl-1,3-thiazol-2-yl)-1-ethenyl]-2-pyridylamino)-2-oxoethyl]benzoic acid (*)

### (A) 6-[(tert-Butoxycarbonyl)amino]picolinic acid

2,6-Pyridinedicarboxylic acid was treated in the same manner as in Example 15 (A) to obtain the title compound.
¹H-NMR (DMSO-d₆) δ : 1.56 (9H, s), 7.76 (1H, d, J=7.2Hz), 7.99 (1H, t, J=7.5Hz), 8.09 (1H, d, J=7.5Hz), 10.09 (1H, s), 13.18 (1H, s)

### (B) tert-Butyl N-(6-[methoxy(methyl)amino]carbonyl-2-pyridyl)carbamate

6-[(tert-Butoxycarbonyl)amino]picolinic acid was treated in the same manner as in Example 15 (B) to obtain the title compound.
¹H-NMR (CDCl₃) δ : 1.64 (9H, s), 3.48 (3H, s), 3.82 (3H, s), 7.39 (1H, d, J=7.5Hz), 7.86 (1H, t, J=8.0Hz), 8.13 (1H, d, J=8.0Hz)

### (C) tert-Butyl N-(6-formyl-2-pyridyl)carbamate

The tert-butyl N-(6-[methoxy(methyl)amino]carbonyl-2-pyridyl)carbamate obtained in (B) was treated in the same manner as in Example 15 (C) to obtain the title compound.
¹H-NMR (CDCl₃) δ : 1.64 (9H, s), 7.64 (1H, broad s), 7.73 (1H, d, J=8.1Hz), 7.95 (1H, t, J=8.1Hz), 8.31 (1H, d, J=8.1Hz), 10.03 (1H, s)

### (D) tert-Butyl N-6-[(E)-2-(4-isopropyl-1,3-thiazol-2-yl)-1-ethenyl]-2-pyridylcarbamate

The diethyl [(4-isopropyl-1,3-thiazol-2-yl)methyl]phosphonate obtained in (C) and the diethyl [(4-isopropyl-1,3-thiazol-2-yl)methyl]phosphonate obtained in Example 15 (E) were treated in the same manner as in Example 15 (F) to obtain the title compound.
¹H-NMR (CDCl₃) δ : 1.45 (6H, d, J=7.0Hz), 1.65 (9H, s), 3.25 (1H, heptuplet, J=7.0Hz), 6.98 (1H, s), 7.18 (1H, d, J=7.5Hz), 7.23-7.42 (2H, m), 7.78 (2H, m), 7.98 (1H, m)
MS (ES+); m/z: 346 (MH⁺)

### (E) 2-[2-(6-[(E)-2-(4-Isopropyl-1,3-thiazol-2-yl)-1-ethenyl]-2-pyridylamino)-2-oxo-ethyl]benzoic acid

The tert-butyl N-6-[(E)-2-(4-isopropyl-1,3-thiazol-2-yl)-1-ethenyl]-2-pyridylcarbamate obtained in (D) was treated in the same manner as in Example 15 (G) and subsequently in the same manner as in Example 1 to obtain the title compound.
¹H-NMR (DMSO-d₆) δ : 1.37 (6H, d, J=6.9Hz), 3.18 (1H, heptuplet, J=6.9Hz), 4.26 (2H, s), 7.50 (5H, m), 7.62 (1H, t, J=7.5Hz), 7.86 (2H, m), 8.01 (2H, m), 10.67 (1H, s), 12.97 (1H, broad s)
MS (ES-); m/z: 406 (M⁺-1)

### Example 19:

### 2-[2-(5-[(E)-2-(3-Isopropyl-2,4-thiadiazol-5-yl)-1-ethenyl]-3-pyridylamino)-2-oxoethyl]benzoic acid (*)

### (A) 5-Chloromethyl-3-isopropyl-1,2,4-thiadiazole

The (3-isopropyl-1,2,4-thiadiazol-5-yl)methanol obtained in Example 13 (B) (0.57 g) was dissolved in methylene chloride (4 mL), and the solution was added with pyridine (0.87 mL) and methanesulfonyl chloride (0.84 mL) under ice cooling, and then the mixture was stirred at room temperature overnight. The reaction mixture was distributed in ethyl acetate and water, washed with 1 N hydrochloric acid, saturated aqueous copper sulfate and saturated brine, and then dried over magnesium sulfate. The solvent was evaporated under reduced pressure. The residue was purified by silica gel chromatography (hexane:methylene chloride, 1:1, v/v) to obtain the title compound as oil (378 mg).
¹H-NMR (CDCl₃) δ : 1.53 (6H, d, J=7.2Hz), 3.40 (1H, m), 5.01 (2H, s)

### (B) Diethyl (3-isopropyl-(1,2,4)-thiadiazol-5-yl)methylphosphonate

The 5-chloromethyl-3-isopropyl-1,2,4-thiadiazole obtained in (A) (378 mg) was dissolved in acetone (15 mL), and the solution was added with a solution of sodium iodide (640 mg) in acetone (2 mL), and stirred in a dark place for 2 hours. The reaction mixture was distributed in ethyl acetate and water, washed with saturated brine, and then dried over magnesium sulfate. The solvent was evaporated under reduced pressure. The residue was added with triethyl phosphite (6 mL), and heated under reflux for 4 hours. The reaction mixture was concentrated to obtain the title compound. This compound was used for the next reaction without purification.
¹H-NMR (CDCl₃) δ : 1.42-1.51 (12H, m), 3.42 (1H, m), 3.60 (2H, d, J=21.4Hz), 4.15-4.28 (4H, m)

### (C) tert-Butyl N-5-[(E)-2-(3-isopropyl-1,2,4-thiadiazol-5-yl)-1-ethenyl]-3-pyridylcarbamate

The diethyl (3-isopropyl-(1,2,4)-thiadiazol-5-yl)methylphosphonate obtained in (B) was treated in the same manner as in Example 15 (F) to obtain the title compound.
¹H-NMR (CDCl₃) δ : 1.54 (6H, d, J=6.9Hz), 1.66 (9H, s), 3.46 (1H, heptuplet, J=6.9Hz), 7.05 (1H, s), 7.55 (1H, d, J=16.5Hz), 7.70 (1H, d, J=16.2Hz), 8.54 (2H, m), 8.58 (1H, m)

### (D) 2-[2-(5-[(E)-2-(3-Isopropyl-1,2,4-thiadiazol-5-yl)-1-ethenyl]-3-pyridylamino)-2-oxoethyl]benzoic acid

The tert-butyl N-5-[(E)-2-(3-isopropyl-1,2,4-thiadiazol-5-yl)-1-ethenyl]-3-pyridylcarbamate obtained in (C) was treated in the same manner as in Example 15 (G) and then in the same manner as in Example 1 to obtain the title compound.
¹H-NMR (DMSO-d₆) δ : 1.44 (6H, d, J=6.9Hz), 3.39 (1H, heptuplet, J=6.9Hz), 4.25 (2H, s), 7.50 (1H, t, J=8.1Hz), 7.67 (1H, t, J=7.5Hz), 7.82 (2H, s), 8.00 (1H, d, J=7.8Hz), 8.82 (1H, m), 8.70 (1H, d, J=2.1Hz), 8.74 (1H, d, J=2.1Hz)
MS (ES-); m/z: 407 (M⁺-1)

### Example 20: 4-[(5-[(E)-2-(4-Isopropyl-1,3-thiazol-2-yl)-1-ethenyl]-3-pyridylamino)-carbonyl]tetrahydro-3-furancarboxylic acid

### (A) 4-(Ethoxycarbonyl)tetrahydro-3-furancarboxylic acid

3,4,4-Tetraethoxycarbonyldihydrofuran (J. Org. Chem., 1963, 28, 802, 2.12 g) was dissolved in dimethyl sulfoxide (5 mL), and the solution was added with lithium chloride (749 mg) and water (200 µL), and then stirred at 160°C for 3 hours. The reaction mixture was cooled and poured into water, and then extracted with ethyl acetate. The extract was dried over sodium sulfate, and the solvent was evaporated under reduced pressure. The residue was distilled under reduced pressure to obtain 3,4-diethoxycarbonyltetrahydrofuran as an oil (642 mg). A 442 mg portion of the product was dissolved in ethanol (3 mL), and the solution was added with potassium hydroxide (135 mg) and stirred for 16 hours. The solvent was evaporated under reduced pressure, and the residue was dissolved in water, and washed twice with ether. The aqueous layer was adjusted to pH 2 and extracted with ethyl acetate, and the extract was dried over sodium sulfate. The solvent was evaporated under reduced pressure to obtain the title compound.
¹H-NMR (CDCl₃) δ : 1.49 (3H, t, J=7.8Hz), 3.58-3.72 (3H, m), 4.07 (1H, dd, J=8.4, 5.7Hz), 4.16 (1 H, dd, J=8.6, 5.0), 4.23 (1H, dd, J=8.0, 4.9Hz), 4.31 (2H, q, J=7.8)
MS (ES-); m/z: 187 (M⁺-1)

### (B) 4-[(5-[(E)-2-(4-Isopropyl-1,3-thiazol-2-yl)-1-ethenyl]-3-pyridylamino)carbonyl]-tetrahydro-3-furancarboxylic acid

The 4-(ethoxycarbonyl)tetrahydro-3-furancarboxylic acid obtained in (A) was treated in the same manner as in Example 15 (H) and (I) to obtain the title compound.
¹H-NMR (CD₃OD) δ : 1.49 (6H, d, J=6.9Hz), 3.25 (1H, heptuplet, J=6.9Hz), 3.38 (1H, dd, J=7.9, 7.9Hz), 3.58 (1H, dd, J=7.5, 7.5Hz), 4.07-4.24 (3H, m), 7.29 (1H, s), 7.50 (1H, d, J=16.3), 7.55 (1H, m), 7.60 (1H, d, J=16.3), 7.74 (1H, m), 8.04 (1H, m)
MS (ES-); m/z: 385 (M⁺-1)

### Example 21: 2-[2-(4-[(E)-2-(4-Iopropyl-1,3-thiazol-2-yl)-1-ethenyl]-2-pyridylamino)-2-oxoethyl]benzoic acid (*)

### (A) 4-[(E)-2-(4-Isopropyl-1,3-thiazol-2-yl)-1-ethenyl]-2-aminopyridine

N-(4-Formyl-2-pyridyl)-2,2-dimethylpropaneamide (1.7 g, 8.2 mmol) and 4-isopropyl-2-methyl-1,3-thiazole (1.2 g, 8.5 mmol) were dissolved in acetic anhydride (10 ml), and the solution was heated under reflux at 100°C for 15 hours and further at 170°C for 7 hours. The reaction mixture was distributed in ethyl acetate and saturated aqueous sodium hydrogencarbonate. The organic layer was washed with saturated brine, and dried over magnesium sulfate. The solvent was evaporated under reduced pressure. The residue was added with 3 N hydrochloric acid (50 mL), and heated under reflux at 100°C for one hour. The reaction mixture was made alkaline with 3 N aqueous sodium hydroxide, and extracted with ethyl acetate, and then the extract was dried over magnesium sulfate. The solvent was evaporated under reduced pressure. The residue was purified by silica gel chromatography (chloroform:methanol, 20:1, v/v) to obtain the title compound as light yellow powder (480 mg, 24%).
¹H-NMR (CD₃OD) δ : 1.33 (6H, d, J=6.8Hz), 3.10 (1H, m), 6.70 (1H, s), 6.82 (1H, d, J=5.6Hz), 7.15 (1H, s), 7.27 (1H, d, J=16.5Hz), 7.43 (1H, d, J=16.5Hz), 7.88 (1H, d, J=5.4Hz)

### (B) 2-[2-(4-[(E)-2-(4-Isopropyl-1,3-thiazol-2-yl)-1-ethenyl]-2-pyridylamino)-2-oxo-ethyl]benzoic acid

The 4-[(E)-2-(4-isopropyl-1,3-thiazol-2-yl)-1-ethenyl]-2-aminopyridine obtained in (A) was treated under the same conditions as shown in Example 1 to obtain the title compound.
¹H-NMR (DMSO-d₆) δ : 1.26 (6H, d, J=6.8Hz), 2.99-3.09 (1H, m), 4.17 (2H, s), 7.32 (1H, s), 7.36 (1H, d, J=16.3Hz), 7.35-7.42 (3H, m), 7.49-7.55 (1H, m), 7.57 (1H, d, J=16.3Hz), 7.89 (1H, dd, J=5.4, 1.2Hz), 8.19 (1H, s), 8.30 (1H, d, J=5.4Hz), 10.61 (1H, s)
FAB-MS; m/z: 408 (MH⁺)

### Example 22: 2-[2-(4-[(4-Isopropyl-1,3-thiazol-2-yl)methoxy]-2-pyridinoamino)-2-oxo-ethyl]benzoic acid (*)

### (A) Ethyl 4-[(4-isopropyl-1,3-thiazol-2-yl)methoxy]-2-pyridinecarboxylate

Ethyl 4-hydroxy-2-pyridinecarboxylate (U.S. Patent No. 5,260,286) was treated under the same conditions as shown in Example 34 to obtain the title compound.
¹H-NMR (CDCl₃) δ : 1.32 (6H, d, J=6.9Hz), 1.44 (3H, t, J=7.1Hz), 3.07-3.17 (1H, m), 4.47 (2H, q, J=7.1Hz), 5.44 (2H, s), 6.94 (1H, d, J=1.0Hz), 7.09 (1H, dd, J=5.6, 2.7Hz), 7.79 (1H, d, J=2.7Hz), 8.58 (1H, d, J=5.6Hz)

### (B) N-tert-Butyl N-4-[(4-isopropyl-1,3-thiazol-2-yl)methoxy]-2-pyridinecarbamate

The ethyl 4-[(4-isopropyl-1,3-thiazol-2-yl)methoxy]-2-pyridinecarboxylate obtained in (A) (198 mg, 0.65 mmol) was dissolved in ethanol (10 ml), and the solution was added dropwise with 1 N aqueous sodium hydroxide (0.71 ml, 0.71 mmol) and stirred at room temperature for 17 hours. After the solvent was evaporated, the residue was added with 1 N aqueous hydrochloric acid so as to be adjusted to approximately pH 2, and then washed with ethyl acetate. The mixture was added with 1 N aqueous sodium hydroxide so as to be adjusted to approximately pH 7, and then the solvent was evaporated. The residue was suspended in tert-butanol (10 ml), and the suspension was added dropwise with diphenylphosphoric acid azide (122 µl, 0.56 mmol) and triethylamine (85 µl, 0.61 mmol), and heated under reflux for 8 hours. The reaction mixture was further added with tert-butanol (10 ml), diphenylphosphoric acid azide (122 µl, 0.56 mmol) and triethylamine (85 µl, 0.61 mmol), and heated under reflux for 6 hours. After the solvent was evaporated, the residue was purified by silica gel column chromatography (chloroform → chloroform:methanol = 10:1, v/v) and preparative silica gel thin layer chromatography (chloroform → chloroform:methanol = 20:1, v/v) to obtain the title compound as light yellow oil (91 mg, 56.0%).
¹H-NMR (CDCl₃) δ : 1.32 (6H, d, J=6.8Hz), 1.54 (9H, s), 3.08-3.17 (1H, m), 5.34 (2H, s), 6.61 (1H, dd, J=5.9, 2.2Hz), 6.91 (1H, s), 7.26 (1H, d, J=2.2Hz), 8.15 (1H, d, J=5.9Hz), 9.36 (1H, br)

### (C) 4-[(4-Isopropyl-1,3-thiazol-2-yl)methoxy]-2-pyridineamine

The N-tert-butyl N-4-[(4-isopropyl-1,3-thiazol-2-yl)methoxy]-2-pyridinecarbamate obtained in (B) (90 mg, 0.26 mmol) was dissolved in dichloromethane (2 ml), and the solution was added dropwise with trifluoroacetic acid (2 ml) under ice cooling, and stirred at room temperature for 30 minutes. After the solvent and excess reagents were evaporated, the reaction mixture was added with saturated aqueous sodium hydrogencarbonate so as to be adjusted to approximately pH 8. Then, the reaction mixture was extracted with chloroform, and the organic layer was washed with saturated brine and dried over anhydrous sodium sulfate. The solvent was evaporated to obtain the title compound as pale orange oil (79 mg, quantitative).
¹H-NMR (CDCl₃) δ : 1.32 (6H, d, J=6.8Hz), 3.05-3.17 (1H, m), 4.65 (2H, br), 5.33 (2H, s), 6.10 (1H, d, J=1.7Hz), 6.36 (1H, dd, J=5.9, 1.7Hz), 6.92 (1H, s), 7.90 (1H, d, J=5.9Hz)

### (D) 2-[2-(4-[(4-Isopropyl-1,3-thiazol-2-yl)methoxy]-2-pyridinoamino)-2-oxoethyl]-benzoic acid

The 4-[(4-isopropyl-1,3-thiazol-2-yl)methoxy]-2-pyridineamine obtained in (C) was treated under the same conditions as shown in Example 1 to obtain the title compound.
¹H-NMR (DMSO-d₆) δ : 1.23 (6H, d, J=6.7Hz), 2.95-3.09 (1H, m), 4.13 (2H, s), 5.42 (2H, s), 6.81 (1H, dd, J=5.6, 2.2Hz), 7.29 (1H, s), 7.32-7.40 (2H, m), 7.48-7.64 (1H, m), 7.71 (1H, d, J=2.2Hz), 7.87-7.92 (1H, m), 8.15 (1H, d, J=5.6Hz), 10.53 (1H, s)
FAB-MS; m/z: 412 (MH⁺)

### Example 23: N-Cyano-2-(2-((5-((E)-2-(4-isopropyl-1,3-thiazol-2-yl)-1-ethenyl)-3-pyridyl)amino)-2-oxoethyl)benzamide (*)

2-[2-(5-[(E)-2-(4-Isopropyl-1,3-thiazol-2-yl)-1-ethenyl]-3-pyridylamino)-2-oxo-ethyl]benzoic acid (49 mg) was dissolved in tetrahydrofuran (2 mL), and the solution was added with pentafluorophenol (22 mg) and dicyclohexylcarbodiimide (25 mg), and stirred for 14 hours. After the solvent was evaporated under reduced pressure, the residue was added with ethyl acetate. The insoluble solids were removed by filtration, and the solvent was evaporated under reduced pressure. The residue was dissolved in dimethylformamide (3 ml), and the solution was added with cyanamide (24 mg) and sodium hexamethyldisilazide (0.58 mmol), and then stirred for 72 hours. The reaction mixture was distributed in ethyl acetate and 1 N hydrochloric acid, and the organic layer was washed with saturated brine, and dried over sodium sulfate. The solvent was evaporated under reduced pressure, and the residue was purified by silica gel chromatography (methylene chloride:methanol, 9:1, v/v) to obtain the title compound as white powder (11 mg).
¹H-NMR (CD₃OD) δ : 1.30 (6H, d), 3.09 (1H, heptuplet), 3.94 (2H, s), 7.10 (1H, s), 7.28 (1H, m), 7.39 (5H, m), 7.75 (1H, d, J=7.5), 8.29 (1H, s), 8.38 (1H, s), 8.68 (1H, s)
MS (ES-); m/z: 430 (MH⁺)

### Example 24: 3-(2-(tert-Butoxycarbonylamino)ethoxy)-5-((3-((E)-2-(4-isopropyl-1,3-thiazol-2-yl)-1-ethenyl)anilino)carbonyl)benzoic acid (*)

### (A) 5-Hydroxyisophthalic acid dimethyl ester

5-Hydroxyisophthalic acid (10.0 g, 54.9 mmol) was dissolved in toluene and methanol (300 ml, 100 ml), and the solution was added with (trimethylsilyl)diazomethane (50 ml, 2.0 M solution in hexane, 100 mmol), and stirred for one hour. After the solvent was evaporated under reduced pressure, the residue was distributed in ethyl acetate and saturated sodium hydrogencarbonate. The organic layer was washed with saturated brine and dried over sodium sulfate, and then the solvent was evaporated to obtain the title compound as colorless crystals (6.2 g, 59%).
¹H-NMR (CDCl₃) δ : 3.95 (6H, s x 2), 5.96 (1H, br), 7.74 (2H, d, J=1.0Hz), 8.25 (1H, d, J=1.0Hz)

### (B) 5-(2-(tert-Butoxycarbonylamino)ethoxy)isophthalic acid dimethyl ester

The 5-hydroxyisophthalic acid dimethyl ester obtained in (A) (2.54 g, 12.1 mmol) and 2-(tert-butoxycarbonylamino)ethanol (1.95 g, 12.1 mmol) were dissolved in THF (110 ml), and the solution was added with diethyl azodicarboxylate (2.11 g, 12.1 mmol) and triphenylphosphine (3.17 g, 12.1 mmol), and stirred at room temperature for 13 hours. After the solvent was evaporated under reduced pressure, the residue was subjected to silica gel chromatography (hexane:ethyl acetate, 2:1, v/v) to obtain a white solid (3.428 g). Since the resulting solid was a mixture of the title compound and the starting material, i.e., 5-hydroxyisophthalic acid dimethyl ester, the following reaction was conducted.

A 2.43 g portion of the resulting white solid was dissolved in methylene chloride (50 ml), and the solution was added with the trifluoroacetic acid (30 ml) and stirred at room temperature for 1 hour. The reaction mixture was evaporated under reduced pressure, and the residue was distributed in ether and 1 N aqueous hydrochloric acid. The aqueous layer was neutralized with saturated sodium hydrogencarbonate, and extracted with chloroform. The organic layer was dried over sodium sulfate, and chloroform was evaporated under reduced pressure to obtain a white solid (330 mg).

The resulting solid was dissolved in THF (40 ml) and saturated sodium hydrogencarbonate (40 ml), and the mixture was added with di-tert-butyl dicarbonate (320 mg) and stirred at room temperature for 12 hours. The reaction mixture was distributed in ethyl acetate and 1 N aqueous hydrochloric acid. The organic layer was washed successively with saturated sodium hydrogencarbonate and saturated brine, and dried over sodium sulfate. The solvent was evaporated under reduced pressure to obtain the title compound as colorless oil (550 mg).
¹H-NMR (CDCl₃) δ : 1.45 (9H, s), 3.57 (2H, brdd), 3.94 (6H, s x 2), 4.11 (2H, t, J=5.37Hz), 4.98 (1H, br), 7.74 (2H, d, J=1.46Hz), 8.29 (1H, t, J=1.46Hz)
EI-MS; m/z: 353 (M⁺), 322 (M⁺-OMe), 88 (BP)

### (C) 5-(2-(tert-Butoxycarbonylamino)ethoxy)isophthalic acid monomethyl ester

The 5-(2-(tert-butoxycarbonylamino)ethoxy)isophthalic acid dimethyl ester obtained in (B) (540 mg, 1.53 mmol) was dissolved in THF (20 ml), and the solution was added with lithium hydroxide monohydrate (75 mg, 1.79 mmol) and water (20 ml), and then stirred at room temperature for one hour. After the reaction mixture was made acidic by addition of 1 N aqueous hydrochloric acid, the mixture was extracted with ethyl acetate. The organic layer was dried over sodium sulfate, and evaporated under reduced pressure. The residue was subjected to silica gel chromatography (hexane:ethyl acetate, 1:1, v/v) to obtain the title compound (180 mg).
¹H-NMR (CDCl₃) δ : 1.46-1.52 (9H, brs x 2), 3.60 (2H, brdd), 3.94 (3H, s), 4.13 (2H, t, J=5.0Hz), 5.06 (1H, br), 7.79 (2H, brs), 8.35 (1H, brs)

### (D) 3-(2-(tert-Butoxycarbonylamino)ethoxy)-5-((3-((E)-2-(4-phenyl-1,3-thiazol-2-yl)-1-ethenyl)anilino)carbonyl)benzoic acid methyl ester

The 5-(2-(tert-butoxycarbonylamino)ethoxy)isophthalic acid monomethyl ester obtained in (C) (122 mg, 0.360 mmol) and 3-((E)-2-(4-phenyl-1,3-thiazol-2-yl)-1-ethenyl)aniline (100 mg, 0.360 mmol) were dissolved in methylene chloride (10 ml), and the solution was added with N,N-bis-(2-oxo-3-oxazolidinyl)phosphinic acid chloride (92 mg, 0.360 mmol) and diisopropylethylamine (0.13 ml, 0.360 mmol), and then stirred at room temperature under nitrogen atmosphere for 14 hours. The reaction mixture was extracted with ethyl acetate. The organic layer was washed successively with 1 N hydrochloric acid, saturated sodium hydrogencarbonate, and saturated brine, and dried over sodium sulfate. The solvent was evaporated under reduced pressure to obtain the title compound as yellow powder (199 mg, 92%).
¹H-NMR (DMSO-d₆) δ : 1.39 (9H, s), 3.34 (2H), 3.92 (3H, s), 4.14 (2H, m), 7.07 (1H, m), 7.38-7.65 (8H, m), 7.73-7.82 (2H, m), 8.01 (1H, s), 8.03 (1H, s), 8.12 (2H, s x 2), 8.17 (1H, s), 10.49 (1H, s)
IR (KBr); cm⁻¹: 3282, 2981, 2615, 2440, 1745, 1608, 1421, 1252, 1207, 1136, 1063, 1011, 839, 688

### (E) 3-(2-(tert-Butoxycarbonylamino)ethoxy)-5-((3-((E)-2-(4-phenyl-1,3-thiazol-2-yl)-1-ethenyl)anilino)carbonyl)benzoic acid

The 3-(2-(tert-butoxycarbonylamino)ethoxy)-5-((3-((E)-2-(4-phenyl-1,3-thiazol-2-yl)-1-ethenyl)anilino)carbonyl)benzoic acid methyl ester obtained in (D) (190 mg, 0.317 mmol) was dissolved in THF (20 ml), and the solution was added with aqueous lithium hydroxide (20 ml, 0.238 M), and stirred at room temperature for one hour. The reaction mixture was made acidic with 1 N aqueous hydrochloric acid, and then the mixture was extracted with ethyl acetate. The organic layer was dried over sodium sulfate, and evaporated under reduced pressure. The residue was washed with ether to obtain the title compound as light yellow powder (110 mg, 59%).
¹H-NMR(DMSO-d₆) δ : 1.39 (9H, s), 3.35 (2H), 4.13 (2H, t, J=5.5Hz), 7.07 (1H, m), 7.36-7.63 (8H, m), 7.74-7.78 (2H, m), 8.01 (1H, s), 8.03 (1H, s), 8.12 (2H, s x 2), 8.17 (1H, s), 10.48 (1H, s)
FAB-MS; m/z: 530 (MH⁺-tBu)
IR (KBr); cm⁻¹: 3298, 2976, 1689, 1252, 1065, 688

### Example 25: 3-(2-Aminoethoxy)-5-((3-((E)-2-(4-isopropyl-1,3-thiazol-2-yl)-1-ethenyl)-anilino)carbonyl)benzoic acid (*)

3-(2-(tert-Butoxycarbonylamino)ethoxy)-5-((3-((E)-2-(4-phenyl-1,3-thiazol-2-yl)-1-ethenyl)anilino)carbonyl)benzoic acid (76 mg, 0.13 mmol) was dissolved in methylene chloride (20 ml), and the solution was added with trifluoroacetic acid (10 ml) and stirred at room temperature for one hour. The reaction mixture was evaporated under reduced pressure, and the residue was washed with ether to obtain the title compound as yellow plate crystals (58 mg, 75%).
¹H-NMR (DMSO-d₆) δ : 3.36 (2H), 4.33 (2H, t, J=4.9Hz), 7.36-7.59 (7H, m), 7.72-7.80 (3H, m), 8.01 (1H, s), 8.03 (1H, s), 8.12 (2H, s x 2), 8.25 (1H, s), 10.54 (1H, s)
EI-MS; m/z: 485 (M⁺)

### Example 26: 3-(2-Hydroxyethoxy)-5-((3-((E)-2-(4-isopropyl-1,3-thiazol-2-yl)-1-ethenyl)anilino)carbonyl)benzoic acid (*)

### (A) 2-(tert-Butyldiphenylsilyloxy)ethanol

Ethylene glycol (4.87 g, 78.5 mmol) and imidazole (5.34 g, 78.5 mmol) were dissolved in DMF (60 ml), and the solution was added with tert-butyldiphenylsilyl chloride (21 ml) at room temperature and stirred at room temperature under nitrogen atmosphere for 14.5 hours. The reaction mixture was distributed in ethyl acetate and 1 N aqueous hydrochloric acid. The organic layer was washed with water and saturated brine and dried over sodium sulfate, and then the solvent was evaporated under reduced pressure. The residue was purified by silica gel chromatography (hexane:ethyl acetate, 3:1, v/v) to obtain the title compound as colorless oil (9.298 g, 39%).
¹H-NMR (CDCl₃) δ : 1.07 (9H, s), 2.13 (1H, br), 3.69 (2H, br), 3.77 (2H, t, J=4.5Hz), 7.37-7.46 (6H, m), 7.66-7.68 (4H, m)

### (B) 5-Hydroxyisophthalic acid dimethyl ester

5-Hydroxyisophthalic acid (10.0 g, 54.9 mmol) was dissolved in toluene and methanol (300 ml, 100 ml), and the solution was added with (trimethylsilyl)diazomethane (50 ml, 2.0 M solution in hexane, 100 mmol), and stirred for one hour. The solvent was evaporated under reduced pressure, and the residue was distributed in ethyl acetate and saturated sodium hydrogencarbonate. The organic layer was washed with saturated brine, and dried over sodium sulfate. The solvent was evaporated under reduced pressure to obtain the title compound as colorless crystals (6.241 g, 59%).
¹H-NMR (CDCl₃) δ : 3.95 (6H, s x 2), 5.96 (1H, br), 7.74 (2H, d, J=1.0Hz), 8.25 (1H, d, J=1.0Hz)

### (C) 5-(2-(tert-Butyldiphenylsilyloxy)ethoxy)isophthalic acid dimethyl ester

The 2-(tert-butyldiphenylsilyloxy)ethanol obtained in (A) (6.90 g, 23.0 mmol) and 5-hydroxyisophthalic acid dimethyl ester (4.83 g, 23.0 mmol) obtained in (B) were dissolved in THF (110 ml), and the solution was added with diethyl azodicarboxylate (4.00 g, 23.0 mmol) and triphenylphosphine (6.03 g, 23.0 mmol), and stirred at room temperature for 13 hours. The solvent was evaporated under reduced pressure, and the residue was purified by silica gel chromatography (hexane:ethyl acetate, 4:1, v/v) to obtain the title compound as colorless oil (9.752 g, 86%).
¹H-NMR (CDCl₃) δ : 1.05 (9H, s), 3.94 (6H, s x 2), 4.02 (2H, t, J=4.88Hz), 4.17 (2H, t, J=4.88Hz), 7.36-7.45 (6H, m), 7.69-7.74 (6H, m), 8.27 (1H, d, J=1.47Hz)
EI-MS; m/z: 461 (M⁺-OMe), 435 (M⁺-tBu, BP)

### (D) 5-(2-(tert-Butyldiphenylsilyloxy)ethoxy)isophthalic acid monomethyl ester

The 5-(2-(tert-butyldiphenylsilyloxy)ethoxy)isophthalic acid dimethyl ester obtained in (C) (1.00 g, 2.03 mmol) was dissolved in THF (20 ml), and the solution was added with lithium hydroxide monohydrate (85 mg, 2.03 mmol) and water (20 ml), and stirred at room temperature for 20 hours. The reaction mixture was made strongly acidic with 1 N aqueous hydrochloric acid, and then extracted with ethyl acetate. The ethyl acetate layer was dried over sodium sulfate, and the ethyl acetate was evaporated under reduced pressure. The residue was purified by silica gel chromatography (hexane:ethyl acetate, 2:1, v/v) to obtain the title compound as colorless oil (241 mg, 25%).
¹H-NMR (CDCl₃) δ : 1.08 (9H, s), 3.97 (3H, s), 4.04 (2H, t, J=4.89Hz), 4.20 (2H, t, J=4.89Hz), 7.38-7.45 (6H, m), 7.70-7.73 (4H, m), 7.80 (2H, d, J=1.46Hz), 8.37 (1H, d, J=1.46Hz)

### (E) 3-(2-(tert-Butyldiphenylsilyloxy)ethoxy)-5-((3-((E)-2-(4-isopropyl-1,3-thiazol-2-yl)-1-ethenyl)anilino)carbonyl)benzoic acid methyl ester

The 5-(2-(tert-butyldiphenylsilyloxy)ethoxy)isophthalic acid monomethyl ester obtained in (D) (590 mg, 1.24 mmol) and 3-((E)-2-(4-isopropyl-1,3-thiazol-2-yl)-1-ethenyl)aniline (310 mg, 1.27 mmol) were dissolved in methylene chloride (30 ml), and the mixture was added with N,N-bis-(2-oxo-3-oxazolidinyl)phosphinic acid chloride (320 mg) and diisopropylethylamine (0.3 ml), and stirred at room temperature under nitrogen atmosphere for 12 hours. The reaction mixture was extracted with ethyl acetate, and the organic layer was washed successively with 1 N aqueous hydrochloric acid, saturated sodium hydrogencarbonate, and saturated brine, and dried over sodium sulfate. The solvent was evaporated under reduced pressure to obtain the title compound as light yellow amorphous (815 mg, 94%).

### (F) 3-(2-Hydroxyethoxy)-5-((3-((E)-2-(4-isopropyl-1,3-thiazol-2-yl)-1-ethenyl)anilino)-carbonyl)benzoic acid methyl ester

The 3-(2-(tert-butyldiphenylsilyloxy)ethoxy)-5-((3-((E)-2-(4-isopropyl-1,3-thiazol-2-yl)-1-ethenyl)anilino)carbonyl)benzoic acid methyl ester obtained in (E) (785 mg, 1.12 mmol) was dissolved in THF (20 ml), and the solution was added with tetrabutylammonium fluoride (3.0 ml, 1.0 M solution in THF, 3.0 mmol), and stirred at room temperature under nitrogen atmosphere for 2 hours. The reaction mixture was distributed in ether and 1 N aqueous hydrochloric acid, and the ether layer was dried over sodium sulfate, and evaporated under reduced pressure. The residue was purified by silica gel chromatography (hexane:ethyl acetate, 1:1, v/v) to obtain the title compound as colorless amorphous (287 mg, 55%).
¹H-NMR (CDCl₃) δ : 1.33 (6H, d, J=6.84Hz), 3.12 (1H, quintet, J=6.84Hz), 3.92 (3H, s), 4.00 (2H, t, J=4.0Hz), 4.14 (2H, t, J=4.0Hz), 6.82 (1H, s), 7.29-7.37 (4H, m), 7.57 (1H, d, J=7.82Hz), 7.62-7.65 (2H, m), 7.88 (1H, s), 8.02 (1H, s), 8.35 (1H, s)
EI-MS; m/z: 466 (M⁺, BP), 436 (M⁺-tBu+1), 435 (M⁺-tBu)

### (G) 3-(2-Hydroxyethoxy)-5-((3-((E)-2-(4-isopropyl-1,3-thiazol-2-yl)-1-ethenyl)anilino)-carbonyl)benzoic acid

The 3-(2-hydroxyethoxy)-5-((3-((E)-2-(4-isopropyl-1,3-thiazol-2-yl)-1-ethenyl)-anilino)carbonyl)benzoic acid methyl ester obtained in (F) (253 mg, 0.543 mmol) was dissolved in THF (30 ml), and the solution was added with aqueous lithium hydroxide (30 ml, 0.238 M), and stirred at room temperature for 2 hours. The reaction mixture was made acidic with 1 N aqueous hydrochloric acid, and then extracted with ethyl acetate. The organic layer was dried over sodium sulfate, and the solvent was evaporated under reduced pressure. The residue was washed with ether to obtain the title compound as light yellow powder (110 mg, 45%).
Elemental Analysis (as C₂₄H₂₄N₂O₅S•0.5H₂O):
Calculated: C, 62.46; H, 5.46; N, 6.07; S, 6.95
Found: C, 62.59; H, 5.48; N, 5.78; S, 6.59
¹H-NMR (DMSO-d₆) δ : 1.27 (6H, d, J=6.83Hz), 3.05 (1H, quintet, J=6.83Hz), 3.77 (2H, t, J=4.88Hz), 4.15 (2H, t, J=4.88Hz), 4.94 (1H, t, J=5.37Hz), 7.25 (1H, s), 7.41 (3H, m), 7.47 (1H, d, J=7.81Hz), 7.65 (1H, s), 7.73 (1H, d, J=7.81Hz), 7.78 (1H, s), 8.08 (1H, s), 8.15 (1H, s), 10.45 (1H, s), 13.25 (1H, br)
EI-MS; m/z: 452 (M⁺, BP)
IR (KBr); cm⁻¹: 3311, 2962, 1701, 1589, 1545, 1439, 1302, 1215, 1057, 687

### Example 27: (4R,5R)-5-(3-[(E)-2-(4-Isopropyl-1,3-thiazol-2-yl)]-1-ethenyl]anilino-carbonyl)-2,2-dimethyl-1,3-dioxolane-4-carboxylic acid (*)

### (A) (4R,5R)-5-(Methoxycarbonyl)-2,2-dimethyl-1,3-dioxolane-4-carboxylic acid

Dimethyl (4R,5R)-2,2-dimethyl-1,3-dioxolane-4,5-dicarboxylate (1.10 g, 5.04 mmol) was dissolved in methanol (30 ml), and the solution was added dropwise with 1 N aqueous sodium hydroxide (5.0 ml, 5.0 mmol) and stirred at room temperature for 15 minutes, and then heated under reflux for 1.5 hours. After the reaction mixture was cooled, the solvent was evaporated. The residue was added with a small amount of water and washed with ether, and then adjusted to about pH 1 with 1 N aqueous hydrochloric acid. The mixture was extracted with ethyl acetate, and the ether layer was washed with saturated brine and dried over anhydrous sodium sulfate. The solvent was evaporated to obtain the title compound as colorless oil containing the dicarboxylic acid (0.74 g). The yield calculated from NMR was 0.50 g, 48.6% for the target compound, and 0.24 g for the dicarboxylic acid.
¹H-NMR (CDCl₃) δ : 1.53 (3H, s), 1.54 (3H, s), 3.85 (3H, s), 4.77-4.91 (2H, m), 5.99 (1H, br)

### (B) Methyl (4R,5R)-5-(3-[(E)-2-(4-isopropyl-1,3-thiazol-2-yl)-1-ethenyl]anilino-carbonyl)-2,2-dimethyl-1,3-dioxolane-4-carboxylate

3-[(E)-2-(4-Isopropyl-1,3-thiazol-2-yl)-1-ethenyl]aniline (202 mg, 0.83 mmol) was dissolved in methylene chloride (10 ml), and the solution was added with the (4R,5R)-5-(methoxycarbonyl)-2,2-dimethyl-1,3-dioxolane-4-carboxylic acid obtained in (A) (containing 1/3 of the dicarboxylic acid, 338 mg, 1.66 mmol), and added dropwise with diisopropylethylamine (433 µl, 2.49 mmol) under ice cooling. The mixture was further added with N,N-bis-(2-oxo-3-oxazolidinyl)phosphinic acid chloride (316 mg, 1.24 mmol), and stirred at the same temperature for 10 minutes and further at room temperature for 13.5 hours. The solvent was evaporated, and the residue was added with water and extracted with chloroform. The organic layer was washed successively with 1 N aqueous hydrochloric acid, water, saturated aqueous sodium hydrogencarbonate, and saturated brine, and dried over anhydrous sodium sulfate. The solvent was evaporated, and the residue was purified by silica gel column chromatography (chloroform → chloroform:methanol = 50:1, v/v) to obtain the title compound as light yellow oil (90 mg, 25.2%).
¹H-NMR (CDCl₃) δ : 1.34 (6H, d, J=7.3Hz), 1.52 (3H, s), 1.55 (3H, s), 3.06-3.17 (1H, m), 3.87 (3H, s), 4.73-4.81 (1H, m), 4.84-4.92 (1H, m), 6.81 (1H, s), 7.23-7.41 (4H, m), 7.45-7.51 (1H, m), 7.80 (1H, s), 8.32 (1H, s)

### (C) (4R,5R)-5-(3-[(E)-2-(4-Isopropyl-1,3-thiazol-2-yl)-1-ethenyl]anilinocarbonyl)-2,2-dimethyl-1,3-dioxolane-4-carboxylic acid

The Methyl (4R,5R)-5-(3-[(E)-2-(4-isopropyl-1,3-thiazol-2-yl)-1-ethenyl]-anilinocarbonyl)-2,2-dimethyl-1,3-dioxolane-4-carboxylate obtained in (B) (90 mg, 0.21 mmol) was dissolved in THF and methanol (3:1, v/v, 4 ml), and the solution was added dropwise with a solution of lithium oxide monohydrate (9 mg, 0.23 mmol) in water (1 ml) under ice cooling, and stirred at room temperature for 2 hours. After the solvent was evaporated, the residue was added with a small amount of water, washed with ether, and then adjusted to about pH 1 with 1 N aqueous hydrochloric acid. The mixture was extracted with ethyl acetate, and the organic layer was washed with saturated brine and dried over anhydrous sodium sulfate. The solvent was evaporated, and the residue was added with hexane and collected by filtration as powder, and the resulting power was washed with hexane to obtain the title compound as light yellow powder (58 mg, 67.4%).
¹H-NMR (CDCl₃) δ : 1.34 (6H, d, J=6.8Hz), 1.59 (3H, s), 1.61 (3H, s), 3.08-3.20 (1H, m), 4.59 (1H, s), 4.75 (1H, s), 6.85 (1H, s), 7.17 (1H, br), 7.32-7.45 (3H, m), 7.48-7.58 (1H, m), 7.80 (1H, s), 8.45 (1H, s)
EI/MS; m/z: 416 (M⁺)
FAB/MS; m/z: 417 (MH⁺)

### Example 28: (4S,5S)-5-(3-[(4-Isopropyl-5-methyl-1,3-thiazol-2-yl)methoxy]anilino-carbonyl)-2,2-dimethyl-1,3-dioxolane-4-carboxylic acid (*)

### (A) Ethyl 4-isopropyl-2-[(3-nitrophenoxyl)methyl]-1,3-thiazole-5-carboxylate

2-(3-Nitrophenoxy)ethanethioamide (775 mg, 3.66 mmol) and ethyl 2-chloro-4-methyl-3-oxopentanoate (624 mg, 3.24 mmol) were dissolved in ethanol (10 ml), and heated under reflux for two days. The reaction mixture was left stand for cooling and then concentrated. The deposited crystals were collected by filtration to obtain the title compound (519 mg, 41%).
¹H-NMR (CDCl₃) δ: 1.31 (6H, d, J=6.8Hz), 1.36 (3H, t, J=7.3Hz), 3.98 (1H, m), 4.33 (2H, q, J=7.3Hz), 5.41 (2H, s), 7.34 (1H, m), 7.47 (1H, m), 7.90 (2H, m)

### (B) {4-Isopropyl-2-[(3-nitrophenoxy)methyl]-1,3-thiazol-5-yl}methanol

The ethyl 4-isopropyl-2-[(3-nitrophenoxyl)methyl]-1,3-thiazole-5-carboxylate obtained in (A) (519 mg, 1.48 mmol) was dissolved in THF (10 ml), and the solution was added with a 1.02 M solution of diisobutylaluminum hydride in toluene (2.96 ml) under ice cooling, and stirred for 30 minutes under ice cooling. The reaction mixture was further added with a 1.02 M solution of diisobutylaluminum hydride in toluene (5.92 ml), and stirred for one hour. The reaction mixture was added with aqueous potassium hydrogensulfate, and then extracted with ethyl acetate. After the organic layer was dried over sodium sulfate, the solvent was evaporated under reduced pressure to obtain the title compound as white crystals (454 mg, 99%).
¹H-NMR (CDCl₃) δ : 1.30 (6H, d, J=6.8Hz), 3.12 (1H, m), 4.83 (2H, d, J=4.9Hz), 5.38 (2H, s), 7.33 (1H, dd, J=8.3, 2.5Hz), 7.45 (1H, t, J=8.3Hz), 7.86-7.90 (2H, m)

### (C) [5-(Chloromethyl)-4-isopropyl-1,3-thiazol-2-yl]methyl (3-nitrophenyl) ether

The {4-isopropyl-2-[(3-nitrophenoxy)methyl]-1,3-thiazol-5-yl}methanol obtained in (B) (454 mg, 1.47 mmol) was dissolved in methylene chloride (10 ml), and the solution was added with thionyl chloride (0.215 ml) and stirred at room temperature for 1.5 hours. The reaction mixture was concentrated, diluted with chloroform, and then washed with saturated aqueous sodium hydrogencarbonate and saturated brine. The reaction mixture was dried over magnesium sulfate, and the solvent was evaporated under reduced pressure to obtain the title compound (473 mg, 98%).

### (D) [4-Isopropyl-5-methyl-1,3-thiazol-2-yl]methyl (3-nitrophenyl) ether

The [4-isopropyl-5-methyl-1,3-thiazol-2-yl]methyl (3-nitrophenyl) ether obtained in (C) (60.7 mg, 0.186 mmol) was dissolved in DMSO (1.5 ml), and the solution was added with sodium borohydride (17.5 mg) at room temperature, and then stirred overnight. The reaction mixture was added with water (20 ml) and extracted with ethyl acetate. The organic layer was dried over magnesium sulfate, and the solvent was evaporated under reduced pressure. The residue was purified by preparative TLC (n-hexane:ethyl acetate, 4:1, v/v) to obtain the title compound (44.0 mg, 81%).
¹H-NMR (CDCl₃) δ : 1.27 (6H, d, J=6.8Hz), 2.37 (3H, s), 3.05 (1H, m), 5.35 (2H, s), 7.33 (1H, dd, J=8.3, 2.0Hz), 7.44 (1H, t, J=8.3Hz), 7.84 (1H, m), 7.90 (1H, m)

### (E) 3-[(4-Isopropyl-5-methyl-1,3-thiazol-2-yl)methoxy]aniline

The [4-isopropyl-5-methyl-1,3-thiazol-2-yl]methyl (3-nitrophenyl) ether obtained in (D) (258 mg, 0.882 mmol) was dissolved in ethanol (10 ml), and the solution was added with stannous chloride (585 mg) and heated under reflux for 5 hours. The reaction mixture was left stand for cooling, and then added with 5 M aqueous sodium hydroxide (20 ml) and stirred for 15 minutes. The reaction mixture was extracted with ethyl acetate, and the organic layer was dried over magnesium sulfate. The solvent was evaporated under reduced pressure to obtain the title compound (311 mg, quantitative yield).

### (F) (4S,5S)-5-(Methoxycarbonyl)-2,2-dimethyl-1,3-dioxolane-4-carboxylic acid

Dimethyl (4S,5S)-2,2-dimethyl-1,3-dioxolane-4,5-dicarboxylate (3.00 g, 13.7 mmol) was dissolved in methanol (100 ml), and the solution was added dropwise with 1 N aqueous sodium hydroxide (13.7 ml, 13.7 mmol) and stirred at room temperature for 16 hours. The solvent was evaporated, and the residue was added with a small amount of water. The mixture was washed with ether, and adjusted to about pH 1 with 1 N aqueous hydrochloric acid. The mixture was extracted with ethyl acetate, washed with saturated brine, and dried over anhydrous sodium sulfate. The solvent was evaporated to obtain the title compound containing the dicarboxylic acid as colorless oil (2.63 g, 93.7%).
¹H-NMR (CDCl₃) δ : 1.52 (6H, d, J=8.3Hz), 3.85 (3H, s), 4.83 (1H, d, J=5.4Hz), 4.89 (1H, d, J=5.4Hz), 7.27 (1H, br)

### (G) Methyl (4S,5S)-5-(3-[(4-isopropyl-5-methyl-1,3-thiazol-2-yl)methoxy]anilino-carbonyl)-2,2-dimethyl-1,3-dioxolane-4-carboxylate

The 3-[(4-isopropyl-5-methyl-1,3-thiazol-2-yl)methoxy]aniline obtained in (E) (159 mg, 0.61 mmol) was dissolved in methylene chloride (10 ml), and the solution was added with the (4S,5S)-5-(methoxycarbonyl)-2,2-dimethyl-1,3-dioxolane-4-carboxylic acid obtained in (F) (186 mg, 0.91 mmol), and then added dropwise with diisopropylethylamine (254 µl, 1.46 mmol) under ice cooling. The reaction mixture was further added with N,N-bis-(2-oxo-3-oxazolidinyl)phosphinic acid chloride (186 mg, 0.73 mmol), and stirred at the same temperature for 10 minutes and then at room temperature for 20 hours. The solvent was evaporated, and the residue was added with water and extracted with chloroform. The organic layer was washed with 1 N aqueous hydrochloric acid, water, saturated aqueous sodium hydrogencarbonate, and then with saturated brine. The organic layer was dried over the anhydrous sodium sulfate, and the solvent was evaporated. The residue was purified by silica gel column chromatography (hexane:ethyl acetate = 10:1 → 5:1 → 2:1, v/v) to obtain the title compound as pale orange oil (139 mg, 51.2%).
¹H-NMR (CDCl₃) δ : 1.26 (6H, d, J=6.8Hz), 1.54 (3H, s), 1.56 (3H, s), 2.36 (3H, s), 2.99-3.10 (1H, m), 3.87 (3H, s), 4.85 (1H, d, J=5.4Hz), 4.88 (1H, d, J=5.4Hz), 5.27 (2H, s), 6.79 (1H, dd, J=7.8, 2.4Hz), 7.15 (1H, d, J=7.8Hz), 7.25 (1H, t, J=7.8Hz), 7.36 (1H, t, J=2.4Hz), 8.24 (1H, s)

### (H) (4S,5S)-5-(3-[(4-Isopropyl-5-methyl-1,3-thiazol-2-yl)methoxy]anilinocarbonyl)-2,2-dimethyl-1,3-dioxolane-4-carboxylic acid

The methyl (4S,5S)-5-(3-[(4-isopropyl-5-methyl-1,3-thiazol-2-yl)methoxy]-anilinocarbonyl)-2,2-dimethyl-1,3-dioxolane-4-carboxylate obtained in (G) (139 mg, 0.31 mmol) was dissolved in THF:methanol (3:1, v/v, 4 ml), and the solution was added dropwise with a solution of lithium oxide monohydrate (14 mg, 0.34 mmol) in water (1 ml) under ice cooling, and then stirred at room temperature for 16 hours. After the solvent was evaporated, the residue was added with a small amount of water, and the mixture was washed with ether and adjusted to about pH 1 with 1 N aqueous hydrochloric acid. The mixture was extracted with ethyl acetate, and the organic layer was washed with saturated brine and dried over anhydrous sodium sulfate, and then the solvent was evaporated. The residue was added with hexane, and collected by filtration as powder. The powder was washed with hexane to obtain the title compound as light orange powder (98 mg, 73.0%).
¹H-NMR (DMSO-d₆) δ : 1.18 (6H, d, J=6.8Hz), 1.42 (6H, s), 2.34 (3H, s), 2.99-3.10 (1H, m), 4.72 (1H, d, J=5.4Hz), 4.77 (1H, d, J=5.4Hz), 5.28 (2H, s), 6.75-6.82 (1H, m), 7.20-7.30 (2H, m), 7.45 (1H, s), 10.21 (1H, s), 13.20 (1H, br)
EI/MS; m/z: 434 (M⁺)
FAB/MS; m/z: 435 (MH⁺)

### Example 29: (1R*,2R*)-2-(3-((E)-2-(4-Isopropyl-1,3-thiazol-2-yl)-1-ethenyl)anilino-carbonyl)cyclohexane-1-carboxylic acid (*)

### (A) Trans-cyclohexane-1,2-dicarboxylic acid anhydride

trans-Cyclohexane-1,2-dicarboxylic acid (5.00 g, 29.0 mmol) was dissolved in acetic anhydride (10 mL), and the solution was heated under reflux for 1 hour. The reaction mixture was concentrated under reduced pressure by azeotropy using toluene and ether. The residue was added with ether, and collected by filtration to obtain the title compound as colorless crystals (3.82 g, 85%).
¹H-NMR (DMSO-d₆) δ : 1.22-1.34 (2H, m), 1.47-1.61 (2H, m), 1.90-2.00 (2H, m), 2.28-2.32 (2H, m), 2.54-2.63 (2H, m)

### (B) (1R*,2R*)-2-(3-((E)-2-(4-Isopropyl-1,3-thiazol-2-yl)-1-ethenyl)anilinocarbonyl)-cyclohexane-1-carboxylic acid

A solution of 3-((E)-2-(4-isopropyl-1,3-thiazol-2-yl)-1-ethenyl)aniline (100 mg, 0.409 mmol) in toluene (5 mL) was added with the trans-cyclohexane-1,2-dicarboxylic acid anhydride obtained in (A) (63.1 mg, 0.409 mmol), and the mixture was stirred at room temperature for 2.5 hours. The solvent was concentrated under reduced pressure by azeotropy using ether, and then the residue was added with ether and collected by filtration to obtain the title compound as light yellow crystals (127 mg, 78%).
¹H-NMR (DMSO-d₆) δ : 1.20-1.39 (2H, m), 1.27 (6H, d, J=6.8Hz), 1.76 (2H, br), 1.90-2.05 (2H, m), 2.46-2.61 (2H, m), 3.04 (1H, septet, J=6.8Hz), 7.24 (1H, s), 7.28-7.42 (4H, m), 7.47 (1H, d, J=7.3Hz), 7.93 (1H, s), 10.00 (1H, s), 12.06 (1H, s)
EI-MS; m/z: 398 (M⁺)

### Example 30: (1S*,2R*)-2-(3-((E)-2-(4-Isopropyl-1,3-thiazol-2-yl)-1-ethenyl)anilino-carbonyl)cyclohexane-1-carboxylic acid (*)

A solution of 3-((E)-2-(4-isopropyl-1,3-thiazol-2-yl)-1-ethenyl)aniline (100 mg, 0.409 mmol) in toluene (5 mL) was added with cis-cyclohexane-1,2-dicarboxylic acid anhydride (63.1 mg, 0.409 mmol), and the mixture was stirred at room temperature for 2.5 hours. The solvent was evaporated under reduced pressure by azeotropy using ether, and then the residue was added with ether and collected by filtration to obtain the title compound as light yellow crystals (120 mg, 73%).
¹H-NMR (DMSO-d₆) δ : 1.21-1.47 (3H, m), 1.27 (6H, d, J=6.8Hz), 1.60-1.80 (3H, m), 1.97-2.18 (2H, m), 2.57-2.65 (1H, m), 2.93-3.00 (1H, m), 3.04 (1H, septet, J=6.8Hz), 7.24 (1H, s), 7.28-7.40 (4H, m), 7.46 (1H, d, J=8.3Hz), 7.93 (1H, s), 9.78 (1H, s), 11.97 (1H, s)
FAB-MS; m/z: 399 (MH⁺)

### Example 31: (1S*,2R*)-2-(3-((E)-2-(4-isopropyl-1,3-thiazol-2-yl)-1-ethenyl)anilino-carbonyl)cyclopropane-1-carboxylic acid (*)

### (A) Methyl (1S*,2R*)-2-chlorocarbonylcyclopropane-1-carboxylate

A solution of (1R*,2S*)-2-(methoxycarbonyl)cyclopropane-1-carboxylic acid (215 mg, 1.49 mmol) in methylene chloride (5 mL) was added with DMF (one drop) and oxalyl chloride (143 mL, 1.64 mmol) at 0°C, and the mixture was stirred at 0°C for 2 hours. The reaction mixture was concentrated under reduced pressure by azeotropy using chloroform to obtain the title compound not purified as colorless oil (241 mg).

### (B) Methyl (1S*,2R*)-2-(3-((E)-2-(4-isopropyl-1,3-thiazol-2-yl)-1-ethenyl)anilino-carbonyl)cyclopropane-1-carboxylate

A solution of 3-((E)-2-(4-isopropyl-1,3-thiazol-2-yl)-1-ethenyl)aniline (182 mg, 744 mmol) in methylene chloride (2 mL) was added with triethylamine (208 mL, 49 mmol) and a solution of the methyl (1S*,2R*)-2-chlorocarbonylcyclopropane-1-carboxylate (128 mg) obtained in (A) in methylene chloride (2 mL) at 0°C. The mixture was stirred at room temperature for 18 hours, and then added with 1 N aqueous hydrochloric acid and extracted with ethyl acetate. The organic layer was washed with saturated aqueous sodium hydrogencarbonate and saturated brine, dried over magnesium sulfate, and then concentrated under reduced pressure. The residue was purified by silica gel column chromatography (hexane:ethyl acetate, 1: 1, v/v) to obtain the title compound as light yellow oil (180 mg, 65%).
¹H-NMR (CDCl₃) δ : 1.33 (6H, d, J=6.8Hz), 1.42-1.50 (1H, m), 1.73 (1H, dd, J=6.8, 12.2Hz), 2.10-2.22 (2H, m), 3.11 (1H, septet, J=6.8Hz), 3.76 (3H, s), 6.80 (1H, s), 7.25-7.31 (4H, m), 7.47 (1H, d, J=7.3Hz), 7.70 (1H, s), 8.36 (1H, br)

### (C) (1S*,2R*)-2-(3-((E)-2-(4-Isopropyl-1,3-thiazol-2-yl)-1-ethenyl)anilinocarbonyl)-cyclopropane-1-carboxylic acid

A solution of the methyl (1S*,2R*)-2-(3-((E)-2-(4-isopropyl-1,3-thiazol-2-yl)-1-ethenyl)anilinocarbonyl)cyclopropane-1-carboxylate obtained in (B) (180 mg, 485 mmol) in THF (3 mL) and methanol (1 mL) was added with lithium hydroxide monohydrate (20.3 mg, 0.485 mmol) at 0°C, and the mixture was stirred at room temperature for 1.5 hours. The mixture was added with lithium hydroxide monohydrate (10.2 mg, 0.242 mmol) again at 0°C, and then stirred at room temperature for 30 minutes. The reaction mixture was concentrated under reduced pressure, and the residue was diluted with water, and washed with ether. The aqueous layer was made acidic with 5% aqueous citric acid, and then extracted with ethyl acetate. The organic layer was washed with 1 N aqueous hydrochloric acid and saturated aqueous sodium hydrogencarbonate. The aqueous layer was extracted with ethyl acetate, and the organic layers were combined, and washed with saturated brine. The organic layer was dried over magnesium sulfate, and concentrated under reduced pressure, and then the residue was added with ether and collected by filtration to obtain the title compound as a white solid (51.0 mg, 30%).
¹H-NMR (DMSO-d₆) δ : 1.20 (1H, br), 1.26 (6H, d, J=6.8Hz), 1.41 (1H, br), 1.86 (2H, t, J=7.3Hz), 3.30 (1H, septet, J=6.8Hz), 7.23 (1H, s), 7.27-7.34 (4H, m), 7.46 (1H, d, J=7.3Hz), 7.82 (1H, br), 11.68 (1H, br)
EI-MS; m/z: 356 (M⁺)

### Example 32: 2-(2-{2,4-Difluoro-5-[(4-isopropyl-1,3-thiazol-2-yl)methoxy]anilino}-2-oxoethyl)benzoic acid (*)

### (A) 2-[(2,4-Difluoro-5-nitrophenoxy)methyl]-4-isopropyl-1,3-thiazole

2,4-Difluoro-5-nitrophenol (Akama et al., Synthesis, 1447, (1997), 200 mg, 1.14 mmol) and 2-hydroxymethyl-4-isopropyl-1,3-thiazole (198 mg, 1.25 mmol) were dissolved in THF (6 ml), and the solution was added with triphenylphosphine (389 mg), added further with diethyl azodicarboxylate (0.234 ml) under argon atmosphere, and then stirred at room temperature for 2 hours. After the solvent was evaporated under reduced pressure, the residue was purified by silica gel chromatography (n-hexane:ethyl acetate, 10:1, v/v) to obtain the title compound as white crystals (313 mg, 87%).
¹H-NMR (CDCl₃) δ : 1.31 (6H, d, J=6.8Hz), 3.11 (1H, m), 5.44 (2H, s), 6.96 (1H, s), 7.08 (1H, t, J=10.3Hz), 7.98 (1H, t, J=8.3Hz)

### (B) 2,4-Difluoro-5-[(4-isopropyl-1,3-thiazol-2-yl)methoxy]aniline

The 2-[(2,4-difluoro-5-nitrophenoxy)methyl]-4-isopropyl-1,3-thiazole obtained in (A) (500 mg, 1.59 mmol) was dissolved in ethyl acetate (20 ml), and the solution was added with 10% palladium/carbon (100 mg) and stirred overnight at 40°C under hydrogen atmosphere. The catalyst was removed by filtration, and the mother liquid was concentrated under reduced pressure. The resulting residue was purified by silica gel chromatography (n-hexane:ethyl acetate, 4:1, v/v) to obtain the title compound (396 mg, 87%).

### (C) 2-(2-{2,4-Difluoro-5-[(4-isopropyl-1,3-thiazol-2-yl)methoxy]anilino}-2-oxoethyl)-benzoic acid

The 2,4-difluoro-5-[(4-isopropyl-1,3-thiazol-2-yl)methoxy]aniline obtained in (B) (396 mg, 1.39 mmol) and anhydrous homophthalic acid (225 mg) were dissolved in toluene (10 ml), and the mixture was heated under reflux for 1 hour. The mixture was further added with anhydrous homophthalic acid (90 mg), and heated under reflux for 12 hours. The deposited solid was collected by filtration to obtain the title compound as a white solid (383 mg, 62%).
¹H-NMR (DMSO-d₆) δ : 1.21 (6H, d, J=6.8Hz), 3.00 (1H, m), 4.12 (2H, s), 5.34 (2H, s), 7.30 (1H, s), 7.35-7.44 (3H, m), 7.52 (1H, t, J=7.3Hz), 7.88 (2H, m), 9.89 (1H, s)
EI-MS; m/z: 446 (M⁺)

### Example 34: 2-(2-{2-Chloro-5-[(4-isopropyl-1,3-thiazol-2-yl)methoxy]anilino}-2-oxoethyl)nicotinic acid (*)

### (A) tert-Butyl 2-(2-ethoxy-2-oxoethyl)nicotinate

2-(2-Ethoxy-2-oxoethyl)nicotinic acid (800 mg, 3.82 mmol, Ames et al., J. Chem. Soc. Perkins 1, 705, (1972)) was dissolved in toluene (15 ml), and the solution was slowly added dropwise with N,N-dimethylformamide di-tert-butylacetal (3.66 ml) with stirring at 80°C. After the mixture was stirred at 80°C for 1.5 hours, the mixture was further added with N,N-dimethylformamide di-tert-butylacetal (0.915 ml), and stirred for 30 minutes. The reaction mixture was left stand for cooling, and then diluted with ethyl acetate and washed with saturated aqueous sodium hydrogencarbonate and saturated brine. After the mixture was dried over magnesium sulfate, the solvent was evaporated under reduced pressure, and the residue was purified by silica gel chromatography (chloroform:methanol, 100:1, v/v) to obtain the title compound (736 mg, 73%).
¹H-NMR (CDCl₃) δ : 1.25 (3H, t, J=7.3Hz), 1.58 (9H, s), 4.18 (2H, q, J=7.3Hz), 4.27 (2H, s), 7.30 (1H, dd, J=7.8, 4.6Hz), 8.22 (1H, dd, J=7.8, 1.5Hz), 8.64 (1H, dd, J=4.6, 1.5Hz)

### (B) 2-[3-(tert-Butoxycarbonyl)-2-pyridyl]acetic acid

The tert-butyl 2-(2-ethoxy-2-oxoethyl)nicotinate obtained in (A) (736 mg, 2.77 mmol) was dissolved in a mixed solvent of THF, methanol and water (17 ml, 10:5:2, v/v), and the solution was added with lithium hydroxide monohydrate (128 mg) and stirred overnight at room temperature. The reaction mixture was neutralized with 10% aqueous citric acid, and then extracted with ethyl acetate. The organic layer was dried over magnesium sulfate, and then the solvent was evaporated under reduced pressure to obtain the title compound as white crystals (516 mg, 78%).

### (C) 2-[(4-Chloro-3-nitrophenoxy)methyl]-4-isopropyl-1,3-thiazole

4-Chloro-3-nitrophenol (500 mg, 2.88 mmol) was dissolved in DMF, and the solution was added with potassium carbonate (796 mg) and 2-chloromethyl-4-isopropyl-1,3-thiazole (557 mg), and stirred overnight at room temperature. The reaction mixture was concentrated under reduced pressure, and the resulting residue was diluted with ethyl acetate, and washed with saturated brine. After the mixture was dried over magnesium sulfate, the solvent was evaporated under reduced pressure, and the residue was purified by silica gel chromatography (n-hexane:ethyl acetate, 12: 1, v/v) to obtain the title compound (767 mg, 85%).
¹H-NMR (CDCl₃) δ : 1.32 (6H, d, J=6.8Hz), 3.10 (1H, m), 5.37 (2H, s), 6.93 (1H, s), 7.17 (1H, dd, J=8.8, 2.9Hz), 7.44 (1H, d, J=9.3Hz), 7.57 (1H, d, J=2.9Hz)

### (D) 2-Chloro-5-[(4-isopropyl-1,3-thiazol-2-yl)methoxy]aniline

The 2-[(4-chloro-3-nitrophenoxy)methyl]-4-isopropyl-1,3-thiazole obtained in (C) (767 mg, 2.45 mmol) was dissolved in ethanol (15 ml), and the solution was added with stannous chloride (1628 mg) and heated under reflux overnight. The reaction mixture was left stand for cooling, and then added with 5 M aqueous sodium hydroxide (20 ml) and stirred for 15 minutes. The reaction mixture was extracted with ethyl acetate, and the organic layer was dried over magnesium sulfate. The solvent was evaporated under reduced pressure to obtain the title compound (647 mg, 93%).

### (E) tert-Butyl 2-(2-{2-chloro-5-[(4-isopropyl-1,3-thiazol-2-yl)methoxy]anilino}-2-oxo-ethyl)nicotinate

The 2-chloro-5-[(4-isopropyl-1,3-thiazol-2-yl)methoxy]aniline obtained in (D) (110 mg, 388 mmol) and the 2-[3-(tert-butoxycarbonyl)-2-pyridyl]acetic acid obtained in (B) (101 mg, 0.426 mmol) were dissolved in methylene chloride (3 ml), and the mixture was added with diisopropylethylamine (0.101 ml) and N,N-bis(2-oxo-3-oxazolidinyl)phosphinic acid chloride (128 mg), and stirred overnight at room temperature. The reaction mixture was further added with diisopropylethylamine (0.253 ml) and N,N-bis(2-oxo-3-oxazolidinyl)phosphinic acid chloride (320 mg), and then stirred overnight. The reaction mixture was diluted with chloroform, and washed with saturated aqueous sodium hydrogencarbonate and saturated brine. After the reaction mixture was dried over magnesium sulfate, the solvent was evaporated under reduced pressure, and the residue was purified by silica gel chromatography (n-hexane:ethyl acetate, 5: 1, v/v) to obtain the title compound (71.5 mg, 37%).
¹H-NMR (CDCl₃) δ : 1.30 (6H, d, J=6.8Hz), 1.62 (9H, s), 3.10 (1H, m), 4.36 (2H, s), 5.29 (2H, s), 6.65 (1H, dd, J=8.8, 2.9Hz), 6.88 (1H, s), 7.21 (1H, d, J=8.8Hz), 7.32 (1H, dd, J=7.8, 4.9Hz), 8.20 (1H, dd, J=7.8, 1.5Hz), 8.28 (1H, d, J=2.9Hz), 8.73 (1H, dd, J=4.9, 1.5Hz), 9.51 (s, 1H)

### (F) 2-(2-{2-Chloro-5-[(4-isopropyl-1,3-thiazol-2-yl)methoxy]anilino}-2-oxoethyl)-nicotinic acid

The tert-butyl 2-(2-{2-chloro-5-[(4-isopropyl-1,3-thiazol-2-yl)methoxy]anilino}-2-oxoethyl)nicotinate obtained in (E) (71.5 mg, 0.142 mmol) was dissolved in chloroform (1 ml), the solution was added with trifluoroacetic acid (1 ml) and stirred at room temperature for 24 hours. The reaction mixture was concentrated under reduced pressure, and the residue was added with diethyl ether. The deposited crystals were collected by filtration, and dried to obtain ditrifluoroacetate of the title compound as white powder (46.3 mg, 48%).
¹H-NMR (DMSO-d₆) δ : 1.23 (6H, d, J=6.8Hz), 3.02 (1H, m), 4.36 (2H, s), 5.35 (2H, s), 6.88 (1H, dd, J=8.8, 2.4Hz), 7.29 (1H, s), 7.41 (1H, d, J=8.8Hz), 7.46 (1H, dd, J=7.8, 4.9Hz), 7.62 (1H, br), 8.25 (1H, d, J=7.8Hz), 8.68 (1H, d, J=4.9Hz), 9.74 (1H, s)

### Example 35: N1-3-[(E)-2-(4-Isopropyl-1,3-thiazol-2-yl)-1-ethenyl]phenyl-2-(2-[(trifluoromethyl)sulfonyl]aminophenyl)acetamide

### (A) Methyl 2-(2-nitrophenyl)acetate (*)

2-(2-Nitrophenyl)acetic acid (5.06 g, 27.9 mmol) was dissolved in methanol (100 ml), and the solution was added dropwise with concentrated sulfuric acid (5 ml) and heated under reflux for 16 hours. After the solvent was evaporated, the residue was added with water and then extracted with ethyl acetate. The organic layer was washed with saturated aqueous sodium hydrogencarbonate and saturated brine, and dried over anhydrous sodium sulfate. The solvent was evaporated to obtain the title compound as pale orange oil (5.37 g, 99.0%).
¹H-NMR (CDCl₃) δ : 3.72 (3H, s), 4.03 (2H, s), 7.36 (1H, d, J=7.3Hz), 7.44-7.51 (1H, m), 7.57-7.63 (1H, m), 8.12 (1H, d, J=7.8Hz)

### (B) Methyl 2-(2-aminophenyl)acetate

The methyl 2-(2-nitrophenyl)acetate obtained in (A) (1.69 g, 8.66 mmol) was dissolved in methanol (20 ml). 10% palladium/carbon (0.17 g) was suspended in the solution, and the suspension was stirred at room temperature under hydrogen atmosphere (1 atm) for 19 hours. The catalyst was removed by filtration and washed with methanol, and then the solvent was evaporated to obtain the title compound as pale orange oil (1.33 g, 93.0%).
¹H-NMR (CDCl₃) δ : 3.57 (2H, s), 3.68 (3H, s), 4.06 (2H, br), 6.67-6.79 (2H, m), 7.04-7.12 (2H, m)

### (C) Methyl 2-(2-[(trifluoromethyl)sulfonyl]aminophenyl)acetate

The methyl 2-(2-aminophenyl)acetate obtained in (B) (1.32 g, 7.99 mmol) was dissolved in methylene chloride (50 ml), and the solution was added dropwise with triethylamine (1.23 ml, 8.79 mmol) under ice cooling, and slowly added dropwise with trifluoroacetic anhydride (1.34 ml, 7.99 mmol). After the reaction mixture was stirred for 15 minutes at the same temperature, the organic layer was separated, and the layer was washed with 10% aqueous citric acid, saturated aqueous sodium hydrogencarbonate and then with saturated brine, and dried over anhydrous sodium sulfate. The solvent was evaporated to obtain the title compound as pale orange oil (1.41 g, 59.4%).
¹H-NMR (CDCl₃) δ : 3.75 (2H, s), 3.77 (3H, s), 7.17-7.45 (3H, m), 7.48-7.65 (1H, m)

### (D) 2-(2-[(Trifluoromethyl)sulfonyl]aminophenyl)acetic acid

The methyl 2-(2-[(trifluoromethyl)sulfonyl]aminophenyl)acetate obtained in (C) (1.41g, 4.74 mmol) was dissolved in THF:methanol (3:1, v/v, 80 ml), and the solution was added dropwise with a solution of lithium hydroxide monohydrate (218 mg, 5.22 mmol) in water (20 ml) under ice cooling, and stirred at room temperature for 3 hours. The reaction mixture was further added with a solution of lithium hydroxide monohydrate (218 mg, 5.22 mmol) in water (20 ml) under ice cooling, and stirred at room temperature for 17 hours. After the solvent was evaporated, the residue was added with a small amount of water and washed with ether, and then the mixture was adjusted to about pH 1 with 1 N aqueous hydrochloric acid and extracted with ethyl acetate. The organic layer was washed with saturated brine, and dried over anhydrous sodium sulfate, and the solvent was evaporated to obtain the title compound as pale orange oil (1.35 g, quantitative).
¹H-NMR (CDCl₃) δ : 3.79 (2H, s), 5.61 (1H, br), 7.23-7.46 (3H, m), 7.51 (1H, d, J=7.8Hz), 8.50 (1H, br)

### (E) N1-3-[(E)-2-(4-isopropyl-1,3-thiazol-2-yl)-1-ethenyl]phenyl-2-(2-[(trifluoromethyl)sulfonyl]aminophenyl)acetamide

The 2-(2-[(trifluoromethyl)sulfonyl]aminophenyl)acetic acid obtained in (D) (515 mg, 1.82 mmol) was added dropwise with thionyl chloride (663 ml, 9.09 mmol) under ice cooling, and the mixture was stirred for 10 minutes at the same temperature, and further stirred for 30 minutes at room temperature. The solvent was evaporated, and the residue was dissolved in methylene chloride (20 ml). The solution was added dropwise with a solution of 3-((E)-2-(4-isopropyl-1,3-thiazol-2-yl)-1-ethenyl)aniline (222 mg, 0.91 mmol) in methylene chloride (10 ml) and the triethylamine (1.27 ml, 9.09 mmol) under ice cooling, and added further with dimethylaminopyridine (222 mg, 1.82 mmol). The reaction mixture was stirred at the same temperature for 30 minutes and at room temperature for 20 hours, and then the solvent was evaporated. The residue was added with water, and extracted with chloroform. The organic layer was washed successively with 1 N aqueous hydrochloric acid, water, saturated aqueous sodium hydrogencarbonate, and then with saturated brine, and dried over anhydrous sodium sulfate, and then, the solvent was evaporated. The residue was purified by silica gel column chromatography (chloroform → chloroform:methanol = 50:1, v/v). The solvent was evaporated, and the residue was added with hexane and collected by filtration as powder. The powder was washed with hexane to obtain the title compound as pale orange powder (98 mg, 21.2%).
¹H-NMR (DMSO-d₆) δ : 1.26 (6H, d, J=6.8Hz), 2.97-3.09 (1H, m), 3.85 (2H, s), 7.23-7.44 (10H, m), 7.50 (1H, d, J=7.8Hz), 7.92 (1H, br), 10.41 (1H, br)
EI/MS; m/z: 509 (M⁺)
FAB/MS; m/z: 510 (MH⁺)

### Example 36: 1-Benzyl-4-((3-((E)-2-(4-isopropyl-1,3-thiazol-2-yl)-1-ethenyl)anilino)-carbonyl)-1H-3-pyrrolecarboxylic acid (*)

### (A) Dimethyl 1-benzyl-2,5-dihydro-1H-3,4-pyrroledicarboxylate

A solution of N-benzyl-N-butoxymethyl-N-[(1,1,1-trimethylsilyl)methyl]amine (803.7 mg) and dimethyl acetylenedicarboxylate (0.353 ml) in methylene chloride (10 ml) was added with trifluoroacetic acid (22 µl) at room temperature, and the mixture was stirred overnight at the same temperature. The reaction mixture was added with chloroform, washed with saturated aqueous sodium hydrogencarbonate and saturated brine, and then dried over anhydrous sodium sulfate. The solvent was evaporated under reduced pressure, and the resulting residue was purified by silica gel chromatography (chloroform:methanol = 99.5:0.5, v/v) to obtain dimethyl 1-benzyl-2,5-dihydro-1H-3,4-pyrroledicarboxylate as a yellow oil (536.9 mg, 68%).
¹H-NMR (CDCl₃) δ : 3.77 (6H, s), 3.80 (2H, s), 3.83 (4H, s), 7.32-7.34 (5H, m)

### (B) 1-Benzyl-4-(methoxycarbonyl)-2,5-dihydro-1H-3-pyrrolecarboxylic acid

A solution of dimethyl 1-benzyl-2,5-dihydro-1H-3,4-pyrroledicarboxylate (148 mg) in methanol (3 ml) was added with 1 N aqueous sodium hydroxide (0.539 ml) at room temperature, and the mixture was stirred at the same temperature for 2.5 hours. The solvent was evaporated, and the resulting residue was added with 1 N hydrochloric acid (0.539 ml) and then added with toluene, and evaporated under reduced pressure. The resulting residue was added with chloroform and anhydrous sodium sulfate, and an insoluble matter was removed by filtration. The solvent was evaporated to obtain 1-benzyl-4-(methoxycarbonyl)-2,5-dihydro-1H-3-pyrrole-carboxylic acid as a pale yellow solid (119.5 mg, 85%).
¹H-NMR (CDCl₃) δ : 3.79 (2H, s), 3.89 (3H, s), 3.85-3.92 (2H, m), 3.95-4.00 (2H, m), 7.20-7.40 (5H, m)

### (C) Methyl 1-benzyl-4-(3-[(E)-2-(4-isopropyl-1,3-thiazol-2-yl)-1-ethenyl]anilino-carbonyl)-2,5-dihydro-1H-3-pyrrolecarboxylate

A solution of 1-benzyl-4-(methoxycarbonyl)-2,5-dihydro-1H-3-pyrrole-carboxylic acid (117.4 mg) and 3-[(E)-2-(4-isopropyl-1,3-thiazol-2-yl)-1-ethenyl]aniline (120.8 mg) in methylene chloride (5 ml) was added with dicyclohexylcarbodiimide (102.0 mg) and N,N-dimethylaminopyridine (60.4 mg) at room temperature, and the mixture was stirred overnight at the same temperature. After the insoluble solids were removed by filtration, the solvent was evaporated. The resulting residue was dissolved in ethyl acetate, and the insoluble solids were removed by filtration. The solvent was evaporated, and the resulting residue was purified by silica gel chromatography (hexane:ethyl acetate = 3:1, v/v) to obtain methyl 1-benzyl-4-(3-[(E)-2-(4-isopropyl-1,3-thiazol-2-yl)-1-ethenyl]anilinocarbonyl)-2,5-dihydro-1H-3-pyrrolecar boxylate as yellow oil (107.1 mg, 49%).
¹H-NMR (CDCl₃) δ : 1.33 (6H, d, J=6.8Hz), 3.05-3.20 (1H, m), 3.80 (2H, s), 3.86 (3H, s), 3.91 (2H, t, J=4.9Hz), 4.06 (2H, t, J=4.9Hz), 7.20-7.40 (9H, m), 7.67 (1H, d, J=7.8Hz), 7.85 (1H, s), 11.65 (1H, s)

### (D) 1-Benzyl-4-((3-((E)-2-(4-isopropyl-1,3-thiazol-2-yl)-1-ethenyl)anilino)carbonyl)-1H-3-pyrrolecarboxylic acid

A solution of methyl 1-benzyl-4-(3-[(E)-2-(4-isopropyl-1,3-thiazol-2-yl)-1-ethenyl]anilinocarbonyl)-2,5-dihydro-1H-3-pyrrolecarboxylate (106.7 mg) in a mixture of methanol (2 ml) and THF (1 ml) was added with an aqueous solution (0.3 ml) of lithium hydroxide monohydrate (9.6 mg) at room temperature, and then the mixture was stirred at the same temperature for 7 hours. The reaction mixture was further added with an aqueous solution (0.5 ml) of lithium hydroxide monohydrate (9.2 mg), and stirred at room temperature for 16 hours. The mixture was added with an aqueous solution (0.3 ml) of lithium hydroxide monohydrate (4.6 mg), and stirred overnight at the same temperature. The solvent was evaporated, and the resulting residue was added with 1 N hydrochloric acid (0.558 ml). The deposited solid was purified by silica gel chromatography (chloroform:methanol = 96:4 → 92:8, v/v) to obtain 1-benzyl-4-((3-((E)-2-(4-isopropyl-1,3-thiazol-2-yl)-1-ethenyl)anilino)carbonyl)-1H-3-pyrrolecarboxylic acid as a colorless solid (32.3 mg, 31%).
¹H-NMR (DMSO-d₆) δ : 1.27 (6H, d, J=6.8Hz), 3.04 (1H, quint., J=6.8Hz), 5.23 (2H, s), 7.24 (1H, s), 7.30-7.45 (9H, m), 7.58 (1H, d, J=7.8Hz), 7.65-7.75 (2H, m), 7.92 (1H, s)
FAB-MS; m/z: 472 (MH⁺)
Elemental Analysis (as C₂₇H₂₅N₃O₃S•0.4H₂O):
Calculated: C, 67.73; H, 5.43; N, 8.78
Found: C, 67.91; H, 5.49; N, 8.47

### Example 37: 4-((3-((E)-2-(4-Isopropyl-1,3-thiazol-2-yl)-1-ethenyl)anilino)carbonyl)-3-furoic acid (*)

### (A) 4-(Methoxycarbonyl)-3-furoic acid

A solution of commercially available dimethyl 3,4-frandicarboxylate (504.1 mg) in methanol (3 ml) was added with 1 N aqueous sodium hydroxide (3.01 ml) at room temperature, and the mixture was stirred at the same temperature for 3 hours. The solvent was evaporated, and the resulting residue was added with water, and washed with diethyl ether. The aqueous layer was made acidic by using 1 N hydrochloric acid, and extracted with ethyl acetate. The organic layer was dried over anhydrous sodium sulfate, and the solvent was evaporated to obtain 4-(methoxycarbonyl)-3-furoic acid as a colorless solid (379.8 mg, 82%).
¹H-NMR (CDCl₃) δ : 4.01 (3H, s), 8.13 (1H, d, J=2.0Hz), 8.25 (1H, d, J=2.0Hz)

### (B) Methyl 4-(3-[(E)-2-(4-isopropyl-1,3-thiazol-2-yl)-1-ethenyl]anilinocarbonyl)-3-furoate

A solution of 4-(methoxycarbonyl)-3-furoic acid (101.1 mg) and 3-[(E)-2-(4-isopropyl-1,3-thiazol-2-yl)-1-ethenyl]aniline (132.0 mg) in methylene chloride (5 ml) was added with dicyclohexylcarbodiimide (122.6 mg) and N,N-dimethylaminopyridine (72.6 mg) at room temperature, and the mixture was stirred at the same temperature for 18 hours. The insoluble solids were removed by filtration, and the solvent was evaporated. The resulting residue was dissolved in ethyl acetate, and the insoluble solids were removed by filtration. The solvent was evaporated, and the resulting residue was purified by silica gel chromatography (chloroform → chloroform:methanol = 99:1, v/v) to obtain methyl 4-(3-[(E)-2-(4-isopropyl-1,3-thiazol-2-yl)-1-ethenyl]anilinocarbonyl)-3-furoate as a light yellow solid (165.3 mg, 77%).
¹H-NMR (CDCl₃) δ: 1.34 (6H, d, J=6.8Hz), 3.12 (1H, quint., J=6.8Hz), 3.99 (3H, s), 6.81 (1H, s), 7.20-7.30 (1H, m), 7.30-7.40 (3H, m), 7.77 (1H, d, J=7.8Hz), 7.91 (1H, s), 8.14 (1H, d, J=2.0Hz), 8.28 (1H, d, J=2.0Hz)

### (C) 4-((3-((E)-2-(4-Isopropyl-1,3-thiazol-2-yl)-1-ethenyl)anilino)carbonyl)-3-furoic acid

A solution of methyl 4-(3-[(E)-2-(4-isopropyl-1,3-thiazol-2-yl)-1-ethenyl]-anilinocarbonyl)-3-furoate (162.1 mg) in a mixture of methanol (2 ml) and THF (1.5 ml) was added with an aqueous solution (0.6 ml) of lithium hydroxide monohydrate (19.3 mg) at room temperature, and the mixture was stirred at the same temperature for 2 hours. The solvent was evaporated, and the resulting residue was added with 1 N hydrochloric acid (0.46 ml), neutralized with 1 N aqueous sodium hydroxide, and then extracted with chloroform and ethyl acetate. The organic layer was dried over anhydrous sodium sulfate, and the solvent was evaporated. The resulting residue was collected by filtration using diethyl ether to obtain 4-((3-((E)-2-(4-isopropyl-1,3-thiazol-2-yl)-1-ethenyl)anilino)carbonyl)-3-furoic acid as a colorless solid (116.1 mg, 74%).
¹H-NMR (DMSO-d₆) δ : 1.27 (6H, d, J=6.8Hz), 3.05 (1H, quint., J=6.8Hz), 7.25 (1H, s), 7.35-7.45 (4H, m), 7.62 (1H, d, J=7.8Hz), 7.94 (1H, s), 8.20-8.35 (2H, m)
FAB-MS; m/z: 383 (MH⁺)
Elemental Analysis (as C₂₀H₁₈N₂O₄S•H₂O):
Calculated: C, 59.99; H, 5.03; N, 7.00
Found: C, 60.26; H, 4.68; N, 6.88

### Example 38: 3-((3-((E)-2-(4-Isopropyl-1,3-thiazol-2-yl)-1-ethenyl)anilino)carbonyl)-5-methyl-4-isoxazolecarboxylic acid (*)

### (A) 4-(tert-Butyl) 3-ethyl 5-methyl-3,4-isoxazoledicarboxylate

A solution of tert-butyl acetoacetate (500.0 mg) and ethyl cyanoformate (0.375 ml) in methylene chloride (10 ml) was added with zinc acetylacetonate (16.7 mg) at room temperature under nitrogen flow, and the mixture was stirred at the same temperature for three days. The solvent was evaporated, and the resulting residue was added with ethyl acetate. The insoluble solids were removed by filtration through Celite. The solvent was evaporated, and the resulting residue was purified by silica gel chromatography (chloroform). The resulting solution of the enamine in chloroform (6 ml) was added with hydroxylamine hydrochloride (113.8 mg) and triethylamine (0.228 ml), and the mixture was stirred at room temperature under nitrogen flow for 15 hours. The reaction mixture was added with 5% aqueous citric acid, and then extracted with chloroform. The organic layer was dried over anhydrous sodium sulfate, and then the solvent was evaporated to obtain 4-(tert-butyl) 3-ethyl 5-methyl-3,4-isoxazoledicarboxylate as light yellow oil (242.0 mg, 30%).
¹H-NMR (CDCl₃) δ : 1.41 (3H, t, J=7.3Hz), 1.54 (9H, s), 2.67 (3H, s), 4.43 (2H, q, J=7.3Hz)

### (B) 4-(tert-Butoxycarbonyl)-5-methyl-3-isoxazolecarboxylic acid

A solution of 4-(tert-butyl) 3-ethyl 5-methyl-3,4-isoxazoledicarboxylate (125.4 mg) in THF (3 ml) was added with an aqueous solution (0.3 ml) of lithium hydroxide monohydrate (9.6 mg) at room temperature, and the mixture was stirred at the same temperature for 7 hours. The reaction mixture was further added with an aqueous solution (0.5 ml) of lithium hydroxide monohydrate (22.7 mg), and stirred at the same temperature for 1.5 hours. The solvent was evaporated, and the resulting residue was added with 5% aqueous citric acid, and then extracted with ethyl acetate. The organic layer was dried over anhydrous sodium sulfate, and the solvent was evaporated to obtain 4-(tert-butoxycarbonyl)-5-methyl-3-isoxazolecarboxylic acid as yellow oil (86.7 mg, 78%).
¹H-NMR (CDCl₃) δ : 1.55 (9H, s), 2.31 (3H, s)

### (C) tert-Butyl 3-(3-[(E)-2-(4-isopropyl-1,3-thiazol-2-yl)-1-ethenyl]anilinocarbonyl)-5-methyl-4-isoxazolecarboxylate

A solution of 4-(tert-butoxycarbonyl)-5-methyl-3-isoxazolecarboxylic acid (86.7 mg) and 3-[(E)-2-(4-isopropyl-1,3-thiazol-2-yl)-1-ethenyl]aniline (93.2 mg) in a mixture of methylene chloride (5 ml) and DMF (0.3 ml) was added with dicyclohexylcarbodiimide (86.6 mg) and N,N-dimethylaminopyridine (51.3 mg) at room temperature, and the mixture was stirred at the same temperature for two days. The solvent was evaporated, and the resulting residue was dissolved in ethyl acetate, and washed with water and saturated brine. After the reaction mixture was dried over anhydrous sodium sulfate, the solvent was evaporated, and the resulting residue was purified by silica gel chromatography (hexane:ethyl acetate = 85:15, v/v) to obtain tert-butyl
3-(3-[(E)-2-(4-isopropyl-1,3-thiazol-2-yl)-1-ethenyl]anilinocarbonyl)-5-methyl-4-isoxazo lecarboxylate as light yellow oil (16.3 mg, 9%)
¹H-NMR (CDCl₃) δ : 1.34 (6H, d, J=6.8Hz), 1.69 (9H, s), 2.52 (3H, s), 3.13 (1H, dq, J=6.8, 0.5Hz), 6.82 (1H, s), 7.30-7.45 (4H, m), 7.77 (1H, br d, J=7.6Hz), 7.91 (1H, d, J=1.5Hz), 12.05, (1H, s)

### (D) 3-((3-((E)-2-(4-Isopropyl-1,3-thiazol-2-yl)-1-ethenyl)anilino)carbonyl)-5-methyl-4-isoxazolecarboxylic acid

A solution of tert-butyl 3-(3-[(E)-2-(4-isopropyl-1,3-thiazol-2-yl)-1-ethenyl]-anilinocarbonyl)-5-methyl-4-isoxazolecarboxylate (16.3 mg) in dioxane (0.5 ml) was added with 4 N hydrochloric acid in dioxane (1 ml) at room temperature, and the mixture was stirred at the same temperature for 4.5 hours. The solvent was evaporated, and the resulting residue was added with dioxane. The solvent was evaporated, and the residue was collected by filtration using diethyl ether to obtain 3-((3-((E)-2-(4-isopropyl-1,3-thiazol-2-yl)-1-ethenyl)anilino)carbonyl)-5-methyl-4-isoxa zolecarboxylic acid as a brown solid (7.6 mg, 49%).
¹H-NMR (DMSO-d₆) δ : 1.27 (6H, d, J=6.8Hz), 2.46 (3H, s), 3.05 (1H, quint., J=6.8Hz), 7.27 (1H, s), 7.35-7.50 (3H, m), 7.50-7.60 (2H, m), 7.95 (1H, s), 11.44 (1H, s)
FAB-MS; m/z: 398 (MH⁺)

### Example 39: 4-((3-((E)-2-(4-Isopropyl-1,3-thiazol-2-yl)-1-ethenyl)anilino)carbonyl)-5-methyl-3-isoxazolecarboxylic acid (*)

### (A) 3-(Ethoxycarbonyl)-5-methyl-4-isoxazolecarboxylic acid

A solution of the 4-(tert-butyl) 3-ethyl 5-methyl-3,4-isoxazoledicarboxylate synthesized in Example 38 (A) (121.5 mg) in methylene chloride (1.5 ml) was added with trifluoroacetic acid (1.5 ml) under ice cooling, and the mixture was stirred at the same temperature for 2 hours. The solvent was evaporated, and the resulting residue was added with water, and the mixture was neutralized with 1 N aqueous sodium hydroxide. The mixture was added with 5% aqueous citric acid, and extracted with ethyl acetate under a salting out condition. The ethyl acetate layer was dried over anhydrous sodium sulfate, and the solvent was evaporated to obtain 3-(ethoxycarbonyl)-5-methyl-4-isoxazolecarboxylic acid as yellow amorphous (74.0 mg, 78%).
¹H-NMR (CDCl₃-CD₃OD) δ : 1.42 (3H, t, J=7.1Hz), 2.74 (3H, s), 4.48 (2H, q, J=7.1Hz)

### (B) Ethyl 4-((3-((E)-2-(4-isopropyl-1,3-thiazol-2-yl)-1-ethenyl)anilino)carbonyl)-5-methyl-3-isoxazolecarboxylate

A solution of 3-(ethoxycarbonyl)-5-methyl-4-isoxazolecarboxylic acid (74.0 mg) and 3-[(E)-2-(4-isopropyl-1,3-thiazol-2-yl)-1-ethenyl]aniline (90.8 mg) in a mixture of methylene chloride (5 ml) and DMF (0.5 ml) was added with dicyclohexylcarbodiimide (84.3 mg) and N,N-dimethylaminopyridine (49.9 mg) at room temperature, and the mixture was stirred at the same temperature for two days. The solvent was evaporated, and the resulting residue was dissolved in ethyl acetate, and washed with water and saturated brine. After the reaction mixture was dried over anhydrous sodium sulfate, the solvent was evaporated, and the resulting residue was purified by silica gel chromatography (hexane:ethyl acetate = 85:15, v/v) to obtain ethyl 4-((3-((E)-2-(4-isopropyl-1,3-thiazol-2-yl)-1-ethenyl)anilino)carbonyl)-5-methyl-3-isoxa zolecarboxylate as a colorless solid (76.0 mg, 48%).
¹H-NMR (CDCl₃) δ : 1.34 (6H, d, J=6.8Hz), 1.52 (3H, t, J=7.1Hz), 2.89 (3H, s), 3.12 (1H, quint., J=6.8Hz), 4.61 (2H, d, J=7.1Hz), 6.81 (1H, s), 7.25-7.40 (3H, m), 7.67 (1H, br d, J=7.8Hz), 7.90 (1H, d, J=1.7Hz), 11.15 (1H, s)

### (C) 4-((3-((E)-2-(4-Isopropyl-1,3-thiazol-2-yl)-1-ethenyl)anilino)carbonyl)-5-methyl-3-isoxazolecarboxylic acid

A solution of ethyl 4-((3-((E)-2-(4-isopropyl-1,3-thiazol-2-yl)-1-ethenyl)-anilino)carbonyl)-5-methyl-3-isoxazolecarboxylate (76.0 mg) in a mixture of methanol (1 ml) and THF (1.5 ml) was added with an aqueous solution (0.5 ml) of lithium hydroxide monohydrate (8.3 mg) at room temperature, and the mixture was stirred at the same temperature for 2 hours. The reaction mixture was added with 1 N hydrochloric acid (0.1 ml), and the solvent was evaporated. The resulting residue was added with chloroform and anhydrous sodium sulfate, and the insoluble solids were collected by filtration. The resulting insoluble solids were added with water and 1 N hydrochloric acid, and extracted with ethyl acetate. The ethyl acetate layer was dried over anhydrous sodium sulfate, and the solvent was evaporated. The resulting residue was collected by filtration using diethyl ether to obtain 4-((3-((E)-2-(4-isopropyl-1,3-thiazol-2-yl)-1-ethenyl)anilino)carbonyl)-5-methyl-3-isoxazolecarboxylic acid as a yellow solid (42.9 mg, 60%).
¹H-NMR (DMSO-d₆) δ : 1.27 (6H, d, J=6.9Hz), 2.70 (3H, s), 3.05 (1H, quint., J=6.9Hz), 7.25 (1H, m), 7.30-7.50 (4H, m), 7.56 (1H, d, J=7.3Hz), 7.95 (1H, s)
FAB-MS; m/z: 398 (MH⁺)
Elemental Analysis (as C₂₀H₁₉N₃O₄S•0.5H₂O•1HCl):
Calculated: C, 54.23; H, 4.78; N, 9.49
Found: C, 53.97; H, 4.71; N, 9.14

### Example 40: 2-(2-(3-((4-Isopropyl-1,3-thiazol-2-yl)methoxy)benzylamino)-2-oxoethyl)-benzoic acid (*)

### (A) Methyl 2-(3-hydroxyphenyl)acetate

A solution of 2-(3-hydroxyphenyl)acetic acid (2.50 g, 16.4 mmol) in toluene (30 mL) and methanol (10 mL) was added with trimethylsilyldiazomethane (2.0 M in hexane, 8.22 mL, 16.4 mmol) at 0°C, and the mixture was stirred at room temperature for 30 minutes. The reaction mixture was further added with trimethylsilyldiazomethane (2.0 M in hexane, 4.11 mL, 8.22 mmol) at 0°C, and stirred at room temperature for 15 minutes. The solvent was concentrated under reduced pressure, and the residue was purified by silica gel column chromatography (chloroform → chloroform:methanol, 10:1, v/v, and hexane → hexane:ethyl acetate, 10:1 → 5:1, v/v) to obtain the title compound as colorless oil (2.70 g, 99%).
¹H-NMR (CDCl₃) δ : 3.58 (2H, s), 3.70 (3H, s), 5.93 (1H, br), 6.74 (1H, dd, J=2.7, 8.1Hz), 6.77 (1H, s), 6.81 (1H, d, J=7.57Hz), 7.17 (1H, t, J=7.8Hz)

### (B) Methyl 3-((4-isopropyl-1,3-thiazol-2-yl)methoxy)phenylacetate

A solution of the methyl 2-(3-hydroxyphenyl)acetate obtained in (A) (2.70 g, 16.3 mmol), (4-isopropyl-1,3-thiazol-2-yl)methanol (2.56 g, 16.3 mmol), and triphenylphosphine (4.27 g, 16.3 mmol) in THF (50 mL) was added with diethyl azodicarboxylate (2.56 mL, 16.3 mmol) at -15°C. After the reaction mixture was stirred at room temperature for 3.5 hours, the solvent was concentrated under reduced pressure by azeotropy using toluene. The residue was added with toluene, and the insoluble solids were separated by filtration, and washed with toluene. The filtrate and the washing liquid were combined and concentrated under reduced pressure, and the residue was purified by silica gel column chromatography (hexane → hexane:ethyl acetate, 10:1 → 4:1, v/v) to obtain the title compound as colorless oil (3.78 g, 76%).
¹H-NMR (CDCl₃) δ : 1.32 (6H, d, J=6.8Hz), 3.11 (1H, septet, J=6.8Hz), 3.60 (2H, s), 3.69 (3H, s), 5.33 (2H, s), 6.90-6.94 (4H, m), 7.25 (1H, t, J=7.8Hz)

### (C) 3-((4-isopropyl-1,3-thiazol-2-yl)methoxy)phenylacetic acid

A solution of the methyl 3-((4-isopropyl-1,3-thiazol-2-yl)methoxy)phenylacetate obtained in (B) (3.78 g, 12.4 mmol) in THF (30 mL) and (10 mL) was added with a solution of lithium hydroxide monohydrate (520 mg, 12.4 mmol) in water (20 mL) at 0°C, and the mixture was stirred at room temperature for 1.5 hours. The reaction mixture was further added with lithium hydroxide monohydrate (260 mg, 6.19 mmol) at 0°C, and stirred at room temperature for 2 hours. The solvent was evaporated under reduced pressure, and the residue was diluted with 1 N aqueous sodium hydroxide, and washed with ether. The aqueous layer was made acidic with 1 N aqueous hydrochloric acid, and extracted with ethyl acetate. The ethyl acetate layer was washed with saturated aqueous sodium hydrogencarbonate. The aqueous layer was extracted with chloroform. The organic layers were combined, dried over magnesium sulfate, and then concentrated under reduced pressure to obtain the title compound as a white solid (2.34 g, 65%).
¹H-NMR (DMSO-d₆) δ : 1.25 (6H, d, J=6.8Hz), 3.03 (1H, septet, J=6.8Hz), 3.54 (2H, s), 5.36 (2H, s), 6.88 (1H, d, J=7.6Hz), 6.93-6.96 (2H, m), 7.24 (1H, t, J=7.8Hz), 7.29 (1H, s), 12.32 (1H, br)

### (D) tert-Butyl N-3-((4-isopropyl-1,3-thiazol-2-yl)methoxy)benzylcarbamate

A solution of the 3-((4-isopropyl-1,3-thiazol-2-yl)methoxy)phenyl acetic acid obtained in (C) (1.00 g, 3.43 mmol) in tert-butanol (20 mL) was added with triethylamine (574 mL, 4.12 mmol) and diphenylphosphoric acid azide (814 mL, 3.78 mmol), and the mixture was heated under reflux for 15.5 hours. The reaction mixture was concentrated under reduced pressure, and the residue was purified by silica gel column chromatography (chloroform → chloroform:methanol, 99:1, v/v, and hexane:ethyl acetate, 6:1 → 5:1, v/v) to obtain the title compound as colorless oil (203 mg, 16%).
¹H-NMR (CDCl₃) δ : 1.32 (6H, d, J=6.8Hz), 1.46 (9H, s), 3.11 (1H, septet, J=6.8Hz), 4.30 (2H, d, J=5.4Hz), 4.85 (1H, br), 5.33 (2H, s), 6.88-6.94 (4H, m), 7.25 (1H, t, J=7.8Hz)

### (E) 3-((4-Isopropyl-1,3-thiazol-2-yl)methoxy)benzylamine

The tert-butyl N-3-((4-isopropyl-1,3-thiazol-2-yl)methoxy)benzylcarbamate obtained in (D) (203 mg, 0.561 mmol) was added with 4 N hydrochloric acid in dioxane (5 mL) at 0°C. Since a uniform mixture was not obtained, the mixture was added with methanol (1 mL), and then stirred at room temperature for 15 minutes. The solvent was evaporated under reduced pressure by azeotropy using chloroform, and the residue was diluted with saturated aqueous sodium hydrogencarbonate, and extracted with chloroform. The organic layer was dried over magnesium sulfate, and concentrated under reduced pressure to obtain the title compound as light yellow oil (152 mg, quantitative).
¹H-NMR (CDCl₃) δ : 1.32 (6H, d, J=6.8Hz), 1.66 (2H, br), 3.11 (1H, septet, J=6.8Hz), 3.84 (2H, s), 5.34 (2H, s), 6.86-6.89 (2H, m), 6.94 (1H, d, J=7.6Hz), 6.99 (1H, br), 7.26 (1H, t, J=7.8Hz)

### (F) 2-(2-(3-((4-Isopropyl-1,3-thiazol-2-yl)methoxy)benzylamino)-2-oxoethyl)benzoic acid

A solution of (E)-3-((4-isopropyl-1,3-thiazol-2-yl)methoxy)benzylamine (52.1 mg, 0.199 mmol) in toluene (2 mL) was added with homophthalic anhydride (32.2 mg, 0.199 mmol), and the mixture was heated under reflux for 5 hours. The deposited solid was collected by filtration, and washed with toluene to obtain the title compound as white crystals (63.3 mg, 75%).
¹H-NMR (DMSO-d₆) δ : 1.25 (6H, d, J=6.8Hz), 3.04 (1H, septet, J=6.8Hz), 3.93 (2H, s), 4.25 (2H, d, J=5.9Hz), 5.35 (2H, s), 6.88-6.94 (3H, m), 7.24 (1H, t, J=7.7Hz), 7.30-7.38 (3H, m), 7.47-7.50 (1H, m), 7.84 (1H, d, J=7.6Hz), 8.37-8.40 (1H, m), 12.89 (1H, br)
EI-MS; m/z: 424 (M⁺)

### Example 41: 3-(3-[(E)-2-(4-Isopropyl-1,3-thiazol-2-yl)-ethyl]phenoxymethyl)benzoic acid (*)

### (A) 3-[(E)-2-(4-Isopropyl-1,3-thiazol-2-yl)-1-ethenyl]phenol

The diethyl [(4-isopropyl-1,3-thiazol-2-yl)methyl]phosphonate obtained in Example 15 (E) and 3-hydroxybenzaldehyde were treated in the same manner as in Example 15 (F) to obtain the title compound.
¹H-NMR (CDCl₃) δ: 1.34 (6H, d, J=6.6Hz), 3.13 (1H, heptuplet, J=6.6Hz), 6.79-6.82 (2H, m), 6.99 (1H, m), 7.08 (1H, d, J=7.5Hz), 7.22 (1H, d, J=7.8Hz), 7.27 (1H, d, J=8.4Hz), 7.29 (1H, s)

### (B) 3-(3-[(E)-2-(4-Isopropyl-1,3-thiazol-2-yl)-1-ethenyl]phenoxymethyl)benzonitrile

The 3-[(E)-2-(4-isopropyl-1,3-thiazol-2-yl)-1-ethenyl] phenol obtained in (A) (221 mg, 0.9 mmol) was dissolved in dimethylformamide (5 mL), and the solution was added with cesium carbonate (588 mg) and stirred for 20 minutes. The reaction mixture was added with 2-bromomethylbenzonitrile (195 mg), and stirred for 16 hours. The reaction mixture was distributed in ethyl acetate and water, and the organic layer was washed with saturated brine, and dried over sodium sulfate. The solvent was evaporated under reduced pressure to obtain the title compound as an oil (320 mg). The compound was used for the next reaction without purification.
¹H-NMR (CDCl₃) δ : 1.34 (6H, d, J=6.9Hz), 3.12 (1H, heptuplet, J=6.9Hz), 5.12 (2H, s), 6.82 (1H, s), 6.92 (1H, d, J=7.8Hz), 7.12 (1H, s), 7.17 (1H, d, J=7.8Hz), 7.31 (3H, m), 7.51 (1H, t, J=8.1Hz), 7.63 (1H, d, J=8.1), 7.68 (1H, d, J=8.1Hz), 7.76 (1H, s)
MS (ES+); m/z: 361 (MH⁺)

### (C) 3-(3-[(E)-2-(4-Isopropyl-1,3-thiazol-2-yl)-ethyl]phenoxymethyl)benzoic acid

The 3-(3-[(E)-2-(4-isopropyl-1,3-thiazol-2-yl)-1-ethenyl]phenoxymethyl)benzonitrile obtained in (B) (50 mg) was dissolved in ethanol (1 mL) and 2 N aqueous sodium hydroxide (1 mL), and the mixture was heated under reflux for 2 hours. After the reaction mixture was cooled, the mixture was made acidic with 1 N hydrochloric acid, and extracted with ethyl acetate. The ethyl acetate layer was dried over sodium sulfate, and the solvent was evaporated under reduced pressure. The residue was purified by silica gel chromatography (methylene chloride:methanol, 99: 1, v/v) to obtain the title compound as white powder (14 mg).
¹H-NMR (DMSO-d₆) δ : 1.34 (6H, d, J=6.9Hz), 3.13 (1H, heptuplet, J=6.9Hz), 5.14 (2H, s), 6.18 (1H, s), 6.95 (1H, d, J=7.5Hz), 7.15 (2H, m), 7.26-7.34 (3H, m), 7.51 (1H, t, J=8.4Hz), 7.71 (1H, d, J=7.0Hz), 8.08 (1H, d, J=7.5Hz), 8.20 (1H, s)
MS (ES-); m/z: 378 (M⁺-1)

### Example 42: 3-[(E)-2-(4-Cyclobutyl-1,3-thiazol-2-yl)-1-ethenyl]phenyl [3-(1H-1,2,3,4-tetrazol-5-yl)propyl] ether (*)

### (A) 3-[(E)-2-(4-Cyclobutyl-1,3-thiazol-2-yl)-1-ethenyl]phenol

The title compound was synthesized in the same manner as in Example 41 (A).
¹H-NMR (CDCl₃) δ : 1.83-2.16 (2H, m), 2.21-2.40 (4H, m), 3.69 (1H, m), 6.80 (1H, d, J=8.1Hz), 6.84 (1H, s), 7.00 (1H, m), 7.09 (1H, d, J=8.1Hz), 7.21-7.30 (3H, m)

### (B) 3-3-[(E)-2-(4-Cyclobutyl-1,3-thiazol-2-yl)-1-ethenyl]phenoxypropyl cyanide

The 3-[(E)-2-(4-cyclobutyl-1,3-thiazol-2-yl)-1-ethenyl]phenol obtained in (A) (141 mg) was dissolved in dimethylformamide (5 mL), and the solution was added with cesium carbonate (358 mg) and stirred for 10 minutes. The reaction mixture was added with 4-bromobutyronitrile (60 µL) and sodium iodide (177 mg), and then the mixture was stirred for 16 hours. The reaction mixture was distributed in ethyl acetate and water, and washed with saturated brine. The ethyl acetate layer was dried over sodium sulfate, and the solvent was evaporated under reduced pressure to obtain the title compound as an oil (119 mg). This compound was used for the next reaction without purification.
¹H-NMR (CDCl₃) δ : 2.01-2.62 (8H, m), 2.72 (2H, t, J=7.2Hz), 3.80 (1H, m), 4.22 (2H, t, J=5.7Hz), 6.95 (1H, s), 6.97 (1H, d, J=8.4Hz), 7.17 (1H, m), 7.25 (1H, d, J=7.5Hz), 7.37-7.42 (3H, m)
MS (ES+); m/z: 325 (MH⁺)

### (C) 3-[(E)-2-(4-Cyclobutyl-1,3-thiazol-2-yl)-1-ethenyl]phenyl [3-(1H-1,2,3,4-tetrazol-5-yl)propyl] ether

Sodium azide (337 mg) was suspended in dimethylformamide (1.5 mL), and the suspension was added with aluminum chloride (243 mg) under ice cooling, and stirred at room temperature for 1.5 hours. The 3-3-[(E)-2-(4-cyclobutyl-1,3-thiazol-2-yl)-1-ethenyl]phenoxypropyl cyanide obtained in (B) (118 mg) was dissolved in dimethylformamide (1.5 mL), and the solution was added to the aforementioned reaction mixture, and then the mixture was stirred at 90°C for 16 hours. After the reaction mixture was cooled, the mixture was made acidic with 1 N hydrochloric acid and extracted with ethyl acetate. The ethyl acetate layer was dried over sodium sulfate, and the solvent was evaporated under reduced pressure. The residue was purified by silica gel chromatography (methylene chloride:methanol, 9: 1, v/v) to obtain the title compound as yellow powder (40 mg).
¹H-NMR (CDCl₃) δ : 1.96-2.18 (4H, m), 2.25-2.50 (4H, m), 3.22 (2H, t, J=7.2Hz), 3.77 (1H, m), 4.15 (2H, t, J=6.0Hz), 6.86 (1H, d, J=8.1Hz), 6.92 (1H, s), 7.05 (1H, d, J=7.2Hz), 7.36-7.38 (3H, m), 7.68 (1H, d, J=15.9Hz)
MS (ES-); m/z: 366 (M⁺-1)

### Example 43: 4-(3-[(E)-2-(4-Isopropyl-1,3-thiazol-2-yl)-1-ethenyl]benzoylamino)benzoic acid (*)

### (A) 3-[(E)-2-(4-Isopropyl-1,3-thiazol-2-yl)-1-ethenyl]benzonitrile

Diethyl [(4-isopropyl-1,3-thiazol-2-yl)methyl]phosphonate and 3-cyanobenzaldehyde were treated in the same manner as in Example 15 (F) to obtain the title compound.
¹H-NMR (CDCl₃) δ : 1.42 (6H, d), 3.23 (1H, heptuplet), 6.99 (1H, s), 7.42 (2H, s), 7.58 (1H, m), 7.68 (1H, m), 7.86 (2H, m)

### (B) 3-[(E)-2-(4-Isopropyl-1,3-thiazol-2-yl)-1-ethenyl]benzoic acid

The 3-[(E)-2-(4-isopropyl-1,3-thiazol-2-yl)-1-ethenyl]benzonitrile obtained in (A) (470 mg) was added with concentrated hydrochloric acid (20 mL), and the mixture was heated under reflux for 1.5 hours. The reaction mixture was distributed in ethyl acetate and water, and the organic layer was washed with saturated brine. The solvent was evaporated under reduced pressure to obtain the title compound (288 mg). ¹H-NMR (DMSO-d₆) δ : 1.35 (6H, d), 3.18 (1H, m), 7.38 (1H, s), 7.62 (3H, m), 7.99 (1H, d), 8.12 (1H, d), 8.29 (1H, s)

### (C) Methyl 4-(3-[(E)-2-(4-isopropyl-1,3-thiazol-2-yl)-1-ethenyl]benzoylamino)benzoate

The 3-[(E)-2-(4-isopropyl-1,3-thiazol-2-yl)-1-ethenyl]benzoic acid obtained in (B) (100 mg) was dissolved in methylene chloride (2.5 mL), and the solution was added with thionyl chloride (2.5 mL) and heated under reflux for 0.5 hours. The solvent was evaporated under reduced pressure, and the residue was added with methylene chloride. The solvent was evaporated again, and the residue was dissolved in methylene chloride (2 mL). The solution was added to a solution of methyl 4-aminobenzoate (190 mg) in pyridine (2 mL) at 0°C, and then the mixture was stirred at room temperature for 14 hours. The reaction mixture was distributed in ethyl acetate and water, and the organic layer was washed with water, 1 N hydrochloric acid, and then with saturated brine, and dried over sodium sulfate. The solvent was evaporated under reduced pressure to obtain the title compound (90 mg). ¹H-NMR (CDCl₃) δ : 1.49 (6H, d), 3.37 (1H, heptuplet), 3.96 (3H, s), 7.12 (1H, s), 7.61 (1H, t), 7.70 (1H, m), 7.81 (1H, m), 7.95 (4H, m), 8.18 (2H, m), 8.28 (1H, m)
MS (ES+); m/z: 407 (MH⁺)

### (D) 4-(3-[(E)-2-(4-Isopropyl-1,3-thiazol-2-yl)-1-ethenyl]benzoylaminobenzoic acid

The methyl 4-(3-[(E)-2-(4-isopropyl-1,3-thiazol-2-yl)-1-ethenyl]benzoylamino)-benzoate obtained in (C) was treated in the same manner as in Example 15 (I) to obtain the title compound.
¹H-NMR (DMSO-d₆) δ : 1.27 (6H, d), 3.05 (1H, heptuplet), 7.28 (1H, s), 7.57 (4H, m), 7.94 (5H, m), 10.55 (1H)
MS (ES-); m/z: 391 (M⁺-1)

### Example 44: N-[4-(1H-1,2,3,4-Tetrazol-5-yl)phenyl]-5-[(E)-2-(4-cyclobutyl-1,3-thiazol-2-yl)-1-ethenyl]-2-furanamide (*)

### (A) N-(4-Cyanophenyl)-5-formyl-2-furanamide

5-Formylfuran-2-carboxylic acid (100 mg) was dissolved in methylene chloride (5 mL), and the solution was added with oxalyl chloride (0.2 mL) and dimethylformamide (several drops) at 0°C. After the reaction mixture was stirred at room temperature for 1 hour, the solvent was evaporated under reduced pressure to obtain an acid chloride. This product was treated with 4-aminobenzonitrile in the same manner as in Example 43 (C) to obtain the title compound.
¹H-NMR (DMSO-d₆) δ : 6.92 (1H, d, J=3.9Hz), 7.48 (1H, d, J=3.9), 7.67 (1H, s), 7.85 (2H, d, J=9), 7.92 (2H, d, J=9), 10.67 (1H, s)

### (B) N-(4-Cyanophenyl)-5-[(E)-2-(4-cyclobutyl-1,3-thiazol-2-yl)-1-ethenyl]-2-furanamide

The N-(4-cyanophenyl)-5-formyl-2-furanamide obtained in (A) was treated in the same manner as in Example 15 (F) to obtain the title compound.
¹H-NMR (CDCl₃) δ : 1.91 (2H, m), 2.10 (1H, m), 2.31 (3H, m), 6.64 (1H, d, J=3.6Hz), 6.93 (1H, s), 7.20 (1H, d, J=16Hz), 7.33 (1H, d, J=3.3Hz), 7.42 (1H, d, J=16Hz), 7.68 (2H, d, J=9Hz), 7.85 (2H, d, J=8.8Hz), 8.29 (1H, s)

### (C) N-[4-(1H-1,2,3,4-Tetrazol-5-yl)phenyl]-5-[(E)-2-(4-cyclobutyl-1,3-thiazol-2-yl)-1-ethenyl]-2-furanamide

The N-(4-cyanophenyl)-5-[(E)-2-(4-cyclobutyl-1,3-thiazol-2-yl)-1-ethenyl]-2-furanamide obtained in (B) was treated in the same manner as in Example 42 (C) to obtain the title compound.
¹H-NMR (DMSO-d₆) δ : 1.92 (2H, m), 2.25 (4H, m), 6.96 (1H, s), 7.37 (3H, m), 7.68 (1H, m), 8.04 (4H, m), 10.48 (1H, s)
MS (ES-); m/z: 417 (M⁺-1)

### Example 45: N-3-[(E)-2-(4-Isopropyl-1,3-thiazol-2-yl)-1-ethenyl]phenyl-3-trifluoro-methanesulfonylaminobenzamide (*)

### (A) N-3-[(E)-2-(4-Isopropyl-1,3-thiazol-2-yl)-1-ethenyl]phenyl-3-nitrobenzamide

3-[(E)-2-(4-Isopropyl-1,3-thiazol-2-yl)-1-ethenyl]aniline (59 mg) was dissolved in methylene chloride, and the solution was added with 3-nitrobenzyl chloride (82 mg) and triethylamine (67 µL) at 0°C, and stirred at room temperature for 4 hours. The reaction mixture was distributed in ethyl acetate and 1 N hydrochloric acid, and the organic layer was washed with water, saturated aqueous sodium hydrogencarbonate, and then with saturated brine, and dried over and sodium sulfate. The solvent was evaporated under reduced pressure, and the residue was purified by silica gel chromatography (methylene chloride:methanol, 90:1, v/v) to obtain the title compound as white powder (86 mg).
¹H-NMR (CDCl₃) δ : 1.43 (6Hd, J=6.9Hz), 3.22 (1H, heptuplet, J=6.9Hz), 6.96 (1H, s), 7.48 (3H, m), 7.66 (1H, s, J=8.3), 7.82 (t, 1H, J=8.0), 8.00 (1H, s), 8.29 (1H, s), 8.38 (1H, d, J=7.5), 8.53 (1H, d, J=7.5), 8.82 (1H, s)

### (B) 3-[(E)-2-(4-Isopropyl-1,3-thiazol-2-yl)-1-ethenyl]aniline

The N-3-[(E)-2-(4-isopropyl-1,3-thiazol-2-yl)-1-ethenyl]phenyl-3-nitrobenzamide obtained in (A) was treated in the same manner as in Example 5 (D) to obtain the title compound.
¹H-NMR (CDCl₃) δ : 1.42 (6H, d, J=6.9Hz), 3.21 (1H, heptuplet, J=6.9Hz), 6.93 (1H, s), 6.97 (1H, d, J=7.7Hz), 7.24-7.49 (6H, m), 7.64 (1H, d, J=8.1Hz), 7.95 (1H, s), 8.06 (1H, s)

### (C) N-3-[(E)-2-(4-Isopropyl-1,3-thiazol-2-yl)-1-ethenyl]phenyl-3-trifluoromethanesulfonylaminobenzamide

The 3-[(E)-2-(4-isopropyl-1,3-thiazol-2-yl)-1-ethenyl]aniline obtained in (B) was treated in the same manner as in Example 35 (C) to obtain the title compound.
MS (ES-); m/z: 949 (M⁺-1)

### Example 46: N-3-[(E)-2-(4-Isopropyl-1,3-thiazol-2-yl)-1-ethenyl]phenyl-3-fluoromethanesulfonylaminobenzamide (*)

The 3-[(E)-2-(4-isopropyl-1,3-thiazol-2-yl)-1-ethenyl]aniline (98 mg) obtained in Example 45 (B) was dissolved in tetrahydrofuran (2 mL), and the solution was added with triethylamine (50 µL), added further with fluoromethylsulfonyl chloride (Chem. Ber. 1991, 124, 1879, 50 µL) at -78°C, and then gradually warmed to room temperature. The reaction mixture was distributed in ethyl acetate and water. The organic layer was dried over sodium sulfate, and the solvent was evaporated under reduced pressure. The residue was purified by silica gel chromatography (methylene chloride:methanol, 90:1, v/v) to obtain the title compound.
¹H-NMR (CDCl₃) δ : 1.38 (6H, d, J=6.9Hz), 3.16 (1H, heptuplet, J=6.9Hz), 4.53 (1H, s), 5.10 (1H, d, J=47.1Hz), 6.78 (1H, s), 7.22-7.40 (3H, m), 7.37 (1H, m), 7.48 (2H, m), 7.64 (1H, t, J=7.7Hz), 7.77 (2H, m), 8.30 (1H, s)
MS (ES-); m/z: 468 (M⁺-1)

### Example 47: 4-Isopropyl-2-(E)-2-[7-(1H-1,2,3,4-tetrazol-5-yl)-3-quinolyl]-1-ethenyl-1,3-thiazole

### (A) 3-[(E)-2-(4-Isopropyl-1,3-thiazol-2-yl)-1-ethenyl]-7-quinolinecarbonitrile

3-Formyl-7-quinolinecarbonitrile was treated in the same manner as in Example 15 (F) to obtain the title compound.
¹H-NMR (CDCl₃) δ : 1.36 (6H, d, J=6.9Hz), 3.14 (1H, heptuplet, J=6.9Hz), 6.93 (1H, s), 7.55 (2H, m), 7.80 (1H, d, J=8.4Hz), 7.92 (1Hd, J=8.4Hz), 8.23 (1H, s), 8.45 (1H, s), 9.23 (1H, s)

### (B) 4-Isopropyl-2-(E)-2-[7-(1H-1,2,3,4-tetrazol-5-yl)-3-quinolyl]-1-ethenyl-1,3-thiazole

The 3-[(E)-2-(4-isopropyl-1,3-thiazol-2-yl)-1-ethenyl]-7-quinolinecarbonitrile obtained in (A) was treated in the same manner as in Example 42 (C) to obtain the title compound.
¹H-NMR (DMSO-d₆) δ : 1.29 (6H, d, J=6.9Hz), 3.13 (1H, heptuplet, J=6.9Hz), 7.33 (1H, s), 7.69 (1H, d, J=15.5Hz), 7.85 (1H, d, J=16Hz), 8.15 (1H, d, J=8.4Hz), 8.27 (1H, d, J=8.7Hz), 8.69 (2H, m), 9.40 (1H, s)
MS (ES-); m/z: 347 (M⁺-1)

### Example 48: 1-Ethyl-7-(E)-2-(4-isopropyl-1,3-thiazol-2-yl)-1-ethenyl]-4-oxo-1,4-dihydro-3-quinolinecarboxylic acid

### (A) Ethyl 7-[(E)-2-(4-isopropyl-1,3-thiazol-2-yl)-1-ethenyl]-4-oxo-1,4-dihydro-3-quinolinecarboxylate

3-[(E)-2-(4-Phenyl-1,3-thiazol-2-yl)-1-ethenyl]aniline (Japanese Patent Unexamined Publication No. 62-142168/1987, 650 mg, 2.7 mmol) and diethyl ethoxymethylenemalonate (610 mg) were dissolved in toluene (3 mL), and the solution was heated under reflux for 1 hour. The solvent was evaporated under reduced pressure, and the residue was added with Dowtherm A (2 mL) and then heated to 220°C for 1 hour with stirring. The reaction mixture was cooled, and added with 2-propanol. The deposited crystals were collected by filtration, and washed with 2-propanol to obtain the title compound as light brown powder (480 mg, 49%).
¹H-NMR (CDCl₃-CD₃OD) δ : 1.36 (6H, d, J=6.8Hz), 1.41 (3H, t, J=7.3Hz), 3.15 (1H, m), 4.38 (2H, q, J=7.3Hz), 7.01 (1H, s), 7.65 (2H, m), 8.39 (1H, d, J=9.3Hz), 8.61 (1H, s)

### (B) 1-Ethyl-7-(E)-2-(4-isopropyl-1,3-thiazol-2-yl)-1-ethenyl]-4-oxo-1,4-dihydro-3-quinolinecarboxylic acid

The ethyl 7-[(E)-2-(4-isopropyl-1,3-thiazol-2-yl)-1-ethenyl]-4-oxo-1,4-dihydro-3-quinolinecarboxylate obtained in (A) (40 mg, 0.11 mmol) was dissolved in THF (1 mL) and DMF (1 mL), and the solution was added with sodium hydride (60% in oil, 5 mg) and ethyl iodide (500 µL) and heated at 80°C with stirring. The solvent was evaporated under reduced pressure, and the residue was distributed in ethyl acetate and 1 N hydrochloric acid. The organic layer was washed with saturated brine, and dried over magnesium sulfate. The solvent was evaporated under reduced pressure to obtain light brown powder. The powder was dissolved in THF (2 mL) and methanol (1 mL), and the solution was added with 1 N aqueous sodium hydroxide (400 µL) and heated under reflux for 2 hours. The reaction mixture was distributed in ethyl acetate and 1 N hydrochloric acid. The organic layer was washed with saturated brine, and dried over magnesium sulfate. The solvent was evaporated under reduced pressure, and the residue was purified by silica gel chromatography (chloroform:methanol, 10:1, v/v) to obtain the title compound as white powder (15 mg, 38%).
¹H-NMR (CDCl₃) δ : 1.36 (6H, d, J=6.8Hz), 1.64 (3H, t, J=7.3Hz), 3.16 (1H, m), 4.42 (2H, q, J=7.3Hz), 6.95 (1H, s), 7.52 (2H, s), 7.67 (1H, s), 7.77 (1H, d, J=8.3Hz), 8.55 (1H, d, J=8.3Hz), 8.79 (1H, s)
FAB-MS; m/z: 369 (MH⁺)

### Example 49: 1-Cyclopropyl-6,7-difluoro-4-oxo-1,4-dihydro-3-quinolinecarboxylic acid

### (A) Ethyl

### 1-cyclopropyl-6-fluoro-7-[(4-isopropyl-1,3-thiazol-2-yl)methoxy]-4-oxo-1,4-dihydro-3-qu inolinecarboxylate

(4-Isopropyl-1,3-thiazol-2-yl)methanol (Japanese Patent Unexamined Publication No. 6-80654/1994, 95 mg, 0.60 mmol) was dissolved in DMF (1.5 ml), added with 18-crown-6 (174 mg) and sodium hydride (95%, 17 mg), and stirred for 10 minutes under nitrogen atmosphere. The reaction mixture was added with ethyl 1-cyclopropyl-6,7-difluoro-4-oxo-1,4-dihydro-3-quinolinecarboxylate (150 mg), and stirred at 80°C for 2 hours. The reaction mixture was distributed between ethyl acetate and 1 N hydrochloric acid. The organic layer was dried over magnesium sulfate, and the solvent was evaporated under reduced pressure. The residue was added with ether, and the produced powder was collected by filtration to obtain the title compound as white powder (175 mg, 80%).
¹H-NMR (CDCl₃) δ : 1.08 (4H, m), 1.31 (6H, d, J=6.8Hz), 1.40 (3H, t, J=7.3Hz), 3.11 (1H, m), 3.36 (1H, m), 4.38 (2H, q, J=7.3Hz), 5.57 (2H, s), 6.91 (1H, s), 7.58 (1H, t, J=6.8Hz), 8.16 (1H, d, J=10.2Hz), 8.53 (1H, s)

### (B) 1-Cyclopropyl-6,7-difluoro-4-oxo-1,4-dihydro-3-quinolinecarboxylic acid

The ethyl 1-cyclopropyl-6-fluoro-7-[(4-isopropyl-1,3-thiazol-2-yl)methoxy]-4-oxo-1,4-dihydro-3-quinolinecarboxylate obtained in (A) (40 mg, 0.09 mmol) was dissolved in acetic acid (1 ml) and sulfuric acid (1 mL), and the mixture was stirred at 130°C for 11 hours. The reaction mixture was distributed in ethyl acetate and iced water. The organic layer was washed with saturated brine, and dried over magnesium sulfate, and then the solvent was evaporated under reduced pressure. The residue was purified by silica gel chromatography (chloroform:methanol, 10: 1, v/v) to obtain the title compound as white powder (37 mg, 99%).
¹H-NMR (CDCl₃-CD₃OD) δ : 1.19 (2H, m), 1.34 (6H, d, J=7.3Hz), 1.47 (2H, m), 3.14 (1H, m), 3.69 (1H, m), 5.63 (2H, s), 7.12 (1H, s), 7.86 (1H, t, J=6.8Hz), 8.10 (1H, d, J=10.8Hz), 8.84 (1H, s)
FAB-MS; m/z: 403 (MH⁺)

### Example 50: 1-Cyclopropyl-6-fluoro-7-(2-quinolylmethoxy)-4-oxo-1,4-dihydro-3-quinolinecarboxylic acid

Quinoline-2-methanol was treated in the same manner as in Example 49 to obtain the title compound.
¹H-NMR (DMSO-d₆) δ : 0.95 (2H, m), 1.29 (2H, m), 3.40 (1H, m), 5.70 (2H, s), 7.63 (1H, dd, J=7.8, 7.1Hz), 7.73 (2H, m), 7.81 (1H, dd, J=8.3, 7.1Hz), 7.88 (1H, d, J=7.8Hz), 8.09 (1H, d, J=8.3Hz), 8.15 (1H, d, J=10.8Hz), 8.28 (1H, d, J=8.1Hz), 8.73 (1H, s)
EI-MS; m/z: 404 (M⁺)

### Example 51: 7-((E)-2-(4-Isopropyl-1,3-thiazol-2-yl)-1-ethenyl)-4-oxo-1,4-dihydro-2-quinolinecarboxylic acid

### (A) Methyl 7-((E)-2-(4-isopropyl-1,3-thiazol-2-yl)-1-ethenyl)-4-oxo-1,4-dihydro-2-quinolinecarboxylate

A solution of 3-[(E)-2-(4-isopropyl-1,3-thiazol-2-yl)-1-ethenyl]aniline (245.0 mg) in methanol (4 ml) was added with dimethyl acetylenedicarboxylate (0.129 ml) at room temperature, and the mixture was stirred at the same temperature for 6 hours. The reaction mixture was further added with dimethyl acetylenedicarboxylate (0.025 ml), and then stirred overnight. The solvent was evaporated, and the resulting residue was purified by silica gel chromatography (hexane:ethyl acetate = 4:1, v/v) to obtain a diester compound as a yellow oil (362.8 mg, 93.6%). The resulting diester (360.0 mg) was added with Dowtherm A (5 ml), and heated at 210°C for 3 hours with stirring. The reaction mixture was cooled, and added with diethyl ether and hexane, and the deposited solid was collected by filtration to obtain methyl 7-((E)-2-(4-isopropyl-1,3-thiazol-2-yl)-1-ethenyl)-4-oxo-1,4-dihydro-2-quinolinecarboxylate as a brown solid (98.2 mg, 29.7%).
¹H-NMR (CDCl₃) δ : 1.35 (6H, d, J=7.1Hz), 3.14 (1H, quint., J=7.1Hz), 4.04 (1H, s), 6.92 (1H, s), 6.98 (1H, s), 7.43 (2H, s), 7.55 (1H, d, J=8.6Hz), 7.74 (1H, s), 8.30 (1H, d, J=8.6Hz)

### (B) 7-((E)-2-(4-Isopropyl-1,3-thiazol-2-yl)-1-ethenyl)-4-oxo-1,4-dihydro-2-quinolinecarboxylic acid

A solution of methyl 7-((E)-2-(4-isopropyl-1,3-thiazol-2-yl)-1-ethenyl)-4-oxo-1,4-dihydro-2-quinolinecarboxylate (36.4 mg) in a mixture of methanol (1 ml), THF (2 ml) and water (0.5 ml) was added with 1N aqueous sodium hydroxide (0.123 ml) at room temperature, and the mixture was stirred at the same temperature for 5 hours. The reaction mixture was further added with 1 N aqueous sodium hydroxide (0.205 ml), and stirred overnight at the same temperature. The solvent was evaporated under reduced pressure, and the residue was added with water and 1N hydrochloric acid (0.328 ml). The deposited crystals were collected by filtration, washed with water, and dried under reduced pressure to obtain 7-((E)-2-(4-isopropyl-1,3-thiazol-2-yl)-1-ethenyl)-4-oxo-1,4-dihydro-2-quinolinecarboxylic acid as a blackish brown solid (29.1 mg, 83.2%).
¹H-NMR (DMSO-d₆) δ : 1.29 (6H, d, J=6.8Hz), 3.07 (1H, quint., J=6.8Hz), 6.63 (1H, s), 7.33 (1H, s), 7.47 (1H, d, J=16.2Hz), 7.57 (1H, d, J=16.2Hz), 7.77 (1H, d, J=8.7HZ), 8.00-8.10 (2H, m)
FAB-MS; m/z: 341 (MH⁺)
Elemental Analysis (as C₁₈H₁₆N₂O₃S•0.75H₂O):
Calculated: C, 61.09; H, 4.98; N, 7.92
Found: C, 61.17; H, 4.91; N, 7.94

### Example 52: (3S)-9-Fluoro-10-[(4-isopropyl-1,3-thiazol-2-yl)methoxy]-3-methyl-7-oxo-2,3-dihydro-7H-[1,4]oxazino-[2,3,4-ij]quinoline-6-carboxylic acid

Ethyl (3R)-9,10-difluoro-3-methyl-7-oxo-2,3-dihydro-7H-[1,4]oxazino[2,3,4-ij]-quinoline-6-carboxylate was treated in the same manner as in Example 49 to obtain the title compound.
¹H-NMR (DMSO-d₆) δ : 1.17 (6H, d, J=6.8Hz), 1.45 (3H, d, J=6.8Hz), 2.98 (1H, m), 4.40 (1H, d, J=10.5Hz), 4.63 (1H, d, J=10.5Hz), 4.94 (1H, m), 5.55 (2H, s), 7.33 (1H, s), 7.66 (1H, d, J=10.5Hz), 8.89 (1H, s)
EI-MS; m/z: 418 (M⁺)

### Example 53: 1-Cyclopropyl-6-fluoro-7-[(4-isopropyl-1,3-thiazol-2-yl)methoxy]-4-oxo-1,4-dihydro[1,8]naphthylidine-3-carboxylic acid

Ethyl 1-cyclopropyl-6,7-difluoro-4-oxo-1,4-dihydro[1,8]naphthylidine-3-carboxylate was treated in the same manner as in Example 49 to obtain the title compound.
¹H-NMR (CDCl₃) δ : 1.02-1.11 (2H, m), 1.20-1.28 (2H, m), 1.32 (6H, d, J=6.8Hz), 3.05-3.18 (1H, m), 3.62-3.72 (1H, m), 5.91 (2H, s), 6.95 (1H, s), 8.35 (1H, d, J=8.8Hz), 8.85 (1H, s), 14.53 (1H, br)
EI/MS; m/z: 403 (M⁺)
FAB/MS; m/z: 404 (MH⁺)

### Examples 54 and 55: 1-Ethyl-7-((E)-2-(4-isopropyl-1,3-thiazol-2-yl)-1-ethenyl)-4-oxo-1,4-dihydro-2-quinolinecarboxylic acid, and 1-ethyl-5-((E)-2-(4-isopropyl-1,3-thiazol-2-yl)-1-ethenyl)-4-oxo-1,4-dihydro-2-quinolinecarboxylic acid

### (A) tert-Butyl N-3-[(E)-2-(4-isopropyl-1,3-thiazol-2-yl)-1-ethenyl]phenylcarbamate

A solution of 3-[(E)-2-(4-isopropyl-1,3-thiazol-2-yl)-1-ethenyl]aniline (98.3 mg) in THF (3 ml) was added with di-tert-butyl dicarbonate (100 mg) and saturated aqueous sodium hydrogencarbonate (1 ml) at room temperature, and the mixture was stirred for 1 hour. While the reaction mixture was stirred overnight, the mixture was added with di-tert-butyl dicarbonate (300 mg) and saturated aqueous sodium hydrogencarbonate (3 ml). The solvent was evaporated, and the resulting residue was added with ethyl acetate. The organic layer was washed with water and saturated brine, and dried over anhydrous sodium sulfate, and then the solvent was evaporated. The obtained residue was purified by silica gel chromatography (hexane:ethyl acetate =9:1, v/v) to obtain tert-butyl N-3-[(E)-2-(4-isopropyl-1,3-thiazol-2-yl)-1-ethenyl]phenylcarbamate as a colorless solid (129.5 mg, 93.5%).
¹H-NMR (CDCl₃) δ : 1.33 (6H, d, J=6.8Hz), 1.53 (9H, s), 3.13 (1H, quint., J=6.8Hz), 6.50 (1H, br s), 6.80 (1H, s), 7.15-7.35 (5H, m), 7.65 (1H, br s)

### (B) tert-Butyl N-ethyl-3-[(E)-2-(4-isopropyl-1,3-thiazol-2-yl)-1-ethenyl]phenylcarbamate

A solution of tert-butyl N-3-[(E)-2-(4-isopropyl-1,3-thiazol-2-yl)-1-ethenyl]-phenylcarbamate (126.1 mg) in DMF (2 ml) was added with sodium hydride (22.0 mg) at room temperature, and the mixture was stirred for 30 minutes at the same temperature. The reaction mixture was then added with ethyl iodide (58.6 µl), and stirred at the same temperature for 1 hour. The reaction mixture was added with water and ethyl acetate, and washed with water and saturated brine. The organic layer was dried over anhydrous sodium sulfate, and the solvent was evaporated to obtain tert-butyl N-ethyl-3-[(E)-2-(4-isopropyl-1,3-thiazol-2-yl)-1-ethenyl]phenylcarbamate as light yellow oil (143.0 mg, quantitative).
¹H-NMR (CDCl₃) δ : 1.17 (3H, t, J=7.1Hz), 1.33 (6H, d, J=6.8Hz), 1.45 (9H, s), 3.11 (1H, quint., J=6.8Hz), 3.68 (3H, q, J=7.1Hz), 6.81 (1H, s), 7.14 (1H, br d, J=7.3Hz), 7.25-7.40 (5H, m)

### (C) N-ethyl-3-[(E)-2-(4-isopropyl-1,3-thiazol-2-yl)-1-ethenyl]aniline

A solution of tert-butyl N-ethyl-3-[(E)-2-(4-isopropyl-1,3-thiazol-2-yl)-1-ethenyl]phenylcarbamate (143.0 mg) in methanol (1 ml) was added with 4 N hydrochloric acid in dioxane (1 ml) at room temperature, and the mixture was stirred at the same temperature for 40 minutes. The solvent was evaporated, and the residue was distributed in ethyl acetate and saturated aqueous sodium hydrogencarbonate. The aqueous layer was extracted with ethyl acetate. The organic layer was washed with saturated brine, and dried over anhydrous sodium sulfate. The solvent was evaporated to obtain N-ethyl-3-[(E)-2-(4-isopropyl-1,3-thiazol-2-yl)-1-ethenyl]aniline as yellow oil (101.4 mg, quantitative).
¹H-NMR (CDCl₃) δ : 1.28 (3H, t, J=7.1Hz), 1.33 (6H, d, J=6.8Hz), 3.11 (1H, quint., J=6.8Hz), 3.19 (3H, q, J=7.1Hz), 6.57 (1H, dd, J=8.0, 2.2Hz), 6.76 (1H, s), 6.78 (1H, s), 6.87 (1H, d, J=7.6Hz), 7.17 (1H, t, J=8.0Hz), 7.26 (1H, s), 7.29(1H, s)

### (D) Methyl 1-ethyl-7-((E)-2-(4-isopropyl-1,3-thiazol-2-yl)-1-ethenyl)-4-oxo-1,4-dihydro-2-quinolinecarboxylate and methyl 1-ethyl-5-((E)-2-(4-isopropyl-1,3-thiazol-2-yl)-1-ethenyl)-4-oxo-1,4-dihydro-2-quinolinecarboxylate

A solution of N-ethyl-3-[(E)-2-(4-isopropyl-1,3-thiazol-2-yl)-1-ethenyl]aniline (186.0 mg) in methanol (4 ml) was added with dimethyl acetylenedicarboxylate (92.3 µl) at room temperature, and the mixture was stirred at the same temperature for 2.5 hours. The solvent was evaporated to obtain a diester compound. The resulting diester (92.5 mg) was added with pyrophosphoric acid (1 ml), and heated at 110°C for 1 hour with stirring. The reaction mixture was cooled and added with water, and then the mixture was extracted with ethyl acetate. The organic layer was washed with water and saturated brine, and dried over anhydrous sodium sulfate. The solvent was evaporated, and the resulting residue was purified by silica gel chromatography (chloroform → chloroform :methanol = 99:1, v/v) to obtain methyl 1-ethyl-5-((E)-2-(4-isopropyl-1,3-thiazol-2-yl)-1-ethenyl)-4-oxo-1,4-dihydro-2-quinolinecarboxylate as yellow oil (31.1 mg, 36.4%).
¹H-NMR (CDCl₃) δ : 1.33 (6H, d, J=6.8Hz), 1.54 (3H, t, J=7.1Hz), 3.11 (1H, quint., J=6.8Hz), 3.99 (3H, s), 4.27 (2H, q, J=7.1Hz), 6.55 (1H, s), 6.80 (1H, s), 7.10 (1H, dd, J=16.1, 0.7Hz), 7.50-7.55 (2H, m), 7.66 (1H, dd, J=8.8, 7.3Hz), 8.84 (1H, d, J=16.1Hz)

Methyl 1-ethyl-7-((E)-2-(4-isopropyl-1,3-thiazol-2-yl)-1-ethenyl)-4-oxo-1,4-dihydro-2-quinolinecarboxylate was obtained as yellow oil (18.7 mg, 21.9%).
¹H-NMR (CDCl₃) δ : 1.36 (6H, d, J=6.8Hz), 1.58 (3H, t, J=7.1Hz), 3.15 (1H, quint., J=6.8Hz), 4.00 (3H, s), 4.31 (2H, q, J=7.1Hz), 6.59 (1H, s), 6.89 (1H, s), 7.45 (1H, d, J=16.1Hz), 7.50 (1H, d, J=16.1Hz), 7.59 (1H, br d, J=8.3Hz), 7.61 (1H, br s), 8.42 (1H, d, J=8.3Hz)

### (E) 1-Ethyl-7-((E)-2-(4-isopropyl-1,3-thiazol-2-yl)-1-ethenyl)-4-oxo-1,4-dihydro-2-quinolinecarboxylic acid

A solution of methyl 1-ethyl-7-((E)-2-(4-isopropyl-1,3-thiazol-2-yl)-1-ethenyl)-4-oxo-1,4-dihydro-2-quinolinecarboxylate (37.0 mg) in a mixture of methanol (1 ml), THF (2 ml) and water (0.5 ml) was added with 1 N aqueous sodium hydroxide (0.145 ml) at room temperature, and the mixture was stirred at the same temperature for 1 hour. The solvent was evaporated, and the residue was added with 1 N hydrochloric acid (0.145 ml). The deposited crystals were collected by filtration, washed with water, and then dried under reduced pressure to obtain 1-ethyl-7-((E)-2-(4-isopropyl-1,3-thiazol-2-yl)-1-ethenyl)-4-oxo-1,4-dihydro-2-quinolinecarboxylic acid as a blackish brown solid (24.9 mg, 69.9%).
¹H-NMR (DMSO-d₆) δ : 1.29 (6H, d, J=6.8Hz), 1.42 (3H, t, J=7.1Hz), 3.08 (1H, quint., J=6.8Hz), 4.40 (2H, q, J=7.1Hz), 6.27 (1H, s), 7.32 (1H, s), 7.67 (1H, d, J=16.4Hz), 7.77 (1H, d, J=16.4Hz), 7.84 (1H, d, J=8.3Hz), 8.12 (1H, s), 8.15 (1H, d, J=8.3Hz)
FAB-MS; m/z: 369 (MH⁺)
Elemental Analysis (as C₂₀H₂₀N₂O₃S•1.33H₂O):
Calculated: C, 61.21; H, 5.82; N, 7.14
Found: C, 61.43; H, 5.86; N, 6.84

### (F) 1-Ethyl-5-((E)-2-(4-isopropyl-1,3-thiazol-2-yl)-1-ethenyl)-4-oxo-1,4-dihydro-2-quinolinecarboxylic acid

A solution of methyl 1-ethyl-5-((E)-2-(4-isopropyl-1,3-thiazol-2-yl)-1-ethenyl)-4-oxo-1,4-dihydro-2-quinolinecarboxylate (64.4 mg) in a mixture of methanol (2 ml), THF (1 ml) and water (0.5 ml) was added with 1 N aqueous sodium hydroxide (0.253 ml) at room temperature, and the mixture was stirred at the same temperature for 1.5 hours. The solvent was evaporated, and the residue was added with 1 N hydrochloric acid (0.253 ml). The deposited crystals were collected by filtration, washed with water, and then dried under reduced pressure to obtain 1-ethyl -5-((E)-2-(4-isopropyl-1,3-thiazol-2-yl)-1-ethenyl)-4-oxo-1,4-dihydro-2-quinolinecarboxylic acid as a dark brown solid (29.9 mg, 48.2%).
¹H-NMR (DMSO-d₆) δ : 1.27 (6H, d, J=6.8Hz), 1.39 (3H, t, J=7.1Hz), 3.05 (1H, quint., J=6.8Hz), 4.32 (2H, q, J=7.1Hz), 6.27 (1H, s), 7.13 (1H, d, J=16.1Hz), 7.22 (1H, s), 7.65 (1H, d, J=7.6Hz), 7.77 (1H, dd, J=8.8, 7.6Hz), 7.86 (1H, d, J=8.8Hz), 8.84 (1H, d,
J=16.1Hz)
FAB-MS; m/z: 369 (MH⁺)
Elemental Analysis (as C₂₀H₂₀N₂O₃S•0.75H₂O):
Calculated: C, 62.89; H, 5.67; N, 7.33
Found: C, 63.07; H, 5.68; N, 7.12

### Example 56: 1-Ethyl-6-((4-isopropyl-1,3-thiazol-2-yl)methoxy)-4-oxo-1,4-dihydro-2-quinolinecarboxylic acid

### (A) (4-Isopropyl-1,3-thiazol-2-yl)methyl (4-nitrophenyl) ether

A solution of (4-isopropyl-1,3-thiazol-2-yl)methanol (473.3 mg), 4-nitrophenol (460.6 mg), and triphenylphosphine (945.7 mg) in THF (25 ml) was added with diethyl azodicarboxylate (0.569 ml) at -10°C under nitrogen flow, and the mixture was stirred at room temperature for 7 hours. The reaction mixture was added with a solution of triphenylphosphine (236.9 mg) and diethyl azodicarboxylate (0.142 ml) in THF (5 ml), which was prepared beforehand, and then the mixture was stirred overnight at room temperature. The solvent was evaporated, and the resulting residue was purified by silica gel chromatography (hexane:ethyl acetate = 9:1, v/v) to obtain (4-isopropyl-1,3-thiazol-2-yl)methyl (4-nitrophenyl) ether as yellow oil (785.8 mg, 93.8%).
¹H-NMR (CDCl₃) δ : 1.32 (6H, d, J=6.8Hz), 3.12 (1H, quint., J=6.8Hz), 5.43 (2H, s), 6.94 (1H, d, J=1.0Hz), 7.09 (1H, d, J=9.0Hz), 8.21 (1H, d, J=9.0Hz)

### (B) 4-[(4-Isopropyl-1,3-thiazol-2-yl)methoxy]aniline

A solution of (4-isopropyl-1,3-thiazol-2-yl)methyl (4-nitrophenyl) ether (775.7 mg) in ethanol (25 ml) was added with tin (II) chloride dihydrate (1.907 g), and the mixture was heated under reflux for 3.5 hours. The solvent was evaporated, and the resulting residue was added with 1 N aqueous sodium hydroxide to be adjusted to pH 9-10, and then extracted with chloroform. The organic layer was dried over anhydrous sodium sulfate, and the solvent was evaporated. The resulting residue was purified by silica gel chromatography (chloroform:methanol = 99:1, v/v) to obtain 4-[(4-isopropyl-1,3-thiazol-2-yl)methoxy]aniline as dark brown oil (386.2 mg, 55.8%).
¹H-NMR (CDCl₃) δ : 1.31 (6H, d, J=6.8Hz), 3.10 (1H, quint., J=6.8Hz), 5.26 (2H, s), 6.64 (1H, d, J=9.0Hz), 6.83 (1H, d, J=9.0Hz), 6.87 (1H, d, J=0.7Hz)

### (C) tert-Butyl N-3-[(4-isopropyl-1,3-thiazol-2-yl)methoxy]phenylcarbamate

A solution of the 4-[(4-isopropyl-1,3-thiazol-2-yl)methoxy]aniline (149.0 mg) synthesized in (B) in a mixture of dioxane (4 ml) and THF (3 ml) was added with di-tert-butyl dicarbonate (261.9 mg) and saturated aqueous sodium hydrogencarbonate (4 ml) at room temperature, and the mixture was stirred for 3 hours. The reaction mixture was added with di-tert-butyl dicarbonate (65.5 mg) and stirred for 2 hours. The solvent was evaporated, and the resulting residue was added with ethyl acetate, and washed with water and saturated brine. The organic layer was dried over anhydrous sodium sulfate, and the solvent was evaporated. The resulting residue was purified by silica gel chromatography (hexane:ethyl acetate = 85:15, v/v) to obtain tert-butyl N-3-[(4-isopropyl-1,3-thiazol-2-yl)methoxy]phenylcarbamate as colorless oil (126.5 mg, 60.5%).
¹H-NMR (CDCl₃) δ : 1.31 (6H, d, J=6.8H), 1.50 (9H, s), 3.10 (1H, d quint., J=6.8, 0.7Hz), 5.29 (2H, s), 6.40-6.50 (1H, m), 6.88 (1H, d, J=0.7Hz), 6.93 (2H, d, J=9.0Hz), 7.27 (2H, br d, J=9.0Hz)

### (B) tert-Butyl N-ethyl-4-[(4-isopropyl-1,3-thiazol-2-yl)methoxy]phenylcarbamate

A solution of tert-butyl N-3-[(4-isopropyl-1,3-thiazol-2-yl)methoxy]phenylcarbamate (124.3 mg) in DMF (2 ml) was added with sodium hydride (21.4 mg) at room temperature, and the mixture was stirred for 30 minutes at the same temperature. The reaction mixture was then added with ethyl iodide (57.1 µl), and stirred at the same temperature for 1 hour. The reaction mixture was added with water and ethyl acetate, and washed with water and saturated brine. The organic layer was dried over anhydrous sodium sulfate, and the solvent was evaporated to obtain tert-butyl N-ethyl-4-[(4-isopropyl-1,3-thiazol-2-yl)methoxy]phenylcarbamate as colorless oil (141.2 mg, quantitative).
¹H-NMR (CDCl₃) δ : 1.12 (3H, t, J=7.1Hz) 1.32 (6H, d, J=6.8Hz), 1.42 (9H, br s), 3.11 (1H, quint., J=6.8Hz), 3.61 (2H, q, J=7.1Hz), 5.32 (2H, s), 6.90 (1H, s), 6.95 (2H, d, J=8.8Hz), 7.05-7.15 (2H, m)

### (C) N-Ethyl-4-[(4-isopropyl-1,3-thiazol-2-yl)methoxy]aniline

A solution of tert-butyl N-ethyl-4-[(4-isopropyl-1,3-thiazol-2-yl)methoxy]-phenylcarbamate (139.2 mg) in methanol (1 ml) was added with 4 N hydrochloric acid in dioxane (1 ml) at room temperature, and stirred at the same temperature for 1 hour. The solvent was evaporated, and the resulting residue was distributed between ethyl acetate and saturated aqueous sodium hydrogencarbonate. The aqueous layer was extracted with ethyl acetate, and the organic layer was washed with saturated brine, and dried over anhydrous sodium sulfate. The solvent was evaporated to obtain N-ethyl-4-[(4-isopropyl-1,3-thiazol-2-yl)methoxy]aniline as colorless oil (85.8 mg, 87.0%).
¹H-NMR (CDCl₃) δ : 1.24 (3H, t, J=7.1Hz), 1.29 (6H, d, J=6.8H), 3.05-3.15 (1H, m), 3.10 (2H, q, J=7.1Hz), 5.25 (2H, s), 6.56 (2H, d, J=8.8Hz), 6.87 (1H, s), 6.88 (2H, d, J=8.8Hz)

### (D) Methyl 1-ethyl-6-((4-isopropyl-1,3-thiazol-2-yl)methoxy)-4-oxo-1,4-dihydro-2-quinolinecarboxylate

A solution of N-ethyl-4-[(4-isopropyl-1,3-thiazol-2-yl)methoxy]aniline (84.3 mg) in methanol (2 ml) was added with dimethyl acetylenedicarboxylate (41.2 µl) at the room temperature, and the mixture was stirred at the same temperature for 2 hours. The solvent was evaporated to obtain a diester, which was then added with pyrophosphoric acid (2 ml) and heated at 110°C for 1 hour with stirring. The reaction mixture was cooled, added with water, and extracted with ethyl acetate. The organic layer was washed with water and saturated brine, and dried over anhydrous sodium sulfate. The solvent was evaporated, and the resulting residue was purified by silica gel chromatography (chloroform → chloroform:methanol = 98:2, v/v) to obtain methyl 1-ethyl-6-((4-isopropyl-1,3-thiazol-2-yl)methoxy)-4-oxo-1,4-dihydro-2-quinolinecarboxylate as a light yellow solid (95.0 mg, 80.6%).
¹H-NMR (CDCl₃) δ : 1.33 (6H, d, J=6.8H), 1.54 (3H, t, J=7.1Hz), 3.11 (1H, d quint., J=6.8, 1.0Hz), 3.99 (3H, s), 4.30 (2H, q, J=7.1Hz), 5.44 (2H, s), 6.60 (1H, s), 6.91 (1H, d, J=1.0Hz), 7.45 (1H, dd, J=9.3, 3.2Hz), 7.56 (1H, d, J=9.3Hz), 7.97 (1H, d, J=3.2Hz)

### (E) 1-Ethyl-6-((4-isopropyl-1,3-thiazol-2-yl)methoxy)-4-oxo-1,4-dihydro-2-quinolinecarboxylic acid

A solution of methyl 1-ethyl-6-((4-isopropyl-1,3-thiazol-2-yl)methoxy)-4-oxo-1,4-dihydro-2-quinolinecarboxylate (93.1 mg) in a mixture of methanol (1 ml), THF (1 ml) and water (0.5 ml) was added with 1 N aqueous sodium hydroxide (0.361 ml) at room temperature, and the mixture was stirred at the same temperature for 1.5 hours. The solvent was evaporated, and the residue was added with 1 N hydrochloric acid (0.361 ml). The deposited crystals were collected by filtration, washed with water, and then dried under reduced pressure to obtain 1-ethyl-6-((4-isopropyl-1,3-thiazol-2-yl)methoxy)-4-oxo-1,4-dihydro-2-quinolinecarboxylic acid as a light yellow solid (64.2 mg, 71.6%).
¹H-NMR (DMSO-d₆) δ : 1.26 (6H, d, J=6.8H), 1.38 (3H, t, J=7.1Hz), 3.05 (1H, quint., J=6.8Hz), 4.34 (2H, q, J=7.1Hz), 5.51 (2H, s), 6.25 (1H, s), 7.31 (1H, d, J=1.0Hz), 7.55 (1H, dd, J=9.3, 3.2Hz), 7.73 (1H, d, J=3.2Hz), 7.88 (1H, d, J=9.3Hz)
FAB-MS; m/z: 373 (MH⁺)
Elemental Analysis (as C₁₉H₂₀N₂O₄S•0.75H₂O):
Calculated: C, 59.13; H, 5.61; N, 7.26
Found: C, 58.86; H, 5.76; N, 6.99

### Example 57: 8-[(E)-2-(4-Isopropyl-1,3-thiazol-2-yl)-1-ethenyl]-4-oxo-4H-pyrido[1,2-a]-pyrimidine-3-carboxylic acid

### (A) Ethyl 8-[(E)-2-(4-isopropyl-1,3-thiazol-2-yl)-1-ethenyl]-4-oxo-4H-pyrido[1,2-a]-pyrimidine-3-carboxylate

The 4-[(E)-2-(4-isopropyl-1,3-thiazol-2-yl)-1-ethenyl]-2-aminopyridine obtained in Example 21 (A) (100 mg, 0.41 mmol) and diethyl ethoxymethylenemalonate (300 µL) were heated in toluene (3 ml) under reflux for 2 hours. The solvent was evaporated under reduced pressure, and the residue was added with propionic acid (5 ml), and heated under reflux for 3 hours. The solvent was evaporated under reduced pressure, and the residue was purified by silica gel chromatography (chloroform:acetone, 10: 1, v/v) to obtain the title compound as light yellow powder (101 mg, 67%).
¹H-NMR (CDCl₃) δ : 1.36 (6H, d, J=7.0Hz), 1.42 (3H, t, J=7.3Hz), 3.17 (1H, m), 4.43 (2H, q, J=7.3Hz), 7.02 (1H, s), 7.45 (1H, d, J=15.1Hz), 7.46 (1H, m), 7.59 (1H, d, J=15.1Hz), 7.73 (1H, s), 9.04 (1H, s), 9.20 (1H, d, J=7.3Hz)

### (B) 8-[(E)-2-(4-Isopropyl-1,3-thiazol-2-yl)-1-ethenyl]-4-oxo-4H-pyrido[1,2-a]pyrimidine-3-carboxylic acid

The ethyl 8-[(E)-2-(4-isopropyl-1,3-thiazol-2-yl)-1-ethenyl]-4-oxo-4H-pyrido-[1,2-a]pyrimidine-3-carboxylate obtained in (A) (55 mg, 0.15 mmol) was added with concentrated hydrochloric acid (3 mL) and acetic acid (5 mL), and the mixture was heated at 100°C for 1.5 hours with stirring. The solvent was evaporated under reduced pressure, and the residue was purified by silica gel chromatography (chloroform:methanol, 10: 1, v/v) to obtain the title compound as light yellow powder (22 mg, 43%).
¹H-NMR (CD₃OD) δ : 1.37 (6H, d, J=6.8Hz), 3.18 (1H, m), 7.19 (1H, s), 7.57 (2H, m), 7.78 (1H, d, J=16.1Hz), 7.84 (1H, d, J=6.6Hz), 7.92 (1H, s), 9.14 (1H, s), 9.23 (1H, d, J=6.8Hz)
FAB-MS; m/z: 342 (MH⁺)

### Example 58: 9-[(4-Isopropyl-1,3-thiazol-2-yl)methoxy]-4-oxo-4H-pyrido[1,2-a]-pyrimidine-3-carboxylic acid

### (A) Ethyl 9-[(4-isopropyl-1,3-thiazol-2-yl)methoxy]-4-oxo-4H-pyrido[1,2-a]pyrimidine-3-carboxylate

2-(Chloromethyl)-4-isopropyl-1,3-thiazole (323 mg, 1.84 mmol) was dissolved in DMF (10 ml), added with ethyl 9-hydroxy-4-oxo-4H-pyrido[1,2-a]pyrimidine-3-carboxylate (Hermecz et al., Synthesis, 1984, 152, 391 mg, 1.67 mmol), and the solution was further added with potassium carbonate (346 mg, 2.51 mmol) and potassium iodide (277 mg, 1.57 mmol) and then heated at about 110°C for 6 hours with stirring. The reaction mixture was added with water, and the deposited solids were collected by filtration and washed with water. The solids were dissolved in chloroform, and dried over anhydrous sodium sulfate, and then the solvent was evaporated. The residue was purified by silica gel column chromatography (chloroform → chloroform:methanol = 50:1, v/v) to obtain the title compound as a light brown solid (43 mg, 7%). The solvent of the filtrate was evaporated, and the residue was added with chloroform. The organic layer was washed with water and saturated brine, and dried over anhydrous sodium sulfate, and then the solvent was evaporated. The residue was purified by silica gel column chromatography (chloroform → chloroform:methanol = 50:1, v/v) and preparative silica gel thin layer chromatography (chloroform:methanol = 20:1, v/v) to obtain the title compound as a light brown solid (324 mg, 52%). The total yield of the title compound was 367 mg (59%).
¹H-NMR (CDCl₃) δ : 1.31 (6H, d, J=6.8Hz), 1.42 (3H, t, J=7.1Hz), 3.09-3.18 (1H, m), 4.43 (2H, q, J=7.1Hz), 5.66 (2H, s), 6.96 (1H, s), 7.19 (1H, t, J=7.6Hz), 7.59 (1H, dd, J=7.8, 1.2Hz), 8.91 (1H, dd, J=7.1, 1.2Hz), 9.08 (1H, s)

### (B) 9-[(4-Isopropyl-1,3-thiazol-2-yl)methoxy]-4-oxo-4H-pyrido[1,2-a]pyrimidine-3-carboxylic acid

The ethyl 9-[(4-isopropyl-1,3-thiazol-2-yl)methoxy]-4-oxo-4H-pyrido[1,2-a]-pyrimidine-3-carboxylate obtained in (A) (63 mg, 0.17 mmol) was dissolved in THF (3 ml), and the solution was added dropwise with 1% aqueous sodium hydroxide (2.7 ml, 0.68 mmol) and stirred at room temperature for 1 hour. The reaction mixture was added with 1 N aqueous hydrochloric acid (0.7 ml, 0.70 mmol) and water, and extracted with ethyl acetate. The organic layer was washed with saturated brine, and dried over anhydrous sodium sulfate, and then, the solvent was evaporated. The residue was added with hexane and ether and collected by filtration as powder, and then the powder was washed with hexane to obtain the title compound as pale orange powder (39 mg, 67%).
¹H-NMR (CDCl₃) δ : 1.31 (6H, d, J=6.9Hz), 3.07-3.18 (1H, m), 5.70 (2H, s), 6.97 (1H, s), 7.37 (1H, t, J=7.6Hz), 7.76 (1H, d, J=7.8Hz), 8.87 (1H, d, J=7.1Hz), 9.32 (1H, s)
EI/MS; m/z: 345 (M⁺)
FAB/MS; m/z: 346 (MH⁺)

### Example 59: 8-[(E)-2-(4-Isopropyl-1,3-thiazol-2-yl)-1-ethenyl]-3-(1H-1,2,3,4-tetrazol-5-yl)-4H-pyrido[1,2-a]pyrimidin-4-one

### (A) 8-[(E)-2-(4-Isopropyl-1,3-thiazol-2-yl)-1-ethenyl]-3-(2-(4-methoxybenzyl-1H-1,2,3,4-tetrazol-5-yl)-4H-pyrido[1,2-a]pyrimidin-4-one

(4-[(E)-2-(4-Isopropyl-1,3-thiazol-2-yl)-1-ethenyl]-2-aminopyridine (150 mg, 0.61 mmol) and ethyl 3-dimethylamino-2-(2-(4-methoxybenzyl)tetrazol-5-yl)acrylate (Sugano et al., Chem. Pharm. Bull., 1991, 39, 1099, 305 mg) were heated in propionic acid (2 ml) under reflux for 4 hours. The solvent was evaporated under reduced pressure, and the residue was purified by silica gel chromatography (chloroform: acetone, 10: 1, v/v) to obtain the title compound as light yellow powder (256 mg, 86%).
¹H-NMR (CDCl₃) δ : 1.36 (6H, d, J=6.8Hz), 3.17 (1H, m), 3.79 (3H, s), 5.81 (2H, s), 6.89 (2H, d, J=8.5Hz), 7.00 (1H, s), 7.43 (4H, m), 7.58 (1H, d, J=16.1Hz), 7.74 (1H, s), 9.21 (2H, m)

### (B) 8-[(E)-2-(4-Isopropyl-1,3-thiazol-2-yl)-1-ethenyl]-3-(1H-1,2,3,4-tetrazol-5-yl)-4H-pyrido[1,2-a]pyrimidin-4-one

The 8-[(E)-2-(4-isopropyl-1,3-thiazol-2-yl)-1-ethenyl]-3-(2-(4-methoxybenzyl-1H-1,2,3,4-tetrazol-5-yl)-4H-pyrido[1,2-a]pyrimidin-4-one obtained in (A) (240 mg, 0.49 mmol) was added with trifluoroacetic acid (3 mL), and the mixture was stirred for two days at room temperature. The solvent was evaporated under reduced pressure, and then the residue was added with toluene and the solvent was evaporated again. The residue was added with chloroform, methanol and ether, and the deposited crystals were collected by filtration, and dried to obtain the title compound as light yellow powder (130 mg, 73%).
¹H-NMR (CD₃OD) δ : 1.37 (6H, d, J=7.1Hz), 3.18 (1H, m), 7.16 (1H, s), 7.58 (1H, m), 7.75 (1H, d, J=16.1Hz), 7.77 (1H, m), 7.89 (1H, d, J=1.7Hz), 9.26 (1H, d, J=7.6Hz), 9.34 (1H, s)
FAB-MS; m/z: 366 (MH⁺)

### Example 60: 8-[(4-Isopropyl-1,3-thiazol-2-yl)methoxy]-3-(2H-1,2,3,4-tetrazol-5-yl)-4H-pyrido[1,2-a]pyrimidin-4-one

### (A) 8-[(4-Isopropyl-1,3-thiazol-2-yl)methoxy]-3-[2-(4-methoxybenzyl)-2H-1,2,3,4-tetrazol-5-yl]-4H-pyrido[1,2-a]pyrimidin-4-one

The 4-[(4-isopropyl-1,3-thiazol-2-yl)methoxy]-2-pyridylamine obtained in Example 22 (C) was treated in the same manner as in Example 59 (A) to obtain the title compound.
¹H-NMR (CDCl₃) δ : 1.33 (6H, d, J=6.9Hz), 3.10-3.18 (1H, m), 3.79 (3H, s), 5.50 (2H, s), 5.79 (2H, s), 6.88 (2H, d, J=8.7Hz), 6.97 (1H, d, J=0.7Hz), 7.02 (1H, dd, J=7.8,2.7Hz), 7.18 (1H, d, J=2.7Hz), 7.40 (2H, d, J=8.7Hz), 9.11 (1H, s), 9.17 (1H, d, J=7.8Hz)

### (B) 8-[(4-Isopropyl-1,3-thiazol-2-yl)methoxy]-3-(2H-1,2,3,4-tetrazol-5-yl)-4H-pyrido[1,2-a]pyrimidin-4-one

The 8-[(4-isopropyl-1,3-thiazol-2-yl)methoxy]-3-[2-(4-methoxybenzyl)-2H-1,2,3,4-tetrazol-5-yl]-4H-pyrido[1,2-a]pyrimidin-4-one obtained in (A) was treated in the same manner as in Example 59 (B) to obtain the title compound.
¹H-NMR (DMSO-d₆) δ : 1.27 (6H, d, J=7.1Hz), 3.02-3.12 (1H, m), 5.74 (2H, s), 7.37 (1H, dd, J=7.8, 2.7Hz), 7.41 (1H, d, J=0.7Hz), 7.50 (1H, d, J=2.7Hz), 9.07 (1H, s), 9.12 (1H, d, J=7.8Hz)
EI-MS; m/z: 369 (M⁺)
FAB-MS; m/z: 370 (MH⁺)

### Example 61: 8-[2-(4-Isopropyl-1,3-thiazol-2-yl)ethyl]-3-(2H-1,2,3,4-tetrazol-5-yl)-4H-pyrido[1,2-a]pyrimidin-4-one

### (A) tert-Butyl N-4-[2-(4-isopropyl-1,3-thiazol-2-yl)ethyl]-2-pyridylcarbamate

tert-Butyl N-(4-methyl-2-pyridyl)carbamate (J. Org. Chem., 1996, 61, 4810.) (0.69 g, 1.99 mmol) was dissolved in THF (30 ml) and added dropwise with a 1.63 N solution of n-butyllithium in hexane (5.89 ml, 9.60 mmol) at -78°C over 5 minutes. The mixture was warmed to room temperature, stirred for 30 minutes, then cooled again to -78°C, added dropwise with a solution of 2-(bromomethyl)-4-isopropyl-1,3 -thiazol (1.27 g, 5.76 mmol) in THF (10 ml) over 10 minutes, and stirred at the same temperature for 30 minutes. After the reaction, the reaction mixture was added with water, warmed to room temperature, then extracted with ethyl acetate, washed with saturated brine, and then dried over anhydrous sodium sulfate. The solvent was evaporated, and the residue was purified by silica gel column chromatography (chloroform) to obtain the title compound (0.69 g, 51.7%) as a pale yellow solid.
¹H-NMR (CDCl₃) δ : 1.29 (6H, d, J=6.9Hz), 1.53 (9H, s), 3.02-3.20 (1H, m), 3.05-3.13 (2H, m), 3.27-3.34 (2H, m), 6.70 (1H, s), 6.78-6.82 (1H, m), 7.87 (1H, brs), 7.93 (1H, brs), 8.13 (1H, d, J=5.1Hz)

### (B) 4-[2-(4-Isopropyl-1,3-thiazol-2-yl)ethyl]-2-pyridineamine

The tert-butyl N-4-[2-(4-isopropyl-1,3-thiazol-2-yl)ethyl]-2-pyridylcarbamate obtained in (A) was treated in the same manner as in Example 22 (C) to obtain the title compound.
¹H-NMR (CDCl₃) δ : 1.29 (6H, d, J=6.8Hz), 2.98-3.01 (2H, m), 3.01-3.07 (1H, m), 3.11-3.15 (2H, m), 4.40 (2H, brs), 6.36 (1H, s), 6.50-6.52 (1H, m), 6.71 (1H, s), 7.96 (1H, d, J=5.1Hz)

### (B) 8-[2-(4-Isopropyl-1,3-thiazol-2-yl)ethyl]-3-[2-(4-methoxybenzyl)-2H-1,2,3,4-tetrazol-5-yl]-4H-pyrido[1,2-a]pyrimidin-4-one

The 4-[2-(4-isopropyl-1,3-thiazol-2-yl)ethyl]-2-pyridineamine obtained in (B) was treated in the same manner as in Example 59 (A) to obtain the title compound.
¹H-NMR (CDCl₃) δ : 1.28 (6H, d, J=6.8Hz), 3.00-3.10 (1H, m), 3.26-3.35 (2H, m), 3.35-3.43 (2H, m), 3.79 (3H, s), 5.80 (2H, s), 6.73 (1H, d, J=1.0Hz), 6.88 (2H, d, J=8.8Hz), 7.14 (1H, dd, J=7.3, 1.8Hz), 7.41 (2H, d, J=8.8Hz), 7.57 (1H, s), 8.11 (1H, d, J=5.1Hz), 9.18 (1H, s)

### (C) 8-[2-(4-Isopropyl-1,3-thiazol-2-yl)ethyl]-3-(2H-1,2,3,4-tetrazol-5-yl)-4H-pyrido[1,2-a]pyrimidin-4-one

The 8-[2-(4-isopropyl-1,3-thiazol-2-yl)ethyl]-3-[2-(4-methoxybenzyl)-2H-1,2,3,4-tetrazol-5-yl]-4H-pyrido[1,2-a]pyrimidin-4-one obtained in (C) was treated in the same manner as in Example 59 (B) to obtain the title compound.
¹H-NMR (DMSO-d₆) δ : 1.20 (6H, d, J=6.9Hz), 2.92-3.02 (1H, m), 3.30 (2H, t, J=7.1Hz), 3.43 (2H, t, J=7.1Hz), 7.08 (1H, d, J=0.7Hz), 7.57 (1H, dd, J=6.9, 0.7Hz), 7.77 (1H, s), 9.05-9.20 (2H, m)
EI-MS; m/z: 367 (M⁺)
FAB-MS; m/z: 368 (MH⁺)

### Example 62: 8-[3-(4-Isopropyl-1,3-thiazol-2-yl)propyl]-3-(2H-1,2,3,4-tetrazol-5-yl)-4H-pyrido[1,2-a]pyrimidin-4-one

### (A) Methyl 4-2-[(tert-buthoxycarbonyl)amino]-4-pyridylbutanoate

tert-Butyl N-(4-methyl-2-pyridyl)carbamate (2.04 g, 9.80 mmol) was dissolved in THF (50 ml), and added dropwise with a 1.5 N solution of n-butyllithium in hexane (16.3 ml, 24.5 mmol) under nitrogen flow at -78°C over 5 minutes. The mixture was warmed to room temperature, stirred for 30 minutes, then cooled again to -78°C, added dropwise with methyl 3-bromopropionate (3.21 ml, 29.4 mmol), and stirred at the same temperature over 30 minutes. The reaction mixture was added with water, warmed to room temperature, then extracted with ethyl acetate, washed with saturated brine, and then dried over anhydrous sodium sulfate. The solvent was evaporated, and the residue was purified by silica gel column chromatography (chloroform→chloroform: ethyl acetate) to obtain the title compound (1.10 g, 38.2%) as a mixture of a colorless oil and a solid.
¹H-NMR (CDCl₃) δ : 1.53 (9H, s), 1.93-2.05 (2H, m), 2.34 (2H, t, J=7.5Hz), 2.65 (2H, t, J=7.7Hz), 3.67 (3H, s), 6.79 (1H, dd, J=5.1, 1.2Hz), 7.81 (1H, brs), 8.11 (1H, brs), 8.15 (1H, d, J=5.1Hz)

### (B) 4-2-[(tert-Buthoxycarbonyl)amino]-4-pyridylbutanoic acid

The methyl 4-2-[(tert-buthoxycarbonyl)amino]-4-pyridylbutanoate obtained in (A) was treated in the same manner as in Example 11 (B) to obtain the title compound.
¹H-NMR (CDCl₃) δ : 1.54 (9H, s), 1.97-2.06 (2H, m), 2.39 (2H, t, J=7.1Hz), 2.70 (2H, t, J=7.6Hz), 6.82 (1H, dd, J=5.4, 1.5Hz), 7.87 (1H, brs), 8.00 (1H, d, J=5.4Hz), 8.32 (1H, brs)

### (C) tert-Butyl N-[4-(4-amino-4-oxobutyl)-2-pyridyl]carbamate

The 4-2-[(tert-buthoxycarbonyl)amino]-4-pyridylbutanoic acid obtained in (B) (0.38 g, 1.36 mmol) was dissolved in THF (20 ml), added dropwise with triethylamine (189.1 µl, 1.36 mmol), and with ethyl chloroformate (129.6 µl, 1.36 mmol) under ice cooling, and stirred at the same temperature for 15 minutes. A solution of concentrated aqueous ammonia (28%, 10 ml) in THF (10 ml) was added dropwise, and the mixture was stirred at the same temperature for 15 minutes. After the THF was evaporated, the residue was added with a small amount of water, extracted with chloroform, washed with saturated brine, and then dried over anhydrous sodium sulfate. The solvent was evaporated to obtain the title compound (0.46 g, quantitative yield) as a colorless solid.
¹H-NMR (CDCl₃) δ : 1.53 (9H, s), 1.97-2.07 (2H, m), 2.23 (2H, t, J=7.5Hz), 2.67 (2H, t, J=7.5Hz), 5.31-5.47 (1H, brs), 5.47-5.60 (1H, brs), 6.81 (1H, d, J=5.1Hz), 7.78 (1H, brs), 7.80 (1H, brs), 8.14 (1H, d, J=5.1Hz)

### (D) tert-Butyl N-[4-(4-amino-4-thioxobutyl)-2-pyridyl]carbamate

The tert-butyl N-[4-(4-amino-4-oxobutyl)-2-pyridyl]carbamate obtained in (C) was treated in the same manner as in Example 11 (D) to obtain the title compound.
¹H-NMR (CDCl₃) δ : 1.52 (9H, s), 2.13-2.22 (2H, m), 2.62 (2H, t, J=7.3Hz), 2.69 (2H, t, J=7.3Hz), 6.82 (1H, d, J=5.1Hz), 6.25-7.47 (3H, m), 7.72 (1H, brs), 8.13 (1H, d, J=5.1Hz)

### (E) tert-Butyl N-4-[3-(4-isopropyl-1,3-thiazol-2-yl)propyl]-2-pyridylcarbamate

The tert-butyl N-[4-(4-amino-4-thioxobutyl)-2-pyridyl]carbamate obtained in (D) (0.36 g, 1.22 mmol) was suspended in ethanol (10 ml) and added with 1-bromo-3-methyl-2-butanone (0.20 g, 1.22 mmol), and the mixture was heated under reflux for 1 hour. After the solvent was evaporated, the residue was added with saturated aqueous sodium hydrogencarbonate, extracted with chloroform, washed with saturated brine, and then dried over anhydrous sodium sulfate. The solvent was evaporated to obtain the title compound (0.40 g, 90.8%) as a pale yellow oil.
¹H-NMR (CDCl₃) δ : 1.29 (6H, d, J=6.9Hz), 1.54 (9H, s), 2.08-2.20 (2H, m), 2.71 (2H, t, J=7.7Hz), 3.01 (2H, t, J=7.7Hz), 3.02-3.12 (1H, m), 6.71 (1H, d, J=1.0Hz), 6.80 (1H, dd, J=5.1, 1.2Hz), 7.85 (1H, brs), 8.19 (1H, d, J=5.1Hz), 8.89 (1H, brs)

### (F) 4-[3-(4-Isopropyl-1,3-thiazol-2-yl)propyl]-2-pyridineamine

The tert-butyl N-4-[3-(4-isopropyl-1,3-thiazol-2-yl)propyl]-2-pyridylcarbamate obtained in (E) was treated in the same manner as in Example 22 (C) to obtain the title compound.
¹H-NMR (CDCl₃) δ : 1.30 (6H, d, J=6.9Hz), 2.03-2.18 (2H, m), 2.60 (2H, t, J=7.7Hz), 3.00 (2H, t, J=7.6Hz), 3.00-3.12 (1H, m), 4.41 (2H, brs), 6.35 (1H, s), 6.51 (1H, d, J=5.1Hz), 6.72 (1H, brs), 7.95 (1H, d, J=5.1Hz)

### (G) 8-[3-(4-Isopropyl-1,3-thiazol-2-yl)propyl]-3-[2-(4-methoxybenzyl)-2H-1,2,3,4-tetrazol-5-yl]-4H-pyrido[1,2-a]pyrimidin-4-one

The 4-[3-(4-isopropyl-1,3-thiazol-2-yl)propyl]-2-pyridineamine obtained in (F) was treated in the same manner as in Example 59 (A) to obtain the title compound.
¹H-NMR (CDCl₃) δ : 1.30 (6H, d, J=7.1Hz), 2.20-2.30 (2H, m), 2.91 (2H, t, J=7.7Hz), 2.98-3.08 (1H, m), 3.09 (2H, t, J=7.4Hz), 3.79 (3H, s), 5.81 (2H, s), 6.74 (1H, s), 6.89 (2H, d, J=8.8Hz), 7.17 (1H, dd, J=7.1, 1.8Hz), 7.42 (2H, d, J=8.8Hz), 7.59 (1H, brs), 9.20 (1H, d, J=7.1Hz), 9.20 (1H, s)

### (H) 8-[3-(4-Isopropyl-1,3-thiazol-2-yl)propyl]-3-(2H-1,2,3,4-tetrazol-5-yl)-4H-pyrido[1,2-a]pyrimidin-4-one

The 8-[3-(4-isopropyl-1,3-thiazol-2-yl)propyl]-3-[2-(4-methoxybenzyl)-2H-1,2,3,4-tetrazol-5-yl]-4H-pyrido[1,2-a]pyrimidin-4-one obtained in (G) was treated in the same manner as in Example 59 (B) to obtain the title compound.
¹H-NMR (DMSO-d₆) δ : 1.22 (6H, d, J=7.1Hz), 2.10-2.20 (2H, m), 2.90-3.08 (5H, m), 7.08 (1H, s), 7.51 (1H, d, J=7.1Hz), 7.73 (1H, brs), 9.08 (1H, s), 9.08-9.17 (1H, m)
EI-MS; m/z: 381 (M⁺)
FAB-MS; m/z: 382 (MH⁺)

### Example 63: 8-[(4-Isopropyl-1,3-thiazol-2-yl)carbonyl]-3-(2H-1,2,3,4-tetrazol-5-yl)-4H-pyrido[1,2-a]pyrimidin-4-one

### (A) Ethyl 2-(2-(tert-buthoxycarbonyl)amino)-4-pyridyl acetate

tert-Butyl N-(4-methyl-2-pyridyl)carbamate (3.08 g, 14.8 mmol) was dissolved in THF (80 ml), and added dropwise with n-butyllithium (1.5 M solution in hexane, 20 ml) at -78°C under argon atmosphere, and then the reaction mixture was warmed to room temperature, and stirred at room temperature for 30 minutes. The mixture was cooled again to -78°C, added dropwise with a solution of ethyl chloroformate (1.42 ml) in THF (10 ml), and stirred at the same temperature for 1 hour. After a saturated aqueous ammonium chloride was added, the reaction mixture was distributed in ethyl acetate and saturated brine. The organic layer was dried over magnesium sulfate, the solvent was evaporated, and then the residue was purified by silica gel chromatography (chloroform) to obtain the title compound (2.6 g, 63%) as white powder.
¹H-NMR (CDCl₃) δ : 1.27 (3H, t, J=7.1Hz), 1.54 (9H, s), 3.61 (2H, s), 4.17 (2H, q, J=7.1Hz), 6.91 (1H, dd, J=5.1, 1.5Hz), 7.91 (1H, s), 8.22 (1H, d, J=5.1Hz), 8.49 (1H, brs)

### (B) tert-Butyl N-(4-(2-amino-2-oxoethyl)-2-pyridyl)carbamate

The ethyl 2-(2-(tert-buthoxycarbonyl)amino)-4-pyridylacetate obtained in (A) (550 mg, 1.96 mmol) was dissolved in THF (5 ml), added with 28% aqueous ammonia (10 ml), and stirred at room temperature for 6 days. The deposited solid was collected by filtration and dried to obtain the title compound (130 mg). The filtrate was extracted with chloroform, the extract was dried over magnesium sulfate, and then the solvent was evaporated to obtain 250 mg of the title compound (the total yield 350 mg, 71%).
¹H-NMR (CDCl₃) δ : 1.53 (9H, s), 3.57 (2H, s), 6.94 (1H, dd, J=5.1, 1.5Hz), 7.61 (1H, s), 7.90 (1H, s), 8.21 (1H, d, J=5.1Hz)

### (C) tert-Butyl N-[4-(2-amino-2-thioxoethyl)-2-pyridyl]carbamate

The tert-butyl N-(4-(2-amino-2-oxoethyl)-2-pyridyl)carbamate obtained in (B) was treated in the same manner as in Example 11 (D) to obtain the title compound.
¹H-NMR (CDCl₃) δ : 1.54 (9H, s), 3.38-3.11 (2H, m), 3.99 (2H, s), 7.10 (1H, dd, J=5.1, 0.7Hz), 7.32 (1H, brs), 7.83 (1H, brs), 8.15 (1H, d, J=5.1Hz)

### (D) (2-Amino-4-pyridyl)(4-isopropyl-1,3-thiazol-2-yl)methanone

The tert-butyl N-[4-(2-amino-2-thioxoethyl)-2-pyridyl]carbamate obtained in (C) (0.37 g, 1.38 mmol) was suspended in ethanol (10 ml), added with 1-bromo-3-methyl-2-butanone (0.23 g, 1.38 mmol), and heated under reflux for 1 hour. Since the material remained according to TLC, 1-bromo-3-methyl-2-butanone (0.23 g, 1.38 mmol) was further added, and the mixture was heated under reflux for another 1 hour. After the solvent was evaporated, the residue was added with saturated aqueous sodium hydrogencarbonate and extracted with chloroform, the extract was washed with saturated brine and then dried over anhydrous sodium sulfate, and the solvent was evaporated. The residue was purified by preparative TLC (chloroform:methanol = 30:1, v/v) to obtain tert-butyl N-4-[(4-isopropyl-1,3-thiazol-2-yl)carbonyl]-2-pyridylcarbamate (94.8 mg) as a pale orange oil and the title compound (71.9 mg, 21.0%) as a pale orange oil. The tert-butyl N-4-[(4-isopropyl-1,3-thiazol-2-yl)carbonyl]-2-pyridylcarbamate (94.8 mg) was dissolved in methylene chloride (2 ml), and added dropwise with trifluoroacetic acid (2 ml) under ice cooling, and the mixture was stirred at room temperature for 1 hour. After the solvent was evaporated, the residue was added with saturated aqueous sodium hydrogencarbonate, and extracted with chloroform, the extract was washed with saturated brine, dried over anhydrous sodium sulfate, and the solvent was evaporated to obtain the title compound (68.8 mg). (The total yield, 140.7 mg, 41.1%).
¹H-NMR (CDCl₃) δ : 1.37 (6H, d, J=6.9Hz), 3.17-3.28 (1H, m), 4.74 (2H, brs), 7.33 (1H, d, J=0.7Hz), 7.51 (1H, brs), 7.60 (1H, dd, J=5.3, 1.3Hz), 8.25 (1H, dd, J=5.3, 0.6Hz)

### (E) 8-[(4-Isopropyl-1,3-thiazol-2-yl)carbonyl]-3-[2-(4-methoxybenzyl)-2H-1,2,3,4-tetrazol-5-yl]-4H-pyrido[1,2-a]pyrimidin-4-one

The (2-amino-4-pyridyl)(4-isopropyl-1,3-thiazol-2-yl)methanone obtained in (D) was treated in the same manner as in Example 59 (A) to obtain the title compound.
¹H-NMR (CDCl₃) δ : 1.41 (6H, d, J=7.1Hz), 3.24-3.32 (1H, m), 3.80 (3H, s), 5.84 (2H, s), 6.90 (2H, d, J=8.8Hz), 7.43 (2H, d, J=8.8Hz), 7.46 (1H, s), 8.06 (1H, dd, J=7.6, 1.7Hz), 9.17 (1H, d, J=1.7Hz), 9.34 (1H, d, J=7.6Hz), 9.34 (1H, s)

### (F) 8-[(4-Isopropyl-1,3-thiazol-2-yl)carbonyl]-3-(2H-1,2,3,4-tetrazol-5-yl)-4H-pyrido[1,2-a]pyrimidin-4-one

The 8-[(4-isopropyl-1,3-thiazol-2-yl)carbonyl]-3-[2-(4-methoxybenzyl)-2H-1,2,3,4-tetrazol-5-yl]-4H-pyrido[1,2-a]pyrimidin-4-one obtained in (E) was treated in the same manner as in Example 59 (B) to obtain the title compound.
¹H-NMR (DMSO-d₆) δ : 1.36 (6H, d, J=6.9Hz), 3.20-3.30 (1H, m), 7.89-7.96 (1H, m), 8.03 (1H, s), 8.98-9.02 (1H, m), 9.02-9.11 (1H, m), 9.17-9.23 (1H, m)
EI-MS; m/z: 367 (M⁺)

### Example 64: 8-2-[4-(tert-Butyl)-1,3-thiazol-2-yl]ethyl-3-(2H-1,2,3,4-tetrazol-5-yl)-4H-pyrido[1,2-a]pyrimidin-4-one

### (A) tert-Butyl N-(4-2-[4-(tert-butyl)-1,3-thiazol-2-yl]ethyl-2-pyridyl)carbamate

tert-Butyl N-(4-methyl-2-pyridyl)carbamate (1.74 g, 8.35 mmol) was dissolved in THF (50 ml), and added dropwise with a 1.63 N solution of n-butyllithium in hexane (12.8 ml, 20.9 mmol) at -78°C over 10 minutes. The mixture was warmed to room temperature, stirred for 30 minutes, cooled again to -78°C, added dropwise with a solution of 2-(bromomethyl)-4-(tert-butyl)-1,3-thiazol (2.94 g, 12.6 mmol) in THF (10 ml) over 10 minutes, and stirred at the same temperature for 30 minutes. The reaction mixture was added with water, warmed to room temperature, and extracted with ethyl acetate, the extract was washed with saturated brine and then dried over anhydrous sodium sulfate, and the solvent was evaporated. The residue was purified by silica gel column chromatography (chloroform) to obtain the title compound (1.90 g, 62.9%) as a pale yellow solid.
¹H-NMR (CDCl₃) δ : 1.33 (9H, s), 1.53 (9H, s), 3.10 (2H, t, J=7.9Hz), 3.30 (2H, t, J=7.9Hz), 6.71 (1H, d, J=0.5Hz), 6.80 (1H, d, J=5.1Hz), 7.87 (1H, brs), 8.12 (1H, brs), 8.14 (1H, d, J=5.1Hz)

### (B) 4-2-[4-(tert-Butyl)-1,3-thiazol-2-yl]ethyl-2-pyridineamine

The tert-butyl N-(4-2-[4-(tert-butyl)-1,3-thiazol-2-yl]ethyl-2-pyridyl)carbamate obtained in (A) was treated in the same manner as in Example 22 (C) to obtain the title compound.
¹H-NMR (CDCl₃) δ : 1.33 (9H, s), 2.98 (2H, t, J=7.8Hz), 3.26 (2H, t, J=7.8Hz), 4.47 (2H, brs), 6.36 (1H, s), 6.51 (1H, dd, J=5.1, 0.5Hz), 6.72 (1H, s), 7.95 (1H, d, J=5.4Hz)

### (C) 8-2-[4-(tert-Butyl)-1,3-thiazol-2-yl]ethyl-3-[2-(4-methoxybenzyl)-2H-1,2,3,4-tetrazol-5-yl]-4H-pyrido[1,2-a]pyrimidin-4-one

The 4-2-[4-(tert-butyl)-1,3-thiazol-2-yl]ethyl-2-pyridineamine obtained in (B) was treated in the same manner as in Example 59 (A) to obtain the title compound.
¹H-NMR (CDCl₃) δ : 1.30 (9H, s), 3.32 (2H, t, J=7.1Hz), 3.41 (2H, t, J=7.1Hz), 3.78 (3H, s), 5.80 (2H, s), 6.73 (1H, s), 6.88 (2H, d, J=8.7Hz), 7.15 (1H, dd, J=7.3, 1.7Hz), 7.41 (2H, d, J=8.7Hz), 7.58 (1H, brs), 9.16 (1H, d, J=7.3Hz), 9.18 (1H, s)

### (D) 8-2-[4-(tert-Butyl)-1,3-thiazol-2-yl]ethyl-3-(2H-1,2,3,4-tetrazol-5-yl)-4H-pyrido[1,2-a]pyrimidin-4-one

The 8-2-[4-(tert-butyl)-1,3-thiazol-2-yl]ethyl-3-[2-(4-methoxybenzyl)-2H-1,2,3,4-tetrazol-5-yl]-4H-pyrido[1,2-a]pyrimidin-4-one obtained in (C) was treated in the same manner as in Example 59 (B) to obtain the title compound.
¹H-NMR (DMSO-d₆) δ : 1.23 (9H, s), 3.30 (2H, t, J=7.1Hz), 3.43 (2H, t, J=7.1Hz), 7.07 (1H, s), 7.54 (1H, d, J=6.9Hz), 7.73 (1H, brs), 9.04 (1H, s), 9.09 (1H, brd, J=5.4Hz)
EI-MS; m/z: 381 (M⁺)
FAB-MS; m/z: 382 (MH⁺)

### Example 65: (E)-3-(2-Hydroxy-8-[2-(4-isopropyl-1,3-thiazol-2-yl)-1-ethenyl]-4H-pyrido[1,2-a]pyrimidin-4-one-3-yl)-2-propenoic acid

### (A) 2-Hydroxy-8-[2-(4-isopropyl-1,3-thiazol-2-yl)-1-ethenyl]-4H-pyrido[1,2-a]pyrimidin-4-one

4-[2-(4-Isopropyl-1,3-thiazol-2-yl)-1-ethenyl]-2-aminopyridine (1.15 g, 4.65 mmol) and di(2,4,6-trichlorophenyl) malonate (2.2 g, 4.75 mmol) were heated under reflux in xylene (6 ml) for 1 hour, and then purified by silica gel chromatography (chloroform-methanol, 20:1, v/v) to obtain the title compound (900 mg, 61%) as white powder.
¹H-NMR (CDCl₃) δ : 1.29 (6H, d, J=6.9Hz), 3.06 (1H, m), 3.34 (2H, m), 3.39 (2H, m), 5.33 (1H, s), 6.73 (1H, s), 7.09 (1H, dd, J=7.1, 1.7Hz), 7.38 (1H, s), 9.01 (1H, d, J=7.1Hz)

### (B) tert-Butyl (E)-3-(2-hydroxy-8-[2-(4-isopropyl-1,3-thiazol-2-yl)-1-ethenyl]-4H-pyrido[1,2-a]pyrimidin-4-one-3-yl)-2-propenoate

Oxalyl chloride (100 µL) was dissolved in methylene chloride (5 ml), added with DMF (155 µL) under ice cooling, and stirred for 15 minutes. The mixture was added with the 2-hydroxy-8-[2-(4-isopropyl-1,3-thiazol-2-yl)-1-ethenyl]-4H-pyrido[1,2-a]pyrimidin-4-one obtained in (A) (95 mg, 0.30 mmol) as powder as it was, and stirred at room temperature for 3 hours. The reaction mixture was neutralized with saturated aqueous sodium hydrogencarbonate and extracted with ethyl acetate, the extract was washed with saturated brine and dried over magnesium sulfate, then the solvent was evaporated under reduced pressure. The residue was purified by preparative thin layer chromatography (chloroform-methanol, 93:7, v/v) to obtain 2-hydroxy-8-[2-(4-isopropyl-1,3-thiazol-2-yl)-1-ethenyl]-4H-pyrido[1,2-a]pyrimidin-4-o ne-3-carbaldehyde (76 mg). This compound was added with (tert-buthoxycarbonylmethylene)triphenylphosphorane (255 mg) and THF (5 ml), and the mixture was stirred with heating at 60°C for 10 hours. After the solvent was evaporated under reduced pressure, the residue was purified by preparative thin layer chromatography (chloroform-methanol, 9:1, v/v) to obtain yellow oil (64 mg, 50%).
¹H-NMR (CDCl₃-CD₃OD) δ : 1.28 (6H, d, J=6.8Hz), 1.51 (9H, s), 3.05 (1H, m), 3.22 (2H, m), 3.42 (2H, m), 6.81 (1H, s), 6.97 (1H, d, J=15.9Hz), 7.23 (1H, s), 7.27 (1H, dd, J=7.1, 1.7Hz), 7.95 (1H, d, J=15.9Hz), 9.01 (1H, d, J=7.1Hz)

### (C) (E)-3(-2-Hydroxy-8-[2-(4-isopropyl-1,3-thiazol-2-yl)-1-ethenyl]-4H-pyrido[1,2-a]pyrimidin-4-one-3-yl)-2-propenoic acid

The tert-butyl (E)-3-(2-hydroxy-8-[2-(4-isopropyl-1,3-thiazol-2-yl)-1-ethenyl]-4H-pyrido[1,2-a]pyrimidin-4-one-3-yl)-2-propenoate obtained in (B) (30 mg, 0.068 mmol) was dissolved in trifluoroacetic acid (1 ml), and stirred for 20 minutes. After the solvent was evaporated under reduced pressure, the residue was purified by preparative thin layer chromatography (chloroform-methanol, 9:1, v/v) to obtain the title compound (10 mg, 38%) as yellow powder.
¹H-NMR (CDCl₃-CD₃OD) δ : 1.28 (6H, d, J=6.8Hz), 3.06 (1H, m), 3.33 (2H, m), 3.41 (2H, m), 6.82 (1H, s), 7.03 (1H, d, J=15.9Hz), 7.23 (2H, m)8.06 (1H, d, J=15.9Hz), 9.01 (1H, d, J=7.1Hz)

### Example 66: 2-Hydroxy-8-[2-(4-isopropyl-1,3-thiazol-2-yl)-1-ethenyl]-3-[(1H-1,2,3,4-tetrazol-5-ylimino)methyl]-4H-pyrido[1,2-a]pyrimidin-4-one

The 2-hydroxy-8-[2-(4-isopropyl-1,3-thiazol-2-yl)-1-ethenyl]-4H-pyrido[1,2-a]pyrimidin-4-one-3-carbaldehyde obtained in Example 65 (B) (50 mg) and 5-aminotetrazol (50 mg) were suspended in methanol (10 ml), and heated under reflux for 9 hours. After the solvet was evaporated under reduced pressure, the residue was purified by preparative thin layer chromatography (chloroform-methanol, 9:1, v/v) to obtain the title compound (40 mg, 67%, E, Z mixture) as yellow powder.
¹H-NMR (CDCl₃-CD₃OD) δ : 1.29 (6H, m), 3.08 (1H, m), 3.13 (2H, m), 3.35 (2H, m), 3.85 (1H, s), 6.79 (1H, s), 6.92 (1H, m), 8.1-8.4 (total 2H, m), 8.96, 9.18 (total 1H, each s)

### Example 67: 2-Hydroxy-8-[2-(4-isopropyl-1,3-thiazol-2-yl)-1-ethenyl]-3-[(1H-1,2,3,4-tetrazol-5-ylimino)methyl]-4H-pyrido[1,2-a]pyrimidin-4-one

### (A) (E)-3-(2-Hydroxy-8-[2-(4-isopropyl-1,3-thiazol-2-yl)-1-ethenyl]-4H-pyrido[1,2-a]pyrimidin-4-one-3-yl)-2-propenenitrile

The 2-hydroxy-8-[2-(4-isopropyl-1,3-thiazol-2-yl)-1-ethenyl]-4H-pyrido[1,2-a]pyrimidin-4-one-3-carbaldehyde obtained in Example 65 (B) (500 mg,1.46 mmol) and cyanomethyltriphenylphosphorane (660 mg) were dissolved in THF (15 ml), and stirred for 30 minutes. The solvent was evaporated under reduced pressure, the residue was added with methylene chloride and ether, and the deposited solid was collected by filtration and dried to obtain the title compound (255 mg, 48%) as a yellow solid.
¹H-NMR (CDCl₃-CD₃OD) δ : 1.28 (6H, d, J=6.8Hz), 3.06 (1H, m), 3.32 (2H, m), 3.41 (2H, m), 6.61 (1H, d, J=16.4Hz), 6.82 (1H, d, J=1.0Hz), 7.22 (2H, m), 7.71 (1H, d, J=16.4Hz), 9.001H, d, J=7.6Hz)

### (B) 2-Hydroxy-8-[2-(4-isopropyl-1,3-thiazol-2-yl)-1-ethenyl]-3-[(1H-1,2,3,4-tetrazol-5-ylimino)methyl]-4H-pyrido[1,2-a]pyrimidin-4-one

Aluminum chloride (112 mg, 0.842 mmol) was suspended in DMF (3 ml), added with sodium azide (180 mg, 2.77 mmol), and stirred for 10 minutes. The mixture was added with the (E)-3-(2-hydroxy-8-[2-(4-isopropyl-1,3-thiazol-2-yl)-1-ethenyl]-4H-pyrido[1,2-a]pyrimidin-4-one-3-yl)-2-propenenitrile obtained in (A) (100 mg, 0.273 mmol), and stirred at 100°C for 2 days. After ice and 1 M hydrochloric acid were added, the resulting homogeneous solution was adjusted to pH 4-5 with saturated aqueous sodium hydrogencarbonate, and the deposited solid was collected by filtration, and purified by preparative thin layer chromatography (chloroform-methanol-water, 8:3:1, v/v, the low layer solution) to obtain the title compound (18 mg, 16%) as yellow powder.
¹H-NMR (DMSO-d₆) δ : 1.20 (6H, d, J=7.6Hz), 3.0-3.3 (5H, m), 7.09 (1H, s), 7.15 (1H, s), 7.26 (1H, m), 7.74 (2H, s), 8.88 (1H, d, J=5.9Hz)

### Example 68: N-(1H-1,2,3,4-Tetrazol-5-yl)-1-ethyl-7-[(E)-2-(4-isopropyl-1,3-thiazol-2-yl)-1-ethenyl]-4-oxo-1,4-dihydro-3-quinolinecarboxamide

### (A) Diethyl 2-(ethyl-3-[(E)-2-(4-isopropyl-1,3-thiazol-2-yl)-1-ethenyl]anilinomethylene)malonate

A solution of N-ethyl-3-[(E)-2-(4-isopropyl-1,3-thiazol-2-yl)-1-ethenyl]aniline (297.4 mg, 1.09 mmol) and diethyl ethoxymethylenemalonate (0.230 ml, 1.14 mmol) in toluene (5 ml) was heated under reflux for 14 hours. The solvent was evaporated, and the resulting residue was purified by silica gel column chromatography (hexane:ethyl acetate = 85:15 → 1:1) to obtain the title compound (448.3 mg, 92.8%) as yellow oil.

### (B) Ethyl 1-ethyl-5-[(E)-2-(4-isopropyl-1,3-thiazol-2-yl)-1-ethenyl]-4-oxo-1,4-dihydro-3-quinolinecarboxylate (isomer A) and ethyl 1-ethyl-7-[(E)-2-(4-isopropyl-1,3-thiazol-2-yl)-1-ethenyl]-4-oxo-1,4-dihydro-3-quinolinecarboxylate (isomer B)

Pyrophosphoric acid (3 ml) was added to diethyl 2-{ethyl-3-[(E)-2-(4-isopropyl-1,3-thiazol-2-yl)-1-ethenyl]anilinomethylene}malonate (448.3 mg, 1.01 mmol), and the mixture was stirred with heating at 110°C for 45 minutes. The reaction mixture was added with water, and extracted with ethyl acetate. The organic layer was washed with water and saturated brine and dried over anhydrous sodium sulfate, then the solvent was evaporated, and the resulting residue was purified by PTLC (chloroform:methanol = 97:3) to obtain isomer A (148.0 mg, 36.8%) as a colorless solid, and then isomer B (189.4 mg, 47.2%) as a colorless solid. Isomer A
¹H-NMR (CDCl₃) δ : 1.33 (6H, d, J=6.8Hz), 1.40 (3H, t, J=7.1Hz), 1.53 (3H, t, J=7.2Hz), 3.11 (1H, dq, J=6.8Hz), 4.24 (2H, q, 7.2Hz), 4.38 (2H, q, J=7.1Hz), 6.80 (1H, s), 7.03 (1H, d, J=16.1Hz), 7.42 (1H, d, J=8.5Hz), 7.49 (1H, d, J=7.6Hz), 7.62 (1H, t, J=8.5Hz), 8.42 (1H, s), 8.81 (1H, d, J=16.1Hz)
Isomer B
¹H-NMR (CDCl₃) δ : 1.35 (6H, d, J=7.1Hz), 1.42 (3H, t, J=7.1Hz), 1.58 (3H, t, J=7.2Hz), 3.15 (1H, dq, J=6.8Hz), 4.28 (2H, q, 7.2Hz), 4.40 (2H, q, J=7.1Hz), 6.90 (1H, s), 7.45 (2H, br s), 7.51 (1H, br s), 7.03 (1H, d, J=16.1Hz), 7.42 (1H, d, J=8.5Hz), 7.59 (1H, dd, J=8.5, 1.5Hz), 8.49 (1H, s), 8.51 (1H, d, J=8.5Hz)

### (C) 1-Ethyl-7-[(E)-2-(4-isopropyl-1,3-thiazol-2-yl)-1-ethenyl]-4-oxo-1,4-dihydro-3-quinolinecarboxylic acid

1 N Aqueous sodium hydroxide (0.707 ml, 0.71 mmol) was added to a solution of ethyl 1-ethyl-7-[(E)-2-(4-isopropyl-1,3-thiazol-2-yl)-1-ethenyl]-4-oxo-1,4-dihydro-3-quinolinecarboxylate (186.8 mg, 0.47 mmol) in a mixture of methanol (1.5 ml)-tetrahydrofuran (1.5 ml)-water (1.5 ml), and the resulting mixture was stirred at room temperature for 6.5 hours. After the solvent was evaporated, water was added, 1 N hydrochloric acid (0.707 ml) was added, and the deposited crystals were collected by filtration, washed with water and dried to obtain the title compound (137.8 mg, 79.4%) as a colorless solid.
¹H-NMR (DMSO-d₆) δ : 1.29 (6H, d, J=6.8Hz), 1.62 (3H, t, J=7.1Hz), 3.09 (1H, dq, J=6.8Hz), 4.66 (2H, q, J=7.1Hz), 7.36 (1H, s), 7.70 (1H, d, J=16.1Hz), 7.88 (1H, d, J=16.1Hz), 8.05 (1H, d, J=8.3Hz), 8.30 (1H, s), 8.35 (1H, d, J=8.3Hz), 9.06 (1H, s)

### (D) N-(1H-1,2,3,4-Tetrazol-5-yl)-1-ethyl-7-[(E)-2-(4-isopropyl-1,3-thiazol-2-yl)-1-ethenyl]-4-oxo-1,4-dihydro-3-quinolinecarboxamide

A solution of 1-ethyl-7-[(E)-2-(4-isopropyl-1,3-thiazol-2-yl)-1-ethenyl]-4-oxo-1,4-dihydro-3-quinolinecarboxylic acid (39.6 mg, 0.11 mmol) in dimethylformamide (2 ml) was added with ethyl chloroformate (15.3 µl, 0.16 mmol) and triethylamine (22.5 µl, 0.16 mmol) under ice cooling, and the mixture was stirred at the same temperature for 3 hours. Then, 5-aminotetrazol (13.7 mg, 0.16 mmol) and triethylamine (44.9 µl, 0.32 mmol) were added, and the mixture was stirred at room temperature overnight. After the solvent was evaporated, water was added, and the deposited solid was collected by filtration, washed thoroughly with chloroform, and dried to obtain the title compound (14.6 mg, 31.2%) as a pale yellow solid.
¹H-NMR (DMSO-d₆) δ : 1.29 (6H, d, J=6.8Hz), 1.48 (3H, t, J=7.1Hz), 3.09 (1H, dq, J=6.8Hz), 4.65-4.75 (2H, m), 7.35 (1H, s), 7.70 (1H, d, J=16.4Hz), 7.86 (1H, d, J=16.4Hz), 8.02 (1H, d, J=8.3Hz), 8.27 (1H, s), 8.40 (1H, d, J=8.3Hz), 9.08 (1H, s).
FAB-MS; m/z: 436 (MH⁺)

### Example 69: 1-Ethyl-7-[(E)-2-(4-isopropyl-1,3-thiazol-2-yl)-1-ethenyl]-3-(1H-1,2,3,4-tetrazol-5-yl)-1,4-dihydro-4-quinolinone

### (A) 1-Ethyl-5-[(E)-2-(4-isopropyl-1,3-thiazol-2-yl)-1-ethenyl]-4-oxo-1,4-dihydro-3-quinolinecarboxamide (isomer A) and 1-ethyl-7-[(E)-2-(4-isopropyl-1,3-thiazol-2-yl)-1-ethenyl]-4-oxo-1,4-dihydro-3-quinolinecarboxamide (isomer B)

A solution (5 ml) of N-ethyl-3-[(E)-2-(4-isopropyl-1,3-thiazol-2-yl)-1-ethenyl]aniline (203.2 mg, 0.75 mmol) and ethyl 2-cyano-3-ethoxy-2-propenoate (total 252.4 mg, 1.49 mmol) in toluene was heated under reflux for a long period of time. The solvent was evaporated, and the resulting residue was added with pyrophosphoric acid (3 ml) and heated to 110°C with stirring for 1 hour. The reaction mixture was added with water and extracted with ethyl acetate. The organic layer was washed with water and saturated brine, and dried over anhydrous sodium sulfate, then the solvent was evaporated, and the resulting residue was purified by silica gel column chromatography (chloroform → chloroform:methanol = 99:1) to obtain isomer A (41.1 mg, 15.1%) as a colorless solid, then a mixture of isomers A and B (22.1 mg, 8.1%), and isomer B (65.3 mg, 23.8%) as a colorless solid.
Isomer A
¹H-NMR (CDCl₃) δ : 1.33 (6H, d, J=6.8Hz), 1.56 (3H, t, J=7.3Hz), 3.13 (1H, dq, J=6.8Hz), 4.32 (2H, q, 7.3Hz), 5.77 (1H, br s), 6.83 (1H, s), 7.08 (1H, d, J=15.9Hz), 7.51 (1H, d, J=8.5Hz), 7.56 (1H, d, J=7.3Hz), 7.70 (1H, m), 8.76 (1H, d, J=15.9Hz), 8.78 (1H, s), 9.76 (1H, br s)
FAB-MS; m/z: 368 (MH⁺)
Isomer B
¹H-NMR (CDCl₃) δ : 1.36 (6H, d, J=6.8Hz), 1.60 (3H, t, J=7.3Hz), 3.15 (1H, dq, J=6.8Hz), 4.35 (2H, q, 7.3Hz), 5.75 (1H, br s), 6.92 (1H, s), 7.49 (2H, s), 7.59 (1H, s), 7.67 (1H, dd, J=8.5, 1.1Hz), 8.52 (1H, d, J=8.5Hz), 8.80 (1H, s), 9.74 (1H, br s)
FAB-MS; m/z: 368 (MH⁺)

### (B) 1-Ethyl-7-[(E)-2-(4-isopropyl-1,3-thiazol-2-yl)-1-ethenyl]-4-oxo-1,4-dihydro-3-quinolinecarbonitrile

A solution (1 ml) of dimethylformamide (24.3 µl, 0.32 mmol) in acetonitrile was added with oxalyl chloride (25.6 µl, 0.30 mmol) under ice cooling, stirred at the same temperature for 10 minutes, then added with a solution (1 ml) of 1-ethyl-7-[(E)-2-(4-isopropyl-1,3-thiazol-2-yl)-1-ethenyl]-4-oxo-1,4-dihydro-3-quinoline carboxamide (72.0 mg, 0.20 mmol) in acetonitrile, and stirred at the same temperature for 10 minutes. The mixture was added with pyridine (47.5 µl, 0.60 mmol), and stirred at the same temperature for 15 minutes. The mixture was further added with pyridine (23.7 µl, 0.30 mmol), and stirred at room temperature for 30 minutes. After a solution (1 ml) of dimethylformamide (18.2 µl, 0.24 mmol) and oxalyl chloride (18.8 µl, 0.22 mmol) in acetonitrile previously prepared at 0°C was added, the mixture was added with pyridine (34.9 µl, 0.44 mmol), and stirred at room temperature for 20 minutes. The solvent was evaporated, and the resulting residue was added with ethyl acetate, washed in turn with 5% aqueous citric acid, saturated aqueous sodium hydrogencarbonate, water, and saturated brine, and dried over anhydrous sodium sulfate. Then the solvent was evaporated, and the resulting residue was purified by silica gel column chromatography (chloroform → chloroform:methanol = 99.5:0.5) to obtain the title compound (61.5 mg, 89.8%) as a pale yellow solid.
¹H-NMR (CDCl₃) δ : 1.36 (6H, d, J=6.8Hz), 1.55-65 (3H, m), 3.15 (1H, dq, J=6.8Hz), 4.25-4.35 (2H, m), 6.93 (1H, s), 7.47 (2H, s), 7.55 (1H, s), 7.66 (1H, dd, J=8.5, 1.2Hz), 8.05 (1H, s), 8.45 (1H, d, J=8.5Hz)
FAB-MS; m/z: 350 (MH⁺)

### (C) 1-Ethyl-7-[(E)-2-(4-isopropyl-1,3-thiazol-2-yl)-1-ethenyl]-3-(1H-1,2,3,4-tetrazol-5-yl)-1,4-dihydro-4-quinolinone

A solution (1 ml) of aluminum chloride (33.7 mg, 0.24 mmol) in dimethylformamide was added with sodium azide (54.7 mg, 0.84 mmol) under ice cooling, and stirred at room temperature for 15 minutes. Then, the mixture was added with a solution (1 ml) of 1-ethyl-7-[(E)-2-(4-isopropyl-1,3-thiazol-2-yl)-1-ethenyl]-4-oxo-1,4-dihydro-3-quinolinecarbonitrile (29.4 mg, 0.08 mmol) in dimethylformamide, and stirred at 85-90°C for 2 days. The reaction mixture was poured to ice water-1 N hydrochloric acid (0.5 ml), and the mixture was gradually added with 1 N hydrochloric acid (0.5 ml), and stirred at room temperature for 30 minutes. The deposited solid was collected by filtration, washed with water and a small amount of ethanol, and then dried to obtain the title compound (14.4 mg, 43.6%) as a colorless solid.
¹H-NMR (DMSO-d₆) δ : 1.29 (6H, d, J=6.8Hz), 1.46 (3H, t, J=7.1Hz), 3.08 (1H, m), 4.55-4.65 (2H, m), 7.34 (1H, s), 7.69 (1H, d, J=16.1Hz), 7.83 (1H, d, J=16.1Hz), 7.96 (1H, d, J=8.5Hz), 8.21 (1H, s), 8.35 (1H, d, J=8.5Hz), 9.11 (1H, s)
FAB-MS; m/z: 393 (MH⁺)

### Example 70: 1-Cyclopropyl-6-fluoro-7-[(4-isopropyl-1,3-thiazol-2-yl)methoxy]-3-(1H-1,2,3,4-tetrazol-5-yl)-1,4-dihydro-4-quinolinone

### (A) 1-Cyclopropyl-6,7-difluoro-4-oxo-1,4-dihydro-3-quinolinecarboxamide

A suspension (2 ml) of 1-cyclopropyl-6,7-difluoro-4-oxo-1,4-dihydro-3-quinolinecarboxylic acid (100.3 mg, 0.38 mmol) in dimethylformamide was added with ethyl chloroformate (54.0 µl, 0.57 mmol) and triethylamine (79.1 µl, 0.57 mmol) at 0°C under nitrogen flow, and stirred at the same temperature for 1 hour, at room temperature for 30 minutes, and at 0°C for 30 minutes, then added with aqueous ammonia (70 µl), and stirred overnight. The mixture was added with ethyl acetate, washed in turn with 5% aqueous citric acid, saturated aqueous sodium hydrogencarbonate, water, and saturated brine, and dried over anhydrous sodium sulfate. Then the solvent was evaporated to obtain the title compound (99.2 mg, 99.3%) as a colorless solid.
¹H-NMR (CDCl₃) δ : 1.15-1.25 (2H, m), 1.35-1.45 (2H, m), 3.45-3.55 (1H, m), 5.72 (1H, br s), 7.81 (1H, dd, J=11.2, 6.3Hz), 8.28 (1H, dd, J=10.3, 8.8Hz), 8.89 (1H, s), 9.52 (1H, br s)

### (B) 1-Cyclopropyl-6,7-difluoro-4-oxo-1,4-dihydro-3-quinolinecarbonitrile

A solution (1 ml) of dimethylformamide (61.9 µl, 0.79 mmol) in acetonitrile was added with oxalyl chloride (63.4 µl, 0.72 mmol) under ice cooling, and stirred at the same temperature for 15 minutes, then added with a suspension (2 ml) of 1-cyclopropyl-6,7-difluoro-4-oxo-1,4-dihydro-3-quinolinecarboxamide (96.0 mg, 0.36 mmol) in acetonitrile, and stirred at the same temperature 5 minutes. The mixture was added with pyridine (0.118 ml, 1.44 mmol), and stirred at the same temperature for 10 minutes, and at room temperature for 20 minutes. The solvent was evaporated, and the resulting residue was added with ethyl acetate, washed in turn with 5% aqueous citric acid, saturated aqueous sodium hydrogencarbonate, water, and saturated brine, and dried over anhydrous sodium sulfate. Then the solvent was evaporated to obtain the title compound (71.0 mg, 79.4%) as a colorless solid.
¹H-NMR (CDCl₃) δ : 1.10-1.20 (2H, m), 1.35-1.45 (2H, m), 3.45-3.55 (1H, m), 7.78 (1H, dd, J=11.2, 6.3Hz), 8.16 (1H, s), 8.20 (1H, dd, J=10.0, 8.8Hz)

### (C) 1-Cyclopropyl-6-fluoro-7-[(4-isopropyl-1,3-thiazol-2-yl)methoxy]-4-oxo-1,4-dihydro-3-quinolinecarbonitrile

A solution (3 ml) of sodium hydride (13.4 mg, 0.34 mmol) in dimethylformamide was added with 18-crown-6 (81.2 mg, 0.31 mmol) and 4-isopropyl-1,3-thiazol-2-ylmethanol (43.9 mg, 0.28 mmol) under nitrogen flow at room temperature, and stirred at the same temperature for 15 minutes. The reaction mixture was added with a solution (3 ml) of 1-cyclopropyl-6,7-difluoro-4-oxo-1,4-dihydro-3-quinolinecarbonitrile (68.8 mg, 0.28 mmol) in dimethylformamide, and stirred at the same temperature for 1.5 hours. The mixture was added with ethyl acetate, 1 N hydrochloric acid (0.335 ml) and water, and distributed. The ethyl acetate layer was washed with water and saturated brine, and dried over anhydrous sodium sulfate. The solvent was evaporated, and the resulting residue was purified by silica gel column chromatography (chloroform → chloroform:methanol = 99:1) to obtain the title compound (77.6 mg, 72.4%) as a pale yellow solid.
¹H-NMR (CDCl₃) δ : 1.05-1.10 (2H, m), 1.31 (6H, d, J=7.1Hz), 1.35-1.45 (2H, m), 3.05-3.15 (1H, m), 3.35-3.45 (1H, m), 5.59 (2H, s), 6.98 (1H, s), 7.63 (1H, d, J=6.6Hz), 8.07 (1H, d, J=10.5Hz), 8.08 (1H, s)

### (D) 1-Cyclopropyl-6-fluoro-7-[(4-isopropyl-1,3-thiazol-2-yl)methoxy]-3-(1H-1,2,3,4-tetrazol-5-yl)-1,4-dihydro-4-quinolinone

A solution (2 ml) of aluminum chloride (41.0 mg, 0.30 mmol) in dimethylformamide was added with sodium azide (66.6 mg, 1.02 mmol) under ice cooling, and stirred at room temperature for 20 minutes. Then the mixture was added with a solution (2 ml) of 1-cyclopropyl-6-fluoro-7-[(4-isopropyl-1,3-thiazol-2-yl)methoxy]-4-oxo-1,4-dihydro-3-quinolinecarbonitrile (39.3 mg, 0.10 mmol) in dimethylformamide, and stirred at 85-90°C for 1 day. The reaction mixture was poured to ice water-1 N hydrochloric acid (1.1 ml), and the mixture was stirred at room temperature for 30 minutes. The deposited solid was collected by filtration, washed with water and a small amount of ethanol, and then dried to obtain the title compound (13.2 mg) as a colorless solid. The mother was adjusted to pH 4 with 1 N aqueous sodium hydroxide, and the deposited solid was collected by filtration, washed with water and a small amount of ethanol, and then dried to obtain the title compound (15.9 mg, total 66.6%) as a colorless solid.
¹H-NMR (DMSO-d₆) δ : 1.10-1.20 (2H, m), 1.26 (6H, d, J=6.8Hz), 1.30-1.40 (2H, m), 3.00-3.15 (1H, m), 3.65-3.75 (1H, m), 5.77 (2H, s), 7.39 (1H, s), 7.92 (1H, d, J=7.3Hz), 8.03 (1H, d, J=11.2Hz), 8.79 (1H, s)
FAB-MS; m/z: 427 (MH⁺)

### Example 71: 1-Ethyl-6-fluoro-7-[(4-isopropyl-1,3-thiazol-2-yl)methyl]-4-oxo-1,4-dihydro-3-cinnolinecarboxylic acid

### (A) Dimethyl 2-[(3,4-difluorophenyl)hydrazono]malonate

3,4-Difluoroaniline (5.00 g, 38.7 mmol) was dissolved in 10% hydrochloric acid (15 ml), slowly added dropwise with an aqueous solution (10 ml) of sodium nitrite (2.81 g, 40.7 mmol) at 0°C, and then stirred for 30 minutes. The insoluble was removed by filtration, and the resulting solution was slowly added dropwise to a reactor filled with dimethyl malonate (8.84 g, 77.5 mmol), sodium acetate (11.4 g, 139 mmol), ethanol (100 ml) and water (20 ml) which were previously cooled, and the mixture was stirred at 0°C overnight. The deposited crystals were collected by filtration, dissolved in chloroform, and washed with water. The chloroform layer was dried, and the solvent was evaporated to obtain the title compound (9.18 g, 87%) as a yellow solid.
¹H-NMR (CDCl₃) δ : 3.88 (3H, s), 3.91 (3H, s), 6.98 (1H, m), 7.16 (1H, m), 7.29 (1H, m), 12.8 (1H, brs)

### (B) Dimethyl 2-[(3,4-difluorophenyl)-2-ethylhydrazono]malonate

The dimethyl 2-[(3,4-difluorophenyl)hydrazono]malonate obtained in (A) (7.18 g, 26.4 mmol) was dissolved in DMF (140 ml), added with 95% sodium hydride (800 mg, 31.7 mmol) under ice cooling under argon atmosphere, and stirred at room temperature for 1 hour. The mixture was cooled again with ice, added with ethyl iodide (4.24 ml, 52.8 mmol), stirred at room temperature for 1 hour, and then stirred at 75°C overnight. The mixture was left stand for cooling, then added with acetic acid (10 ml), and concentrated, and the resulting residue was diluted with chloroform, and washed with saturated brine. The organic layer was dried and concentrated, and the resulting residue was purified by silica gel column chromatography (hexane-ethyl acetate, 20:1, v/v) to obtain the title compound (4.86 g, 61%).
¹H-NMR(CDCl₃) δ : 1.26 (3H, t, J=7.3Hz), 3.64 (3H, s), 3.84 (3H, s), 3.85 (m, 2H), 6.99 (1H, m), 7.15-7.19 (2H, m)

### (C) Methyl 1-ethyl-6,7-difluoro-4-oxo-1,4-dihydro-3-cinnolinecarboxylate

The dimethyl 2-[(3,4-difluorophenyl)-2-ethylhydrazono]malonate (4.86 g,16.2 mmol) obtained in (B) was added with polyphosphoric acid (100 ml), and stirred at 110°C for 20 hours. The reaction mixture was left stand for cooling, then diluted with water, and extracted with ethyl acetate twice. The organic layer was dried and then concentrated, and the resulting residue was purified by silica gel column chromatography (chloroform-ethyl acetate, 4:1, v/v) to obtain the title compound (959 mg, 21%) as yellow crystals.
¹H-NMR (CDCl₃) δ : 1.57 (3H, t, J=7.3Hz)3.99 (3H, s), 4.48 (q, 2H, J=7.3Hz), 7.35 (1H, dd, J=10.7, 6.1Hz), 8.21 (2H, 1H, dd, J=9.3, 8.8Hz)

### (D) Methyl 1-ethyl-6-fluoro-7-[(4-isopropyl-1,3-thiazol-2-yl)methyl]-4-oxo-1,4-dihydro-3-cinnolinecarboxylate

(4-Isopropyl-1,3-thiazol-2-yl)methanol (182 mg, 0.60 mmol) was dissolved in DMF (3 ml), added with 18-crown-6 (337 mg, 1.28 mmol), and sodium hydride (95%, 32.2 mg, 1.28 mmol), and stirred under argon atmosphere for 15 minutes. The mixture was added with the methyl 1-ethyl-6,7-difluoro-4-oxo-1,4-dihydro-3-cinnolinecarboxylate obtained in (C) (311 mg, 1.16 mmol), and stirred at room temperature for 1 hour. Then the reaction mixture was added with aqueous ammonium chloride, and extracted with ethyl acetate. The organic layer was dried over magnesium sulfate, and the solvent was evaporated under reduced pressure. The resulting residue was purified by preparative TLC (chloroform-ethyl acetate, 4:1, v/v) to obtain the title compound (380 mg, 81%) as yellow crystals.
¹H-NMR (CDCl₃) δ : 1.32 (6H, d, J=6.8Hz), 1.51 (3H, t, J=7.1Hz), 3.11 (1H, m), 3.98 (3H, s), 4.48 (2H, q, J=7.1Hz), 5.59 (2H, s), 6.99 (1H, s), 7.26 (1H, d, J=6.4Hz), 8.07 (1H, d, J=10.5Hz)

### (E) 1-Ethyl-6-fluoro-7-[(4-isopropyl-1,3-thiazol-2-yl)methyl]-4-oxo-1,4-dihydro-3-cinnolinecarboxylic acid

The methyl 1-ethyl-6-fluoro-7-[(4-isopropyl-1,3-thiazol-2-yl)methyl]-4-oxo-1,4-dihydro-3-cinnolinec arboxylate obtained in (D) (380 mg, 0.937 mmol) was dissolved in acetic acid (5 ml)-hydrochloric acid (5 ml), and stirred at 110°C for 2 hours. The reaction mixture was distributed in ethyl acetate-ice water, the organic layer was dried over magnesium sulfate, and then the solvent was evaporated to obtain the title compound (322 mg, 88%) as brown powder.
¹H-NMR (CDCl₃) δ : 1.32 (6H, d, J=7.1Hz), 1.57 (3H, t, J=7.3Hz), 3.12 (1H, m), 4.73 (2H, q, J=7.3Hz), 5.72 (2H, s), 7.04 (1H, s), 7.58 (1H, d, J=6.6Hz), 8.10 (1H, d, J=10.0Hz)

### Example 72: 1-Ethyl-6-fluoro-7-[(4-isopropyl-1,3-thiazol-2-yl)methyl]-3-(1H-1,2,3,4-tetrazol-5-yl)-1,4-dihydro-4-cinnolinone

### (A) N³-(2-Cyanoethyl)-1-ethyl-6-fluoro-7-[(4-isopropyl-1,3-thiazol-2-yl)methyl]-4-oxo-1,4-dihydro-3-cinnolinecarboxamide

The 1-ethyl-6-fluoro-7-[(4-isopropyl-1,3-thiazol-2-yl)methyl]-4-oxo-1,4-dihydro-3-cinnolinecarboxylic acid obtained in Example 71 (E) (322 mg, 0.823 mmol) was dissolved in DMF (6 ml), added with ethyl chloroformate (0.117 ml, 1.23 mmol) and triethylamine (0.172 ml, 1.23 mmol) under ice cooling, and stirred for 1 hour. The mixture was warmed to room temperature, and further stirred for 30 minutes. The mixture was cooled again with ice, stirred for 1 hour, then added with cyanoethylamine (0.273 ml, 3.70 mmol), and stirred at room temperature for 3 days. The reaction mixture was diluted with ethyl acetate, washed with aqueous citric acid, saturated aqueous sodium hydrogencarbonate and water, and dried. The solvent was evaporated and the resulting residue was purified by silica gel column chromatography (chloroform-methanol, 200:1, v/v) to obtain the title compound (276 mg, 76%) as yellow crystals.
¹H-NMR (CDCl₃) δ : 1.32 (6H, d, J=7.1Hz), 1.55 (3H, t, J=7.3Hz), 2.78 (2H, t, J=6.8Hz), 3.11 (1H, m), 3.76 (2H, m), 4.64 (2H, q, J=7.3Hz), 5.62 (2H, s), 7.00 (1H, s), 7.39 (1H, d, J=6.6Hz), 8.08 (1H, d, J=10.3Hz), 10.4 (1H, m)

### (B) 2-(5-{1-Ethyl-6-fluoro-7-[(4--isopropyl-1,3-thiazol-2-yl)methyl]-4-oxo-1,4-dihydro-3-cinnolinyl}-1H-1,2,3,4-tetrazol-1-yl)ethyl cyanide

The N³-(2-cyanoethyl)-1-ethyl-6-fluoro-7-[(4-isopropyl-1,3-thiazol-2-yl)methyl]-4-oxo-1,4-dihydro-3-cinnolinecarboxamide obtained in (A) (276 mg, 0.622 mmol) was dissolved in acetonitrile (6 ml), added with sodium azide (48.5 mg, 0.747 mmol), and trifluoromethanesulfonic anhydride (0.126 ml, 0.747 mmol) under ice cooling under argon atmosphere, and stirred at room temperature for 2 hours. The mixture was further added with sodium azide (97 mg) and trifluoromethanesulfonic anhydride (0.252 ml), and stirred overnight. The mixture was further added with sodium azide (97 mg) and trifluoromethanesulfonic anhydride (0.252 ml), and stirred for 7 hours. The reaction mixture was added with saturated aqueous sodium hyrogencarbonate, and extracted with ethyl acetate, and then the extract was dried. The solvent was evaporated, and the resulting residue was purified by silica gel column chromatography (toluene-acetone, 8:1, v/v) to obtain the title compound (134 mg, 46%) as yellow crystals.
¹H-NMR (CDCl₃) δ : 1.34 (6H, d, J=6.8Hz), 1.58 (3H, t, J=7.3Hz), 3.12 (1H, m), 3.19 (2H, t, J=7.1Hz), 4.58 (2H, q, J=7.3Hz), 4.78 (2H, t, J=7.1Hz), 5.64 (2H, s), 7.02 (1H, s), 7.43 (1H, d, J=6.6Hz), 8.07 (1H, d, J=10.3Hz)

### (C) 1-Ethyl-6-fluoro-7-[(4-isopropyl-1,3-thiazol-2-yl)methyl]-3-(1H-1,2,3,4-tetrazol-5-yl)-1,4-dihydro-4-cinnolinone

The 2-(5-{1-ethyl-6-fluoro-7-[(4--isopropyl-1,3-thiazol-2-yl)methyl]-4-oxo-1,4-dihydro-3-cinnolinyl}-1H-1,2,3,4-tetrazol-1-yl)ethyl cyanide obtained in (B) (134 mg,0.286 mmol) was dissolved in methylene chloride, added with DBU (0.107 ml, 0.714 mmol), and stirred at room temperature for 3 hours. The mixture was further added with DBU (0.107 ml, 0.714 mmol), stirred for 2 hours, added with another DB U (0.107 ml, 0.714 mmol), and stirred for 2 hours. The reaction mixture was diluted with chloroform, washed with 1 N hydrochloric acid, and water, and then dried. The solvent was evaporated, and the resulting residue was purified by preparative TLC (chloroform-methanol-water, 8:3:0.5, v/v/v) to obtain the title compound (101 mg, 85%) as yellow crystals.
¹H-NMR (CD₃OD) δ : 1.35 (6H, d, J=6.8Hz), 1.57 (3H, t, J=6.8Hz), 3.14 (1H, m), 4.72 (2H, m), 5.67 (2H, s), 7.11 (1H, s), 7.49 (1H, m), 7.59 (1H, m)
MS; m/z: 416 (MH⁺)

### Test Example 1: Effect of combinational use with an antimicrobial agent on multidrug resistant Pseudomonas aeruginosa

As multidrug resistant *Pseudomonas aeruginosa, Pseudomonas aeruginosa* PAM 1723 strain highly expressing the drug efflux pump was used. As antimicrobial drugs for the combinational uses, levofloxacin (LVFX) as a quinolone antibacterial agent and azthreonam (AZT) as a monobactam antibiotic were used.

Each of the compounds shown in Table 1 with Example No., which falls within the compounds of the present invention, was subjected to the measurement of its minimum concentration (µg/ml) that was required to enhance the antibacterial activity of the antibacterial agent when the test compound was used in combination with the antibacterial drug applied at a concentration of 1/4, or 1/8 or less of the minimum inhibitory concentration against the PAM 1723 strain. Each value in Table 1 shows a minimum concentration (µg/ml) of each compound that was required to enhance the antibacterial activity when the test compound was used in combination with the antibacterial drug applied at a concentration of 1/8 or less of the minimum inhibitory concentration. As for levofloxacin, effects were indicated as combinational uses for 7.5 hours, and as for azthreonam, effects as combinational uses for 18 hours was indicated. The Müller-Hinton broth (MHB, Difco) was used as a medium, and the inoculated amount of the bacteria was 1 × 10⁶ CFU/ml. Effects were determined by optically measuring the transmittance of the bacterial culture with time, and visually observing the turbidity of the medium. As clearly shown in Table 1, the compounds of the present invention exhibited effect on the drug resistant *Pseudomonas aeruginosa* in the combinational uses mainly by inhibiting acquisition of resistance due to the drug efflux pump, and accordingly, it can be concluded that the class of compounds are expected to be useful clinically.

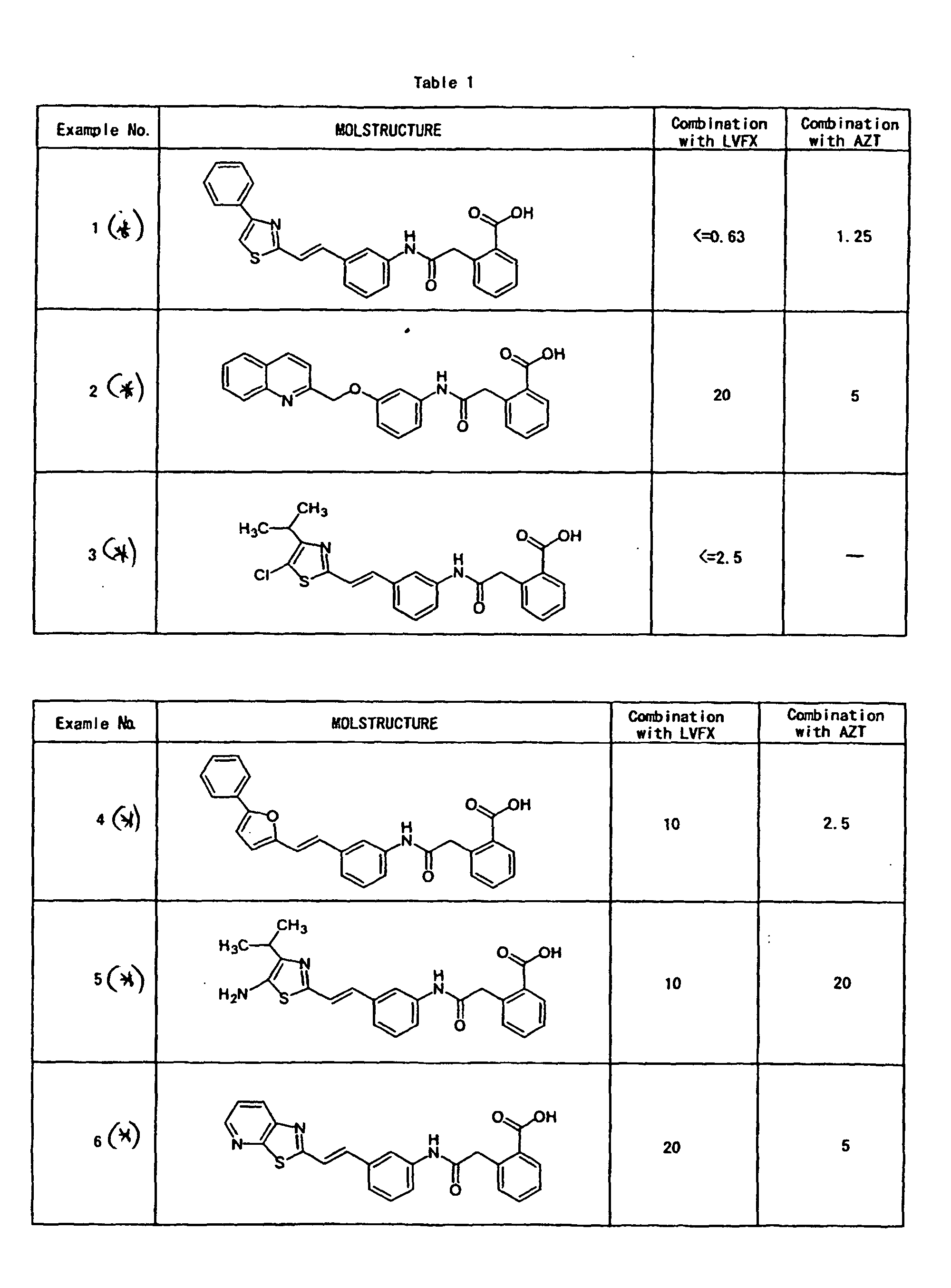

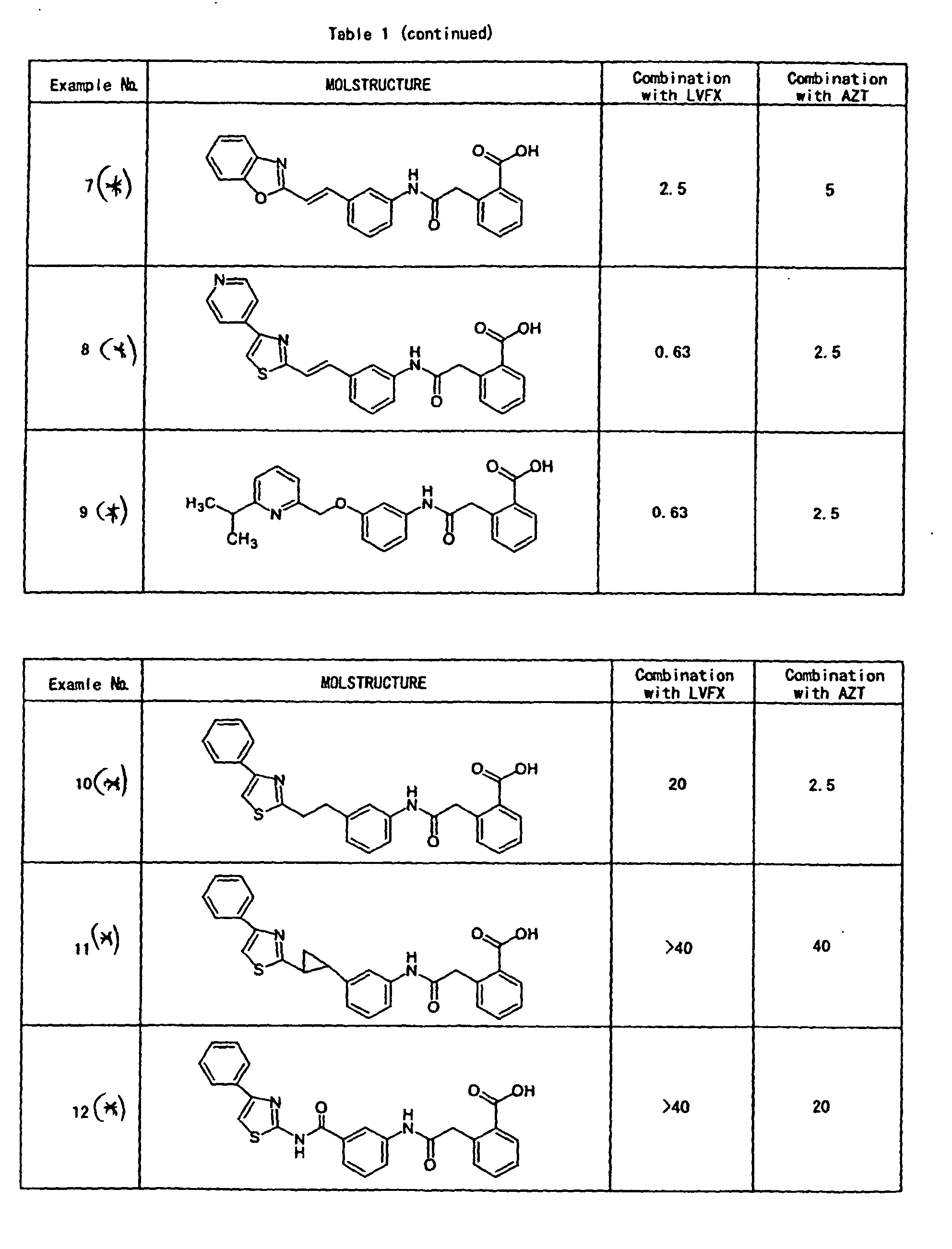

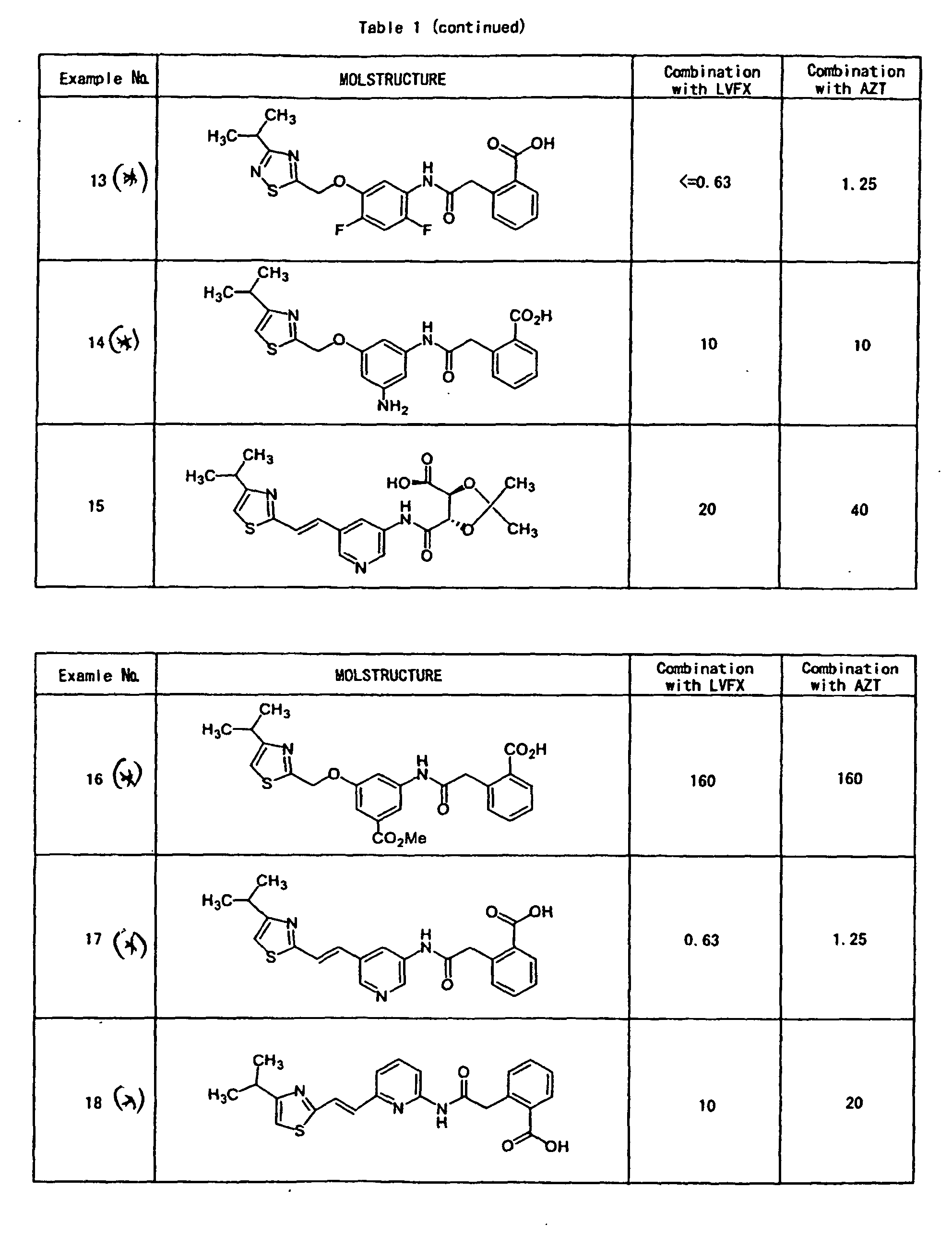

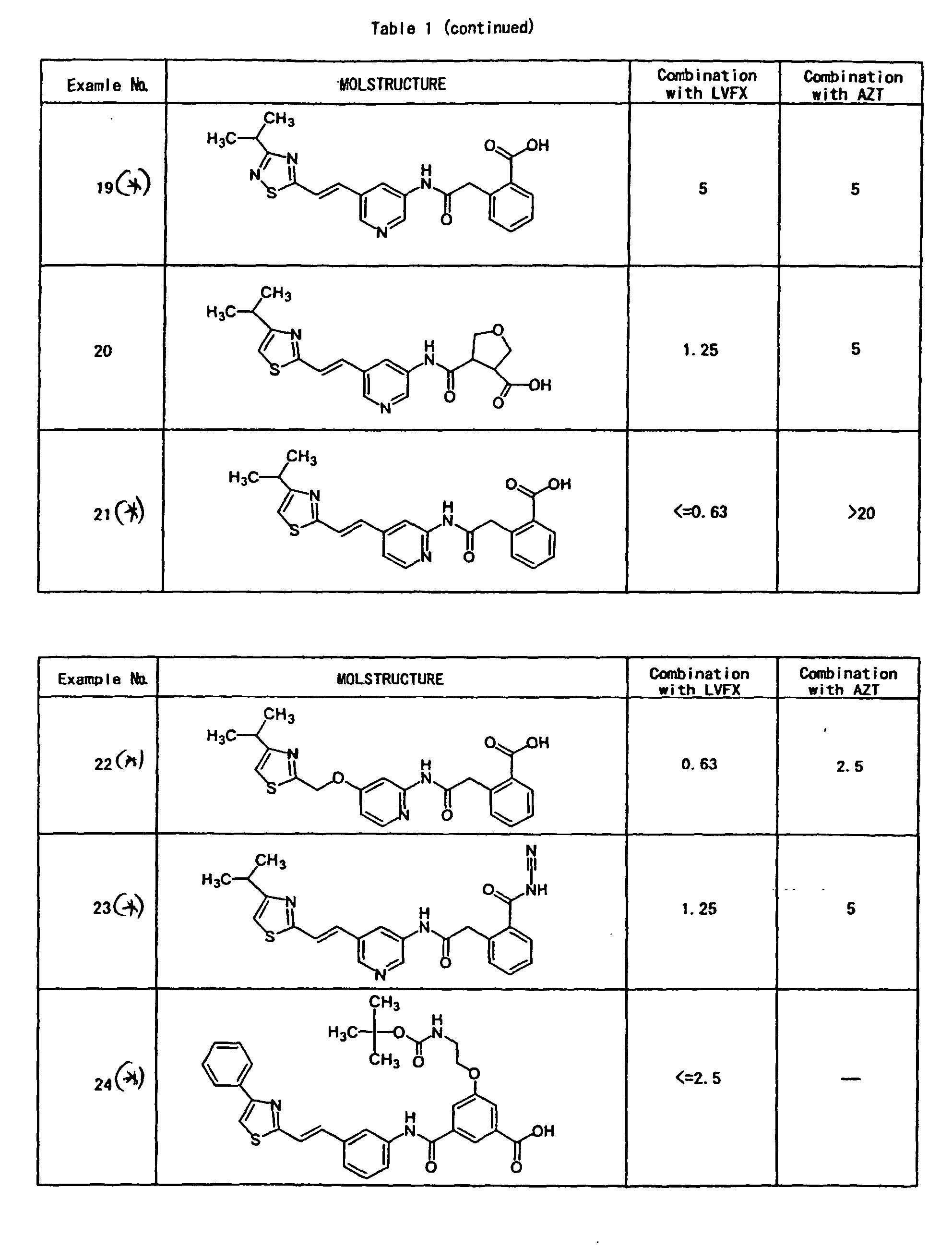

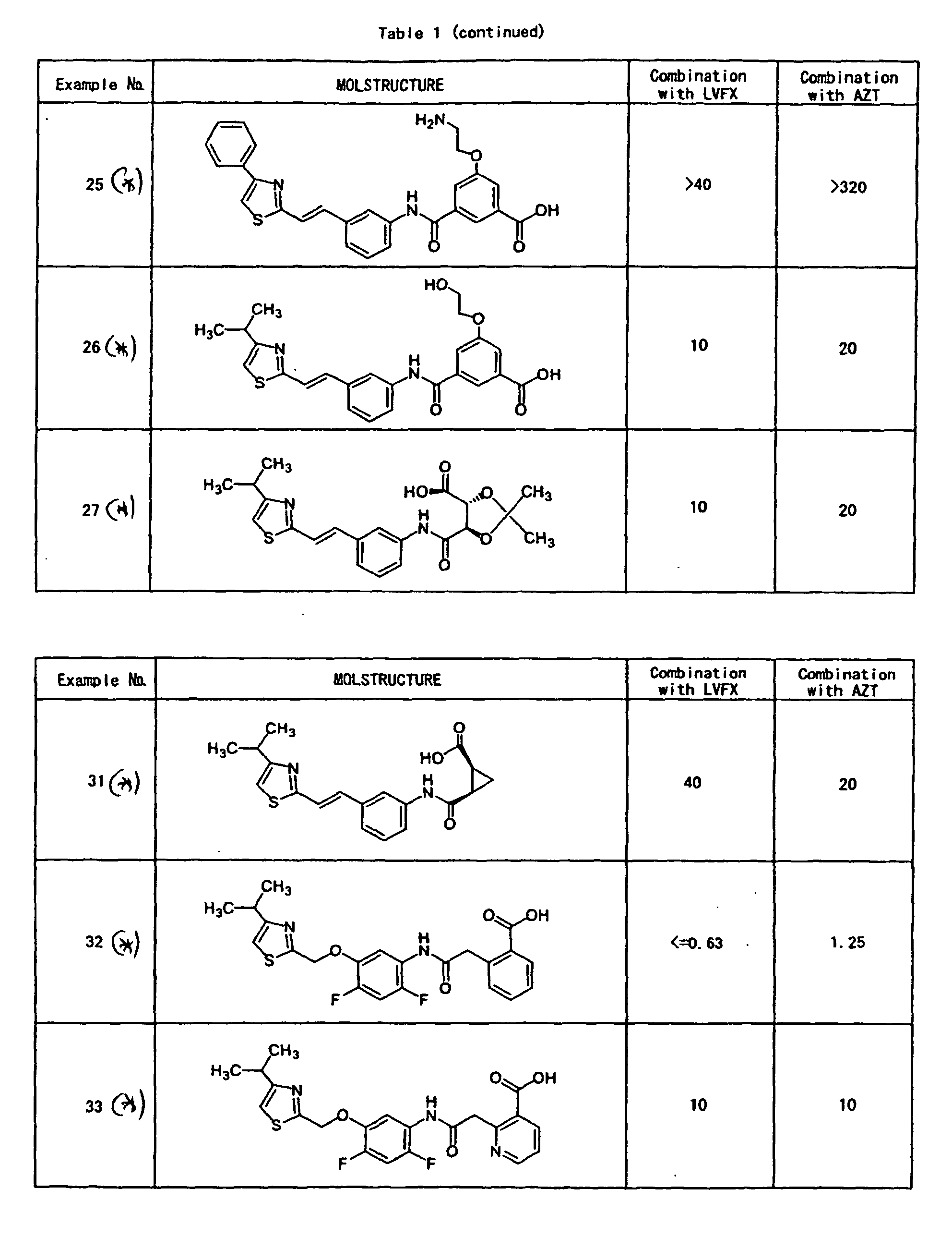

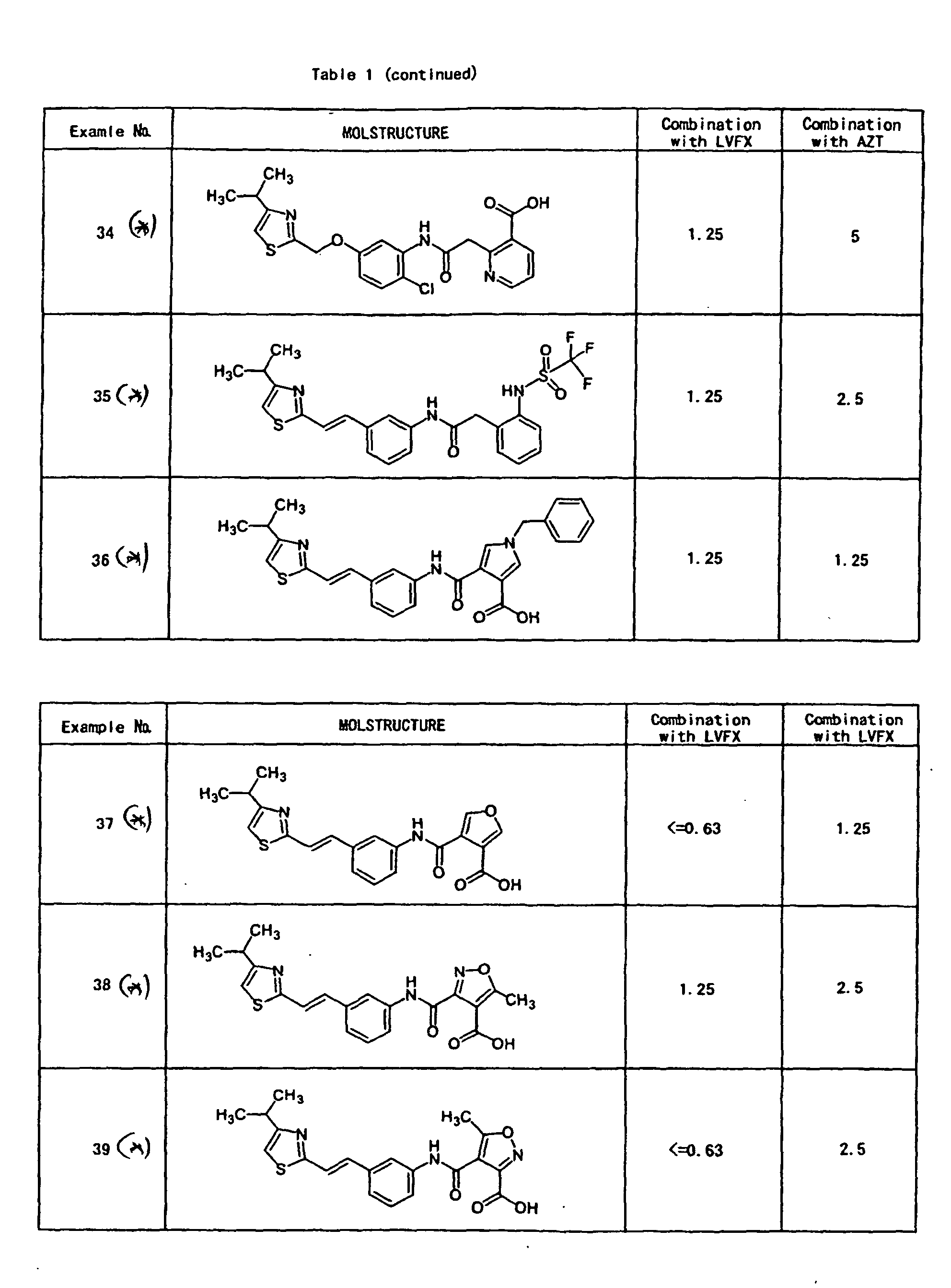

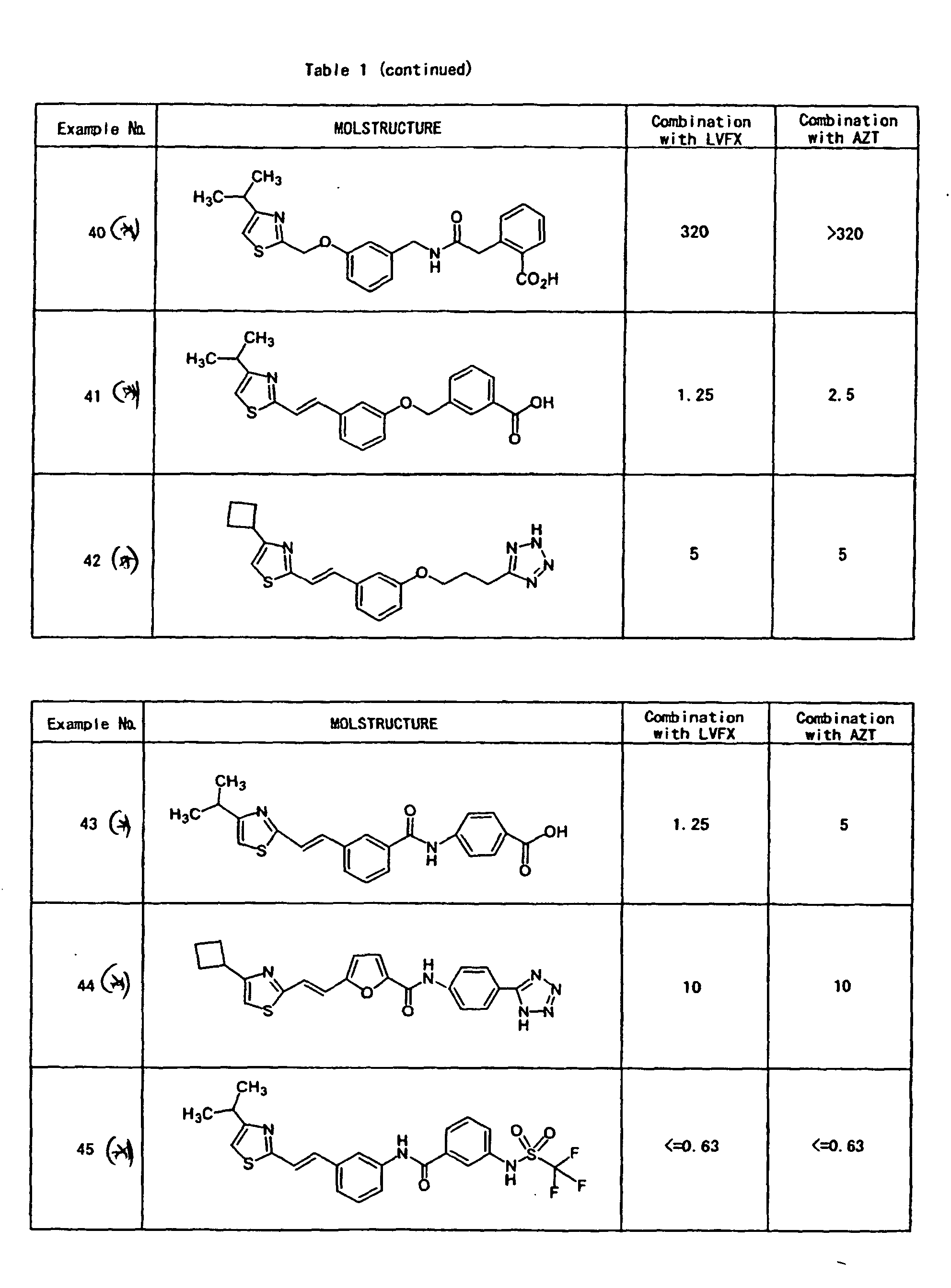

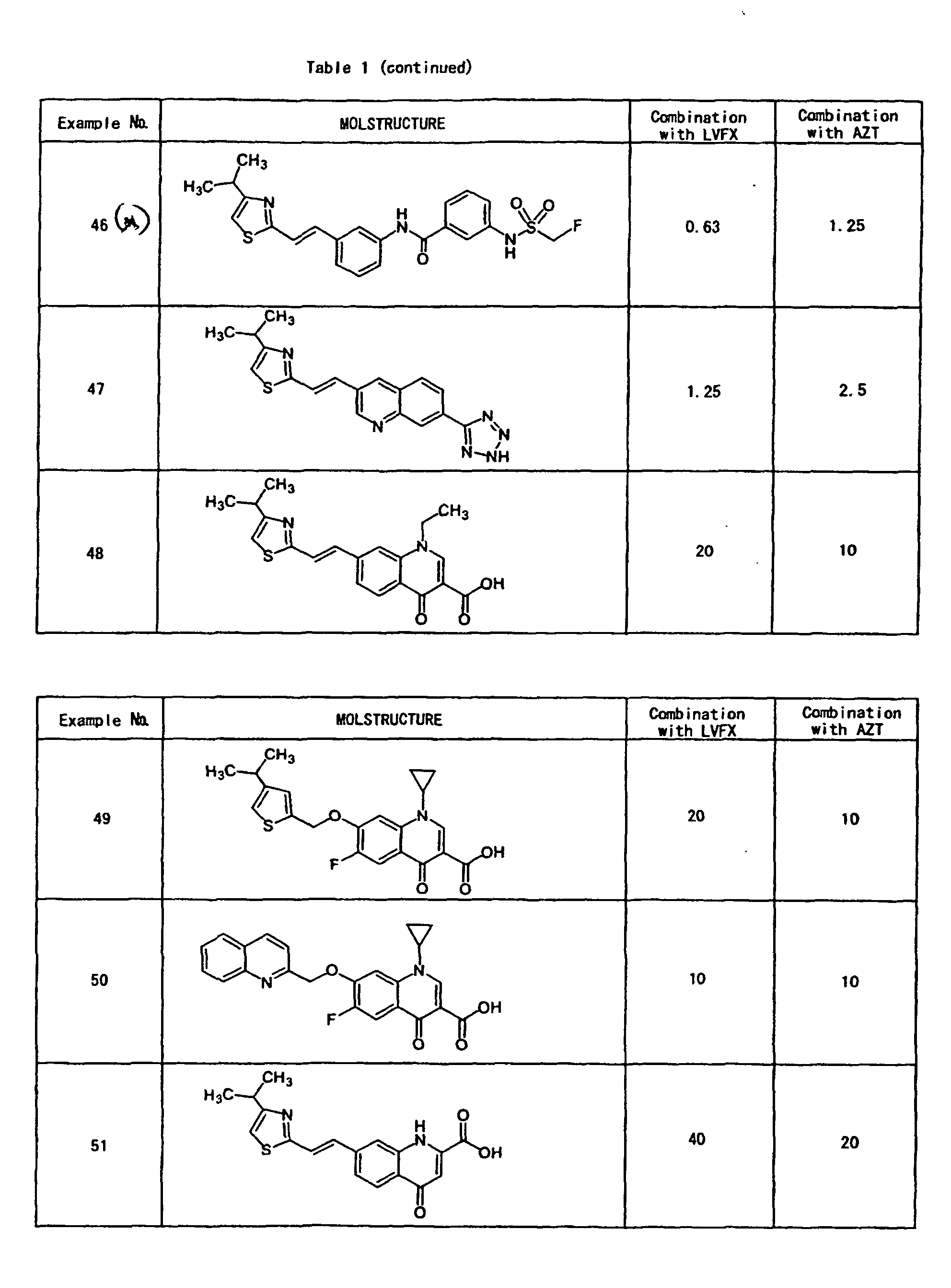

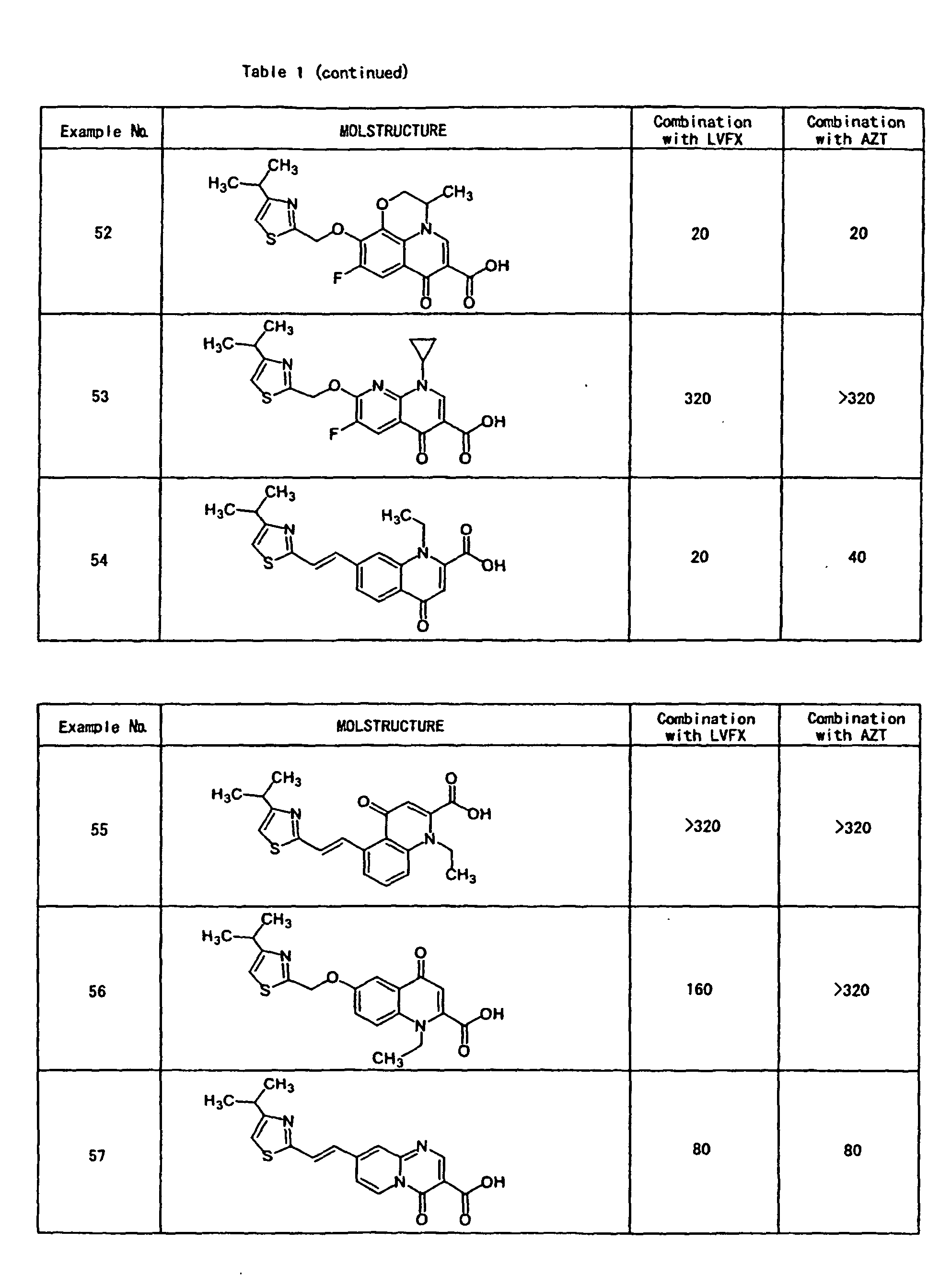

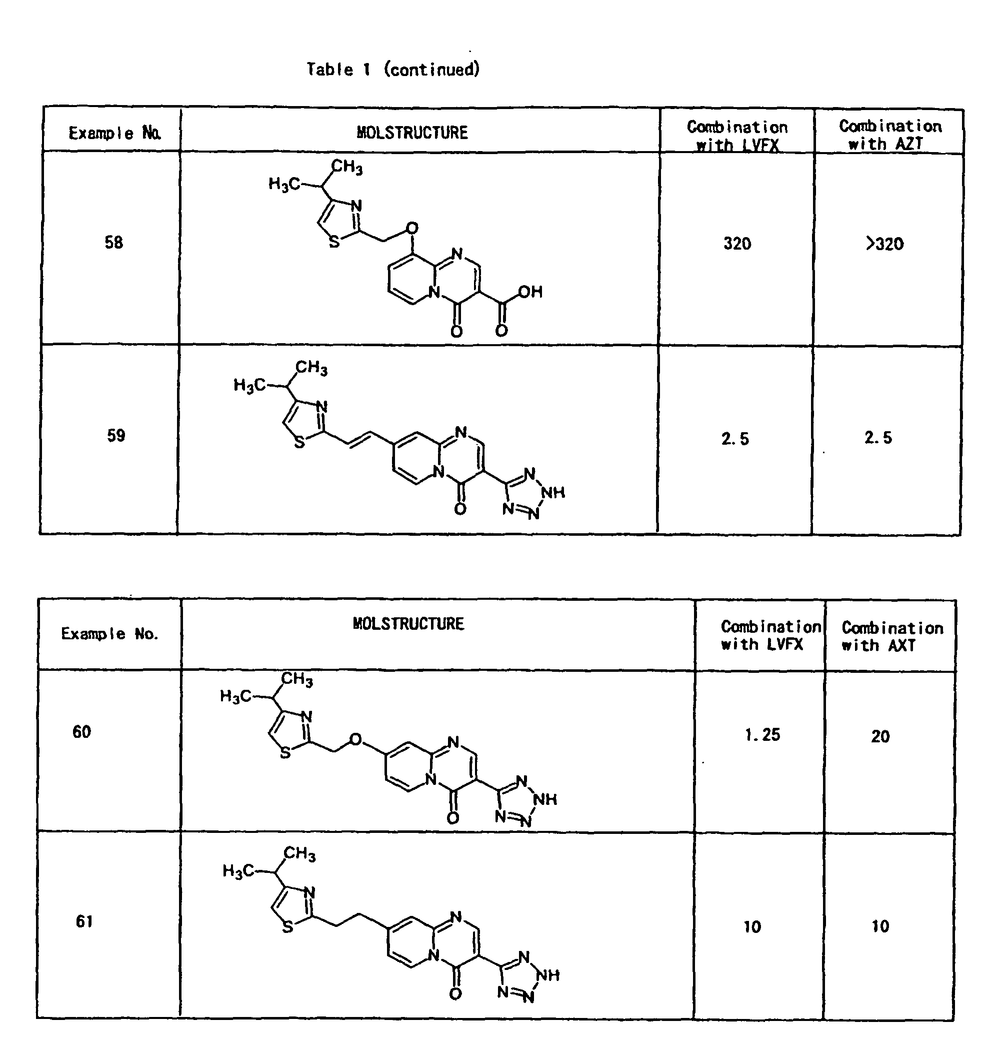

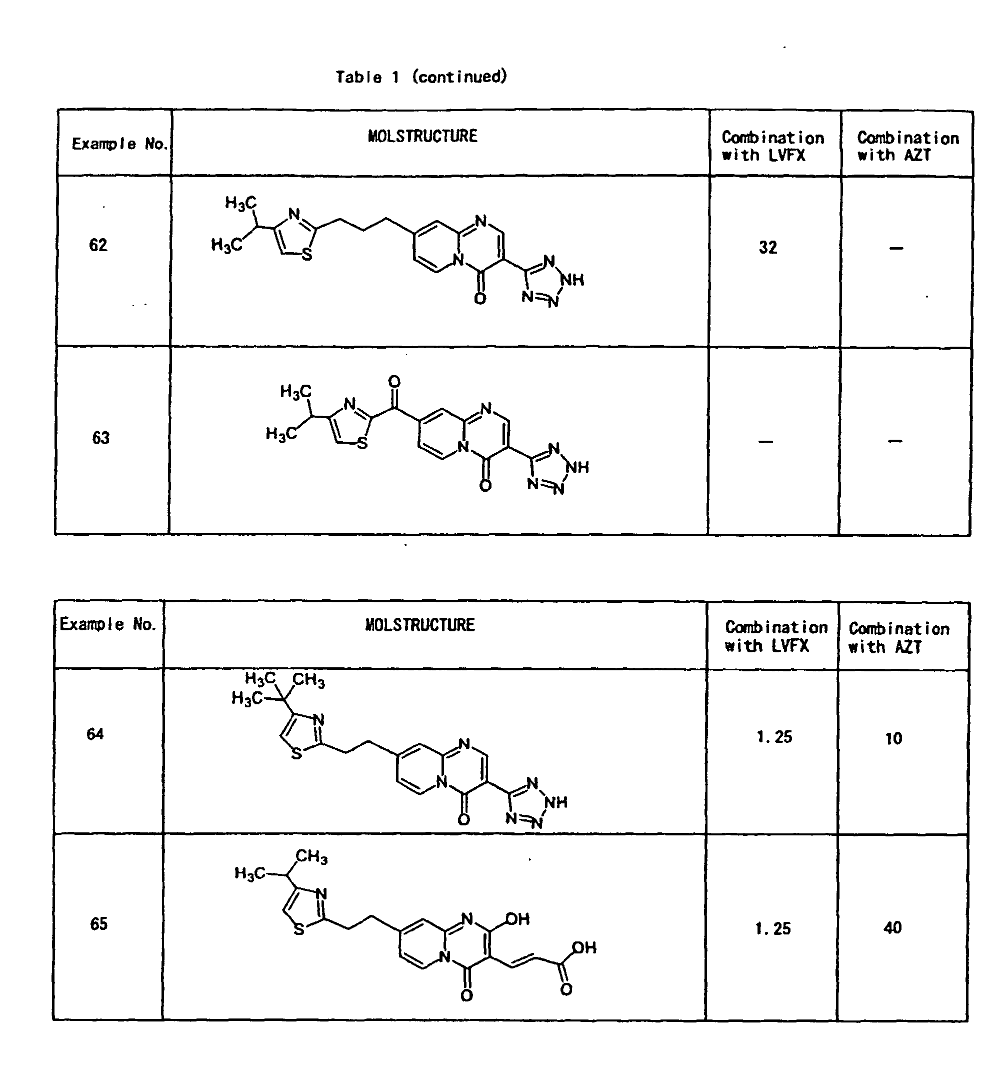

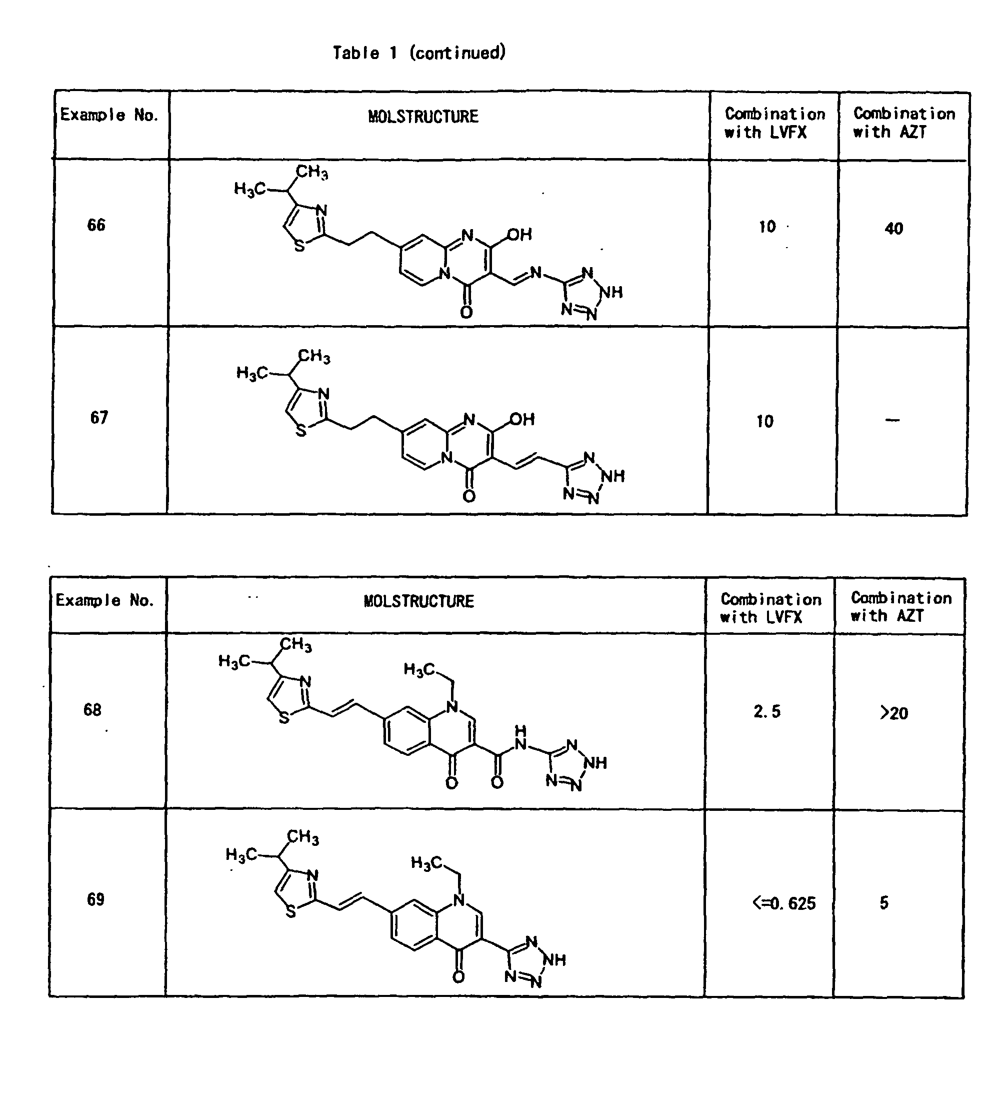

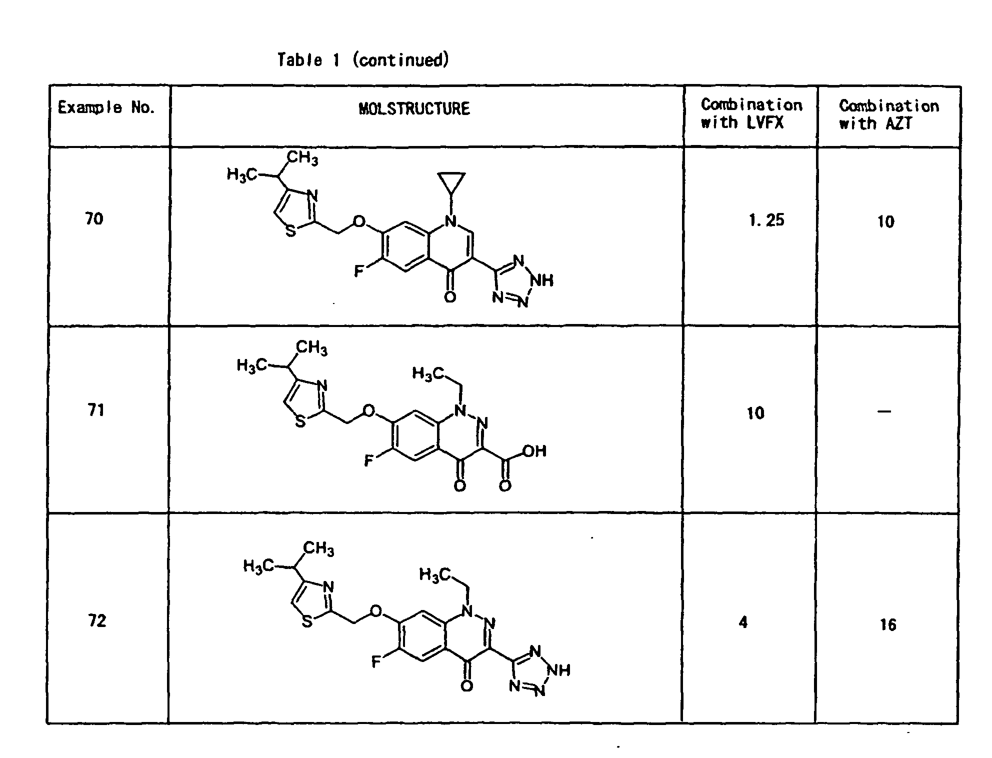

### Example 73

### (1) Calculation of pharmacophore

By using the conformation search function of CATALYST™, calculation was performed on the compounds of Examples 1, 10, 15, 27, 37, 49 and 70 shown in Table 1 in the best quality at the energy tolerance of the conformation generated of 20 kcal/mol and the maximum number of conformations of 250. Then, all the conformations generated were input in the compound information. Pharmacophores were calculated using all of the above compounds by means of CATALYST/HipHop, one of the functions of pharmacophore generation (Hypothesis) by CATALYST™. Among the results obtained, one pharmacophore having the highest adequacy was selected.

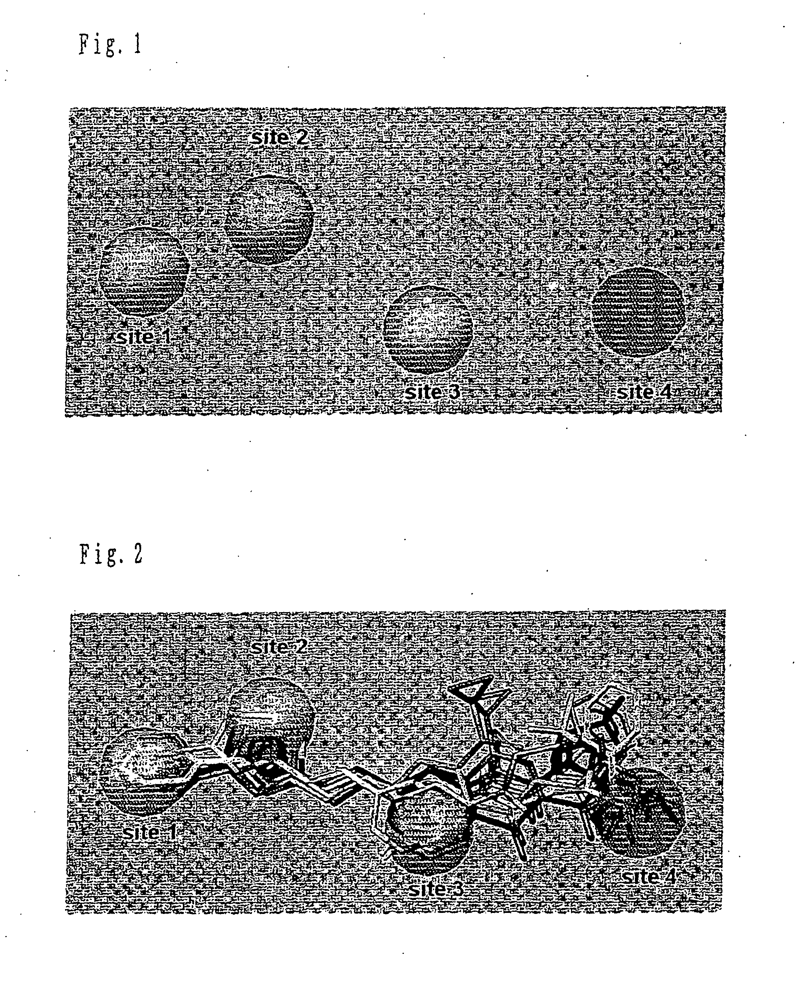

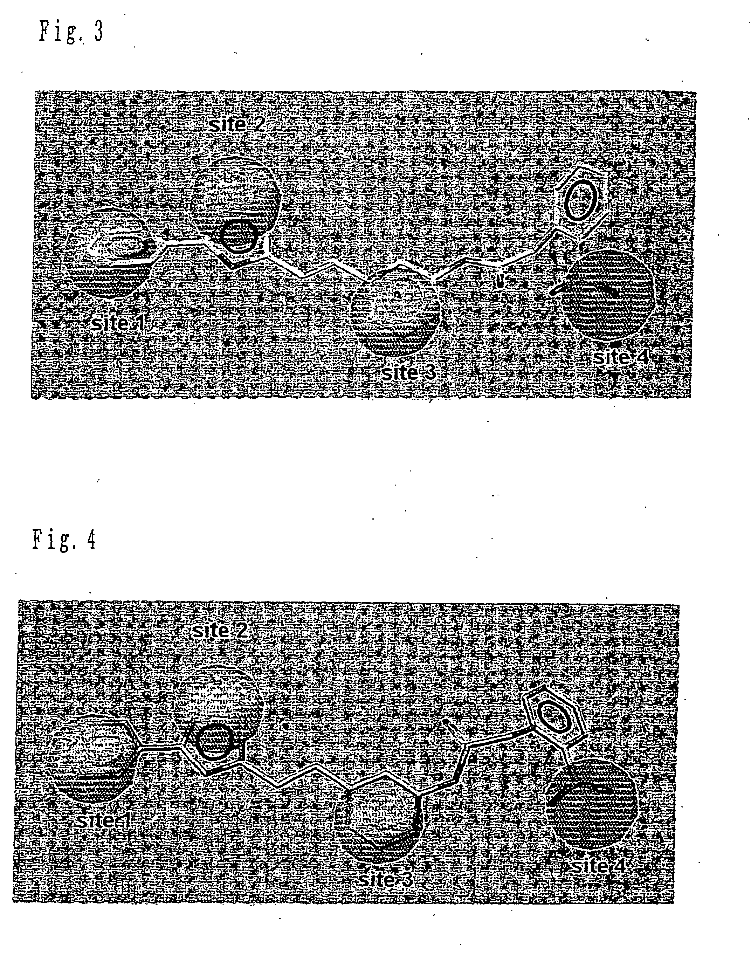

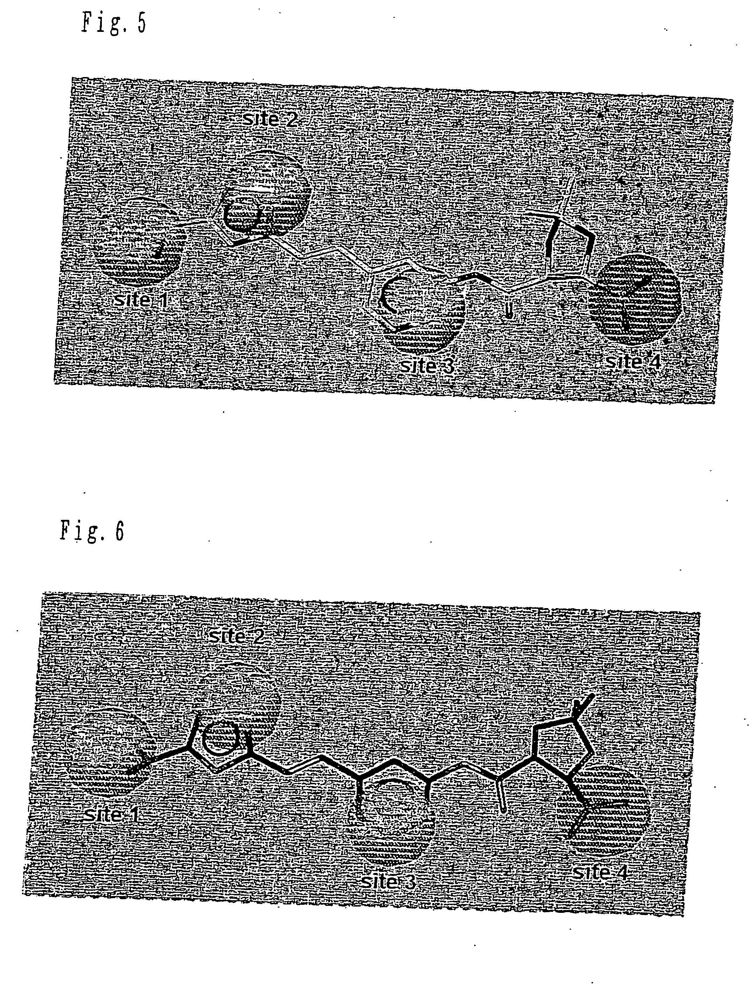

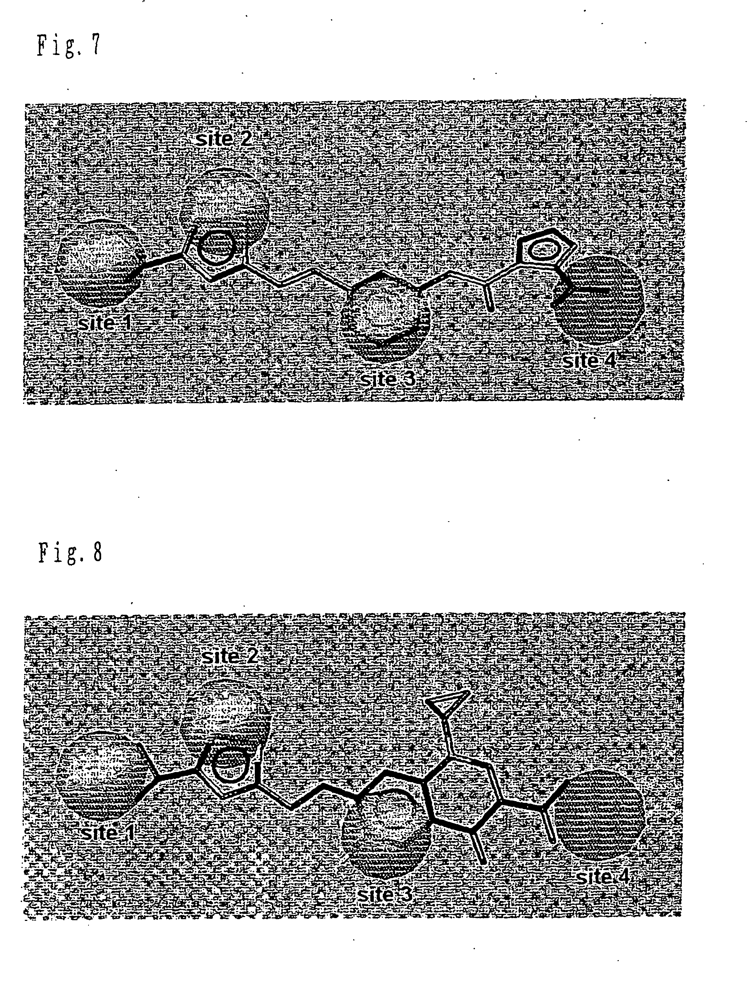

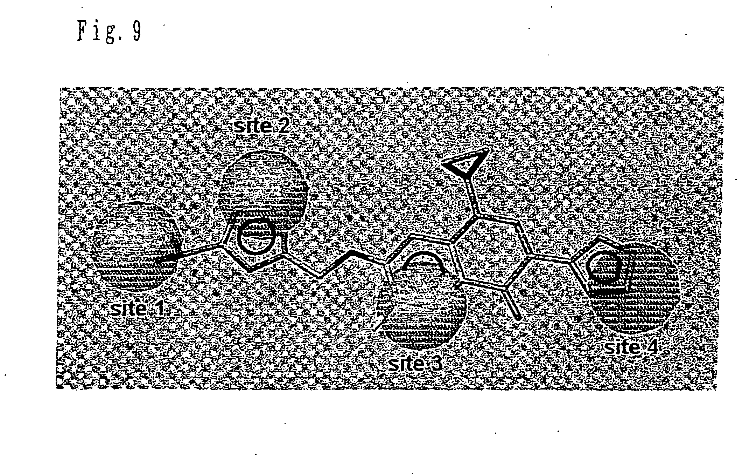

The resulting pharmacophore is shown in Fig. 1. Each site in the figure is shown as a sphere having the radius of 1.7 A from its center. Each tolerance of Site 1 and Site 4 is indicated as a sphere having the radius (tolerance) of 2 A from each center. The result obtained by overlaying all the compounds on the resulting pharmacophore is shown in Fig. 2. For overlaying the compounds, the Compare/Fit function of CATALYST™ was used. In Fig. 2, hydrogen atoms are not shown. In the figure, each site is indicated as the scope of the spherical part, and Site 1 and Site 4 are shown as spheres having the radius of 1.7 Å (The actual tolerance is a sphere having the radius of 2 Å.). Figs. 3 to 9 show the mode of overlay of each compound on the pharmacophore. Fig. 3 shows the result obtained by the compound of Example 1, Fig. 4 for the compound of Example 10, Fig. 5 for the compound of Example 15, Fig. 6 for the compound of Example 27, Fig. 7 for the compound of Example 37, Fig. 8 for the compound of Example 49, and Fig. 9 for the compound of Example 70.

### (2) Evaluation whether or not a compound fits the pharmacophore

Evaluation whether or not a compound fits the pharmacophore can be performed as follows. The conformation of the compound is calculated using a program based on the molecular force field method such as, for example, CATALYST™, CHARMm, and MMFF, or a program based on the molecular orbital method such as MOPAC (these programs are given as examples and programs are not limited to these mentioned above). Each conformation is individually overlaid on the pharmacophore by using a program such as CATALYST™ or manually. A compound whose partial structures occupy the aforementioned four sites, or a compound whose partial structures are in contact with the four sites can be considered to fit the pharmacophore and determined to have an inhibitory activity against the drug efflux pump of *Pseudomonas aeruginosa* More specifically, when the compound is overlaid on the pharmacophore, and for each of the cites, when constitutional atoms in the partial structure that conform with the feature of the site is located within 0.5 to 3 Å from the site, more preferably, when the atoms are within the tolerance of 2 Å for Site 1 and Site 4 and within the tolerance of 1.7 Å for Site 2 and Site 4, the compound is determined to fit the pharmacophore.

For example, as shown in Figs. 3 to 7 and Fig. 9, the partial structure of each compound occupies the inside of the sphere generated from the center of each site with the radius of 1.7 Å, and satisfies the aforementioned tolerance. The compound shown in Fig. 8 has the partial structures for Site 2 and Site 3 which are within the spheres generated from the center of each site with the radius of 1.7 Å. As for Site 1 and Site 4, the partial structures are in contact with the spheres similarly generated from the center of each site with the radius of 1.7 Å and are within 2 Å_{.} Therefore, the compound is determined to satisfy the aforementioned tolerance.

### Industrial Applicability

The medicament of the present invention has an inhibitory activity against the drug efflux pumps of microorganisms, and the medicament has preventing action on the acquisition of resistance to an antimicrobial drug by a microorganism, as well as resistant-eliminating action on a microorganism with acquired resistance. Therefore, the medicament of the present invention can achieve excellent effect in preventive and/or therapeutic treatment of a microbial infection when used, for example, in combination with the administration of an antimicrobial drug.

## Claims

1. Use of the compound represented by the general formula (II), 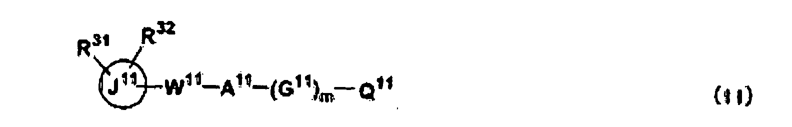 wherein R³¹ and R³² independently represent hydrogen atom, a halogen atom, carboxyl group, an alkoxycarbonyl group, an amino group which may be substituted, an alkyl group which may be substituted, an aryl group which may be substituted, or a heterocyclic group which may be substituted, or R³¹ and R³² represent a 6-membered ring formed by R³¹ and R³² bound to each other together with two adjacent ring-membered atoms of J¹¹;
J¹¹ represents a 5- or 6-membered heteroaromatic ring;
W¹¹ represents a group selected from the group consisting of -CH=CH-, -CH₂CH₂-, -OCH₂-, -SCH₂-, -OCH₂O-, -CH₂O-, -CH₂-, -CO-, -CH₂CH₂CH₂-, -CH₂NH-, -NHCH₂-, -CH₂S-, -CONH-, -CH₂SCH₂-, -CH=CH-CONH- and -CH₂OCH₂- (each group binds at its left end to a ring-membered atom of J¹¹) or single bond;
A¹¹ represents a pyridinediyl group which may be substituted, a pyrido[1,2-a]pyrimidinediyl group which may be substituted, a quinolin-4-one-diyl group which may be substituted, an azaquinolin-4-one-diyl group which may be substituted, a quinolinediyl group which may be substituted, a thiadiazolo[3,2-a]pyrimidinediyl group which may be substituted, or a thiazolo[3,2-a]pyrimidinediyl group which may be substituted;
G¹¹ represents oxygen atom, carbonyl group, ethynyl group, -CH=N-, -N(R³³)CO-, -N(R³⁴)-SO₂-, -SO₂-N(R³⁵)-, -CO-N(R³⁸)-, -C(=CHR³⁷)- or -C(R³⁸)=C(R³⁹) wherein R³³, R³⁴, R³⁵, R³⁸, R³⁷, R³⁸ and R³⁹ independently represent hydrogen atom or an alkyl group which may be substituted; m represents an integer of 0 or 1; and Q¹¹ represents an acidic group, a physiologically acceptable salt thereof, or a hydrate thereof, as an active ingredient for the production of a medicament for improving the efficacy of an antimicrobial agent in the preventive and/or therapeutic treatment of microbial infectious diseases.

2. Use according to claim 1, wherein the microbial infectious disease is caused by bacteria resistant to antimicrobial agents.

3. Use according to claims 1 or 2, wherein the resistant bacteria is Pseudomonas aeruginosa.

## Patentansprüche

1. Verwendung der durch die allgemeine Formel (II) dargestellten Verbindung 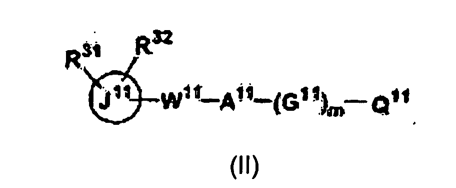 worin
R³¹ und R³² unabhängig voneinander ein Wasserstoff-Atom bedeuten, ein Halogen-Atom, eine Carboxyl-Gruppe, eine Alkoxycarbonyl-Gruppe, eine Amino-Gruppe, die substituiert sein kann, eine Alkyl-Gruppe, die substituiert sein kann, eine Aryl-Gruppe, die substituiert sein kann, oder eine heterocyclische Gruppe, die substituiert sein kann, oder R³¹ und R³² für einen 6-gliedrigen Ring stehen, der gebildet wird durch R³¹ und R³², die zusammen mit zwei benachbarten Ringatomen von J¹¹ aneinander gebunden sind;
J¹¹ einen 5- oder 6-gliedrigen heteroaromatischen Ring bedeutet;
W¹¹ eine Gruppe bedeutet, die ausgewählt ist aus der Gruppe bestehend aus -CH=CH-, -CH₂CH₂-, -OCH₂-, -SCH₂-, -OCH₂O-, -CH₂O-, -CH₂-, -CO-, -CH₂CH₂CH₂-, -CH₂NH-, -NHCH₂-, -CH₂S-, -CONH-, -CH₂SCH₂-, -CH=CH-CONH- und -CH₂OCH₂- (jede Gruppe bindet an ihrem linken Ende an ein Ringatom von J¹¹) oder einer Einfachbindung;
A¹¹ eine Pyridindiyl-Gruppe bedeutet, die substituiert sein kann, eine Pyrido[1,2-a]pyrimidindiyl-Gruppe, die substituiert sein kann, eine Chinolin-4-ondiyl-Gruppe, die substituiert sein kann, eine Azachinolin-4-ondiyl-Gruppe, die substituiert sein kann, eine Chinolindiyl-Gruppe, die substituiert sein kann, eine Thiadiazolo[3,2-a]pyrimidindiyl-Gruppe, die substituiert sein kann, oder eine Thiazolo[3,2-a]pyrimidindiyl-Gruppe, die substituiert sein kann;
G¹¹ ein Sauerstoff-Atom bedeutet, eine Carbonyl-Gruppe, Ethinyl-Gruppe, -CH=N-, -N(R³³)-CO-, -N(R³⁴)-SO₂-, -SO₂-N(R³⁵)-, -CO-N-(R³⁶)-, -C(=CHR³⁷)- oder -C(R³⁸)=C(R³⁹)-, worin R³³, R³⁴, R³⁵, R³⁶, R³⁷, R³⁸ und R³⁹ unabhängig voneinander ein Wasserstoff-Atom oder eine Alkyl-Gruppe bedeuten, die substituiert sein kann; m eine ganze Zahl 0 oder 1 bedeutet; und Q¹¹ eine saure Gruppe bedeutet;
ein physiologisch annehmbares Salz oder ein Hydrat derselben als wirksamer Bestandteil für die Herstellung eines Medikaments zur Verbesserung der Wirksamkeit eines antimikrobiellen Mittels bei der vorbeugenden und/oder therapeutischen Behandlung mikrobieller Infektionskrankheiten.

2. Verwendung nach Anspruch 1, wobei die mikrobielle Infektionskrankheit durch Bakterien verursacht wird, die gegen antimikrobielle Mittel resistent sind.

3. Verwendung nach Anspruch 1 oder 2, wobei das resistente Bakterium *Pseudomonas aeruginosa* ist.

## Revendications

1. Utilisation du composé représenté par la formule générale (II) : 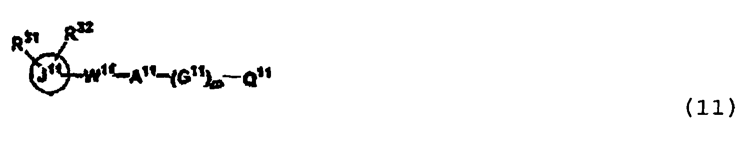 dans laquelle R³¹ et R³² représentent indépendamment un atome d'hydrogène, un atome d'halogène, un groupe carboxy, un groupe alcoxycarbonyle, un groupe amino qui peut être substitué, un groupe alkyle qui peut être substitué, un groupe aryle qui peut être substitué, ou un groupe hétérocyclique qui peut être substitué, ou bien R³¹ et R³² représentent un cycle à 6 chaînons formé par R³¹ et R³² liés l'un à l'autre conjointement avec deux atomes adjacents formant des chaînons du cycle J¹¹ ;
J¹¹ représente un cycle hétéroaromatique à 5 ou 6 chaînons ;
W¹¹ représente un groupe choisi dans le groupe constitué par -CH=CH-, -CH₂CH₂-, -OCH₂-, -SCH₂-, -OCH₂O-, -CH₂O, -CH₂-, -CO-, -CH₂CH₂CH₂-, -CH₂NH-, -NHCH₂-, -CH₂S-, -CONH-, -CH₂SCH₂-, -CH=CH-CONH- et -CH₂OCH₂- (chaque groupe est lié, à son extrémité gauche, à un atome formant un chaînon du cycle J¹¹), ou une liaison simple ;
A¹¹ représente un groupe pyridinediyle qui peut être substitué, un groupe pyrido[1,2-a]pyrimidinediyle qui peut être substitué, un groupe quinolin-4-onediyle qui peut être substitué, un groupe azaquinolin-4-onediyle qui peut être substitué, un groupe quinolinediyle qui peut être substitué, un groupe thiadiazolo[3,2-a]pyrimidinediyle qui peut être substitué, ou un groupe thiazolo[3,2-a]pyrimidinediyle qui peut être substitué ;
G¹¹ représente un atome d'oxygène, un groupe carbonyle, un groupe éthynyle, -CH=N-, -N(R³³)CO-, -N(R³⁴)-SO₂-, -SO₂-N(R³⁵)-, -CO-N(R³⁶)-, -C(=CHR³⁷) ou -C(R³⁸)=C(R³⁹) où R³³, R³⁴, R³⁵, R³⁶, R³⁷, R³⁸ et R³⁹ représentent indépendamment un atome d'hydrogène ou un groupe alkyle qui peut être substitué ; m représente l'entier 0 ou 1 ; et Q¹¹ représente un groupe acide,
d'un sel physiologiquement acceptable de celui-ci, ou d'un hydrate de celui-ci, en tant qu'ingrédient actif pour la production d'un médicament destiné à améliorer l'efficacité d'un agent antimicrobien dans le traitement préventif et/ou thérapeutique de maladies infectieuses microbiennes.

2. Utilisation selon la revendication 1, dans laquelle la maladie infectieuse microbienne est provoquée par une bactérie résistante aux agents antimicrobiens.

3. Utilisation selon la revendication 1 ou 2, dans laquelle la bactérie résistante est Pseudomonas aeruginosa.
